# EUROPEAN PATENT APPLICATION

(11) **EP 4 825 855 A1**
(43) Date of publication of application: **21.10.2026**
(21) Application number: 24902913.3
(22) Date of filing: 12.12.2024
(51) Int. Cl.: C07D 403/12, A61K 31/41, A61P 35/00

(54) **COMPOUND CONTAINING PYRAZOLE RING AND BICYCLIC HETEROARYL GROUP, PHARMACEUTICAL COMPOSITION THEREOF AND USE THEREOF**

(30) Priority: 13.12.2023 CN 202311716882; 01.11.2024 CN 202411548739; 09.12.2024 CN 202411799022
(71) Applicant: Chia Tai Tianqing Pharmaceutical Group Co., Ltd., Lianyungang, Jiangsu 222062 (CN)
(72) Inventor: LIU, Fei, Lianyungang, Jiangsu 222062 (CN); FENG, Taotao, Lianyungang, Jiangsu 222062 (CN); MENG, Qingyi, Lianyungang, Jiangsu 222062 (CN); XU, Hongjiang, Lianyungang, Jiangsu 222062 (CN); SHI, Wei, Lianyungang, Jiangsu 222062 (CN); BI, Cheng, Lianyungang, Jiangsu 222062 (CN)
(74) Representative: Lavoix
(86) International application number: PCT/CN2024/138866
(87) International publication number: WO 2025/124496

(57) **Abstract**

The present disclosure belongs to the field of pharmaceutical chemistry, and relates to a compound containing a pyrazole ring and a bicyclic heteroaryl group, with the structure as shown in formula (I). The present application also relates to a method for preparing the compound, a pharmaceutical composition containing the compound, and the use thereof in the treatment of related diseases (such as cancer or immune diseases).

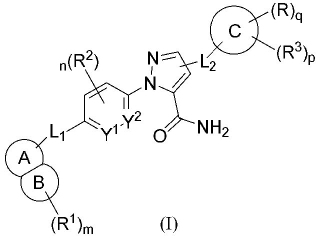

## Description

### CROSS-REFERENCE TO RELATED APPLICATIONS

The present disclosure claims the benefit and priority to the following 3 Chinese patent applications, the contents of which are incorporated herein by reference in their entireties:
Chinese Patent Application No. CN202311716882.7 filed with China National Intellectual Property Administration on December 13, 2023;
Chinese Patent Application No. CN202411548739.6 filed with China National Intellectual Property Administration on November 01, 2024; and
Chinese Patent Application No. CN202411799022.9 filed with China National Intellectual Property Administration on December 09, 2024.

### TECHNICAL FIELD

The present disclosure pertains to the field of pharmaceutical chemistry, and relates to a compound containing a pyrazole ring and a bicyclic heteroaryl group, a preparation method therefor, a pharmaceutical composition comprising the compound, and use thereof for treating related diseases (e.g., cancer or immune diseases).

### BACKGROUND

Bruton's tyrosine kinase (BTK) is mainly expressed in B cells, distributed in the lymphatic, hematopoietic and blood systems, and is a member of the non-receptor type tyrosine kinase Tec family, which also includes TEC, ITK/TSK/EMT, and BMX, which have high structural homology. BTK plays a crucial role in B-cell signaling pathways that link cell surface B-cell receptor stimulation to downstream intracellular responses, and is a key regulator of B-cell development, activation, signaling, and survival. In recent years, researches on B cells, particularly on B cell non-Hodgkin lymphoma and rheumatoid arthritis find that BTK is often abnormally expressed. The BTK signal transduction pathway-based small molecule targeted drug is developed, and a brand new way is provided for the treatment of B cell tumors such as leukemia and multiple myeloma and B cell immune diseases. Currently, irreversible inhibitors such as ibrutinib on the market often have mutation in BTK binding site, which leads to reduction of drug activity and drug resistance, so that more BTK inhibitors are clinically needed and have higher selectivity for BTK, thereby avoiding toxic and side effects caused by off-target effect.

### SUMMARY

The present application relates to a compound of formula (I), a stereoisomer thereof, or a pharmaceutically acceptable salt thereof: 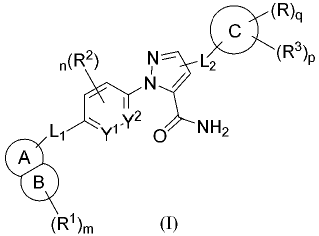 wherein,
Y¹ and Y² are each independently selected from the group consisting of CH and N;
ring A or ring B is each independently selected from the group consisting of phenyl and 5- to 6-membered heteroaryl; ring C is selected from the group consisting of 6- to 12-membered aryl, 4- to 12-membered heterocyclyl, and 5- to 12-membered heteroaryl;
each R¹ is independently selected from the group consisting of deuterium, hydroxy, amino, cyano, nitro, halogen, C₁₋₆ alkyl, hydroxy C₁₋₆ alkyl, 3- to 6-membered cycloalkyl, C₁₋₆ alkoxy, C₁₋₆ alkylthio, C₁₋₆ alkylamino, di-C₁₋₆ alkylamino, C₁₋₆ haloalkyl, C₁₋₆ haloalkoxy, C₁₋₆ haloalkylthio, C₁₋₆ haloalkylamino, and di-C₁₋₆ haloalkylamino;
each R² is independently selected from the group consisting of deuterium, hydroxy, amino, cyano, halogen, C₁₋₆ alkyl, C₂₋₆ alkenyl, C₂₋₆ alkynyl, hydroxy C₁₋₆ alkyl, 3- to 6-membered cycloalkyl, C₁₋₆ alkoxy, C₁₋₆ alkylthio, C₁₋₆ alkylamino, di-C₁₋₆ alkylamino, C₁₋₆ haloalkyl, C₁₋₆ haloalkoxy, C₁₋₆ haloalkylthio, C₁₋₆ haloalkylamino, and di-C₁₋₆ haloalkylamino;
each R³ is independently selected from the group consisting of deuterium, hydroxy, amino, cyano, halogen, C₁₋₆ alkyl, C₁₋₆ alkoxy, C₁₋₆ alkylamino, di-C₁₋₆ alkylamino, C₂₋₈ alkenyl, and C₂₋₈ alkynyl, wherein the hydroxy, amino, C₁₋₆ alkyl, C₁₋₆ alkoxy, C₁₋₆ alkylamino, di-C₁₋₆ alkylamino, C₂₋₈ alkenyl, or C₂₋₈ alkynyl is optionally substituted with one or more R^{3a};
each R^{3a} is independently selected from the group consisting of deuterium, hydroxy, halogen, amino, cyano, =O, C₁₋₆ alkyl, C₁₋₆ alkoxy, C₁₋₆ alkylamino, di-C₁₋₆ alkylamino, 3- to 6-membered cycloalkyl, and 4- to 6-membered heterocyclyl, wherein the hydroxy, amino, C₁₋₆ alkyl, C₁₋₆ alkoxy, C₁₋₆ alkylamino, di-C₁₋₆ alkylamino, 3- to 6-membered cycloalkyl, or 4- to 6-membered heterocyclyl is optionally substituted with one or more R^{3aa}.
each R^{3aa} is independently selected from the group consisting of deuterium, hydroxy, halogen, amino, cyano, =O, C₁₋₆ alkyl, C₁₋₆ alkoxy, -N(C₁₋₆ alkyl)₂, -NHC₁₋₆ alkyl, C₂₋₆ alkenyl, C₂₋₆ alkynyl, 3- to 6-membered cycloalkyl, and 4- to 6-membered heterocyclyl;
each R is independently selected from the group consisting of 3- to 12-membered cycloalkyl, 4- to 12-membered heterocyclyl, 6- to 12-membered aryl, and 5- to 12-membered heteroaryl, wherein the 3- to 12-membered cycloalkyl, 4- to 12-membered heterocyclyl, 6- to 12-membered aryl, or 5- to 12-membered heteroaryl is optionally substituted with one or more R^{a};
each R^{a} is independently selected from the group consisting of deuterium, hydroxy, halogen, amino, cyano, =O, - N(C₁₋₆ alkyl)₂, -NHC₁₋₆ alkyl, C₁₋₆ alkyl, hydroxy C₁₋₆ alkyl, C₁₋₆ haloalkyl, deuterated C₁₋₆ alkyl, amino C₁₋₆ alkyl, C₁₋₆ alkoxy C₁₋₆ alkylene, deuterated C₁₋₆ alkoxy C₁₋₆ alkylene, C₁₋₆ haloalkoxy C₁₋₆ alkylene, C₁₋₆ alkylamino C₁₋₆ alkylene, di-C₁₋₆ alkylamino C₁₋₆ alkylene, C₁₋₆ alkylthio C₁₋₆ alkylene, 3- to 6-membered cycloalkyl, 4- to 6-membered heterocyclyl, 3- to 6-membered cycloalkyl C₁₋₆ alkylene, and 4- to 6-membered heterocyclyl C₁₋₆ alkylene;
L₁ is selected from the group consisting of a bond, -C₁₋₆ alkylene-, -C(O)NHC₁₋₆ alkylene-, -NHC(O)C₁₋₆ alkylene-, and -NHC₁₋₆ alkylene-, wherein the -C₁₋₆ alkylene-, -C(O)NHC₁₋₆ alkylene-, -NHC(O)C₁₋₆ alkylene-, or -NHC₁₋₆ alkylene- is optionally substituted with one or more groups selected from the group consisting of deuterium, hydroxy, halogen, amino, cyano, =O, and C₁₋₆ alkyl;
L₂ is selected from the group consisting of a bond, -NH-, -N(C₁₋₃ alkyl)-, -O-, and -S-;
m is selected from the group consisting of 0, 1, 2, 3, 4, 5, and 6;
n is selected from the group consisting of 0, 1, 2, 3, and 4;
p is selected from the group consisting of 0, 1, 2, 3, and 4;
q is selected from the group consisting of 0, 1, and 2;
provided that p + q ≥ 1.

In some embodiments, the present application relates to a compound of formula (I), a stereoisomer thereof, or a pharmaceutically acceptable salt thereof,
wherein,
Y¹ and Y² are each independently selected from the group consisting of CH and N;
ring A or ring B is each independently selected from the group consisting of phenyl and 5- to 6-membered heteroaryl; ring C is selected from the group consisting of 6- to 12-membered aryl, 4- to 12-membered heterocyclyl, and 5- to 12-membered heteroaryl;
each R¹ is independently selected from the group consisting of hydroxy, amino, cyano, nitro, halogen, C₁₋₆ alkyl, hydroxy C₁₋₆ alkyl, 3- to 6-membered cycloalkyl, C₁₋₆ alkoxy, C₁₋₆ alkylthio, C₁₋₆ alkylamino, di-C₁₋₆ alkylamino, C₁₋₆ haloalkyl, C₁₋₆ haloalkoxy, C₁₋₆ haloalkylthio, C₁₋₆ haloalkylamino, and di-C₁₋₆ haloalkylamino;
each R² is independently selected from the group consisting of hydroxy, amino, cyano, halogen, C₁₋₆ alkyl, C₂₋₆ alkenyl, C₂₋₆ alkynyl, hydroxy C₁₋₆ alkyl, 3- to 6-membered cycloalkyl, C₁₋₆ alkoxy, C₁₋₆ alkylthio, C₁₋₆ alkylamino, di-C₁₋₆ alkylamino, C₁₋₆ haloalkyl, C₁₋₆ haloalkoxy, C₁₋₆ haloalkylthio, C₁₋₆ haloalkylamino, and di-C₁₋₆ haloalkylamino;
each R³ is independently selected from the group consisting of hydroxy, amino, cyano, halogen, C₁₋₆ alkyl, C₁₋₆ alkoxy, C₁₋₆ alkylamino, and di-C₁₋₆ alkylamino, wherein the C₁₋₆ alkyl, C₁₋₆ alkoxy, C₁₋₆ alkylamino, or di-C₁₋₆ alkylamino is optionally substituted with one or more R^{3a};
each R^{3a} is independently selected from the group consisting of hydroxy, halogen, amino, cyano, =O, C₁₋₆ alkoxy, C₁₋₆ alkylamino, di-C₁₋₆ alkylamino, 3- to 6-membered cycloalkyl, and 4- to 6-membered heterocyclyl;
each R is independently selected from the group consisting of 3- to 12-membered cycloalkyl, 4- to 12-membered heterocyclyl, 6- to 12-membered aryl, and 5- to 12-membered heteroaryl, wherein the 3- to 12-membered cycloalkyl, 4- to 12-membered heterocyclyl, 6- to 12-membered aryl, or 5- to 12-membered heteroaryl is optionally substituted with one or more R^{a};
each R^{a} is independently selected from the group consisting of hydroxy, halogen, amino, cyano, =O, C₁₋₆ alkyl, hydroxy C₁₋₆ alkyl, C₁₋₆ haloalkyl, amino C₁₋₆ alkyl, C₁₋₆ alkoxy C₁₋₆ alkylene, C₁₋₆ alkylamino C₁₋₆ alkylene, di-C₁₋₆ alkylamino C₁₋₆ alkylene, C₁₋₆ alkylthio C₁₋₆ alkylene, 3- to 6-membered cycloalkyl, 4- to 6-membered heterocyclyl, 3- to 6-membered cycloalkyl C₁₋₆ alkylene, and 4- to 6-membered heterocyclyl C₁₋₆ alkylene;
L₁ is selected from the group consisting of a bond, -C₁₋₆ alkylene-, -C(O)NHC₁₋₆ alkylene-, -NHC(O)C₁₋₆ alkylene-, and -NHC₁₋₆ alkylene-, wherein the -C₁₋₆ alkylene-, -C(O)NHC₁₋₆ alkylene-, -NHC(O)C₁₋₆ alkylene-, or -NHC₁₋₆ alkylene- is optionally substituted with one or more groups selected from the group consisting of hydroxy, halogen, amino, cyano, =O, and C₁₋₆ alkyl;
L₂ is selected from the group consisting of a bond, -NH-, -N(C₁₋₃ alkyl)-, -O-, and -S-;
m is selected from the group consisting of 0, 1, 2, 3, 4, 5, and 6;
n is selected from the group consisting of 0, 1, 2, 3, and 4;
p is selected from the group consisting of 0, 1, 2, 3, and 4;
q is selected from the group consisting of 0, 1, and 2;
provided that p + q ≥ 1.

In some embodiments, at least one of Y¹ and Y² is CH. In some embodiments, Y¹ is CH, and Y² is N. In some embodiments, Y¹ is N, and Y² is CH. In some embodiments, Y¹ and Y² are both CH.

In some embodiments, ring A or ring B is each independently selected from the group consisting of phenyl, pyridinyl, pyrimidinyl, pyridazinyl, pyrazinyl, pyranyl, furanyl, pyrrolyl, thienyl, pyrazolyl, imidazolyl, thiazolyl, oxazolyl, and isoxazolyl.

In some embodiments, ring A or ring B is each independently selected from the group consisting of phenyl and 6-membered heteroaryl. In some embodiments, ring A is selected from phenyl, and ring B is selected from 5- to 6-membered heteroaryl. In some embodiments, ring A is selected from phenyl, and ring B is selected from 6-membered heteroaryl.

In some embodiments, ring A is selected from the group consisting of phenyl and pyridinyl. In some embodiments, ring B is selected from the group consisting of phenyl and pyridinyl.

In some embodiments, ring A is selected from phenyl. In some embodiments, ring B is selected from pyridinyl.

In some embodiments, the moiety 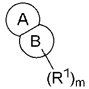 is selected from the group consisting of 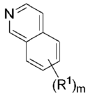 and 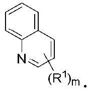 In some embodiments, the moiety 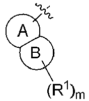 is selected from the group consisting of

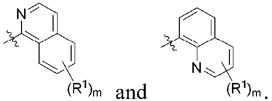

In some embodiments, the moiety 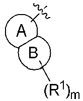 is selected from the group consisting of 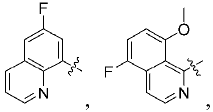 and 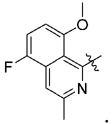 In some embodiments, the moiety 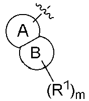 is selected from the group consisting of 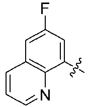 and 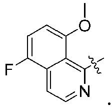 In some embodiments, the moiety 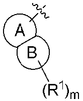 is selected from

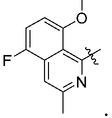

In some embodiments, ring C is selected from the group consisting of 6- to 10-membered aryl, 5- to 10-membered heterocyclyl, and 5- to 10-membered heteroaryl. In some embodiments, ring C is selected from the group consisting of phenyl, 5- to 6-membered heterocyclyl, and 5- to 6-membered heteroaryl.

In some embodiments, ring C is selected from the group consisting of 6- to 12-membered aryl and 5- to 12-membered heteroaryl; in some embodiments, ring C is selected from the group consisting of 6- to 10-membered aryl and 5- to 6-membered heteroaryl. In some embodiments, ring C is selected from the group consisting of phenyl and 5- to 6-membered heteroaryl.

In some embodiments, ring C is selected from the group consisting of phenyl, pyridinyl, pyrimidinyl, pyridazinyl, pyrazinyl, and pyranyl.

In some embodiments, ring C is selected from the group consisting of phenyl and pyridinyl. In some embodiments, ring C is selected from pyridinyl.

In some embodiments, the moiety 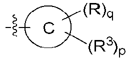 is selected from the group consisting of 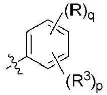 and 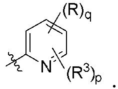 In some embodiments, the moiety 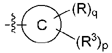 is selected from the group consisting of 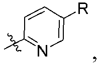 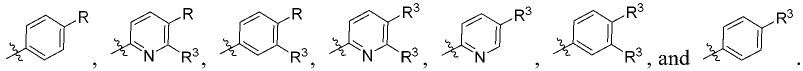 In some embodiments, the moiety 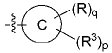 is selected from the group consisting of 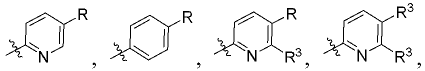 and 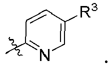 In some embodiments, the moiety 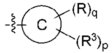 is selected from the group consisting of

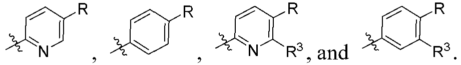

In some embodiments, the moiety 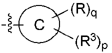 is selected from the group consisting of

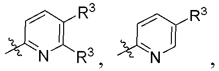

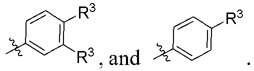

In some embodiments, the moiety 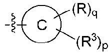 is selected from the group consisting of 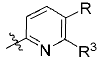 and

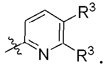

In some embodiments, the moiety 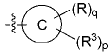 is selected from

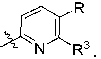

In some embodiments, each R¹ is independently selected from the group consisting of hydroxy, amino, cyano, halogen, C₁₋₄ alkyl, C₃₋₆ cycloalkyl, C₁₋₄ alkoxy, C₁₋₄ haloalkyl, C₁₋₄ alkylamino, and di-C₁₋₄ alkylamino.

In some embodiments, each R¹ is independently selected from the group consisting of halogen, C₁₋₆ alkyl, and C₁₋₆ alkoxy. In some embodiments, each R¹ is independently selected from the group consisting of halogen, C₁₋₄ alkyl, and C₁₋₄ alkoxy.

In some embodiments, each R¹ is independently selected from the group consisting of hydroxy, amino, cyano, fluoro, chloro, bromo, methyl, ethyl, n-propyl, isopropyl, n-butyl, isobutyl, tert-butyl, cyclopropyl, cyclobutyl, cyclopentyl, cyclohexyl, methoxy, ethoxy, trifluoromethyl, difluoromethyl, trifluoroethyl, dimethylamino, and diethylamino.

In some other embodiments, each R¹ is independently selected from the group consisting of fluoro, methyl, and methoxy. In some embodiments, each R¹ is independently selected from the group consisting of fluoro and methoxy. In some embodiments, each R² is independently selected from the group consisting of hydroxy, amino, cyano, halogen, methyl, ethyl, n-propyl, isopropyl, trifluoromethyl, and difluoromethyl.

In some embodiments, R² is selected from fluoro.

In some embodiments, each R³ is independently selected from the group consisting of deuterium, hydroxy, amino, cyano, halogen, C₁₋₄ alkyl, C₁₋₄ alkoxy, C₁₋₄ alkylamino, di-C₁₋₄ alkylamino, C₂₋₆ alkenyl, and C₂₋₆ alkynyl, wherein the hydroxy, amino, C₁₋₄ alkyl, C₁₋₄ alkoxy, C₁₋₄ alkylamino, di-C₁₋₄ alkylamino, C₂₋₆ alkenyl, or C₂₋₆ alkynyl is optionally substituted with one or more R^{3a}.

In some embodiments, each R³ is independently selected from the group consisting of amino, C₁₋₆ alkyl, and C₂₋₈ alkenyl, wherein the amino, C₁₋₆ alkyl, or C₂₋₈ alkenyl is optionally substituted with one or more R^{3a}. In some embodiments, each R³ is independently selected from the group consisting of amino, C₁₋₄ alkyl, and C₂₋₆ alkenyl, wherein the amino, C₁₋₄ alkyl, or C₂₋₆ alkenyl is optionally substituted with one or more R^{3a}.

In some embodiments, each R³ is independently selected from the group consisting of C₁₋₄ alkyl, C₁₋₄ alkoxy, C₁₋₄ alkylamino, and di-C₁₋₄ alkylamino, wherein the C₁₋₄ alkyl, C₁₋₄ alkoxy, C₁₋₄ alkylamino, or di-C₁₋₄ alkylamino is optionally substituted with one or more R^{3a}. In some embodiments, each R³ is independently selected from the group consisting of amino, C₂₋₆ alkenyl, and C₂₋₆ alkynyl, wherein the amino, C₂₋₆ alkenyl, or C₂₋₆ alkynyl is optionally substituted with one or more R^{3a}.

In some embodiments, each R³ is independently selected from the group consisting of deuterium, cyano, fluoro, chloro, bromo, and the following groups optionally substituted with one or more R^{3a}: hydroxy, amino, methyl, ethyl, n-propyl, isopropyl, n-butyl, isobutyl, tert-butyl, methoxy, ethoxy, propoxy, tert-butyloxy, methylamino, ethylamino, dimethylamino, diethylamino, ethenyl, propenyl, butenyl, pentenyl, propynyl, butynyl, and pentynyl.

In some embodiments, each R³ is independently selected from the group consisting of the following groups optionally substituted with one or more R^{3a}: methyl, ethyl, n-propyl, isopropyl, n-butyl, isobutyl, tert-butyl, methoxy, ethoxy, tert-butoxy, methylamino, ethylamino, dimethylamino, and diethylamino. In some embodiments, each R³ is independently selected from the group consisting of the following groups optionally substituted with one or more R^{3a}: amino and pentenyl.

In some embodiments, each R³ is independently selected from the group consisting of deuterium, cyano, fluoro, chloro, and the following groups optionally substituted with one or more R^{3a}: hydroxy, amino, methyl, ethyl, n-propyl, isopropyl, n-butyl, isobutyl, tert-butyl, -CH=CH₂, -CH₂CH=CH₂, -CH₂CH₂CH=CH₂, -CH₂CH=CHCH₃, - CH₂CH₂CH₂CH=CH₂, -CH₂CH₂CH=CHCH₃, and -CH₂CH=CHCH₂CH₃.

In some embodiments, each R³ is independently selected from the group consisting of the following groups optionally substituted with one or more R^{3a}: amino, methyl, ethyl, n-propyl, and -CH₂CH₂CH₂CH=CH₂.

In some embodiments, each R^{3a} is independently selected from the group consisting of deuterium, halogen, cyano, =O, hydroxy, amino, C₁₋₄ alkyl, C₁₋₄ alkoxy, C₁₋₄ alkylamino, di-C₁₋₄ alkylamino, 3- to 6-membered cycloalkyl, and 4- to 6-membered heterocycloalkyl, wherein the hydroxy, amino, C₁₋₄ alkyl, C₁₋₄ alkoxy, C₁₋₄ alkylamino, di-C₁₋₄ alkylamino, 3- to 6-membered cycloalkyl, or 4- to 6-membered heterocycloalkyl is optionally substituted with one or more R^{3aa}.

In some embodiments, each R^{3a} is independently selected from the group consisting of hydroxy, halogen, C₁₋₆ alkoxy, 3- to 6-membered cycloalkyl, and 4- to 6-membered heterocyclyl, wherein the hydroxy, C₁₋₆ alkoxy, 3- to 6-membered cycloalkyl, or 4- to 6-membered heterocyclyl is optionally substituted with one or more R^{3aa}.

In some embodiments, each R^{3a} is independently selected from the group consisting of hydroxy, halogen, amino, cyano, =O, C₁₋₆ alkoxy, C₁₋₆ alkylamino, di-C₁₋₆ alkylamino, 3- to 6-membered cycloalkyl, and 4- to 6-membered heterocyclyl, wherein the hydroxy, amino, C₁₋₆ alkoxy, C₁₋₆ alkylamino, di-C₁₋₆ alkylamino, 3- to 6-membered cycloalkyl, or 4- to 6-membered heterocyclyl is optionally substituted with one or more R^{3aa}.

In some embodiments, each R^{3a} is independently selected from the group consisting of hydroxy, halogen, C₁₋₄ alkoxy, 3- to 6-membered cycloalkyl, and 4- to 6-membered heterocyclyl, wherein the hydroxy, C₁₋₄ alkoxy, 3- to 6-membered cycloalkyl, or 4- to 6-membered heterocyclyl is optionally substituted with one or more R^{3aa}.

In some embodiments, each R^{3a} is independently selected from the group consisting of hydroxy, halogen, amino, cyano, =O, C₁₋₄ alkoxy, C₁₋₄ alkylamino, di-C₁₋₄ alkylamino, 3- to 6-membered cycloalkyl, and 4- to 6-membered heterocyclyl.

In some embodiments, each R^{3a} is independently selected from the group consisting of hydroxy, halogen, amino, cyano, =O, methyl, ethyl, n-propyl, isopropyl, methoxy, ethoxy, methylamino, ethylamino, dimethylamino, diethylamino, cyclopropyl, cyclobutyl, cyclopentyl, cyclohexyl, azetidinyl, oxetanyl, tetrahydrofuranyl, tetrahydropyrrolyl, tetrahydrothienyl, piperidinyl, piperazinyl, and morpholinyl, wherein the hydroxy, amino, methyl, ethyl, n-propyl, isopropyl, methoxy, ethoxy, methylamino, ethylamino, dimethylamino, diethylamino, cyclopropyl, cyclobutyl, cyclopentyl, cyclohexyl, azetidinyl, oxetanyl, tetrahydrofuranyl, tetrahydropyrrolyl, tetrahydrothienyl, piperidinyl, piperazinyl, or morpholinyl is optionally substituted with one or more R^{3aa}.

In some embodiments, each R^{3a} is independently selected from the group consisting of hydroxy, halogen, amino, cyano, =O, methoxy, ethoxy, methylamino, ethylamino, dimethylamino, diethylamino, cyclopropyl, cyclobutyl, cyclopentyl, cyclohexyl, azetidinyl, oxetanyl, tetrahydrofuranyl, tetrahydropyrrolyl, tetrahydrothienyl, piperidinyl, piperazinyl, and morpholinyl.

In some embodiments, each R^{3a} is independently selected from the group consisting of fluoro, hydroxy, methoxy, ethoxy, cyclopropyl, cyclobutyl, cyclopentyl, cyclohexyl, and piperidinyl, wherein the hydroxy, methoxy, ethoxy, cyclopropyl, cyclobutyl, cyclopentyl, cyclohexyl, or piperidinyl is optionally substituted with one or more R^{3aa}.

In some embodiments, each R^{3aa} is independently selected from the group consisting of deuterium, hydroxy, halogen, amino, cyano, =O, -N(C₁₋₃ alkyl)₂, -NHC₁₋₃ alkyl, C₁₋₃ alkyl, 3- to 6-membered cycloalkyl, and 4- to 6-membered heterocycloalkyl.

In some embodiments, each R^{3aa} is independently selected from the group consisting of deuterium, C₁₋₆ alkyl, - N(C₁₋₆ alkyl)₂, and 3- to 6-membered cycloalkyl. In some embodiments, each R^{3aa} is independently selected from the group consisting of deuterium, C₁₋₃ alkyl, -N(C₁₋₃ alkyl)₂, and 3- to 6-membered cycloalkyl.

In some embodiments, each R^{3aa} is independently selected from the group consisting of deuterium, halogen, -N(C₁₋₃ alkyl)₂, C₁₋₃ alkyl, and 3- to 5-membered cycloalkyl.

In some embodiments, each R^{3aa} is independently selected from the group consisting of deuterium, fluoro, -N(CH₃)₂, methyl, and cyclopropyl.

In some embodiments, each R^{3a} is independently selected from the group consisting of hydroxy, fluoro, methoxy, ethoxy, -OCD₃, cyclopropyl,

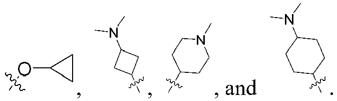

In some embodiments, each R is independently selected from the group consisting of the following groups optionally substituted with one or more R^{a}: 3- to 8-membered cycloalkyl, 4- to 8-membered heterocyclyl, 6- to 10-membered aryl, and 5- to 10-membered heteroaryl. In some embodiments, each R is independently selected from the group consisting of the following groups optionally substituted with one or more R^{a}: 3- to 6-membered cycloalkyl, 4- to 6-membered heterocyclyl, phenyl, and 5- to 6-membered heteroaryl.

In some embodiments, each R is independently selected from 4- to 12-membered heterocyclyl, wherein the 4- to 12-membered heterocyclyl is optionally substituted with one or more R^{a}. In some embodiments, each R is independently selected from 4- to 8-membered heterocyclyl, wherein the 4- to 8-membered heterocyclyl is optionally substituted with one or more R^{a}. In some embodiments, each R is independently selected from 4- to 6-membered heterocyclyl, wherein the 4- to 6-membered heterocyclyl is optionally substituted with one or more R^{a}. In some embodiments, each R is independently selected from the group consisting of the following groups optionally substituted with one or more R^{a}: cyclopropyl, cyclobutyl, cyclopentyl, cyclohexyl, azetidinyl, oxetanyl, tetrahydrofuranyl, tetrahydropyrrolyl, tetrahydrothienyl, piperidinyl, tetrahydropyranyl, tetrahydrothiopyranyl, piperazinyl, morpholinyl, 1,4-dioxanyl, thiomorpholinyl, furanyl, pyrrolyl, pyrazolyl, imidazolyl, thienyl, thiazolyl, oxazolyl, isoxazolyl, phenyl, pyridinyl, pyrimidinyl, pyridazinyl, pyrazinyl, and pyranyl.

In some embodiments, each R is independently selected from the group consisting of the following groups optionally substituted with one or more R^{a}: 3- to 6-membered cycloalkyl and 4- to 6-membered heterocycloalkyl. In some embodiments, each R is independently selected from 4- to 6-membered heterocycloalkyl optionally substituted with one or more R^{a}.

In some embodiments, each R is independently selected from the group consisting of the following groups optionally substituted with one or more R^{a}: piperidinyl and piperazinyl.

In some embodiments, each R^{a} is independently selected from the group consisting of deuterium, hydroxy, halogen, amino, cyano, =O, -N(C₁₋₃ alkyl)₂, -NHC₁₋₃ alkyl, C₁₋₄ alkyl, hydroxy C₁₋₄ alkyl, C₁₋₄ haloalkyl, deuterated C₁₋₄ alkyl, amino C₁₋₄ alkyl, C₁₋₃ alkoxy C₁₋₃ alkylene, deuterated C₁₋₃ alkoxy C₁₋₃ alkylene, C₁₋₃ haloalkoxy C₁₋₃ alkylene, C₁₋₃ alkylamino C₁₋₃ alkylene, di-C₁₋₃ alkylamino C₁₋₃ alkylene, C₁₋₃ alkylthio C₁₋₃ alkylene, 3- to 6-membered cycloalkyl, 4- to 6-membered heterocycloalkyl, 3- to 6-membered cycloalkyl C₁₋₃ alkylene, and 4- to 6-membered heterocycloalkyl C₁₋₃ alkylene.

In some embodiments, each R^{a} is independently selected from the group consisting of -N(C₁₋₆ alkyl)₂, C₁₋₆ alkyl, deuterated C₁₋₆ alkyl, C₁₋₆ alkoxy C₁₋₆ alkylene, deuterated C₁₋₆ alkoxy C₁₋₆ alkylene, 3- to 6-membered cycloalkyl, and 4- to 6-membered heterocyclyl. In some embodiments, each R^{a} is independently selected from the group consisting of -N(C₁₋₃ alkyl)₂, C₁₋₃ alkyl, deuterated C₁₋₄ alkyl, C₁₋₃ alkoxy C₁₋₃ alkylene, deuterated C₁₋₃ alkoxy C₁₋₃ alkylene, 3- to 6-membered cycloalkyl, and 4- to 6-membered heterocyclyl. In some embodiments, each R^{a} is independently selected from the group consisting of -N(C₁₋₃ alkyl)₂, C₁₋₃ alkyl, deuterated C₁₋₄ alkyl, C₁₋₃ alkoxy C₁₋₃ alkylene, deuterated C₁₋₃ alkoxy C₁₋₃ alkylene, 3- to 6-membered cycloalkyl, and 4- to 6-membered heterocycloalkyl.

In some embodiments, each R^{a} is independently selected from the group consisting of hydroxy, halogen, amino, - N(C₁₋₃ alkyl)₂, -NHC₁₋₃ alkyl, C₁₋₄ alkyl, C₁₋₄ haloalkyl, deuterated C₁₋₄ alkyl, C₁₋₃ alkoxy C₁₋₃ alkylene, deuterated C₁₋₃ alkoxy C₁₋₃ alkylene, C₁₋₃ haloalkoxy C₁₋₃ alkylene, 3- to 6-membered cycloalkyl, and 4- to 6-membered heterocycloalkyl.

In some embodiments, each R^{a} is independently selected from the group consisting of hydroxy, halogen, amino, cyano, =O, C₁₋₄ alkyl, hydroxy C₁₋₄ alkyl, C₁₋₄ haloalkyl, amino C₁₋₄ alkyl, C₁₋₃ alkoxy C₁₋₃ alkylene, C₁₋₃ alkylamino C₁₋₃ alkylene, di-C₁₋₃ alkylamino C₁₋₃ alkylene, C₁₋₃ alkylthio C₁₋₃ alkylene, 3- to 6-membered cycloalkyl, 4- to 6-membered heterocyclyl, 3- to 6-membered cycloalkyl C₁₋₃ alkylene, and 4- to 6-membered heterocyclyl C₁₋₃ alkylene.

In some embodiments, each R^{a} is independently selected from the group consisting of C₁₋₃ alkoxy C₁₋₃ alkylene, C₁₋₃ alkylamino C₁₋₃ alkylene, di-C₁₋₃ alkylamino C₁₋₃ alkylene, 3- to 6-membered cycloalkyl, and 4- to 6-membered heterocycloalkyl.

In some embodiments, each R^{a} is independently selected from the group consisting of 3- to 6-membered cycloalkyl and 4- to 6-membered heterocycloalkyl.

In some embodiments, each R^{a} is independently selected from the group consisting of deuterium, hydroxy, halogen, amino, cyano, =O, -NHCH₃, -NHCH₂CH₃, -N(CH₃)₂, -N(CH₂CH₃)₂, methyl, ethyl, n-propyl, isopropyl, n-butyl, isobutyl, tert-butyl, hydroxymethyl, hydroxyethyl, trifluoromethyl, difluoromethyl, monofluoromethyl, trifluoroethyl, pentafluoroethyl, trideuteriomethyl, dideuteriomethyl, monodeuteriomethyl, -CH₂NH₂, - CH₂CH₂NH₂, -CH₂OCH₃, -CH₂OCH₂CH₃, -CH₂CH₂OCH₃, -CH₂CH₂OCH₂CH₃, -CH₂CH₂OCD₃, -CH₂NHCH₃, - CH₂CH₂NHCH₃, -CH₂N(CH₃)₂, -CH₂CH₂N(CH₃)₂, -CH₂SCH₃, -CH₂CH₂SCH₃, cyclopropyl, cyclobutyl, cyclopentyl, cyclohexyl, azetidinyl, oxetanyl, tetrahydrofuranyl, tetrahydropyrrolyl, tetrahydrothienyl, piperidinyl, piperazinyl, morpholinyl, cyclopropylmethyl, cyclobutylmethyl, cyclopentylmethyl, cyclohexylmethyl, azetidinylmethyl, oxetanylmethyl, tetrahydrofuranylmethyl, tetrahydropyrrolylmethyl, tetrahydrothienylmethyl, piperidinylmethyl, piperazinylmethyl, and morpholinylmethyl.

In some embodiments, each R^{a} is independently selected from the group consisting of hydroxy, halogen, amino, cyano, =O, methyl, ethyl, n-propyl, isopropyl, n-butyl, isobutyl, tert-butyl, hydroxymethyl, hydroxyethyl, trifluoromethyl, difluoromethyl, monofluoromethyl, trifluoroethyl, pentafluoroethyl, aminomethyl, aminoethyl, methoxymethyl, ethoxymethyl, methoxyethyl, ethoxyethyl, methylaminomethyl, methylaminoethyl, dimethylaminomethyl, dimethylaminoethyl, methylthiomethyl, methylthioethyl, cyclopropyl, cyclobutyl, cyclopentyl, cyclohexyl, azetidinyl, oxetanyl, tetrahydrofuranyl, tetrahydropyrrolyl, tetrahydrothienyl, piperidinyl, piperazinyl, morpholinyl, cyclopropylmethyl, cyclobutylmethyl, cyclopentylmethyl, cyclohexylmethyl, azetidinylmethyl, oxetanylmethyl, tetrahydrofuranylmethyl, tetrahydropyrrolylmethyl, tetrahydrothienylmethyl, piperidinylmethyl, piperazinylmethyl, and morpholinylmethyl.

In some embodiments, each R^{a} is independently selected from the group consisting of -N(CH₃)₂, methyl, trideuteriomethyl, -CH₂CH₂OCH₃, -CH₂CH₂OCD₃, cyclopropyl, and oxetanyl.

In some embodiments, each R³ is independently selected from the group consisting of

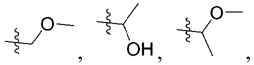

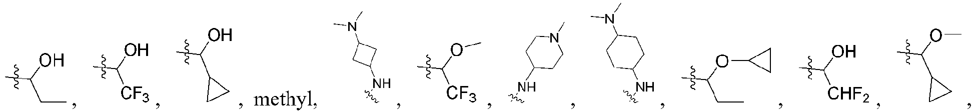

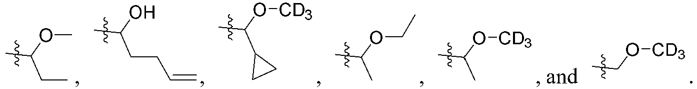

In some embodiments, each R³ is independently selected from the group consisting of

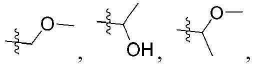

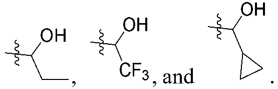

In some embodiments, each R is independently selected from the group consisting of

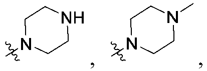

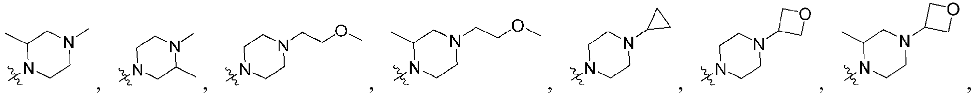

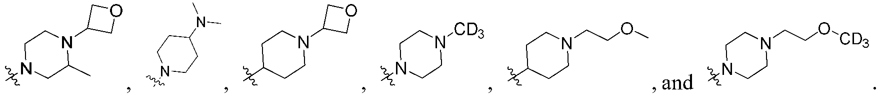

In some embodiments, each R is independently selected from the group consisting of 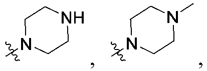 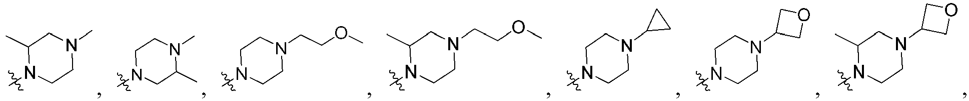 and

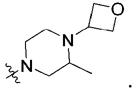

In some embodiments, L₁ is selected from the group consisting of a bond, -C₁₋₃ alkylene-, -C(O)NHC₁₋₃ alkylene-, -NHC(O)C₁₃ alkylene-, and -NHC₁₋₃ alkylene-, wherein the -C₁₋₃ alkylene-, -C(O)NHC₁₋₃ alkylene-, -NHC(O)C₁₋₃ alkylene-, or -NHC₁₋₃ alkylene- is optionally substituted with one or more groups selected from the group consisting of hydroxy, halogen, amino, cyano, =O, and C₁₋₃ alkyl.

In some embodiments, L₁ is selected from the group consisting of -C(O)NHC₁₋₆ alkylene- and -NHC₁₋₆ alkylene-. In some embodiments, L₁ is selected from the group consisting of a bond, -methylene-, -C(O)NHCH₂-, - NHC(O)CH₂-, and -NHCH₂-.

In some embodiments, L₁ is selected from the group consisting of -C(O)NHCH₂- and -NHCH₂-.

In some embodiments, L₁ is selected from the group consisting of *-C(O)NHC₁₋₆ alkylene-, *-NHC(O)C₁₋₆ alkylene-, and *-NHC₁₋₆ alkylene-, wherein * indicates linkage to the moiety 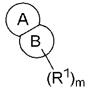 of the compound of formula (I). In some embodiments, L₁ is selected from the group consisting of *-C(O)NHC₁₋₆ alkylene- and *-NHC₁₋₆ alkylene-, wherein * indicates linkage to the moiety 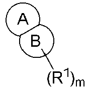 of the compound of formula (I).

In some embodiments, L₁ is selected from the group consisting of *-C(O)NHCH₂- and *-NHCH₂-, wherein * represents linkage to the moiety 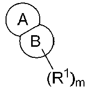 of the compound of formula (I).

In some embodiments, L₂ is selected from -NH-.

In some embodiments, m is selected from the group consisting of 0, 1, 2, and 3.

In some embodiments, m is selected from the group consisting of 1 and 2.

In some embodiments, n is selected from the group consisting of 0, 1, and 2.

In some embodiments, n is 0.

In some embodiments, p is selected from the group consisting of 0, 1, 2, and 3.

In some embodiments, p is selected from the group consisting of 1 and 2.

In some embodiments, p is selected from the group consisting of 0 and 1.

In some embodiments, q is selected from the group consisting of 1 and 2.

In some embodiments, q is 1.

In some embodiments, the C₁₋₆ alkyl or C₁₋₆ alkylene is selected from the group consisting of C₁₋₄ alkyl and C₁₋₄ alkylene. In some embodiments, the C₁₋₄ alkyl is selected from the group consisting of C₁₋₃ alkyl and C₁₋₂ alkyl. In some embodiments, the C₁₋₄ alkylene is selected from the group consisting of C₁₋₃ alkylene, C₁₋₂ alkylene, and methylene.

In some embodiments, the halo is selected from the group consisting of fluoro, chloro, bromo, and iodo. In some embodiments, the halo is selected from a substitution with 1, 2, 3, 4, or 5 halogens.

In some embodiments, the deuterated means that any number of H atoms present on the substituent that can be substituted are substituted with deuterium atoms; for example, non-limiting examples of monodeuterated ethyl include, but are not limited to: -CHD-CH₃ and -CH₂-CH₂D.

In some embodiments, the heteroaryl contains 1, 2, 3, or 4 heteroatoms selected from the group consisting of N, O, and S. In some embodiments, the heteroaryl contains 1, 2, 3, or 4 heteroatoms selected from the group consisting of N and O. In some embodiments, the heteroaryl contains 1 N atom.

In some embodiments, the heterocycloalkyl contains 1, 2, 3, or 4 heteroatoms selected from the group consisting of N, O, and S. In some embodiments, the heterocycloalkyl contains 1, 2, 3, or 4 heteroatoms selected from the group consisting of N and O. In some embodiments, the heterocycloalkyl contains 1 or 2 heteroatoms selected from the group consisting of N and O. In some embodiments, the heterocycloalkyl contains 2 heteroatoms selected from N. In some embodiments, the heterocyclyl contains 1, 2, 3, 4, or 5 heteroatoms selected from the group consisting of N, O, and S. In some embodiments, the heterocyclyl contains 1, 2, 3, 4, or 5 heteroatoms selected from the group consisting of N and O. In some embodiments, the heterocyclyl contains 1, 2, or 3 heteroatoms selected from the group consisting of N and O. In some embodiments, the heterocyclyl contains 1 or 2 heteroatoms selected from the group consisting of N and O.

In some embodiments, the heterocyclyl is selected from the group consisting of heterocycloalkyl and unsaturated heterocyclyl.

In some embodiments, the heterocycloalkyl includes a monocyclic ring, a spiro ring, or a bridged ring.

In some embodiments, the cycloalkyl includes a monocyclic ring, a spiro ring, or a bridged ring.

In some embodiments, the heterocyclyl includes a monocyclic ring, a spiro ring, or a bridged ring.

In some embodiments, the "one or more" is selected from the group consisting of one, two, three, four, five, and six.

In some embodiments, the "one or more" is selected from the group consisting of one, two, three, and four. In some embodiments, the "one or more" is selected from the group consisting of one, two, and three. In some embodiments, the "one or more" is selected from the group consisting of one and two.

In some embodiments, the "3- to 12-membered" is selected from the group consisting of 4- to 12-membered, 3- to 10-membered, 5- to 10-membered, 3- to 9-membered, 3- to 8-membered, 3- to 6-membered, and 5- to 6-membered. In some embodiments, the "4- to 12-membered" is selected from the group consisting of 4- to 10-membered, 5- to 10-membered, 4- to 9-membered, 4- to 8-membered, 4- to 6-membered, and 5- to 6-membered. In some embodiments, the "6- to 10-membered" is selected from the group consisting of 6- to 9-membered, 6- to 8-membered, and 6-membered. In some embodiments, the "5- to 12-membered" is selected from the group consisting of 5- to 10-membered, 5- to 9-membered, 5- to 8-membered, and 5- to 6-membered.

In some embodiments, the compound of formula (I), the stereoisomer thereof, or the pharmaceutically acceptable salt thereof is selected from the group consisting of compounds of formula (I-A), formula (II), and formula (II-A), stereoisomers thereof, or pharmaceutically acceptable salts thereof: 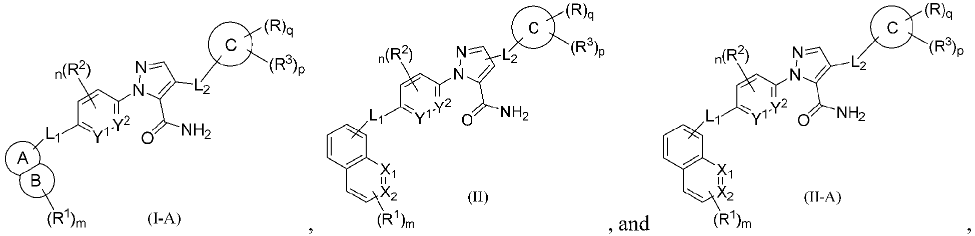 wherein R¹, m, L₁, Y₁, Y₂, R², n, L₂, ring A, ring B, ring C, R, q, R³, and p are as defined in the present disclosure, and X₁ and X₂ are each independently selected from the group consisting of CH and N. In some embodiments, X₁ is selected from N, and X₂ is selected from CH. In some embodiments, X₁ is selected from CH, and X₂ is selected from N.

In some embodiments, the compound of formula I, the stereoisomer thereof, or the pharmaceutically acceptable salt thereof is selected from the group consisting of compounds of formula (III-A) and formula (III-B), stereoisomers thereof, or pharmaceutically acceptable salts thereof: 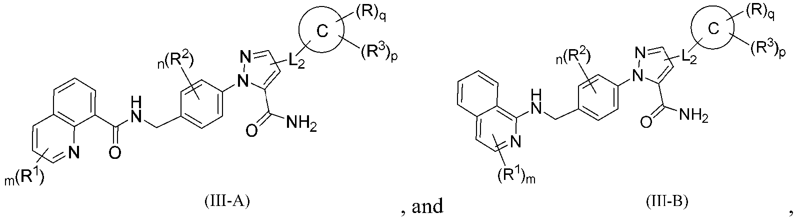 wherein R¹, m, R², n, L₂, ring C, R, q, R³, and p are as defined in the present disclosure.

It should be understood that any embodiment of the compounds of the present disclosure as described above, and any specific substituents set forth herein with respect to particular R¹, L₁, Y₁, Y₂, R², L₂, ring A, ring B, ring C, R, and R³ substituents in the compounds of the present disclosure as described above, may be independently combined with other embodiments of the present disclosure and/or substituents of the compounds to form embodiments of the present disclosure not specifically set forth above. Furthermore, where a list of substituents is disclosed in the detailed description and/or claims with respect to any particular R¹, L₁, Y₁, Y₂, R², L₂, ring A, ring B, ring C, R, and R³ substituents, it should be understood that one or more substituents may be deleted from the list and the remaining list of substituents will be considered an embodiment of the present disclosure.

The present application also relates to a compound of a formula selected from the group consisting of the following formulas, a stereoisomer thereof, or a pharmaceutically acceptable salt thereof:

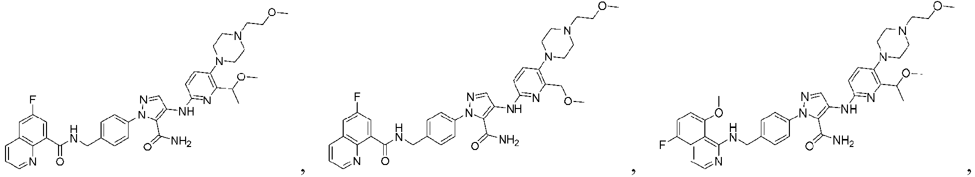

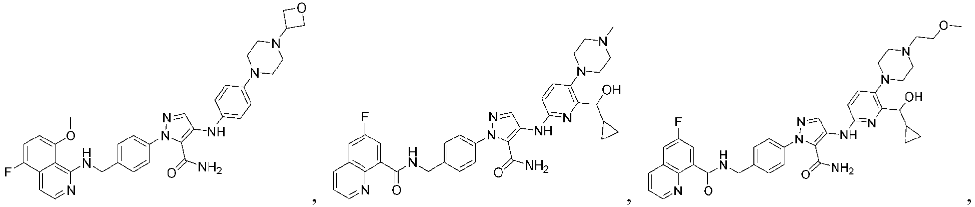

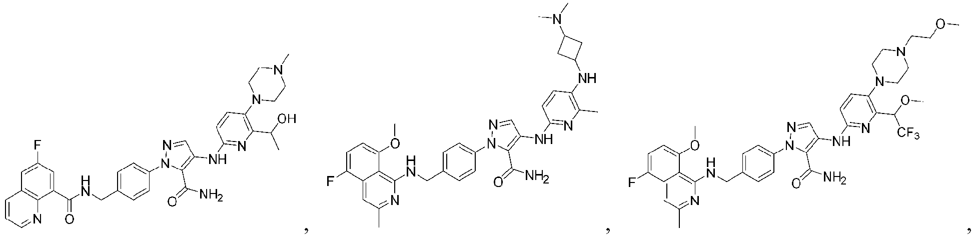

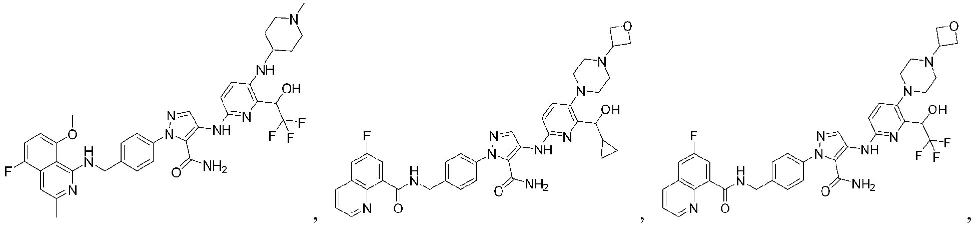

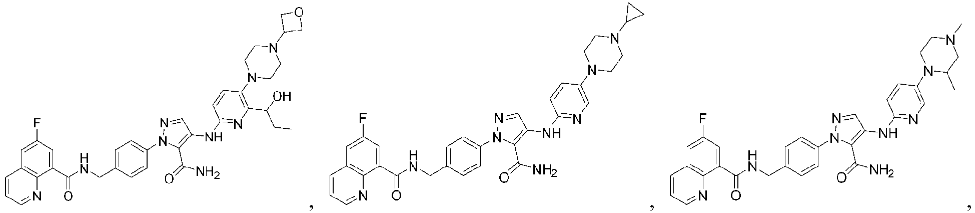

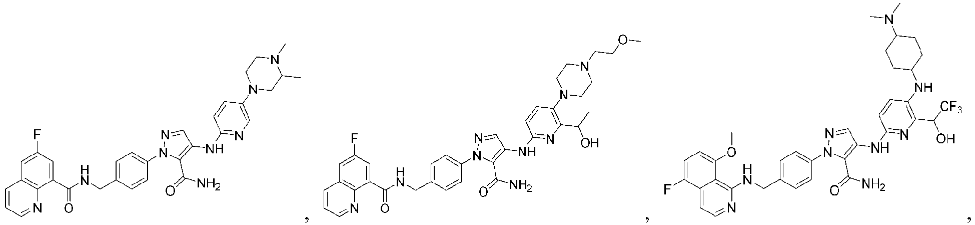

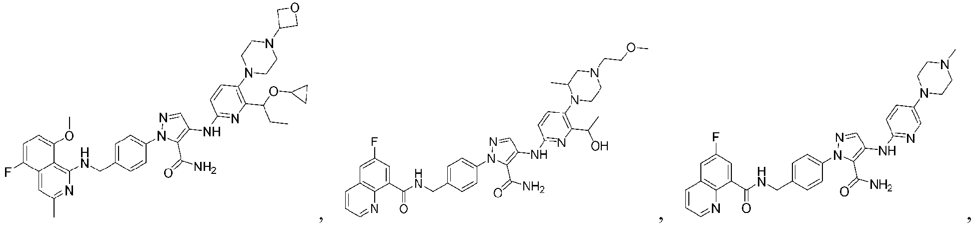

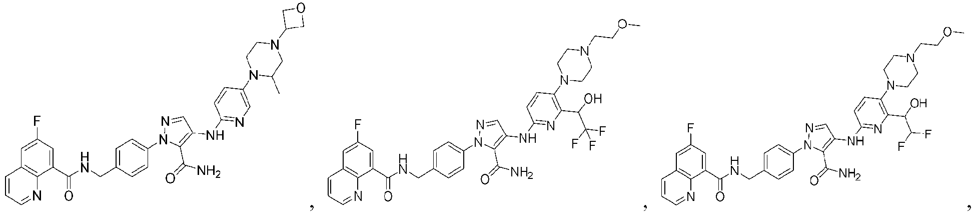

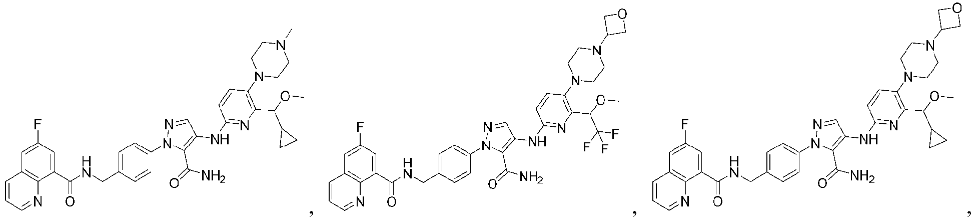

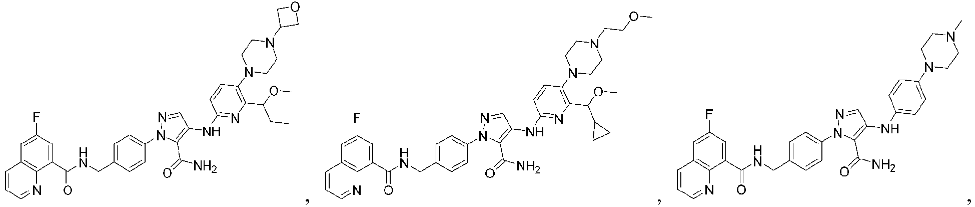

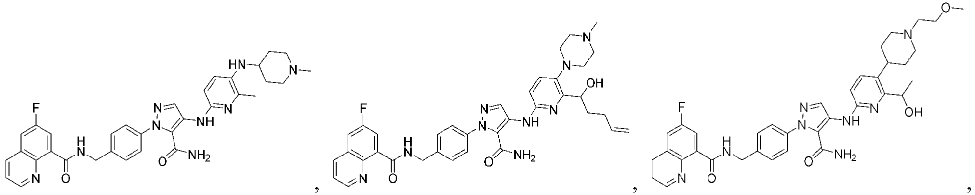

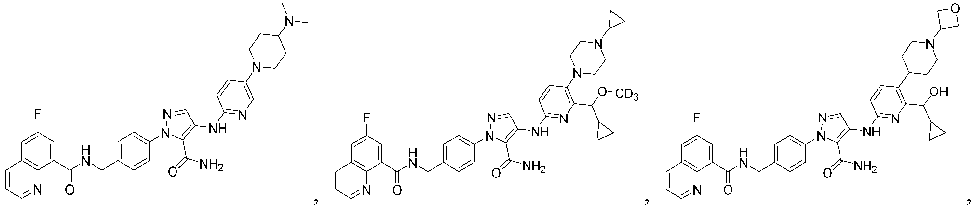

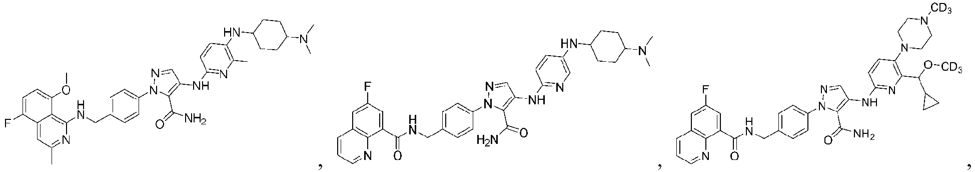

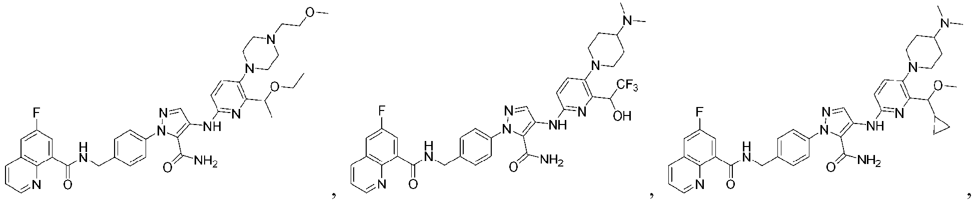

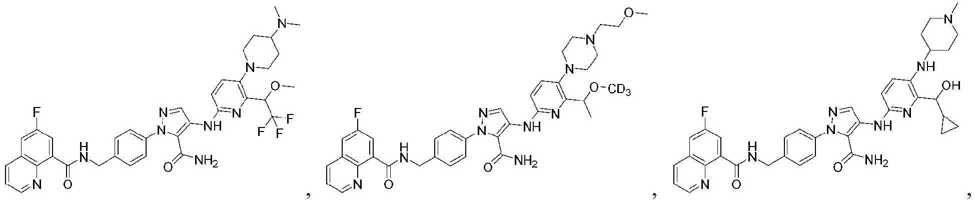

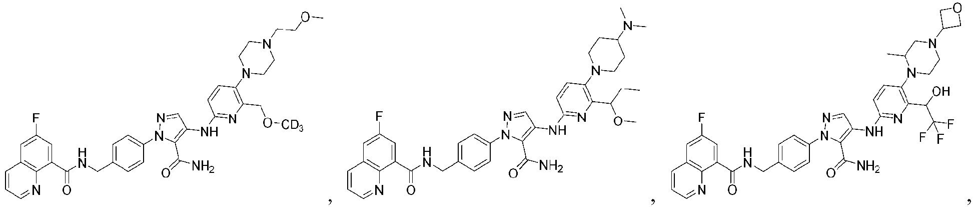

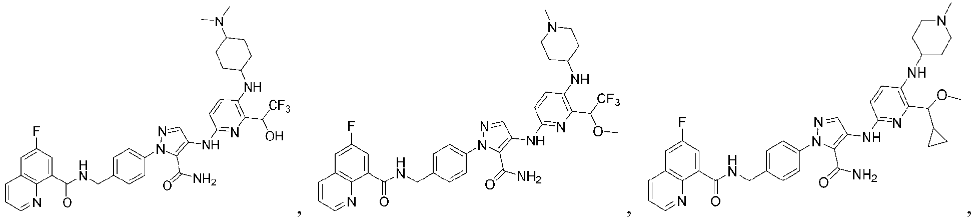

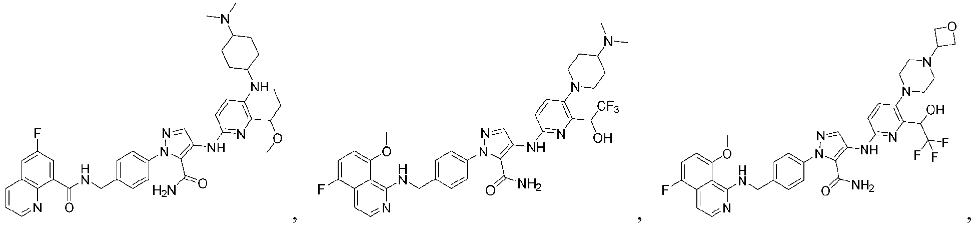

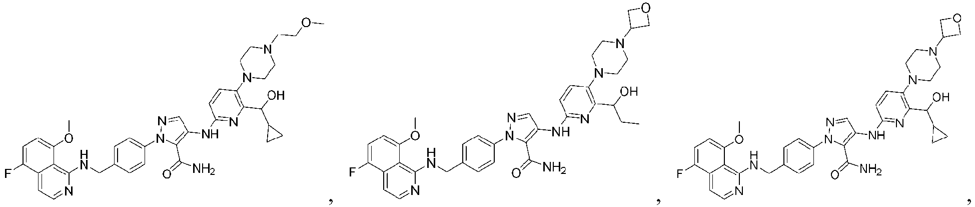

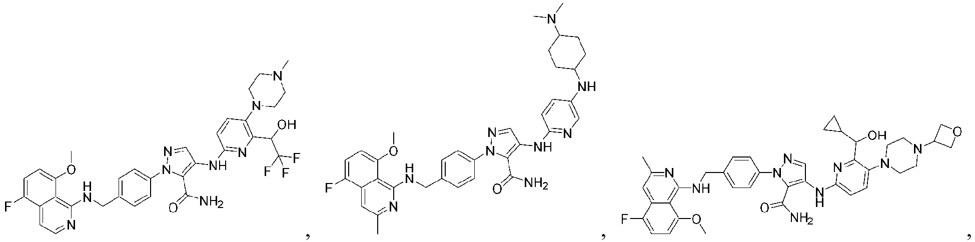

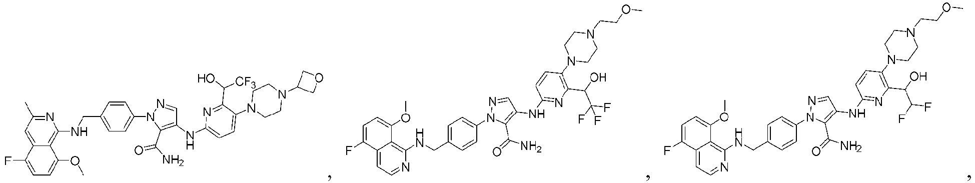

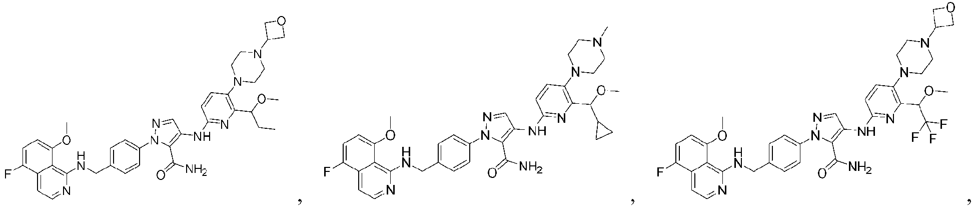

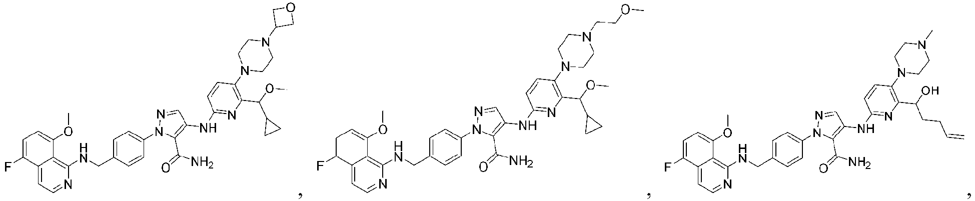

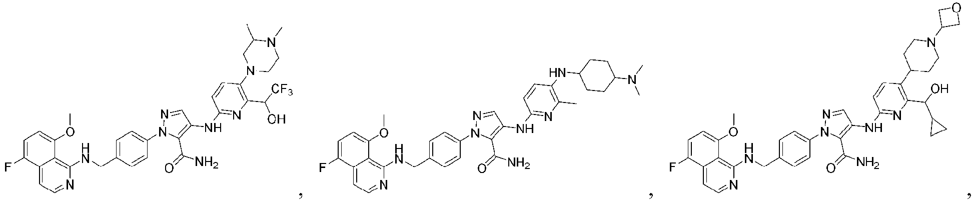

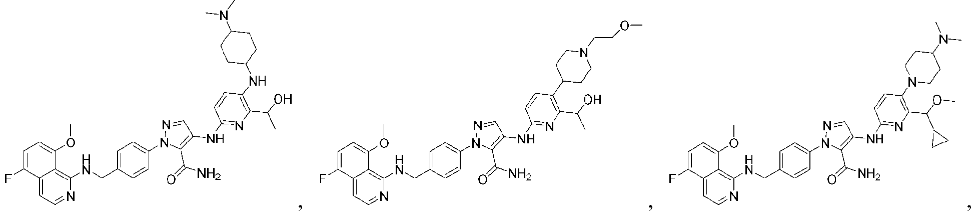

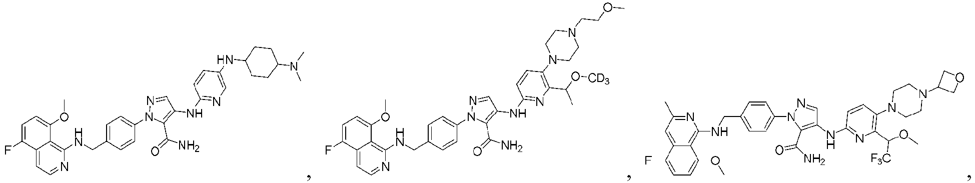

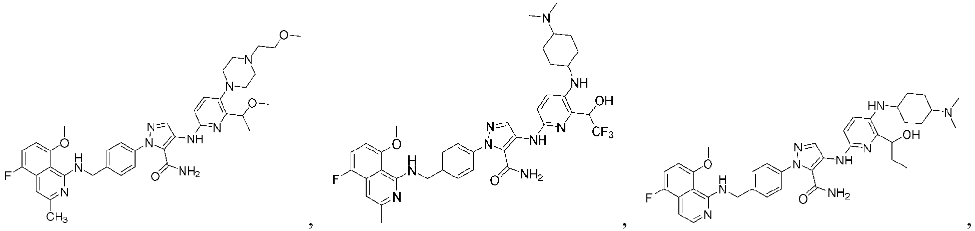

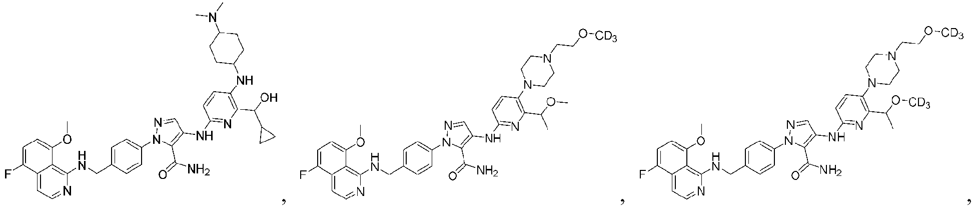

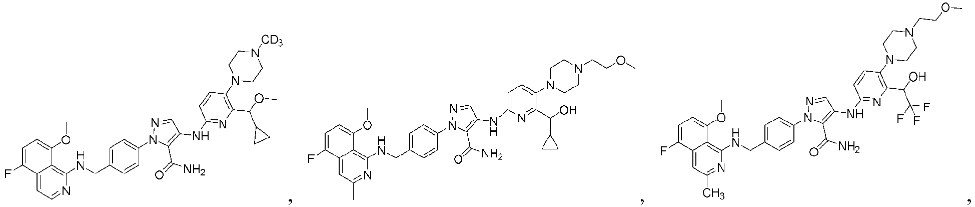

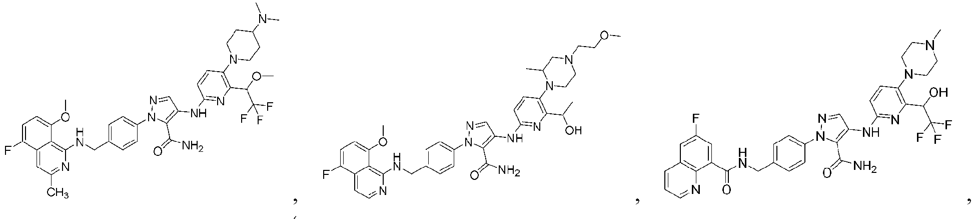

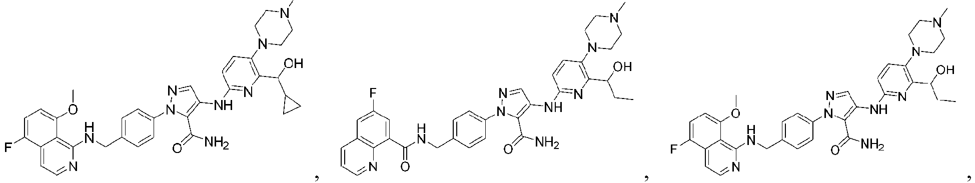

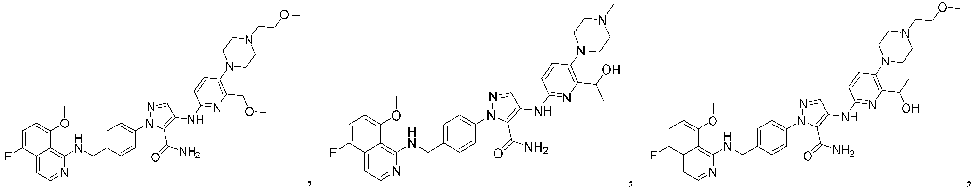

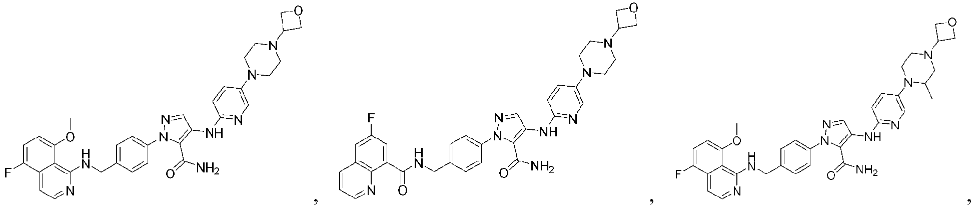

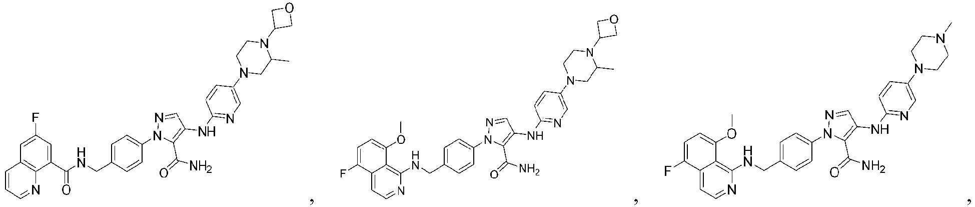

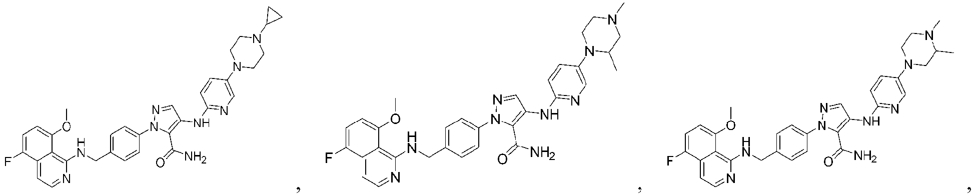

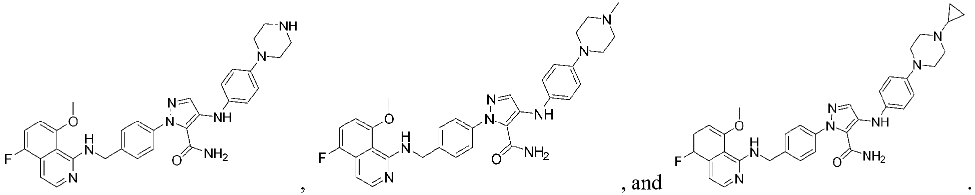

The present application also relates to a compound of a formula selected from the group consisting of the following formulas, a stereoisomer thereof, or a pharmaceutically acceptable salt thereof:

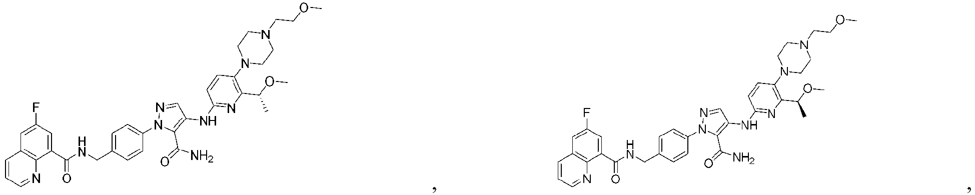

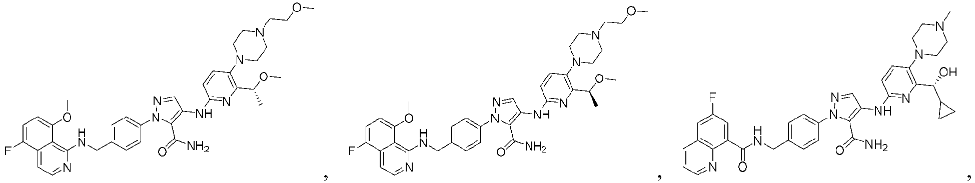

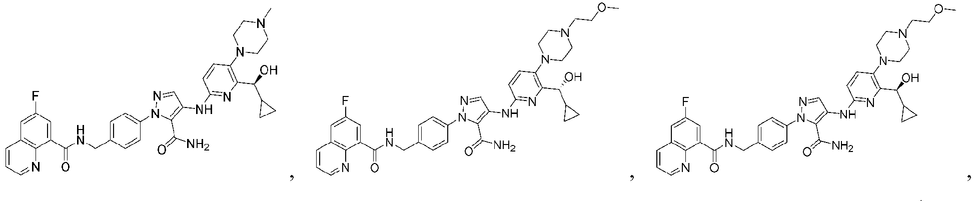

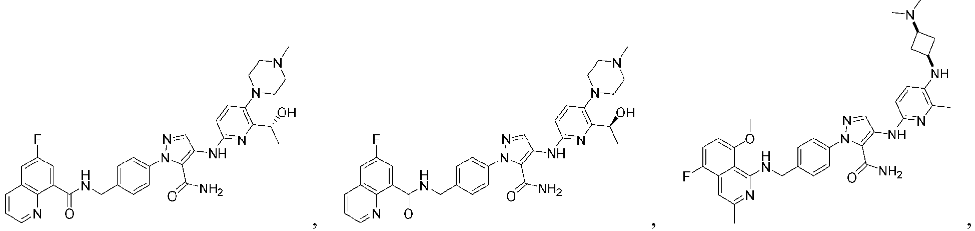

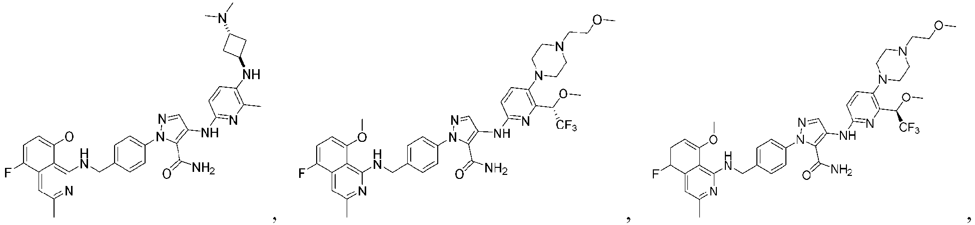

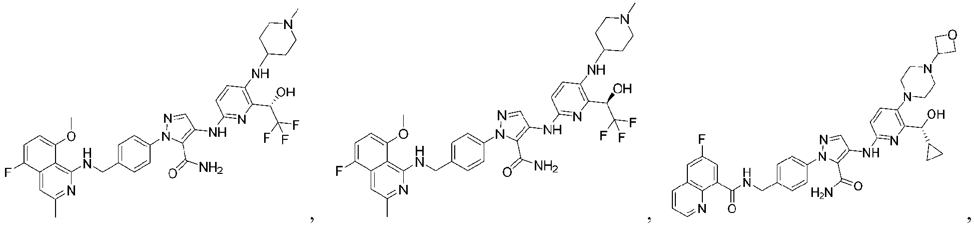

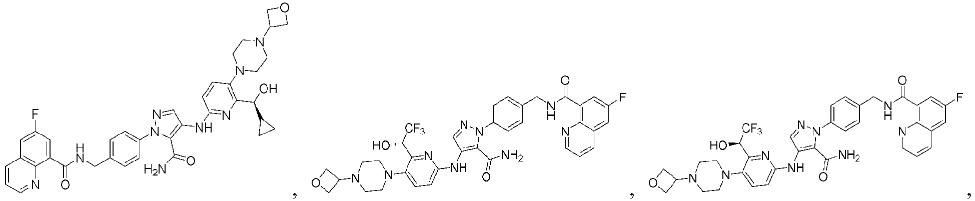

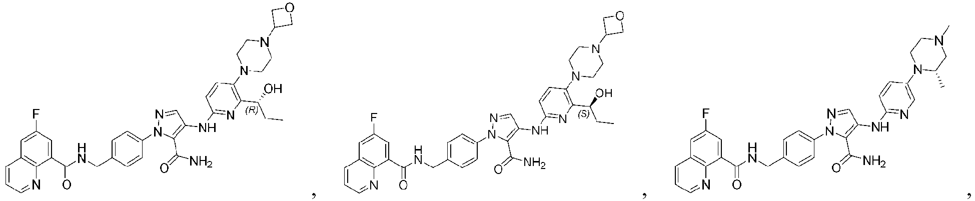

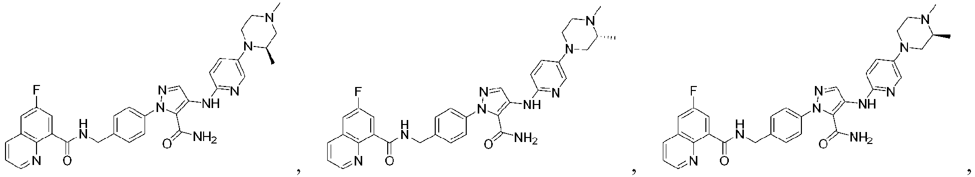

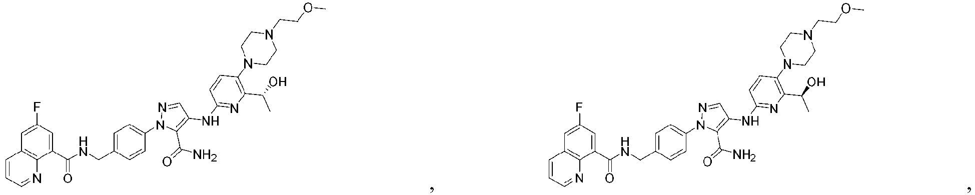

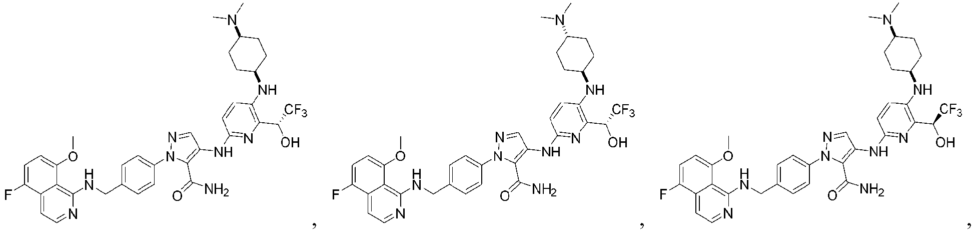

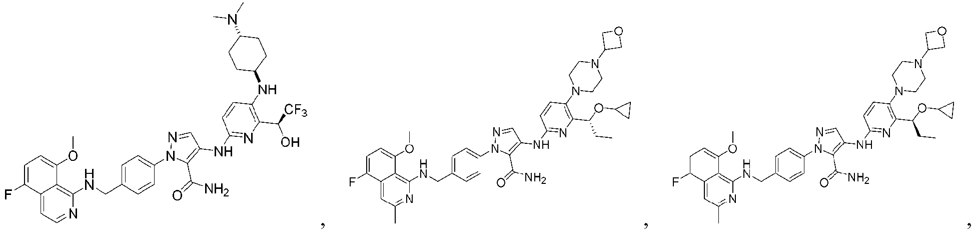

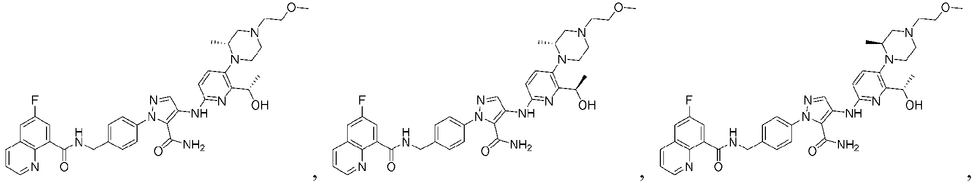

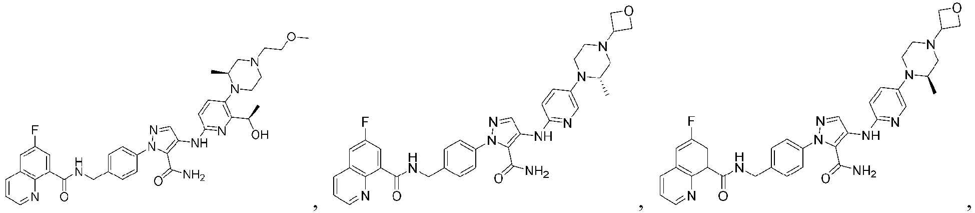

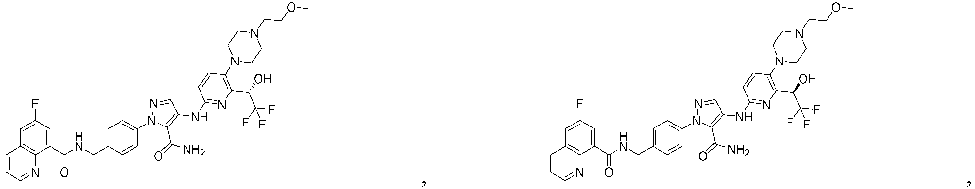

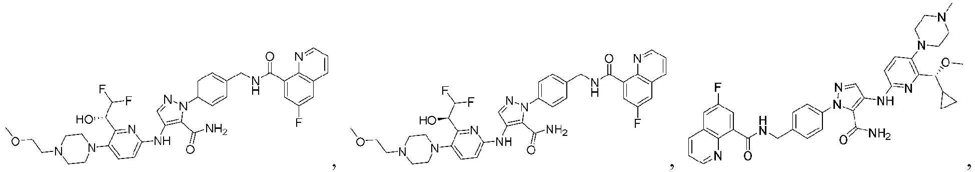

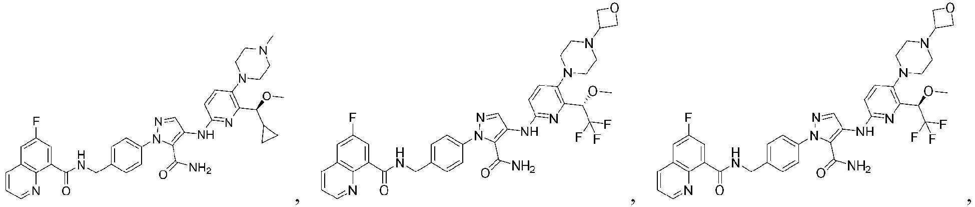

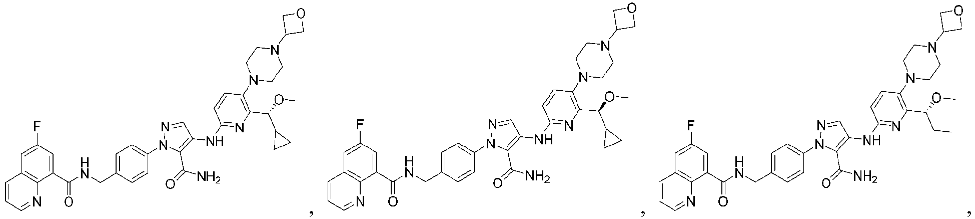

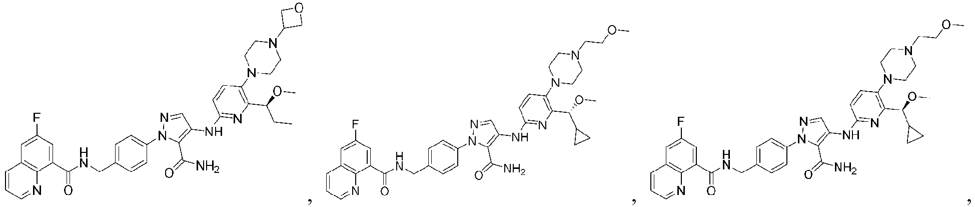

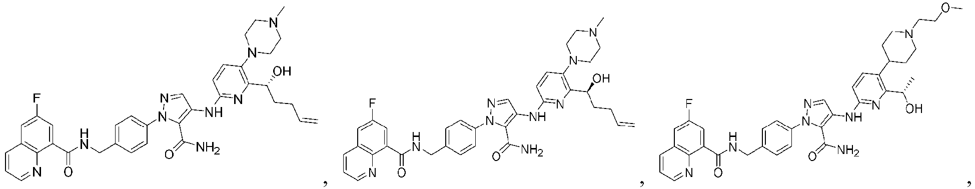

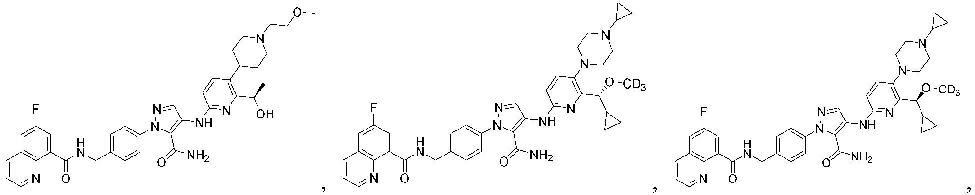

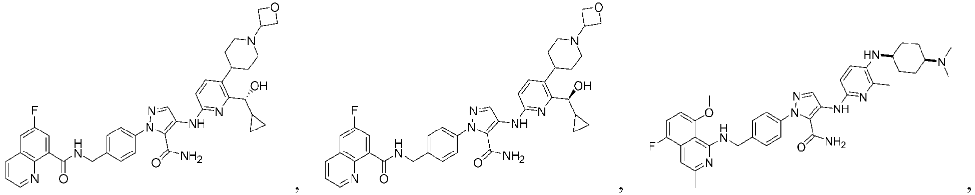

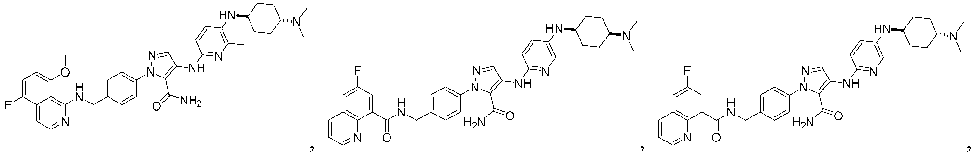

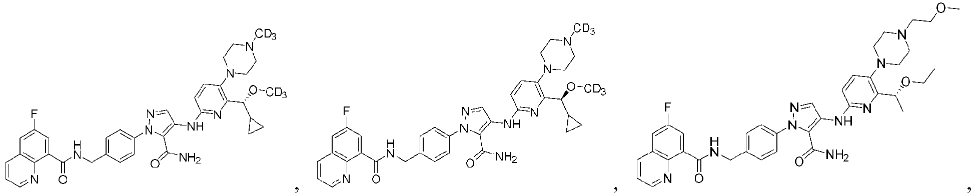

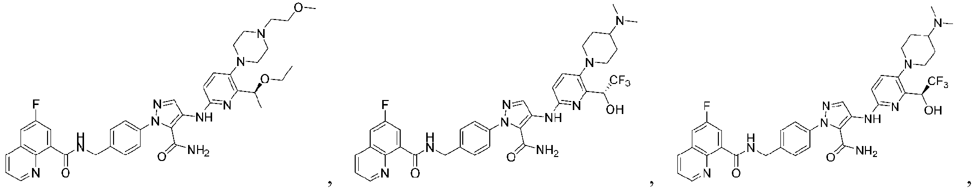

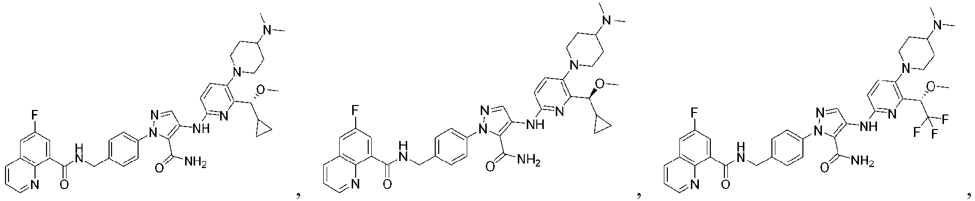

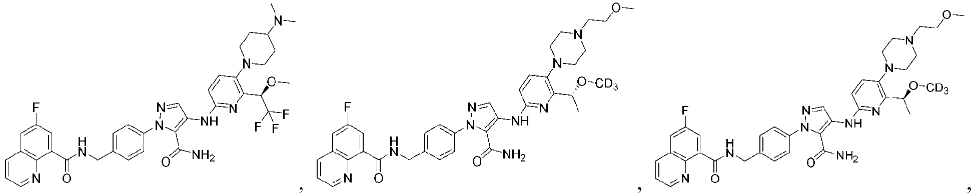

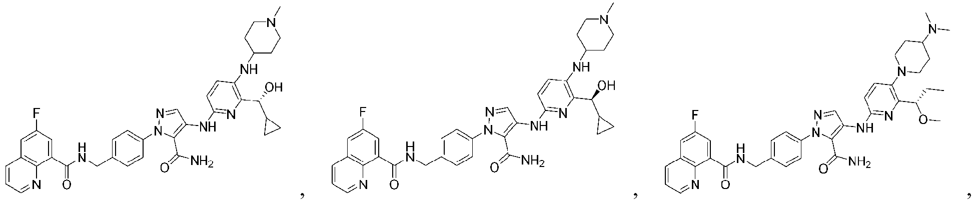

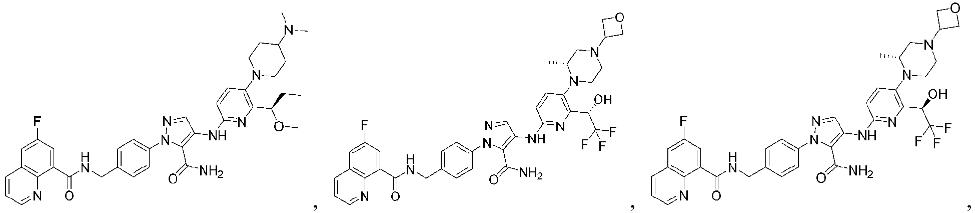

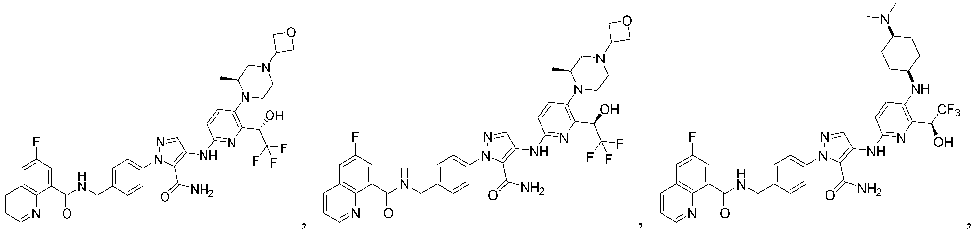

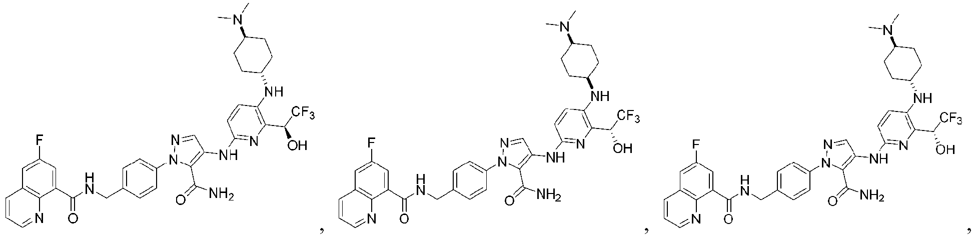

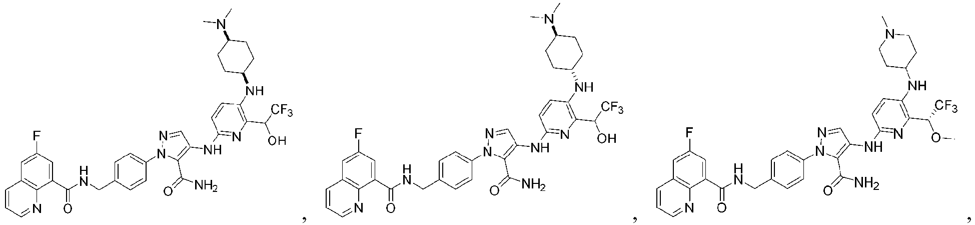

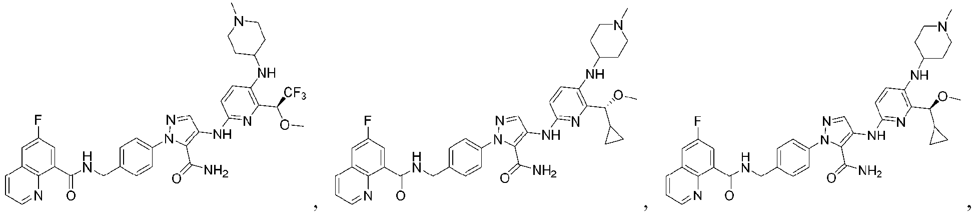

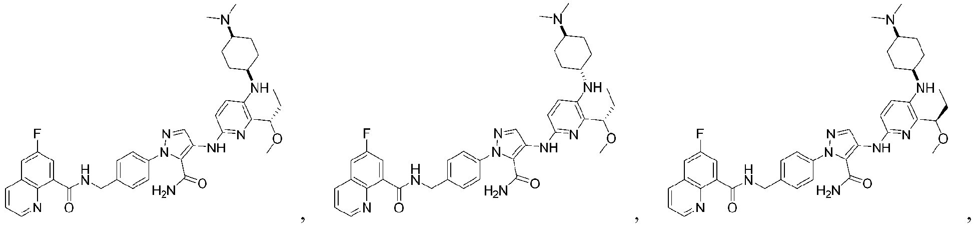

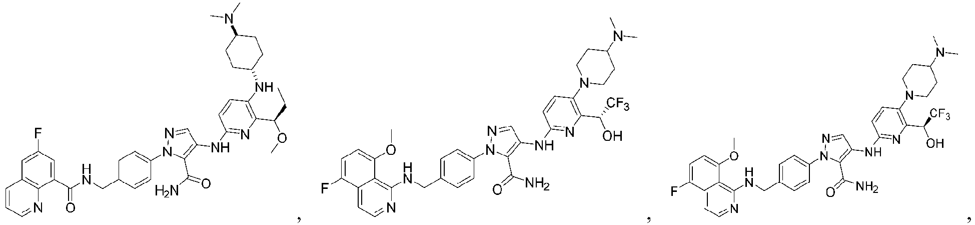

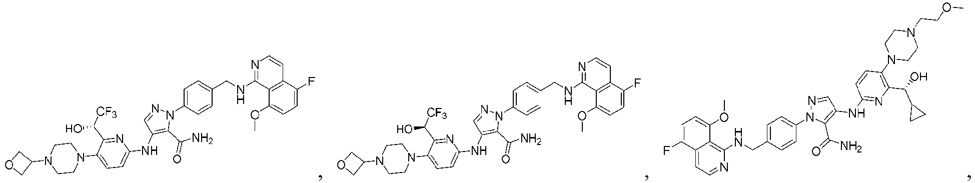

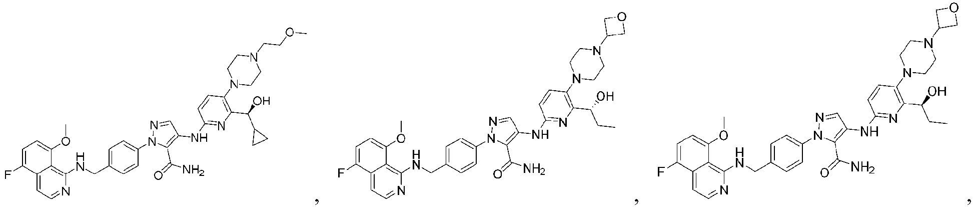

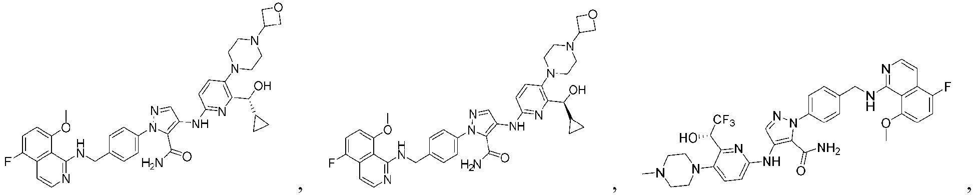

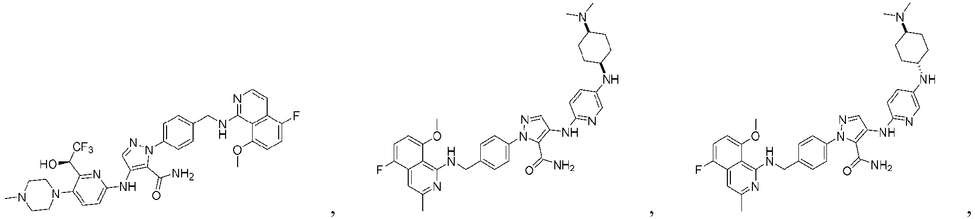

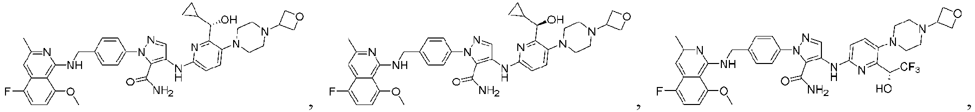

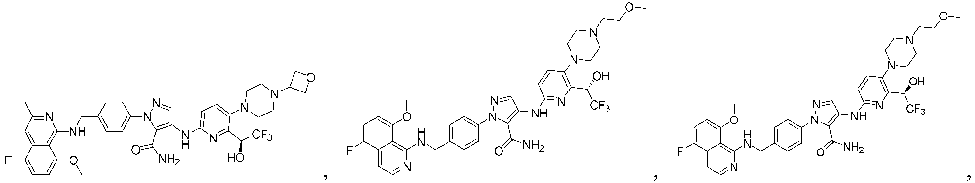

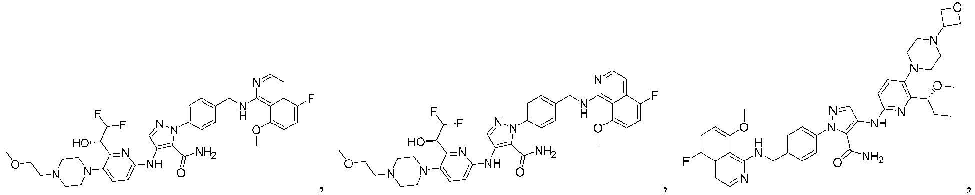

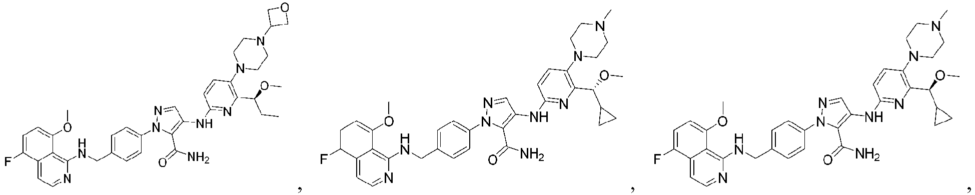

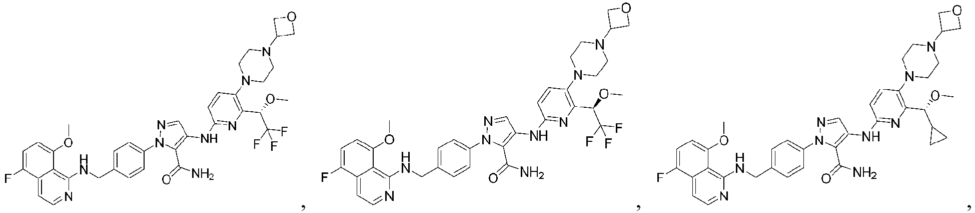

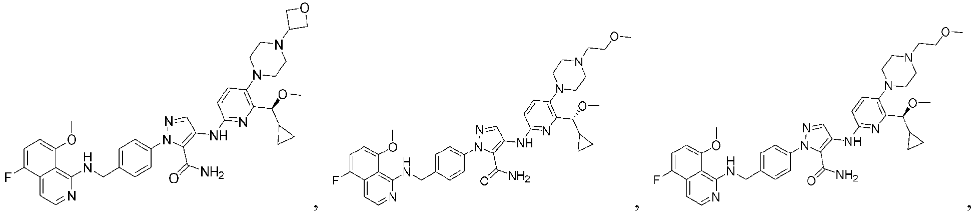

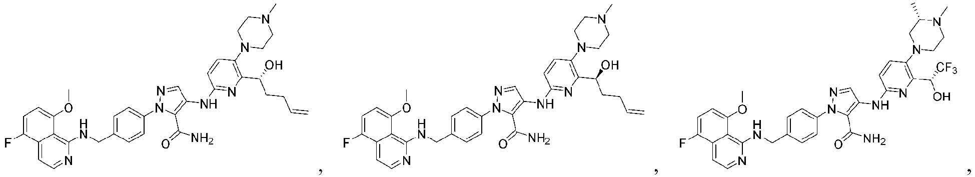

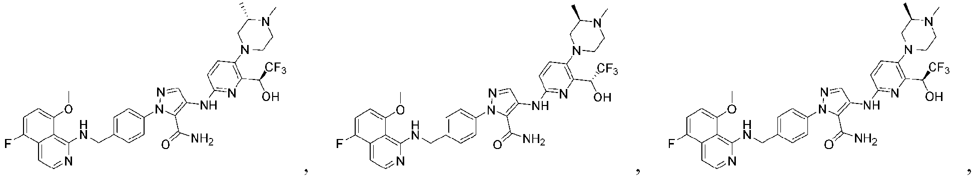

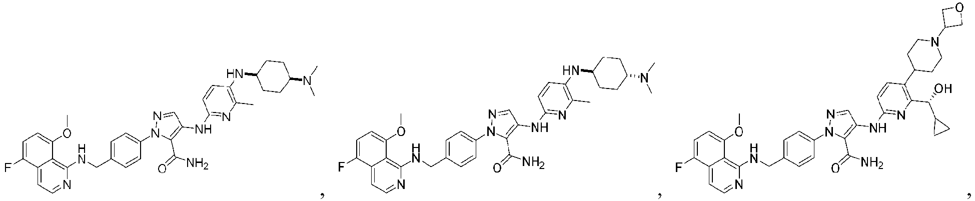

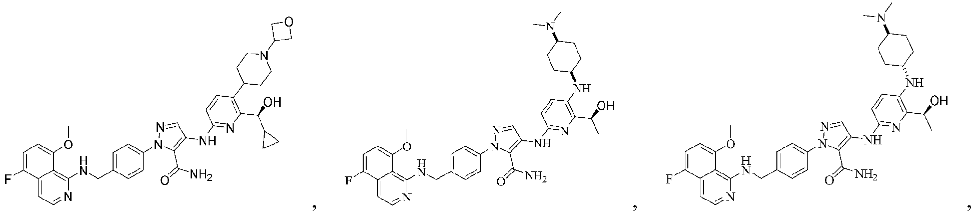

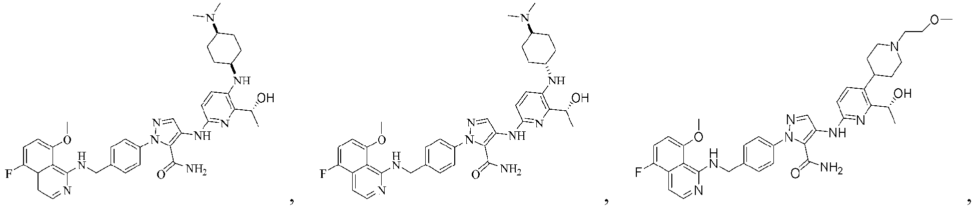

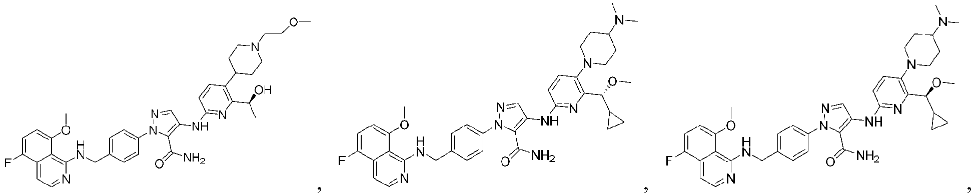

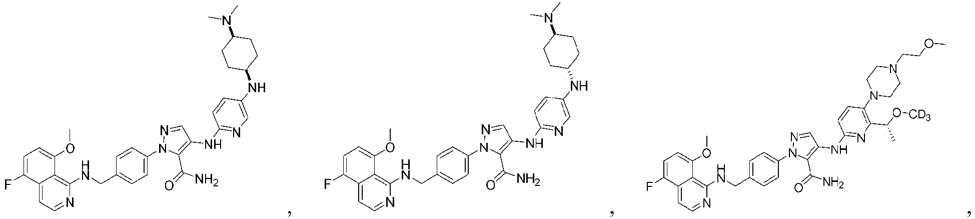

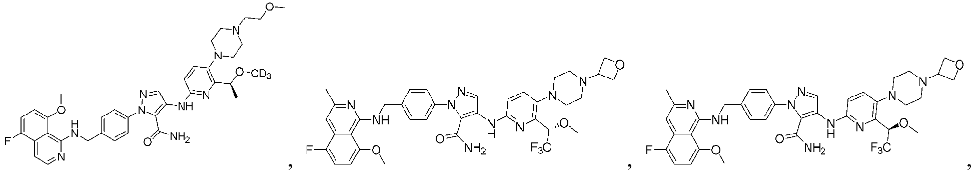

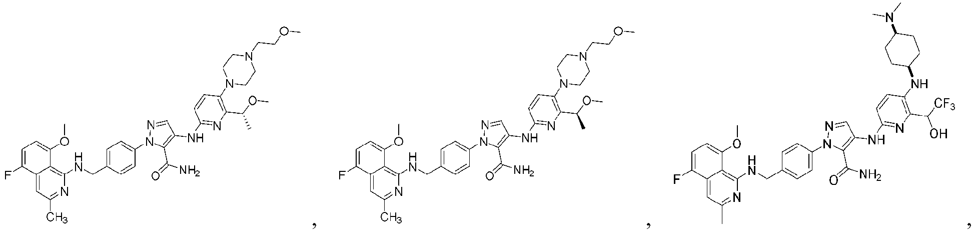

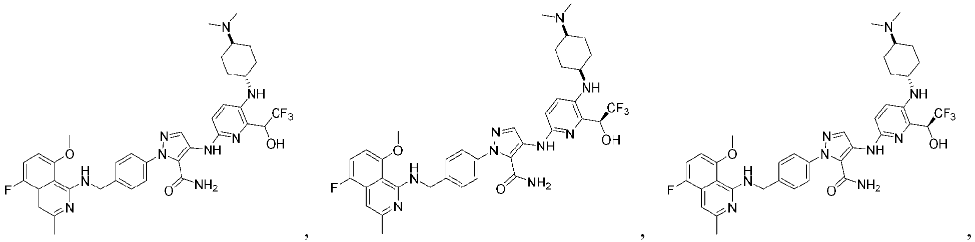

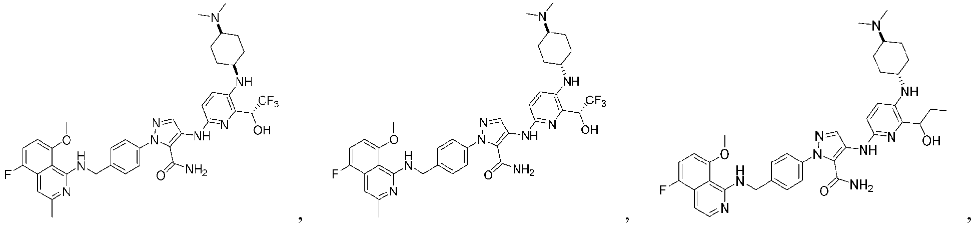

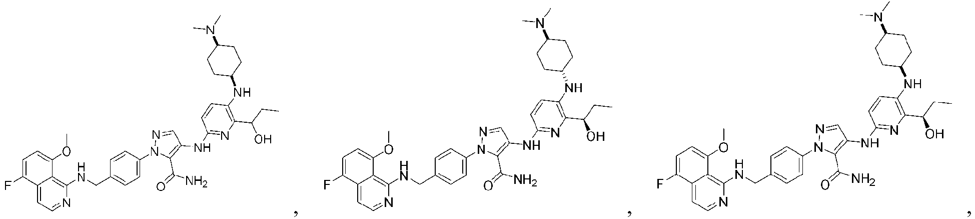

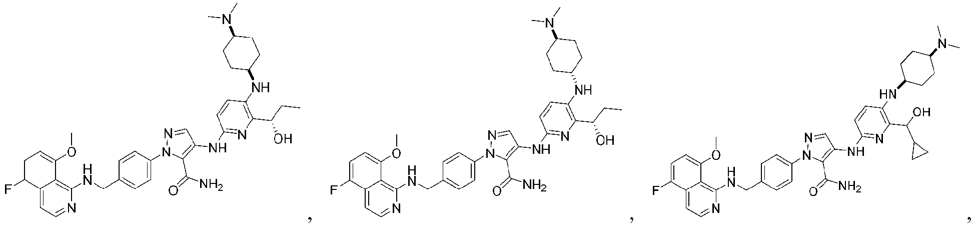

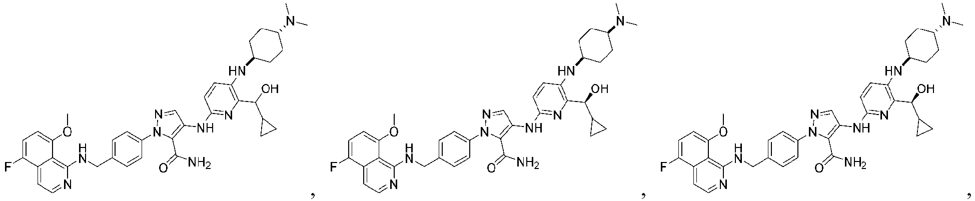

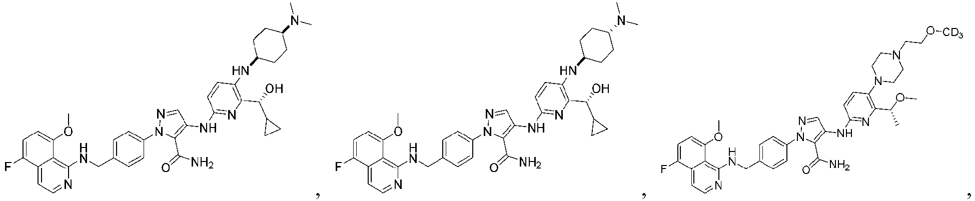

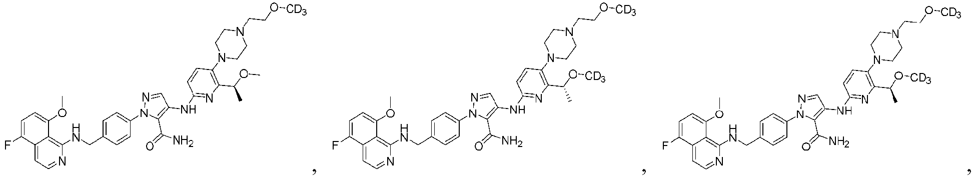

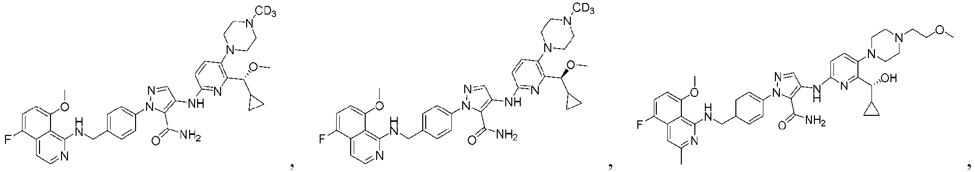

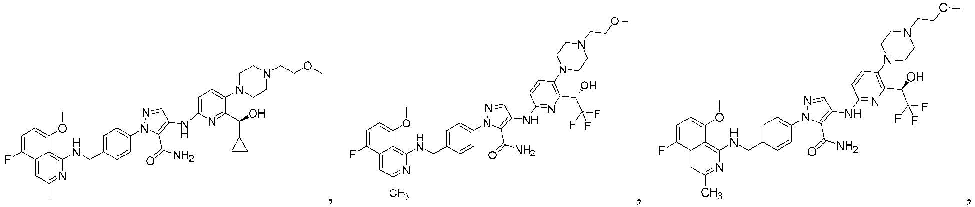

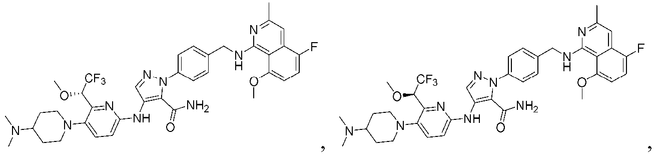

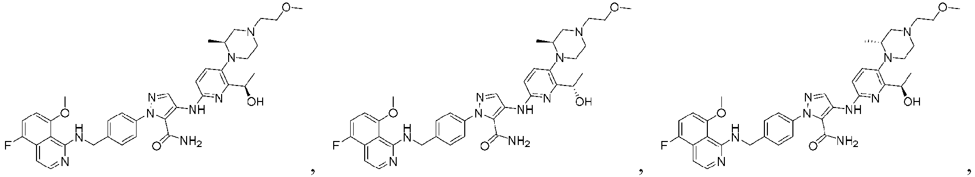

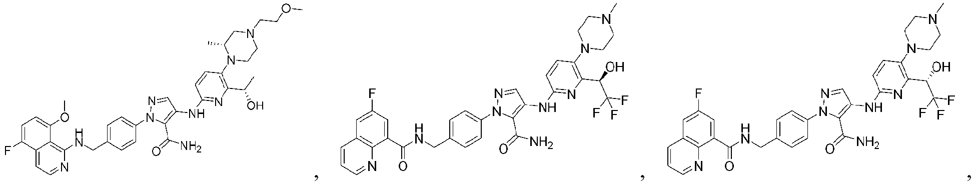

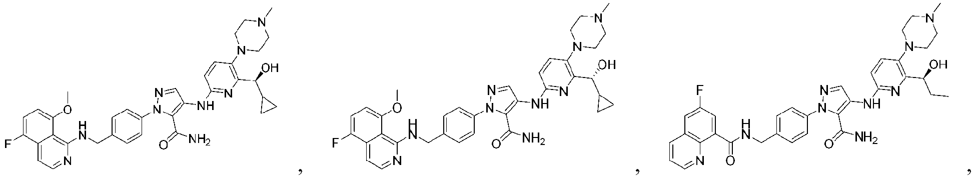

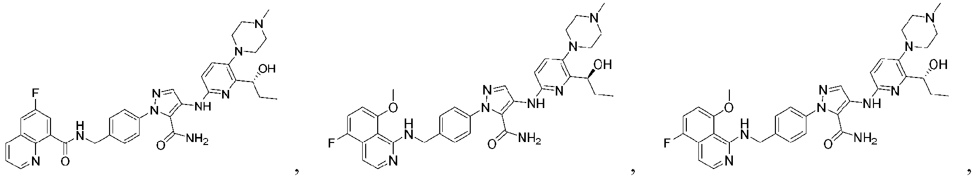

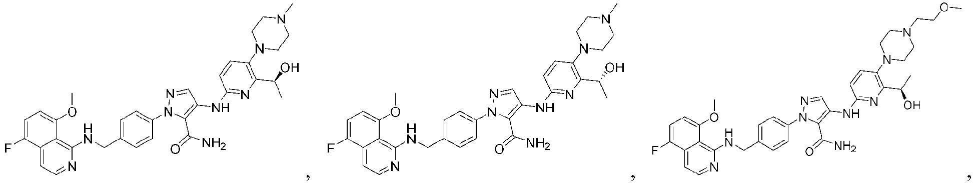

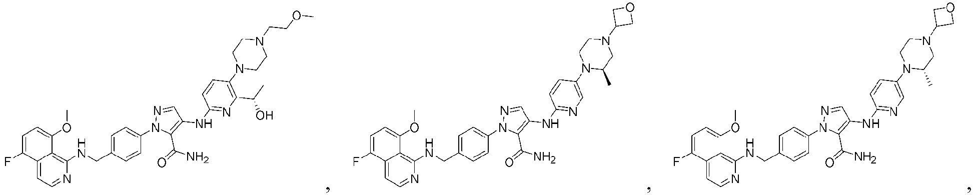

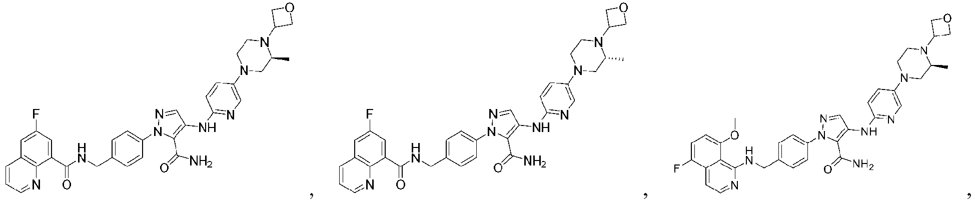

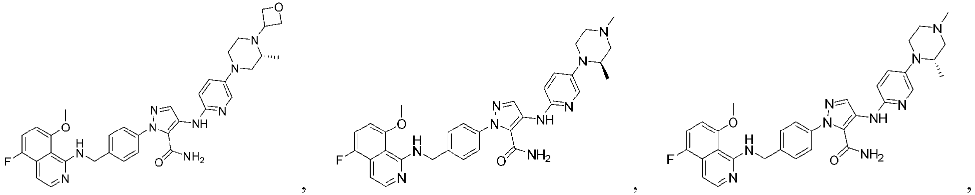

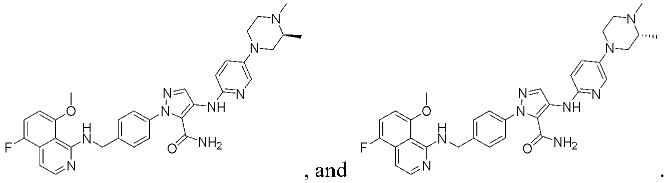

In another aspect, the present application relates to a pharmaceutical composition comprising the compound, the stereoisomer thereof, or the pharmaceutically acceptable salt thereof disclosed herein. In some embodiments, the pharmaceutical composition of the present application further comprises a pharmaceutically acceptable excipient. In another aspect, the present application relates to a method for treating a BTK-related disease in a subject (e.g., a mammal), comprising administering to a subject (e.g., a mammal, preferably a human) in need of such treatment a therapeutically effective amount of the compound, the stereoisomer thereof, or the pharmaceutically acceptable salt thereof, or the pharmaceutical composition thereof disclosed herein.

In another aspect, the present application relates to use of the compound, the stereoisomer thereof, or the pharmaceutically acceptable salt thereof, or the pharmaceutical composition thereof disclosed herein for preparing a medicament for treating a BTK-related disease.

In another aspect, the present application relates to use of the compound, the stereoisomer thereof, or the pharmaceutically acceptable salt thereof, or the pharmaceutical composition thereof disclosed herein for treating a BTK-related disease.

In another aspect, the present application relates to the compound, the stereoisomer thereof, or the pharmaceutically acceptable salt thereof, or the pharmaceutical composition thereof disclosed herein for use in treating a BTK-related disease.

In some embodiments, the BTK-related disease is selected from the group consisting of cancer and an immune disease. In some embodiments, the cancer is selected from lymphoma, e.g., diffuse large B-cell lymphoma. The compounds of the present application, including the compound of formula (I) (such as the example compounds), exhibit good inhibitory activity against wild-type BTK kinase, C481S mutant BTK kinase, and tumor cells, and exhibit good *in vitro* liver microsomal stability, *in vivo* pharmacokinetic properties, and/or good *in vivo* anti-tumor activity.

### Definitions

Unless otherwise stated, the following terms used in the present application shall have the following meanings. A certain term, unless otherwise specifically defined, should not be considered uncertain or unclear, but interpreted according to its common meaning in the art. When referring to a trade name, it is intended to refer to its corresponding commercial product or its active ingredient.

The term "substituted" means that any one or more hydrogen atoms on a specific atom are substituted with a substituent, as long as the valence of the specific atom is normal and the resulting compound is stable. When the substituent is oxo (i.e., =O), it means that two hydrogen atoms are substituted; oxo substitutions do not occur on aromatic groups.

The term "optional" or "optionally" means that the subsequently described event or circumstance may or may not occur, and the description includes instances where the event or circumstance occurs and instances where it does not. For example, ethyl "optionally" substituted with halogen means that the ethyl may be unsubstituted (CH₂CH₃), monosubstituted (e.g., CH₂CH₂F), polysubstituted (e.g., CHFCH₂F, CH₂CHF₂, etc.), or fully substituted (CF₂CF₃). It will be appreciated by those skilled in the art that for any group comprising one or more substituents, no substitution or substitution pattern that is sterically impossible and/or cannot be synthesized is introduced.

Cₘ₋ₙ used herein means that the moiety has an integer number of carbon atoms in the given range. For example, "C₁₋₆" means that the group may have 1 carbon atom, 2 carbon atoms, 3 carbon atoms, 4 carbon atoms, 5 carbon atoms, or 6 carbon atoms.

When any variable (e.g., R) occurs more than once in the constitution or structure of a compound, the definition of the variable in each case is independent. Therefore, for example, if a group is substituted with 2 R, the definition of each R is independent.

When a variable is selected from a covalent bond, it means that the two groups are directly connected. For example, in A-L-Z, when L represents a covalent bond, it means that the structure is actually A-Z. When L in the group A-L-Z is not a covalent bond, the direction of connection between A and Z is not limited. For example, when -L- is - C(O)-NH-methylene-, A-L-Z includes A-C(O)-NH-methylene-Z and Z-C(O)-NH-methylene-A.

When a bond of a substituent is crosslinked to two atoms on a ring, the substituent may be bonded to any atoms on the ring. For example, a structural unit 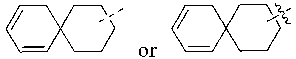 represents that substitution may occur in any one position of cyclohexyl or cyclohexadienyl. For another example, a structural unit 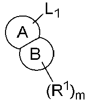 partially represents that R¹ may be substituted at any one position of ring A or ring B, and L1 may also be linked to any one position of ring A or ring B. For another example, a structural unit 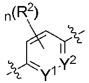 represents that R¹ may be substituted at any one position (e.g., Y¹ or Y²) on this ring.

The term "halo" or "halogen" refers to fluorine, chlorine, bromine, and iodine.

The term "hydroxy" refers to an -OH group.

The term "cyano" refers to a -CN group.

The term "amino" refers to an -NH₂ group.

The term "alkyl" refers to hydrocarbyl with a general formula of CₙH₂ₙ₊₁. The alkyl may be linear or branched. For example, the term "C₁₋₆ alkyl" refers to alkyl containing 1 to 6 carbon atoms (e.g., methyl, ethyl, n-propyl, isopropyl, n-butyl, isobutyl, sec-butyl, tert-butyl, n-pentyl, 1-methylbutyl, 2-methylbutyl, 3-methylbutyl, neopentyl, hexyl, 2-methylpentyl, etc.). Similarly, the alkyl moieties (i.e., alkyl) of alkoxy, alkylamino, dialkylamino, alkylsulfonyl, and alkylthio have the same definition as described above.

Unless otherwise specified, the term "C₁₋₆ alkylene", by itself or as part of another substituent, refers to a linear or branched divalent hydrocarbon group consisting of 1 to 6 carbon atoms, including those having 1 to 6, 1 to 4, 1 to 3, or 1 to 2 carbon atoms. For example, the term "C₁₋₆ alkylene" refers to alkylene containing 1 to 6 carbon atoms. Non-limiting examples of alkylene include, but are not limited to, methylene (-CH₂-), ethylene (-CH₂CH₂-), propylene (-CH₂CH₂CH₂- or -CH₂CH(CH₃)-), butylene (-CH₂CH₂CH₂CH₂-, -CH₂CH(CH₃)CH₂-, or - CH₂CH₂CH(CH₃)-), and the like.

The term "alkoxy" refers to -O-alkyl.

The term "alkylamino" refers to -NH-alkyl.

The term "dialkylamino" refers to -N(alkyl)₂.

The term "alkenyl" refers to linear or branched unsaturated aliphatic hydrocarbyl consisting of carbon atoms and hydrogen atoms and having at least one double bond, including, but not limited to, those having 2 to 8, 2 to 6, 2 to 4, or 2 to 3 carbon atoms. Non-limiting examples of alkenyl include, but are not limited to, ethenyl, 1-propenyl, 2-propenyl, 1-butenyl, isobutenyl, 1,3-butadienyl, and the like.

The term "alkynyl" refers to linear or branched unsaturated aliphatic hydrocarbyl consisting of carbon atoms and hydrogen atoms and having at least one triple bond, including, but not limited to, those having 2 to 8, 2 to 6, 2 to 4, or 2 to 3 carbon atoms. Non-limiting examples of alkynyl include, but are not limited to, ethynyl (-C≡CH), 1-propynyl (-C≡C-CH₃), 2-propynyl (-CH₂-C≡CH), 1,3-butadiynyl (-C≡C-C≡CH), and the like.

The term "cycloalkyl" refers to a carbocyclic ring that is fully saturated and may exist as a monocyclic ring, a bridged ring, or a spiro ring. Unless otherwise indicated, the carbocyclic ring is usually a 3- to 12-membered ring, a 3- to 10-membered ring, a 4- to 8-membered ring, a 5- to 8-membered ring, or a 5- to 6-membered ring. Non-limiting examples of cycloalkyl include, but are not limited to, cyclopropyl, cyclobutyl, cyclopentyl, cyclohexyl, norbornyl (bicyclo[2.2.1]heptyl), bicyclo[2.2.2]octyl, adamantyl, and the like.

The term "monocycloalkyl" refers to a cycloalkyl group that exists as a single ring.

The term "bridged cycloalkyl" refers to a fully saturated 5- to 14-membered, preferably 6- to 10-membered, carbocyclic ring in which two rings share two or more atoms. According to the number of the formed rings, the bridged cycloalkyl may be bicyclic, tricyclic, tetracyclic or polycyclic bridged cycloalkyl, preferably bicyclic or tricyclic bridged cycloalkyl, and more preferably bicyclic bridged cycloalkyl. Non-limiting examples of bridged cycloalkyl include: 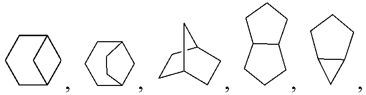 and the like.

The term "spirocycloalkyl" refers to a fully saturated 5- to 20-membered carbocyclic ring in which monocyclic rings share one carbon atom (referred to as the spiro atom). It is preferably 6- to 14-membered, and is more preferably 6- to 10-membered. According to the number of spiro atoms shared among the rings, the spiro heterocyclic ring may be a monospiro ring, a bispiro ring, or a polyspiro ring, preferably a monospiro ring or a bispiro ring. Non-limiting examples of the spiro ring include: 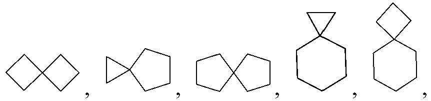 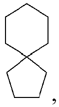 and the like.

The term "heterocycloalkyl" refers to a cyclic group that is fully saturated and may exist as a monocyclic ring, a bridged ring, or a spiro ring. Unless otherwise specified, the heterocyclyl is usually a 4- to 12-membered (e.g., 5- to 10-membered, or 6- to 10-membered) ring containing 1 to 3 heteroatoms (preferably 1 or 2 heteroatoms) independently selected from the group consisting of sulfur, oxygen, and/or nitrogen. Non-limiting examples of 4-membered heterocycloalkyl include, but are not limited to, azetidinyl, oxetanyl, and thietanyl; examples of 5-membered heterocycloalkyl include, but are not limited to, tetrahydrofuranyl, tetrahydrothienyl, pyrrolidinyl, isoxazolidinyl, oxazolidinyl, isothiazolidinyl, thiazolidinyl, imidazolidinyl, and tetrahydropyrazolyl; examples of 6-membered heterocycloalkyl include, but are not limited to, piperidinyl, tetrahydropyranyl, tetrahydrothiopyranyl, morpholinyl, piperazinyl, 1,4-oxathianyl, 1,4-dioxanyl, thiomorpholinyl, 1,3-dithianyl, and 1,4-dithianyl; examples of 7-membered heterocycloalkyl include, but are not limited to, azepanyl, oxepanyl, and thiepanyl. Monocyclic heterocycloalkyl having 5 or 6 ring atoms is preferred.

The term "spiro-heterocycloalkyl" refers to a fully saturated 5- to 20-membered polycyclic ring in which monocyclic rings share one carbon atom (referred to as the spiro atom), wherein one or more ring atoms in the polycyclic ring are selected from the group consisting of heteroatoms (preferably 1 or 2 heteroatoms) of sulfur, oxygen and/or nitrogen, and the remaining ring atoms are carbon atoms. It is preferably 6- to 14-membered, and is more preferably 6- to 10-membered. According to the number of spiro atoms shared among the rings, the spiro heterocyclic ring is a monospiro heterocyclic ring, a bispiro heterocyclic ring, or a polyspiro heterocyclic ring, preferably a monospiro heterocyclic ring or a bispiro heterocyclic ring, and more preferably a 4-membered/4-membered, 4-membered/5-membered, 4-membered/6-membered, 5-membered/5-membered, or 5-membered/6-membered monospiro heterocyclic ring. Non-limiting examples of spiro heterocyclic rings include: 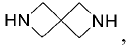 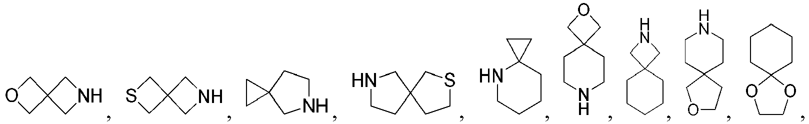 and the like.

The term "heterocyclyl" refers to a non-aromatic ring that is fully saturated or partially unsaturated (but not a fully unsaturated heteroaromatic group) and may exist as a monocyclic ring, a bridged ring, a fused ring, or a spiro ring. Unless otherwise indicated, the heterocyclic ring is usually a 3- to 12-membered, 3- to 10-membered, 8- to 10-membered, 9- to 10-membered, 4- to 8-membered, 5- to 8-membered, 5- to 6-membered, 3- to 7-membered, or 4-to 6-membered ring containing 1 to 3 heteroatoms (preferably 1 or 2 heteroatoms) independently selected from the group consisting of sulfur, oxygen, nitrogen, phosphorus, silicon, and/or boron. Non-limiting examples of heterocyclyl include, but are not limited to, oxiranyl, tetrahydrofuranyl, dihydrofuranyl, pyrrolidinyl, N-methylpyrrolidinyl, dihydropyrrolyl, piperidinyl, piperazinyl, pyrazolidinyl, 4H-pyranyl, morpholinyl, thiomorpholinyl, tetrahydrothienyl, and the like.

The term "aryl" refers to an all-carbon aromatic monocyclic or fused polycyclic group having a conjugated π-electron system. For example, the aryl may have 6-20 carbon atoms, 6-14 carbon atoms, 6-12 carbon atoms, or 6-10 carbon atoms. Non-limiting examples of aryl include, but are not limited to, phenyl, naphthyl, anthryl, 1,2,3,4-tetrahydronaphthalene, and the like.

The term "heteroaryl" refers to a monocyclic or fused polycyclic aromatic ring having at least one ring atom selected from the group consisting of N, O, and/or S, with the remaining ring atoms being C atoms. There are typically 5- to 14-membered, 5- to 12-membered, 5- to 10-membered, 5- to 8-membered, 5- to 7-membered, or 5- to 6-membered rings. Unless otherwise specified, the heteroaryl may be monocyclic, bicyclic, or tricyclic. Unless otherwise specified, the heteroaryl may have a single 5- to 8-membered ring, especially 5- or 6-membered ring, or two or more fused rings containing 6 to 14, especially 6 to 10 ring atoms. Non-limiting examples of heteroaryl include, but are not limited to, pyrrolyl, furanyl, thienyl, imidazolyl, oxazolyl, pyrazolyl, pyridinyl, pyrimidinyl, pyrazinyl, quinolyl, isoquinolyl, indolyl, quinazolinyl, quinoxalinyl, tetrazolyl, triazolyl, triazinyl, and the like.

The "cycloalkyl", "heterocycloalkyl", "heterocyclyl", "aryl", and "heteroaryl" are each independently optionally substituted with one or more substituents selected from the group consisting of the following substituents: oxo, hydroxy, amino, nitro, halogen, cyano, alkyl, alkenyl, alkynyl, alkoxy, haloalkoxy, alkylamino, dialkylamino, haloalkylamino, halodialkylamino, carboxyl, -C(O)O-alkyl, -OC(O)-alkyl, -C(O)NH₂, -C(O)NH-alkyl, - C(O)N(alkyl)₂, -NHC(O)-alkyl, -C(O)-alkyl, -S(O)-alkyl, -S(O)₂-alkyl, -S(O)₂NH₂, -S(O)₂NH-alkyl, - S(O)₂N(alkyl)₂, cycloalkyl, cycloalkylalkylene, cycloalkyloxy, heterocyclyl, heterocyclylalkylene, heterocyclyloxy, heterocycloalkyl, heterocycloalkylalkylene, heterocycloalkyloxy, heteroaryl, heteroarylalkylene, heteroaryloxy, aryl, arylalkylene, and aryloxy.

The term "treat", "treating", or "treatment" refers to administering the compound or formulation described in the present application to ameliorate or eliminate a disease or one or more symptoms related to the disease, including:
(i) inhibiting a disease or disease state, i.e., arresting its progression; and
(ii) alleviating a disease or disease state, i.e., causing the regression of the disease or disease state.

The term "therapeutically effective amount" refers to an amount of the compound of the present application for (i) treating a specific disease, condition, or disorder; or (ii) alleviating, ameliorating, or eliminating one or more symptoms of a specific disease, condition, or disorder; or (iii) preventing or delaying onset of one or more symptoms of a specific disease, condition, or disorder described herein. The amount of the compound of the present application composing the "therapeutically effective amount" varies depending on the compound, the disease state and its severity, the mode of administration, and the age of the mammal to be treated, but may be determined routinely by those skilled in the art in accordance with their knowledge and the present disclosure.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The pharmaceutically acceptable salt, for example, may be a metal salt, an ammonium salt, a salt formed with an organic base, a salt formed with an inorganic acid, a salt formed with an organic acid, a salt formed with a basic or acidic amino acid, and the like.

The term "pharmaceutical composition" refers to a mixture consisting of one or more of the compounds or the salts thereof of the present application and a pharmaceutically acceptable excipient. The pharmaceutical composition is intended to facilitate the administration of the compound of the present application to an organism.

The term "pharmaceutically acceptable excipient" refers to those that do not have a significant irritating effect on an organism and do not impair the biological activity and properties of the active compound. Suitable excipients are well known to those skilled in the art, such as carbohydrates, wax, water-soluble and/or water-swellable polymers, hydrophilic or hydrophobic materials, gelatin, oil, solvents, and water.

The term "subject" includes mammals. Examples of mammals include, but are not limited to, any member of the class Mammalia: humans, non-human primates (e.g., chimpanzees and other apes and monkeys); livestock animals, such as cattle, horses, sheep, goats, and pigs; domestic animals, such as rabbits, dogs, and cats; laboratory animals, including rodents, such as rats, mice, guinea pigs, and the like. In one embodiment associated with the methods and compositions provided herein, the mammal is a human.

The word "comprise" and variations thereof such as "comprises" or "comprising" should be understood in an open, non-exclusive sense, i.e., "including but not limited to".

The compounds and intermediates of the present application may also exist in different tautomeric forms, and all such forms are included within the scope of the present application. The term "tautomer" or "tautomeric form" refers to structural isomers of different energies that may interconvert via a low energy barrier. For example, a proton tautomer (also referred to as a prototropic tautomer) includes interconversion via proton transfer, such as keto-enol isomerization and imine-enamine isomerization. A specific example of a proton tautomer is an imidazole moiety in which a proton may transfer between two ring nitrogens. A valence tautomer includes the interconversion via recombination of some bonding electrons.

The present application also includes isotopically labeled compounds of the present application, which are identical to those recited herein but have one or more atoms replaced by an atom having an atomic mass or mass number different from the atomic mass or mass number generally found in nature. Examples of isotopes that can be incorporated into the compounds of the present application include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, and ³⁶Cl.

Certain isotopically labeled compounds of the present application (e.g., those labeled with ³H and ¹⁴C) may be used to analyze compounds and/or substrate tissue distribution. Tritiated (i.e., ³H) and carbon-14 (i.e., ¹⁴C) isotopes are particularly preferred for their ease of preparation and detectability. Positron emitting isotopes, such as ¹⁵O, ¹³N, ¹¹C, and ¹⁸F, may be used in positron emission tomography (PET) studies to determine substrate occupancy. Isotopically labeled compounds of the present application can generally be prepared by following procedures analogous to those disclosed in the schemes and/or examples below while substituting a non-isotopically labeled reagent with an isotopically labeled reagent.

Furthermore, substitution with heavier isotopes such as deuterium (i.e., ²H) may provide certain therapeutic advantages (e.g., increased *in vivo* half-life or reduced dose) resulting from greater metabolic stability and hence may be preferred in some circumstances in which deuterium substitution may be partial or complete, wherein partial deuterium substitution refers to substitution of at least one hydrogen with at least one deuterium.

The compounds of the present application may be in the form of a geometric isomer or stereoisomer. All such compounds are contemplated herein, including cis- and trans-isomers, (-)- and (+)-enantiomers, (R)- and (S)-enantiomers, diastereoisomers, (D)-isomers, (L)-isomers, and racemic mixtures and other mixtures thereof, such as an enantiomerically or diastereoisomerically enriched mixture, all of which are encompassed within the scope of the present application. The compound of the present application containing asymmetrical carbon atoms may be separated in an optically pure form or in a racemic form. The optically pure form may be resolved from a racemic mixture or may be synthesized using a chiral raw material or a chiral reagent. The cis and trans isomers of the present application are distinguished by " 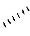 " and " 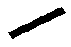 ". When two " 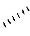 " appear simultaneously or two " 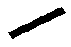 " appear simultaneously in a structure, it means cis; when one " 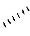 " and one " 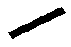 " appear in a structure, it means trans. Non-limiting examples of the cis- and trans-isomers include, but are not limited to, 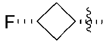 and 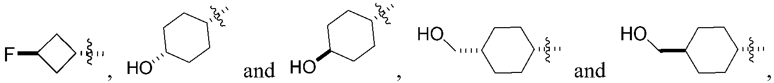 and the like. The (R)- and (S)-enantiomers of the present application are distinguished by " 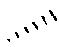 " and " 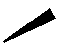 ", and non-limiting examples of enantiomers include, but are not limited to, 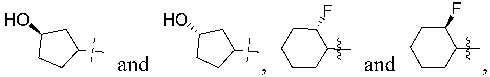 and the like.

The pharmaceutical composition of the present application may be prepared by combining the compound of the present application with a suitable pharmaceutically acceptable excipient, and may be formulated, for example, into solid, semisolid, liquid, or gaseous formulations such as tablets, pills, capsules, powders, granules, ointments, emulsions, suspensions, suppositories, injections, inhalants, gels, microspheres, and aerosols.

Typical routes of administration of the compound or the pharmaceutically acceptable salt thereof or the pharmaceutical composition thereof of the present application include, but are not limited to, oral, rectal, local, inhalation, parenteral, sublingual, intravaginal, intranasal, intraocular, intraperitoneal, intramuscular, subcutaneous, and intravenous administration.

The pharmaceutical composition of the present application may be manufactured using methods well known in the art, such as conventional mixing, dissolving, granulating, dragee-making, levigating, emulsifying, and lyophilizing. In all of the administration methods of the compound of general formula I described herein, the daily dose administered is from 0.01 mg/kg of body weight to 200 mg/kg of body weight, given in individual or separated doses.

The compounds of the present application can be prepared using a variety of synthetic methods well known to those skilled in the art, including the specific embodiments listed below, embodiments formed by combining them with other chemical synthetic methods, and equivalent substitutions well known to those skilled in the art. The preferred embodiments include, but are not limited to, the examples of the present application.

The chemical reactions in the specific embodiments of the present application are conducted in a proper solvent that has to be suitable for the chemical changes in the present application and the reagents and materials required. In order to obtain the compounds of the present application, it is sometimes necessary for those skilled in the art to modify or select a synthetic procedure or a reaction process based on the existing embodiments.

An important consideration in synthetic route planning in the art is the selection of suitable protective groups for reactive functional groups (e.g., amino in the present application); for example, reference may be made to Greene's Protective Groups in Organic Synthesis (4th Ed.) Hoboken, New Jersey: John Wiley & Sons, Inc.

In some embodiments, the compounds of the present application can be prepared by those skilled in the art of organic synthesis according to the following scheme: 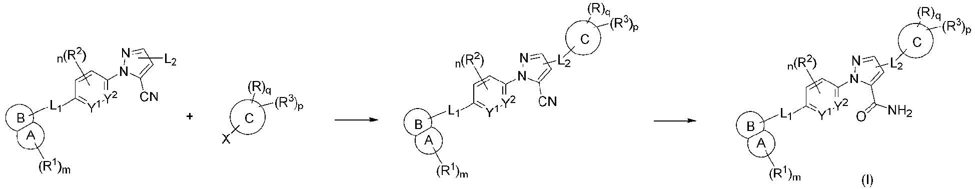 wherein ring A, ring B, R¹, m, L₁, Y₁, Y₂, R², n, L₂, ring C, R, q, R³, and p are as defined in the present disclosure, and X is selected from the group consisting of leaving groups including, but not limited to, F, Cl, Br, and I.

In some embodiments, the compounds of the present application can further be prepared by those skilled in the art of organic synthesis according to the following scheme: 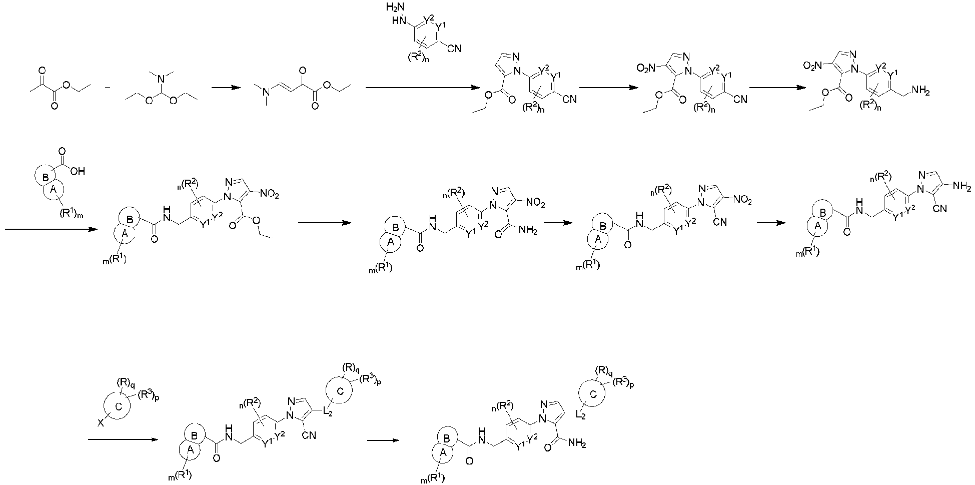 wherein ring A, ring B, R¹, m, L₁, Y₁, Y₂, R², n, L₂, ring C, R, q, R³, and p are as defined in the present disclosure, and X is selected from the group consisting of leaving groups including, but not limited to, F, Cl, Br, and I.

In some embodiments, the compounds of the present application can further be prepared by those skilled in the art of organic synthesis according to the following scheme: 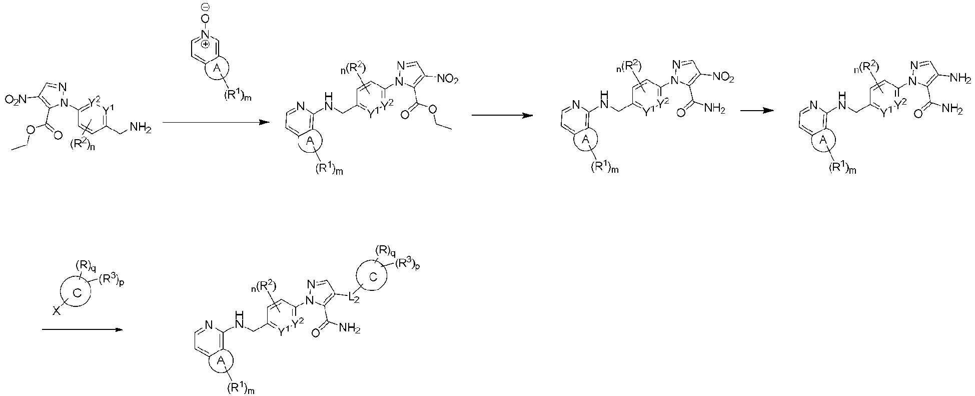 wherein ring A, R¹, m, Y₁, Y₂, R², n, L₂, ring C, R, q, R³, and p are as defined in the present disclosure, and X is selected from the group consisting of leaving groups including, but not limited to, F, Cl, Br, and I.

Each of the products from the reactions of the route described above may be obtained by conventional separation techniques including, but not limited to, filtration, distillation, crystallization, chromatography, and the like. The starting materials may be self-synthesized or purchased from commercial establishments (such as, but not limited to, Adrich or Sigma). These materials can be characterized using conventional means, such as physical constants and spectral data. The compounds described herein can be synthesized as a single isomer or a mixture of isomers. The following abbreviations are used in the present application:
DCM represents dichloromethane; THF represents tetrahydrofuran; DMSO represents dimethyl sulfoxide; EA represents ethyl acetate; CsF represents cesium fluoride; TMSCF₃ represents (trifluoromethyl)trimethylsilane; DPPF represents 1,1'-bis(diphenylphosphino)ferrocene.

### DETAILED DESCRIPTION

For clarity, the content of the present disclosure is further described with the following examples, which are, however, not intended to limit the scope of the present application. It will be apparent to those skilled in the art that various changes and modifications can be made to the specific embodiments of the present disclosure without departing from the spirit and scope of the present disclosure. All reagents used in the present application are commercially available and can be used without further purification.

### Example 1: Preparation of Compounds 1, 1-a, and 1-b

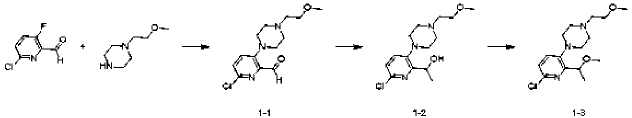

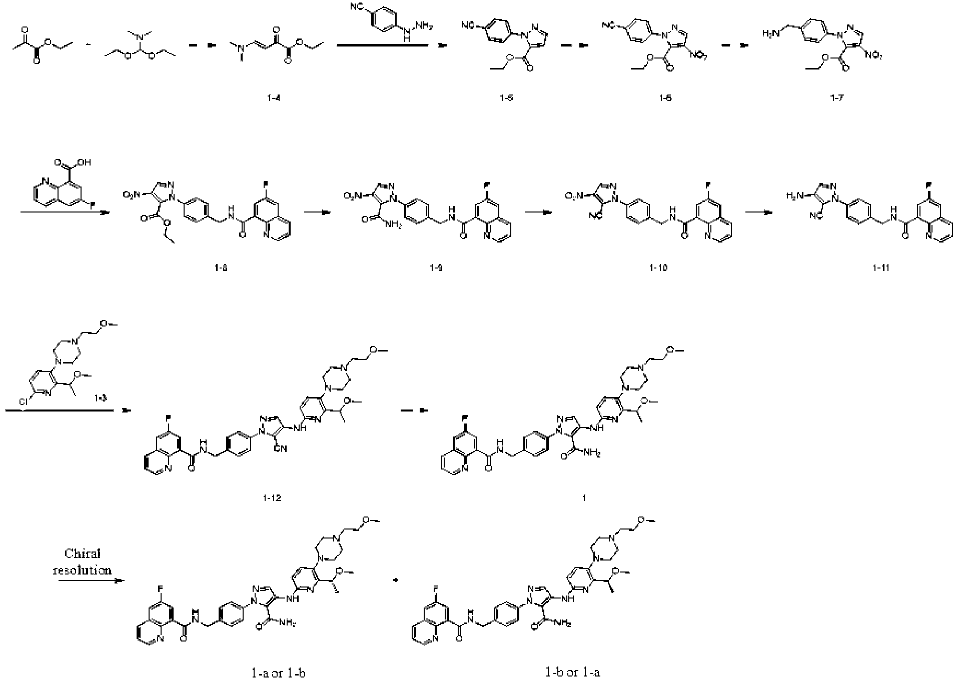

### Preparation of intermediate 1-1:

2-Chloro-5-fluoropyridine-6-carbaldehyde (10 g) and 1-(2-methoxyethyl)piperazine (9.9 g) were added to N,N-dimethylformamide (100 mL), and potassium carbonate (22 g) was added. The mixture was stirred with the temperature controlled at 80 °C for 2 h.

After the reaction was completed, purified water (300 mL) was added, and the reaction system was extracted with DCM (300 mL). The organic phase was dried over anhydrous sodium sulfate and filtered, and the filtrate was concentrated under reduced pressure to give intermediate **1-1** (19.2 g).

LC-MS: m/z 284.15 (M+H)⁺.

### Preparation of intermediate 1-2:

Intermediate **1-1** (10 g) was added to THF (50 mL). The mixture was stirred with the temperature controlled at - 20 °C, and a methylmagnesium bromide solution (3 M, 17.8 mL) was slowly added. After the dropwise addition was completed, the mixture was slowly heated to 0 °C and stirred for 2 h.

After the reaction was completed, purified water (150 mL) was added to quench the reaction, and the reaction solution was stirred for 10 min. DCM (200 mL) was added, and the reaction system was extracted and then left to stand for liquid separation. The organic phase was dried over anhydrous sodium sulfate and filtered under vacuum, and the filtrate was concentrated under reduced pressure to give intermediate **1-2** (9.7 g).

LC-MS: m/z 300.25 (M+H)⁺.

### Preparation of intermediate 1-3:

Intermediate **1-2** (9.7 g) was added to THF (100 mL). The mixture was stirred with the temperature controlled at 0 °C, and sodium hydride (60%, 1.4 g) was slowly added. After the mixture was stirred for 20 min, iodomethane (5.1 g) was added, and the mixture was transferred to room temperature and stirred for 12 h.

After the reaction was completed, purified water (150 mL) was added to quench the reaction, and the reaction solution was stirred for 10 min. Ethyl acetate (200 mL) was added, and the reaction system was extracted and then left to stand for liquid separation. The organic phase was dried over anhydrous sodium sulfate and filtered under vacuum, and the filtrate was concentrated under reduced pressure to give intermediate **1-3** (9 g).

LC-MS: m/z 314.31 (M+H)⁺.

### Preparation of intermediate 1-4:

Ethyl pyruvate (19.5 g) and N,N-dimethylformamide diethyl acetal (24.7 g) were added to DCM (500 mL), and the mixture was stirred at room temperature for 12 h.

After the reaction was completed, the reaction solution was concentrated under reduced pressure to give intermediate **1-4** (28.7 g).

### Preparation of intermediate 1-5:

Intermediate **1-4** (28.7 g) was dissolved in N,N-dimethylformamide (100 mL), and concentrated hydrochloric acid (12 M, 9.8 mL) and *p*-cyanophenylhydrazine (15.6 g) were added. The mixture was stirred at room temperature for 4 h.

After the reaction was completed, purified water (200 mL) was added to the reaction solution, and a solid precipitated. The resulting mixture was filtered, and the filter cake was intermediate **1-5** (18 g).

### Preparation of intermediate 1-6:

Intermediate **1-5** (18 g) was dissolved in trifluoroacetic anhydride (200 mL), and concentrated nitric acid (65%, 17.6 g) was added with the temperature controlled at -15 °C. After the addition was completed, the mixture was reacted for 30 min at the same temperature, transferred to an ice bath, and reacted for 2 h with the temperature controlled at 0 °C.

After the reaction was completed, purified water (500 mL) at 0 °C was added, and a solid precipitated. The resulting mixture was filtered, and the filter cake was washed with purified water and dried under reduced pressure at 50 °C for 12 h to give intermediate **1-6** (16 g).

LC-MS: m/z 303.21 (M+H)⁺.

### Preparation of intermediate 1-7:

Intermediate **1-6** (16 g) was added to borane-THF (1 M, 200 mL), and the mixture was stirred at room temperature for 12 h.

After the reaction was completed, ethanol (50 mL) was slowly added to quench the reaction, and the mixture was stirred for 1 h. The reaction solution was concentrated under reduced pressure, and adjusted to pH 2-3 with diluted hydrochloric acid (1 M, 50 mL). Purified water (200 mL) was added, and the reaction system was extracted with DCM (300 mL). The aqueous phase was adjusted to pH > 7 with a saturated aqueous NaHCO₃ solution (200 mL) and extracted with DCM/methanol (6:1, 500 mL). The organic phase was dried over anhydrous sodium sulfate with stirring and then filtered, and the filtrate was concentrated under reduced pressure to give intermediate **1-7** (6.9 g). LC-MS: m/z 291.28 (M+H)⁺.

### Preparation of intermediate 1-8:

Intermediate **1-7** (4.6 g), 6-fluoroquinoline-8-carboxylic acid (3 g), O-(7-azabenzotriazol-1-yl)-N,N,N',N'-tetramethyluronium hexafluorophosphate (8.9 g), and N,N-diisopropylethylamine (4.1 g) were added to N,N-dimethylformamide (50 mL), and the mixture was stirred at room temperature for 4 h.

After the reaction was completed, purified water (200 mL) was added, the reaction system was extracted with ethyl acetate (250 mL), and the aqueous phase was extracted with ethyl acetate (150 mL × 2). The organic phases were combined, dried over anhydrous sodium sulfate, and filtered, and the filtrate was concentrated under reduced pressure and purified by column chromatography (petroleum ether/ethyl acetate = 3/1). The eluate was concentrated under reduced pressure to give intermediate **1-8** (2 g).

LC-MS: m/z 464.46 (M+H)⁺.

### Preparation of intermediate 1-9:

Intermediate **1-8** (2 g) was added to ammonia-methanol (7 M, 100 mL), and the mixture was stirred with the temperature controlled at 60 °C. After the reaction was completed, the reaction solution was concentrated under reduced pressure to give intermediate **1-9** (2 g), which was directly used in the next step without further purification. LC-MS: m/z 435.11(M+H)⁺.

### Preparation of intermediate 1-10:

Intermediate **1-9** (2 g) was added to phosphorus oxychloride (3 mL), and the mixture was reacted overnight with the temperature controlled at 70 °C. After the reaction was completed, purified water (50 mL) was added to quench the reaction, and the mixture was stirred for 30 min. Ethyl acetate (50 mL) was added for extraction. The organic phase was dried over anhydrous sodium sulfate and filtered, and the filtrate was concentrated under reduced pressure to give intermediate **1-10** (1.5 g), which was directly used in the next step without further purification.

LC-MS: m/z 417.23 (M+H)⁺.

### Preparation of intermediate 1-11:

Intermediate **1-10** (1.5 g) was added to THF (25 mL), palladium on carbon (10%, 500 mg) was added, and the mixture was stirred overnight at room temperature under hydrogen atmosphere. After the reaction was completed, the reaction solution was filtered through celite, and the filtrate was concentrated under reduced pressure to give intermediate **1-11** (1 g), which was directly used in the next step without further purification.

LC-MS: m/z 387.12 (M+H)⁺.

### Preparation of intermediate 1-12:

Intermediate **1-11** (100 mg) and intermediate **1-3** (81 mg) were added to 1,4-dioxane (5 mL), tris(dibenzylideneacetone)dipalladium (47 mg), 4,5-bis-diphenylphosphino-9,9-dimethylxanthene (59 mg), and cesium carbonate (169 mg) were added, and the mixture was reacted at 85 °C for 12 h under nitrogen atmosphere. After the reaction was completed, ethyl acetate (100 mL) and purified water (100 mL) were added. The mixture was stirred for 10 min and then left to stand for liquid separation. The aqueous phase was extracted with ethyl acetate (50 mL × 3), and the organic phases were combined, washed with saturated brine, dried over anhydrous sodium sulfate, and filtered. The filtrate was concentrated under reduced pressure to give intermediate **1-12** (500 mg).

LC-MS: m/z 664.45 (M+H)⁺.

### Preparation of compound 1:

Intermediate **1-12** (500 mg) was added to ethanol (2 mL), a sodium hydroxide solution (1 M, 2 mL) was added, and the mixture was reacted for 2 h with the temperature controlled at 70 °C.

After the reaction was completed, ethyl acetate (100 mL) and purified water (100 mL) were added, and the mixture was stirred for 10 min and then left to stand for liquid separation. The aqueous phase was extracted with ethyl acetate (50 mL × 3). The organic phases were combined, washed with saturated brine, dried over anhydrous sodium sulfate, and filtered, and the filtrate was concentrated under reduced pressure to give a concentrate, which was separated by preparative liquid chromatography to give compound **1.**

LC-MS: m/z 682.23 (M+H)⁺.

### Preparation of compounds 1-a and 1-b:

Compound 1 was subjected to chiral resolution to give compound 1-a and compound 1-b. The resolution conditions were as follows: a CHIRALPAK AD-H preparative column (4.6 × 250 mm, 5.0 µm) was used; by taking n-hexane as mobile phase A and ethanol as mobile phase B, gradient elution was performed at a flow rate of 0.8 mL/min; the detection wavelength was 254 nm, and the retention times were 32.83 min (1-a) and 38.35 min (1-b), respectively. Compound 1-a LC-MS: m/z 682.33 (M+H)⁺.

Compound 1-b LC-MS: m/z 682.23 (M+H)⁺.

Compound 1-a had a shorter retention time in the chiral chromatography column than compound 1-b, and compound 1-b had a longer retention time in the chiral chromatography column than compound 1-a.

### Example 2: Preparation of Compound 2

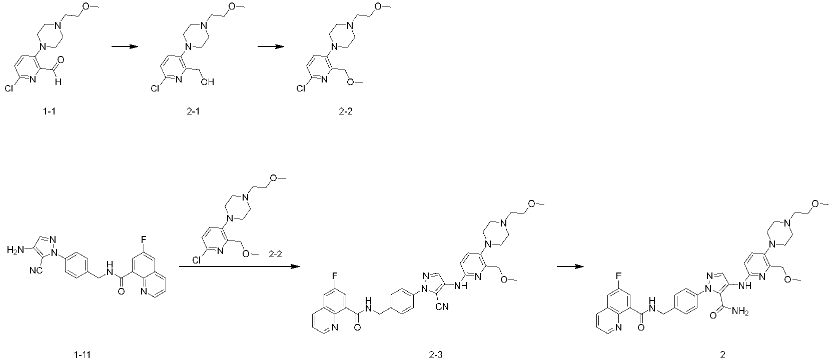

### Preparation of intermediate 2-1:

Intermediate **1-1** (9 g) was added to methanol (150 mL), and sodium borohydride (1.8 g) was slowly added. The mixture was stirred at room temperature for 12 h.

After the reaction was completed, purified water (500 mL) was added to quench the reaction, and the resulting mixture was stirred for 10 min. DCM (600 mL) was added, and the reaction system was extracted and then left to stand for liquid separation. The organic phase was dried over anhydrous sodium sulfate and filtered under vacuum, and the filtrate was concentrated under reduced pressure to give intermediate **2-1** (9 g), which was directly used in the next step without further purification.

LC-MS: m/z 286.13 (M+H)⁺.

### Preparation of intermediate 2-2:

Intermediate **2-1** (9 g) was added to THF (150 mL), and the mixture was stirred with the temperature controlled at 0 °C. Sodium hydride (60%, 1.4 g) was slowly added, and after the mixture was stirred for 20 min, iodomethane (4.9 g) was added. The mixture was transferred to room temperature and stirred for 4 h.

After the reaction was completed, purified water (150 mL) was added to quench the reaction, and the reaction solution was stirred for 10 min. Ethyl acetate (200 mL) was added, and the reaction system was extracted and then left to stand for liquid separation. The organic phase was dried over anhydrous sodium sulfate and filtered under vacuum, and the filtrate was concentrated under reduced pressure to give intermediate **2-2** (8.4 g).

LC-MS: m/z 300.32 (M+H)⁺.

### Preparation of intermediate 2-3:

Intermediate **1-11** (100 mg) and intermediate **2-2** (78 mg) were added to tert-butanol (5 mL), and palladium(II) acetate (12 mg), 4,5-bis-diphenylphosphino-9,9-dimethylxanthene (60 mg), and cesium carbonate (168 mg) were added. The mixture was reacted at 85 °C for 2 h under nitrogen atmosphere.

After the reaction was completed, ethyl acetate (100 mL) and purified water (100 mL) were added. The mixture was stirred for 10 min and then left to stand for liquid separation. The aqueous phase was extracted with ethyl acetate (50 mL × 3), and the organic phases were combined, washed with saturated brine, dried over anhydrous sodium sulfate, and filtered. The filtrate was concentrated under reduced pressure to give intermediate **2-3** (200 mg). LC-MS: m/z 650.42 (M+H)⁺.

### Preparation of compound 2:

Intermediate **2-3** (200 mg) was added to ethanol (2 mL), and a sodium hydroxide solution (1 M, 2 mL) was added. The mixture was reacted for 2 h with the temperature controlled at 70 °C.

After the reaction was completed, ethyl acetate (100 mL) and purified water (100 mL) were added, and the mixture was stirred for 10 min and left to stand for liquid separation. The aqueous phase was extracted with ethyl acetate (50 mL × 3). The organic phases were combined, washed with saturated brine, dried over anhydrous sodium sulfate, and filtered, and the filtrate was concentrated under reduced pressure to give a concentrate, which was separated by preparative liquid chromatography to give compound **2.**

LC-MS: m/z 668.32 (M+H)⁺.

¹H NMR (500 MHz, DMSO-*d*₆) δ 11.25 (s, 1H), 9.05 (dd, *J* = 4.2, 1.8 Hz, 1H), 8.57 (dd, *J* = 8.5, 1.8 Hz, 1H), 8.44-7.96 (m, 5H), 7.59-7.36 (m, 6H), 6.78 (d, *J =* 8.8 Hz, 1H), 4.76 (d, *J =* 6.0 Hz, 2H), 4.45 (s, 2H), 3.46 (t, *J* = 5.8 Hz, 7H), 2.81 (t, *J =* 4.8 Hz, 4H), 2.58 (s, 2H), 2.07 (s, 3H).

### Example 3: Preparation of Compounds 3, 3-a, and 3-b

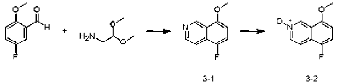

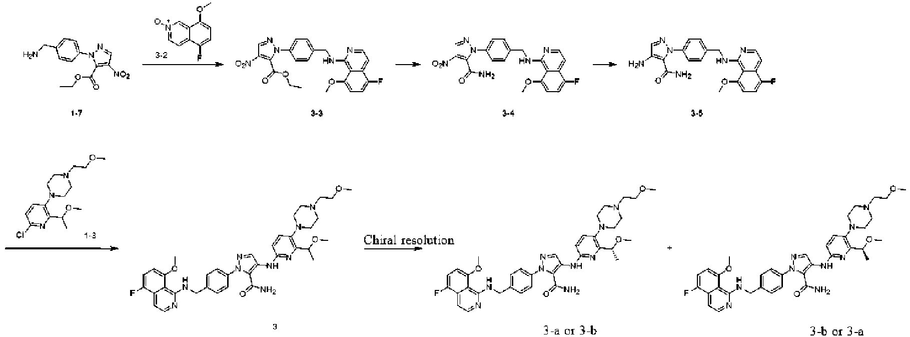

### Preparation of intermediate 3-1:

5-Fluoro-2-methoxybenzaldehyde (5 g), aminoacetaldehyde dimethyl acetal (3.41 g), and 4A molecular sieve (3 g) were added to toluene (50 mL), and the mixture was reacted at 120 °C for 7 h with a water separator.

After the reaction was completed, the reaction mixture was filtered, and the filtrate was concentrated under reduced pressure. The concentrate was dissolved in DCM (80 mL) with stirring, and aluminum trichloride (11 g) was added in portions. The mixture was stirred at room temperature for 6 h. After the reaction was completed, ice water was added, and the mixture was stirred, followed by liquid separation. The aqueous phase was extracted with DCM, and the organic phases were combined, washed twice with water, dried over anhydrous sodium sulfate, and filtered. The filtrate was concentrated under reduced pressure to give intermediate **3-1** (1.5 g).

LC-MS: m/z 178.10 (M+H)⁺.

### Preparation of intermediate 3-2:

Intermediate **3-1** (1.5 g) was added to DCM (20 mL), m-chloroperoxybenzoic acid (2.58 g) was added in portions, and the mixture was stirred at room temperature overnight.

After the reaction was completed, 1 mol/L Na₂S₂O₃ was added to quench the reaction, followed by liquid separation. A saturated NaHCO₃ solution was added to the organic phase, followed by liquid separation. The aqueous phase was extracted three times with DCM, the organic phases were combined, washed with brine, dried, and filtered, and the filtrate was concentrated under reduced pressure to give intermediate **3-2** (1.6 g).

LC-MS: m/z 194.18 (M+H)⁺.

### Preparation of intermediate 3-3:

Intermediate **1-7** (1 g), intermediate **3-2** (700 mg), bromotripyrrolidinophosphonium hexafluorophosphate (3.8 g), and N,N-diisopropylethylamine (1.9 g) were added to THF (20 mL), and the mixture was stirred at room temperature for 12 h.

After the reaction was completed, a saturated aqueous sodium bicarbonate solution (50 mL) and ethyl acetate (100 mL) were added, and the mixture was stirred for 10 min and then left to stand for liquid separation. The organic phase was dried over sodium sulfate and filtered, and the filtrate was concentrated under reduced pressure. The residue was purified by column chromatography (petroleum ether/ethyl acetate = 3/1). The eluate was concentrated under reduced pressure to give intermediate **3-3** (300 mg).

LC-MS: m/z 466.26 (M+H)⁺.

### Preparation of intermediate 3-4:

Intermediate **3-3** (300 mg) was added to ammonia-methanol (7 M, 10 mL), and the mixture was stirred with the temperature controlled at 60 °C. After the reaction was completed, the reaction solution was concentrated under reduced pressure to give intermediate **3-4** (400 mg), which was directly used in the next step without further purification.

LC-MS: m/z 437.25(M+H)⁺.

### Preparation of intermediate 3-5:

Intermediate **3-4** (400 mg) was added to THF (25 mL), palladium on carbon (10%, 200 mg) was added, and the mixture was stirred overnight at room temperature under hydrogen atmosphere. After the reaction was completed, the reaction solution was filtered through celite, and the filtrate was concentrated under reduced pressure to give intermediate **3-5** (250 mg), which was directly used in the next step without further purification.

LC-MS: m/z 407.21 (M+H)⁺.

### Preparation of compound 3:

Intermediate **3-5** (250 mg) and intermediate **1-3** (193 mg) were added to tert-butanol (5 mL), methanesulfonato(2-di-tert-butylphosphino-2',4',6'-triisopropyl-1,1'-biphenyl)(2'-amino-1,1'-biphenyl-2-yl)palladium(II) (22 mg), 2-di-tert-butylphosphino-2',4',6'-triisopropylbiphenyl (40 mg), and cesium carbonate (168 mg) were added, and the mixture was stirred at 85 °C for 2 h under nitrogen atmosphere.

After the reaction was completed, ethyl acetate (100 mL) and purified water (100 mL) were added, and the mixture was stirred for 10 min and left to stand for liquid separation. The aqueous phase was extracted with ethyl acetate (50 mL × 3). The organic phases were combined, washed with saturated brine, dried over anhydrous sodium sulfate, and filtered, and the filtrate was concentrated under reduced pressure to give a concentrate, which was separated by preparative liquid chromatography to give compound **3.**

LC-MS: m/z 684.40 (M+H)⁺.

### Preparation of compounds 3-a and 3-b:

Compound 3 was subjected to chiral resolution to give compound 3-a and compound 3-b. The resolution conditions were as follows: a CHIRALPAK AD-H preparative column (4.6 × 250 mm, 5.0 µm) was used; by taking n-hexane as mobile phase A and ethanol as mobile phase B, gradient elution was performed at a flow rate of 0.6 mL/min; the detection wavelength was 254 nm, and the retention times were 32.38 min (3-a) and 39.79 min (3-b), respectively. Compound 3-a LC-MS: m/z 684.32 (M+H)⁺.

Compound 3-b LC-MS: m/z 684.41 (M+H)⁺.

Compound 3-a had a shorter retention time in the chiral chromatography column than compound 3-b, and compound 3-b had a longer retention time in the chiral chromatography column than compound 3-a.

### Example 4: Preparation of Compound 4

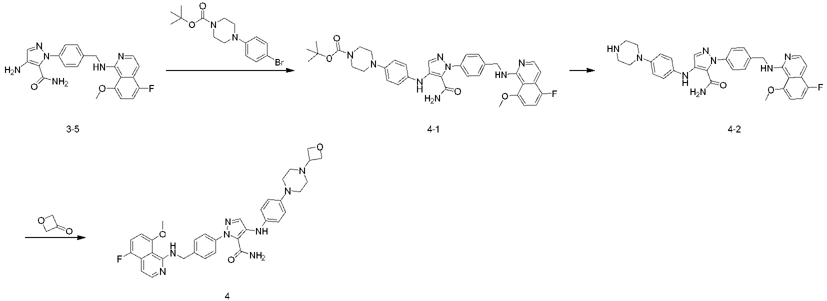

### Preparation of compound 4-1:

Referring to the preparation steps of compound **3** in Example **3,** intermediate **1-3** was replaced with tert-butyl 4-(4-bromophenyl)piperazine-1-carboxylate, and after the reaction was completed, ethyl acetate was added to give intermediate **4-1** (300 mg).

LC-MS: m/z 667.34 (M+H)⁺.

### Preparation of intermediate 4-2:

Intermediate **4-1** (300 mg) was added to DCM (5 mL), trifluoroacetic acid (2 mL) was added, and the mixture was stirred at room temperature. After the reaction was completed, the reaction solution was concentrated under reduced pressure to give intermediate **4-2** (600 mg), which was directly used in the next step without further purification. LC-MS: m/z 567.21 (M+H)⁺.

### Preparation of compound 4:

Intermediate **4-2** (300 mg) was added to DCM (10 mL), and 3-oxetanone (28 mg) and sodium triacetoxyborohydride (112 mg) were added. The mixture was stirred at room temperature for 12 h.

After the reaction was completed, DCM (30 mL) and purified water (20 mL) were added, and the mixture was stirred for 10 min and left to stand for liquid separation. The aqueous phase was extracted with DCM (20 mL × 3), and the organic phases were combined, washed with saturated brine, dried over anhydrous sodium sulfate, and filtered. The filtrate was concentrated under reduced pressure to give a concentrate, which was separated by preparative liquid chromatography to give compound **4.**

LC-MS: m/z 623.22 (M+H)⁺.

### Example 5: Preparation of Compounds 5, 5-a, and 5-b

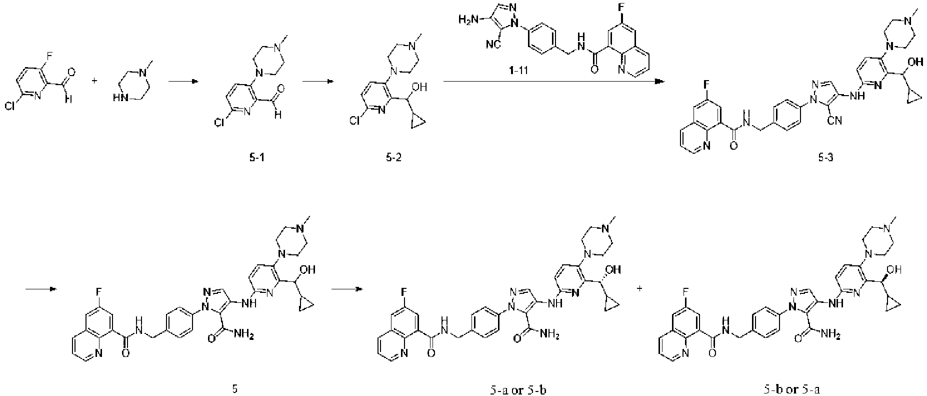

### Preparation of intermediate 5-1:

2-Chloro-5-fluoropyridine-6-carbaldehyde (5 g), N-methylpiperazine (3.77 g), and potassium carbonate (13 g) were added to N,N-dimethylformamide (50 mL), and the mixture was stirred with the temperature controlled at 80 °C for 1 h.

After the reaction was completed, the reaction solution was filtered, and the filtrate was extracted with purified water (100 mL) and ethyl acetate (100 mL). The organic phase was dried over anhydrous sodium sulfate and filtered, and the filtrate was concentrated under reduced pressure to give intermediate 5-1 (6.29 g).

LC-MS: m/z 240.14 (M+H)⁺.

### Preparation of intermediate 5-2:

Intermediate **5-1** (2.5 g) was added to THF (20 mL). The mixture was stirred with the temperature controlled at - 20 °C, and a solution of cyclopropylmagnesium bromide (1 M, 20 mL) was added slowly and dropwise. After the dropwise addition was completed, the mixture was slowly heated to 0 °C and stirred for 2 h.

After the reaction was completed, purified water (50 mL) was added to quench the reaction, and the reaction system was stirred for 10 min. Ethyl acetate (50 mL) was added, and the reaction system was extracted and then left to stand for liquid separation. The organic phase was dried over anhydrous sodium sulfate and filtered under vacuum, and the filtrate was concentrated under reduced pressure to give intermediate **5-2** (2.3 g).

LC-MS: m/z 282.18 (M+H)⁺.

### Preparation of intermediate 5-3:

Referring to the preparation steps of compound **3** in Example **3,** intermediate **3-5** was replaced with intermediate **1-11,** and intermediate **1-3** was replaced with intermediate **5-2;** after the reaction was completed, ethyl acetate was added to give intermediate **5-3** (232 mg).

LC-MS: m/z 632.39 (M+H)⁺.

### Preparation of compound 5:

Intermediate **5-3** (232 mg) was added to a DMSO/ethanol (1:1) mixed solvent (15 mL) and stirred, and a sodium hydroxide solution (5 M, 3 mL) and hydrogen peroxide (3 mL) were added. The mixture was stirred at room temperature for 2.5 h.

After the reaction was completed, ethyl acetate (30 mL) and purified water (30 mL) were added, and the mixture was stirred for 10 min and then left to stand for liquid separation. The aqueous phase was extracted with ethyl acetate (30 mL × 2). The organic phases were combined, washed with saturated brine, dried over anhydrous sodium sulfate, and filtered, and the filtrate was concentrated under reduced pressure to give a concentrate, which was separated by preparative liquid chromatography to give compound **5.**

LC-MS: m/z 650.34(M+H)⁺.

### Preparation of compounds 5-a and 5-b:

Compound 5 was subjected to chiral resolution to give compound **5-a** and compound **5-b.** The resolution conditions were as follows: a CHIRALPAK IC preparative column (4.6 × 250 mm, 5.0 µm) was used; by taking 0.1% ethanolamine in ethanol as the mobile phase, isocratic elution was performed at a flow rate of 0.8 mL/min; the detection wavelength was 254 nm, and the retention times were 22.970 min (5-a) and 30.947 min (5-b), respectively. Compound 5-a LC-MS: m/z 650.38 (M+H)⁺.

Compound 5-b LC-MS: m/z 650.36 (M+H)⁺.

Compound 5-a had a shorter retention time in the chiral chromatography column than compound 5-b, and compound 5-b had a longer retention time in the chiral chromatography column than compound 5-a.

### Example 6: Preparation of Compound 6

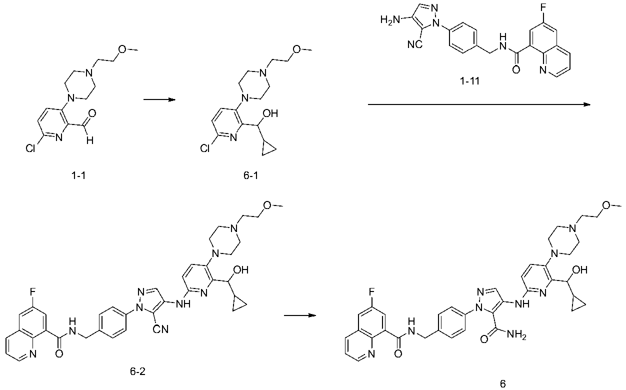

### Preparation of intermediate 6-1:

Intermediate **1-1** (3 g) was added to THF (20 mL). The mixture was stirred with the temperature controlled at -20 °C under nitrogen atmosphere, and a solution of cyclopropylmagnesium bromide (1 M, 15 mL) was added slowly and dropwise. After the dropwise addition was completed, the reaction system was slowly heated to 0 °C and stirred for 3 h.

After the reaction was completed, purified water (50 mL) was added to quench the reaction, and the reaction system was stirred for 10 min. Ethyl acetate (50 mL) was added, and the reaction system was extracted and then left to stand for liquid separation. The organic phase was dried over anhydrous sodium sulfate and filtered under vacuum, and the filtrate was concentrated under reduced pressure to give intermediate **6-1** (3.2 g).

LC-MS: m/z 326.19 (M+H)⁺.

### Preparation of intermediate 6-2:

Referring to the preparation steps of compound **3** in Example **3,** intermediate **3-5** was replaced with intermediate **1-11,** and intermediate **1-3** was replaced with intermediate **6-1;** after the reaction was completed, ethyl acetate was added to give intermediate **6-2** (279 mg).

LC-MS: m/z 676.36(M+H)⁺.

### Preparation of compound 6:

Intermediate **6-2** (279 mg) was added to a mixed solvent of DMSO/ethanol (1:1) (15 mL) and stirred, and a sodium hydroxide solution (5 M, 3 mL) and hydrogen peroxide (3 mL) were added. The mixture was stirred at room temperature for 2.5 h.

After the reaction was completed, ethyl acetate (30 mL) and purified water (30 mL) were added, and the mixture was stirred for 10 min and then left to stand for liquid separation. The aqueous phase was extracted with ethyl acetate (30 mL × 2). The organic phases were combined, washed with saturated brine, dried over anhydrous sodium sulfate, and filtered, and the filtrate was concentrated under reduced pressure to give a concentrate, which was separated by preparative liquid chromatography to give compound **6.**

LC-MS: m/z 694.42(M+H)⁺.

### Example 7: Preparation of Compound 7

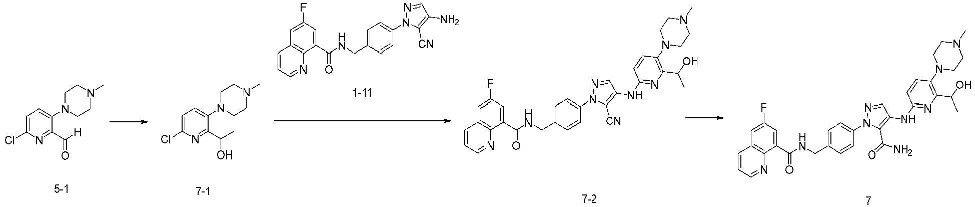

### Preparation of intermediate 7-1:

Intermediate **5-1** (1.2 g) was added to THF (90 mL), and methylmagnesium bromide (3 M, 3 mL) was added dropwise with the temperature controlled at -20 °C under nitrogen atmosphere. After the dropwise addition was completed, the mixture was stirred for 3 h with the temperature controlled at 0 °C. After the reaction was completed, a saturated ammonium chloride solution (50 mL) was added to quench the reaction, and the reaction system was extracted with ethyl acetate (60 mL × 3). The organic phases were combined, washed with a saturated sodium chloride solution, dried over anhydrous sodium sulfate, and filtered under vacuum, and the filtrate was concentrated under reduced pressure to give intermediate **7-1** (1.1 g).

LC-MS: m/z 256.15 (M+H)⁺.

### Preparation of intermediate 7-2:

Referring to the preparation steps of compound **3** in Example **3,** intermediate 3-5 was replaced with intermediate 1-11 and intermediate 1-3 was replaced with intermediate **7-1.** After the reaction was completed, ethyl acetate was added to give intermediate **7-2** (310 mg).

LC-MS: m/z 606.32 (M+H)⁺.

### Preparation of compound 7:

Intermediate **7-2** (310 mg) was added to DMSO (5 mL) and ethanol (5 mL), and a sodium hydroxide solution (1 M, 5 mL) was added, followed by the dropwise addition of hydrogen peroxide (3 mL). After the dropwise addition was completed, the mixture was stirred for 3 h.

After the reaction was completed, ethyl acetate (30 mL) and purified water (20 mL) were added, and the mixture was stirred for 10 min and then left to stand for liquid separation. The aqueous phase was extracted with ethyl acetate (30 mL × 3). The organic phases were combined, washed with saturated brine, dried over anhydrous sodium sulfate, and filtered, and the filtrate was concentrated under reduced pressure to give a concentrate, which was separated by preparative liquid chromatography to give compound **7.**

LC-MS: m/z 624.40 (M+H)⁺.

### Example 8: Preparation of Compounds 8-a and 8-b

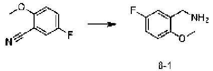

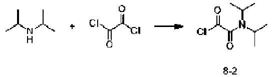

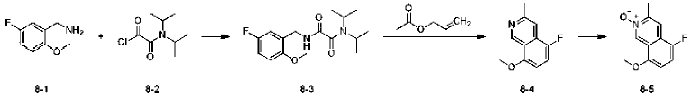

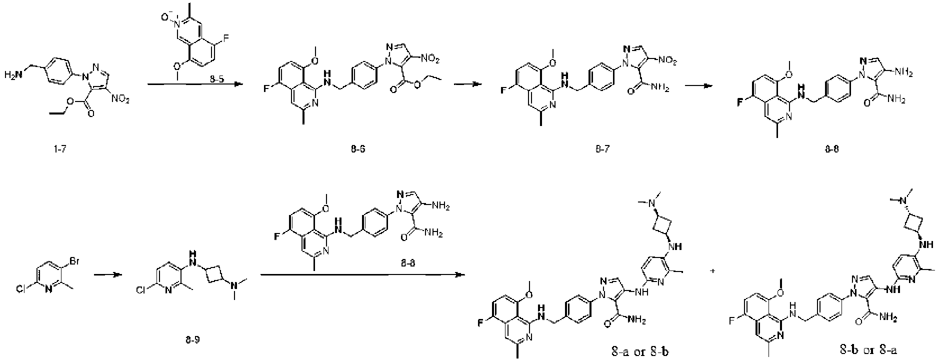

### Preparation of intermediate 8-1:

5-Fluoro-2-methoxybenzonitrile (20 g) was added to tetrahydrofuran (400 mL), and the mixture was placed in an ice bath. Lithium aluminum hydride (20 g) was slowly added. After the addition was completed, the ice bath was removed, and the mixture was heated to room temperature and reacted for 2 h.

After the reaction was completed, 5 mL of water was added to quench the reaction, and the resulting mixture was stirred for 5 min. Then, a 1 M aqueous sodium hydroxide solution (45 mL) was added slowly and dropwise, and ethyl acetate (150 mL) and water (200 mL) were added. The reaction system was extracted and then left to stand for liquid separation, the organic phase was dried over anhydrous sodium sulfate and filtered, and the filtrate was concentrated under reduced pressure to give intermediate **8-1** (20 g).

LC-MS: m/z 156.23 (M+H)⁺.

### Preparation of intermediate 8-2:

Diisopropylamine (20 g) was added to dichloromethane (400 mL), and the mixture was placed in an ice bath. Triethylamine (21 g) and oxalyl chloride (37 g) were slowly added. After the addition was completed, the ice bath was removed, and the mixture was heated to room temperature and reacted for 12 h.

After the reaction was completed, the reaction solution was concentrated under reduced pressure at 40 °C to give intermediate **8-2** (18 g).

### Preparation of intermediate 8-3:

**8-1** (20 g) was added to dichloromethane (300 mL), and the mixture was placed in an ice bath. **8-2** (18 g) and triethylamine (15 g) were slowly added. After the addition was completed, the ice bath was removed, and the mixture was heated to room temperature and reacted for 3 h.

After the reaction was completed, 45 mL of water was added to quench the reaction, and the mixture was stirred for 5 min. Ethyl acetate (250 mL) and water (300 mL) were added, and the mixture was extracted and then left to stand for liquid separation. The organic phase was dried over anhydrous sodium sulfate and filtered, and the filtrate was concentrated under reduced pressure to give intermediate **8-3** (23 g).

LC-MS: m/z 311.25 (M+H)⁺.

### Preparation of intermediate 8-4:

**8-3** (23 g), allyl acetate (13.5 g), palladium(II) acetate (1.5 g), silver carbonate (37.8 g), and dibutyl phosphate (4.3 g) were added to 1,2-dichloroethane (500 mL), and the mixture was reacted in an oil bath at 100 °C for 12 h.

After the starting materials were completely consumed as monitored, sodium hydroxide (35 g) was added. The mixture was cooled to 80 °C and then reacted for 6 h.

After the reaction was completed, ethyl acetate (600 mL) and water (500 mL) were added, and the mixture was extracted and then left to stand for liquid separation. The organic phase was dried over anhydrous sodium sulfate and filtered, and the filtrate was concentrated under reduced pressure and purified and separated by column chromatography (ethyl acetate/petroleum ether = 1/5) to give intermediate **8-4** (16.5 g).

LC-MS: m/z 192.20 (M+H)⁺.

### Preparation of intermediate 8-5:

**8-4** (16.5 g) was added to dichloromethane (150 mL), and m-chloroperoxybenzoic acid (60%, 34.5 g) was added. The mixture was reacted overnight at room temperature.

After the reaction was completed, an aqueous sodium bicarbonate solution (200 mL) was added, and the aqueous phase was extracted with ethyl acetate (500 mL × 2). The organic phases were combined, dried over anhydrous sodium sulfate, and filtered, and the filtrate was concentrated under reduced pressure to give intermediate **8-5** (10.3 g). LC-MS: m/z 208.37 (M+H)⁺.

### Preparation of intermediate 8-6:

Intermediate **1-7** (14 g), intermediate **8-5** (10.3 g), bromotripyrrolidinophosphonium hexafluorophosphate (90 g), and DIPEA (49 g) were added to THF (1 L), and the mixture was reacted at room temperature for 12 h.

After the reaction was completed, a saturated aqueous sodium bicarbonate solution (500 mL) was added, and the mixture was stirred for 10 min. Purified water (1.5 L) was added, and the mixture was extracted with ethyl acetate (1.2 L × 3). The organic phases were combined, dried over sodium sulfate, and filtered. The filtrate was concentrated under reduced pressure and purified by column chromatography (petroleum ether/ethyl acetate = 5/1). The eluate was concentrated under reduced pressure to give intermediate **8-6** (8 g).

LC-MS: m/z 480.23 (M+H)⁺.

### Preparation of intermediate 8-7:

Intermediate **8-6** (8 g) was added to ammonia-methanol (7 M, 800 mL), and the mixture was stirred with the temperature controlled at 60 °C. After the reaction was completed, the reaction solution was concentrated under reduced pressure to give intermediate **8-7** (7.4 g).

LC-MS: m/z 451.25(M+H)⁺.

### Preparation of intermediate 8-8:

Intermediate **8-7** (7.4 g) was added to THF (500 mL), palladium on carbon (10%, 3 g) was added, and the mixture was stirred overnight at room temperature under hydrogen atmosphere. After the reaction was completed, the reaction solution was filtered through celite, and the filtrate was concentrated under reduced pressure to give intermediate **8-8** (6.5 g).

LC-MS: m/z 421.21 (M+H)⁺.

### Preparation of intermediate 8-9:

2-Methyl-3-bromo-5-chloropyridine (718 mg), 3-dimethylaminocyclobutanamine hydrochloride (710 mg), tris(dibenzylideneacetone)dipalladium(0) (510 mg), 4,5-bis-diphenylphosphino-9,9-dimethylxanthene (630 mg), sodium tert-butoxide (1680 mg), and toluene (80 mL) were added. The reaction system was purged three times with nitrogen and stirred at 100 °C for 5 h.

After the reaction was completed, the reaction solution was cooled to room temperature, and ethyl acetate (50 mL) and purified water (30 mL) were added. The resulting mixture was stirred for 10 min and then left to stand for liquid separation, and the aqueous phase was extracted with ethyl acetate (50 mL × 2). The organic phases were combined, washed with saturated brine, dried over anhydrous sodium sulfate, and filtered, and the filtrate was concentrated under reduced pressure and purified by column chromatography (dichloromethane/methanol = 20/1) to give intermediate **8-9** (569 mg).

LC-MS: m/z 240.15 (M+H)⁺.

### Preparation of compounds 8-a and 8-b:

Intermediate **8-9** (138 mg) and intermediate **8-8** (290 mg) were added to tert-butanol (15 mL), methanesulfonato(2-di-tert-butylphosphino-2',4',6'-trilsopropyl-1,1'-biphenyl)(2'-amino-1,1'-biphenyl-2-yl)palladium(II) (72 mg), 2-ditert-butylphosphino-2',4',6'-triisopropylbiphenyl (95 mg), and lithium bis(trimethylsilyl)amide (1 M, 7.5 mL) were added, and the mixture was stirred at 80 °C for 2 h under nitrogen atmosphere.

After the reaction was completed, ethyl acetate (100 mL) and purified water (100 mL) were added, and the mixture was stirred for 10 min and then left to stand for liquid separation. The aqueous phase was extracted with ethyl acetate (50 mL × 3). The organic phases were combined, washed with saturated brine, dried over anhydrous sodium sulfate, and filtered, and the filtrate was concentrated under reduced pressure to give a concentrate, which was separated by preparative liquid chromatography to give compound **8-a** and compound **8-b.** The conditions were as follows: a YMC SA preparative column (30 × 250 mm, 10 µm) was used; by taking n-hexane as mobile phase A and ethanol as mobile phase B, gradient elution was performed at a flow rate of 30 mL/min; the detection wavelength was 254 nm, and the retention times were 19 min (8-a) and 27 min (8-b), respectively.

Compound **8-a** LC-MS: m/z 624.41 (M+H)⁺.

¹H NMR (500 MHz, DMSO-*d*₆) δ 8.34 (t, *J =* 5.9 Hz, 1H), 7.80 (s, 1H), 7.48 (d, *J =* 8.1 Hz, 2H), 7.38 (d, *J =* 8.2 Hz, 2H), 7.31 (t*, J =* 9.3 Hz, 1H), 6.82 (dd, *J* = 8.7, 4.1 Hz, 1H), 6.72 (s, 1H), 6.66 (*d, J =* 8.5 Hz, 1H), 6.56 (*d, J =* 8.5 Hz, 1H), 4.86-4.58 (m, 3H), 3.97 (s, 3H), 3.72 (s, 1H), 2.83 (p, *J =* 6.8 Hz, 1H), 2.34 (s, 3H), 2.20 (dt, *J =* 12.7, 7.2 Hz, 2H), 2.08 (s, 5H), 1.97 (ddd, *J =* 12.0, 7.5, 3.9 Hz, 2H), 1.24 (d, *J =* 5.1 Hz, 5H), 0.85 (tt, *J =* 7.7, 4.4 Hz, 2H).

Compound **8-b** LC-MS: m/z 624.43 (M+H)⁺.

¹H NMR (500 MHz, DMSO-*d*₆) δ 8.35 (q, J = 4.3 Hz, 1H), 8.11 (s, 1H), 7.79 (s, 1H), 7.54-7.46 (m, 2H), 7.43-7.36 (m, 2H), 7.31 (dd, J = 9.9, 8.7 Hz, 1H), 6.82 (dd, J = 8.7, 3.8 Hz, 2H), 6.72 (s, 1H), 6.55 (d, J = 8.6 Hz, 1H), 4.80 (d, J = 5.9 Hz, 2H), 4.55 (d, J = 7.7 Hz, 1H), 3.97 (s, 4H), 3.41 (q, J = 7.4 Hz, 2H), 2.47 (dp, J = 6.8, 2.1 Hz, 2H), 2.41-2.32 (m, 4H), 2.26 (s, 3H), 1.67 (qd, J = 8.6, 2.6 Hz, 2H), 1.33-1.20 (m, 4H), 0.91-0.79 (m, 2H).

Compound 8-a had a shorter retention time in the chiral chromatography column than compound 8-b, and compound 8-b had a longer retention time in the chiral chromatography column than compound 8-a.

### Example 9: Preparation of Compound 9

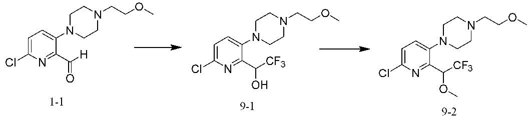

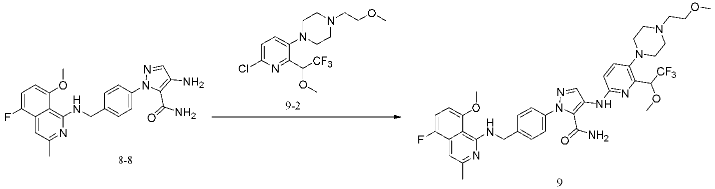

### Preparation of intermediate 9-1:

Intermediate **1-1** (320 mg), (trifluoromethyl)trimethylsilane (241 mg), and cesium fluoride (257 mg) were added to tetrahydrofuran (10 mL). The mixture was stirred at room temperature for 12 h under nitrogen atmosphere.

After the reaction was completed, a saturated ammonium chloride solution (10 mL) was added to quench the reaction, and the reaction system was stirred for 10 min. Ethyl acetate (20 mL × 2) was added, and the reaction system was extracted and then left to stand for liquid separation. The organic phase was dried over anhydrous sodium sulfate and filtered under vacuum, and the filtrate was concentrated under reduced pressure to give intermediate **9-1** (240 mg).

LC-MS: m/z 354.16 (M+H)⁺.

### Preparation of intermediate 9-2:

Intermediate **9-1** (460 mg) was added to THF (10 mL), and the mixture was stirred with the temperature controlled at 0 °C. Sodium hydride (60%, 100 mg) was slowly added, and after the mixture was stirred for 20 min, iodomethane (202 mg) was added. The mixture was transferred to room temperature and stirred for 3 h.

After the reaction was completed, purified water (15 mL) was added to quench the reaction, and the reaction solution was stirred for 10 min. Ethyl acetate (20 mL) was added, and the reaction system was extracted and then left to stand for liquid separation. The organic phase was dried over anhydrous sodium sulfate and filtered under vacuum, and the filtrate was concentrated under reduced pressure to give intermediate **9-2** (287 mg).

LC-MS: m/z 368.18 (M+H)⁺.

### Preparation of compound 9:

Intermediate **8-8** (100 mg) and intermediate **9-2** (105 mg) were added to dioxane (3 mL), and (2-dicyclohexylphosphino-3,6-dimethoxy-2',4',6'-triisopropyl-1,1'-biphenyl)[2-(2-aminoethylphenyl)]palladium(II) chloride (39 mg), 2-(dicyclohexylphosphino)-3,6-dimethoxy-2',4',6'-triisopropyl-1,1'-biphenyl (52 mg), and lithium bis(trimethylsilyl)amide (1 M, 0.24 mL) were added. The mixture was stirred at 85 °C for 2 h under nitrogen atmosphere.

After the reaction was completed, ethyl acetate (100 mL) and purified water (100 mL) were added, and the mixture was stirred for 10 min and then left to stand for liquid separation. The aqueous phase was extracted with ethyl acetate (50 mL × 3). The organic phases were combined, washed with saturated brine, dried over anhydrous sodium sulfate, and filtered, and the filtrate was concentrated under reduced pressure to give a concentrate, which was separated by preparative liquid chromatography to give compound **9.**

LC-MS: m/z 752.39 (M+H)⁺.

¹H NMR (500 MHz, DMSO-*d*₆) δ 8.57 (s, 1H), 8.35 (d, *J =* 6.1 Hz, 2H), 7.72 (d, *J =* 9.0 Hz, 1H), 7.51-7.48 (m, 2H), 7.42-7.39 (m, 2H), 7.31 (dd, *J =* 10.0, 8.7 Hz, 1H), 6.99 (d, *J =* 8.9 Hz, 1H), 6.81 (dd, J = 8.7, 4.2 Hz, 1H), 6.72 (s, 1H), 5.60-5.56 (m, 1H), 4.80 (d, *J =* 5.9 Hz, 2H), 3.97 (s, 3H), 3.46 (t, *J =* 5.8 Hz, 2H), 3.32 (s, 3H), 3.25 (s, 3H), 2.79-2.73 (m, 4H), 2.59-2.52 (m, 6H), 2.34 (s, 3H).

### Example 10: Preparation of Compound 10

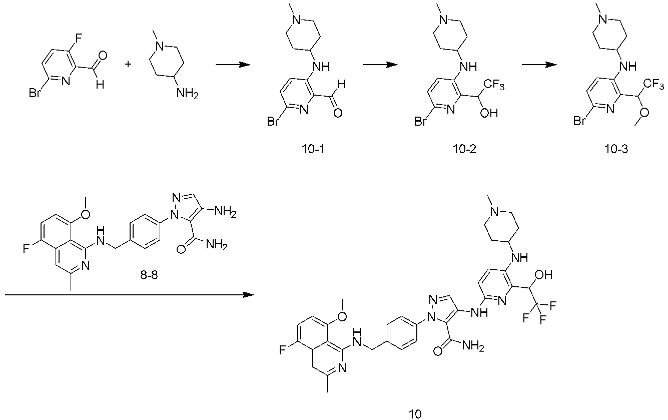

### Preparation of intermediate 10-1:

2-Bromo-5-fluoropyridine-6-carbaldehyde (9 g), 4-amino-N-methylpiperidine (5 g), and potassium carbonate (18.3 g) were added to N,N-dimethylformamide (100 mL), and the mixture was stirred with the temperature controlled at 80°C for 3 h.

After the reaction was completed, the reaction solution was filtered, and the filtrate was extracted with purified water (200 mL) and ethyl acetate (200 mL). The organic phase was dried over anhydrous sodium sulfate and filtered, and the filtrate was concentrated under reduced pressure to give intermediate **10-1** (11.2 g).

LC-MS: m/z 298.24 (M+H)⁺.

### Preparation of intermediate 10-2:

Intermediate **10-1** (11.2 g) was added to THF (50 mL), and (trifluoromethyl)trimethylsilane (11 g) and tetrabutylammonium fluoride (1 M, 40 mL) were added. After the addition was completed, the mixture was stirred at room temperature for 2 h.

After the reaction was completed, purified water (50 mL) was added to quench the reaction, and the reaction system was stirred for 10 min. Ethyl acetate (50 mL) was added, and the reaction system was extracted and then left to stand for liquid separation. The organic phase was dried over anhydrous sodium sulfate and filtered under vacuum, and the filtrate was concentrated under reduced pressure and separated by preparative liquid chromatography to give intermediate **10-2** (500 mg).

LC-MS: m/z 368.22 (M+H)⁺.

### Preparation of intermediate 10-3:

Intermediate **10-2** (200 mg) was added to THF (4 mL), and the mixture was cooled to 0 °C. Sodium hydride (43 mg) was added, and the mixture was stirred for 10 min. Then iodomethane (84 mg) was added dropwise. After the addition was completed, the mixture was heated to room temperature and stirred for 2 h.

After the reaction was completed, purified water (20 mL) was added to quench the reaction. Ethyl acetate (30 mL) was added, and the reaction system was extracted and then left to stand for liquid separation. The organic phase was dried over anhydrous sodium sulfate and filtered under vacuum, and the filtrate was concentrated under reduced pressure to give intermediate **10-3** (254 mg).

LC-MS: m/z 382.42 (M+H)⁺.

### Preparation of compound 10:

Intermediate **8-8** (70 mg) and intermediate **10-3** (61 mg) were added to tert-butanol (5 mL), methanesulfonato(2-di-tert-butylphosphino-2',4',6'-triisopropyl-1,1'-biphenyl)(2'-amino-1,1'-biphenyl-2-yl)palladium(II) (26 mg), 2-di-tert-butylphosphino-2',4',6'-triisopropylbiphenyl (21 mg), and lithium bis(trimethylsilyl)amide (1 M, 0.3 mL) were added, and the mixture was stirred at 80 °C for 2 h under nitrogen atmosphere.

After the reaction was completed, ethyl acetate (100 mL) and purified water (100 mL) were added, and the mixture was stirred for 10 min and then left to stand for liquid separation. The aqueous phase was extracted with ethyl acetate (50 mL × 3). The organic phases were combined, washed with saturated brine, dried over anhydrous sodium sulfate, and filtered, and the filtrate was concentrated under reduced pressure to give a concentrate, which was separated by preparative liquid chromatography to give compound **10.**

LC-MS: m/z 708.41 (M+H)⁺.

¹H NMR (500 MHz DMSO-*d*₆) δ 8.35 (t, *J =* 6.0 Hz, 1H), 8.23 (s, 1H), 8.06 (s, 1H), 7.49 (d, *J =* 8.5 Hz, 2H), 7.39 (d, *J* = 8.5 Hz, 2H), 7.32 (m, 1H), 7.17 (d, *J =* 9.0 Hz, 1H), 6.82 (m, 2H), 6.77 (d, *J =* 6.6 Hz, 1H), 6.72 (s, 1H), 5.27 (m, 1H), 4.84 (d, *J =* 8.2 Hz, 1H), 4.80 (d, *J =* 5.8 Hz, 2H), 3.97 (s, 3H), 3.17 (m, 1H), 2.68 (m, 2H), 2.34 (s, 3H), 2.16 (s, 3H), 2.00 (m, 2H), 1.83 (m, 2H), 1.38 (m, 2H).

### Example 11: Preparation of Compounds 11, 11-a, and 11-b

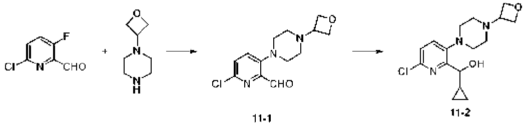

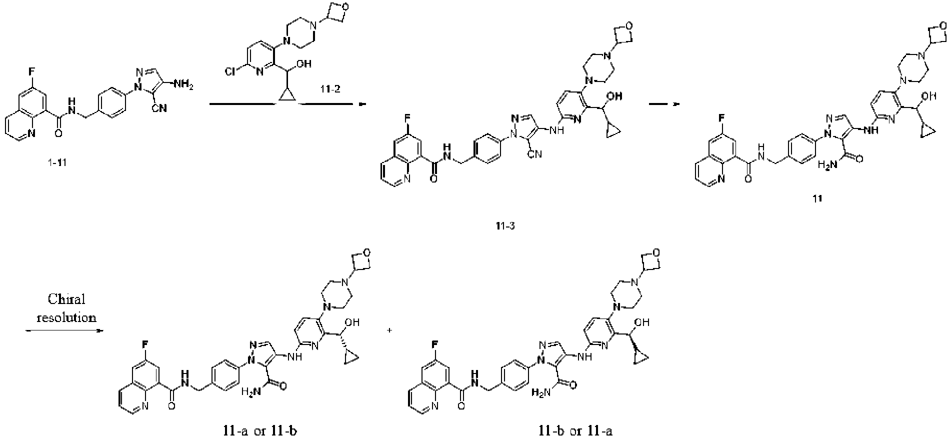

### Preparation of intermediate 11-1:

2-Chloro-5-fluoropyridine-6-carbaldehyde (24 g) and 1-(3-oxetanyl)piperazine (22.2 g) were added to N,N-dimethylformamide (240 mL), and potassium carbonate (64.8 g) was added. The mixture was stirred with the temperature controlled at 80 °C for 2 h.

After the reaction was completed, the reaction solution was filtered, and the filtrate was poured into a saturated aqueous ammonium chloride solution (500 mL). The resulting mixture was extracted with ethyl acetate (250 mL), followed by layer separation and liquid separation. The organic phase was washed with saturated brine, followed by layer separation and liquid separation. The organic phase was dried over anhydrous sodium sulfate and filtered, and the filtrate was concentrated under reduced pressure to give intermediate **11-1** (58 g).

LC-MS: m/z 282.11 (M+H)⁺.

### Preparation of intermediate 11-2:

Intermediate **11-1** (6 g) was added to THF (60 mL), and the mixture was stirred with the temperature controlled at 0 °C. A solution of cyclopropylmagnesium bromide in tetrahydrofuran (1 M, 65 mL) was slowly added. After the dropwise addition was completed, the mixture was transferred to room temperature and stirred overnight.

After the reaction was completed, the reaction solution was transferred to an ice-water bath at 0 °C for cooling, and a saturated ammonium chloride solution (150 mL) was added slowly and dropwise to quench the reaction. The mixture was stirred for 10 min with the temperature maintained. DCM (200 mL) was added, and the reaction system was extracted and then left to stand for liquid separation. The organic phase was dried over anhydrous sodium sulfate and filtered under vacuum, and the filtrate was concentrated under reduced pressure and subjected to column chromatography to give intermediate **11-2** (2.5 g).

LC-MS: m/z 324.18 (M+H)⁺.

### Preparation of intermediate 11-3:

Intermediate 11-2 (200 mg) and intermediate 1-11 (200 mg) were added to a mixed solution of tert-butanol and tetrahydrofuran (1:1, 100 mL), and 2-di-tert-butylphosphino-2',4',6'-triisopropylbiphenyl (110 mg), methanesulfonato(2-di-tert-butylphosphino-2',4',6'-triisopropyl-1,1'-biphenyl)(2'-amino-1,1'-biphenyl-2-yl)palladium(II) (206 mg) and cesium carbonate (506 mg) were added. The mixture was reacted at 85 °C for 2 h under nitrogen atmosphere.

After the reaction was completed, the reaction solution was filtered, and the filtrate was extracted with ethyl acetate (100 mL) and purified water (50 mL). The mixture was stirred for 10 min and then left to stand for liquid separation. The aqueous phase was extracted with ethyl acetate (50 mL × 3), and the organic phases were combined, washed with saturated brine, dried over anhydrous sodium sulfate, and filtered. The filtrate was concentrated under reduced pressure to give intermediate **11-3** (1.2 g).

LC-MS: m/z 674.35 (M+H)⁺.

### Preparation of compound 11:

Intermediate **11-3** (1.2 g) was added to ethanol (12 mL), and a sodium hydroxide solution (1 M, 6 mL) was added. The mixture was reacted for 2 h with the temperature controlled at 70 °C.

After the reaction was completed, ethyl acetate (100 mL) and purified water (50 mL) were added, and the mixture was stirred for 10 min and then left to stand for liquid separation. The aqueous phase was extracted with ethyl acetate (50 mL × 3). The organic phases were combined, washed with saturated brine, dried over anhydrous sodium sulfate, and filtered, and the filtrate was concentrated under reduced pressure to give a concentrate, which was separated by preparative liquid chromatography to give compound **11.**

LC-MS: m/z 692.23 (M+H)⁺.

¹H NMR (500 MHz, DMSO-*d*₆) δ 11.25 (s, 1H), 9.04 (dd, *J* = 4.4, 1.7 Hz, 1H), 8.56 (dd, *J* = 8.4, 1.8 Hz, 1H), 8.38-7.72 (m, 5H), 7.57 (d, *J =* 8.7 Hz, 1H), 7.52 (d, *J =* 8.2 Hz, 1H), 7.45 (d, *J =* 8.1 Hz, 1H), 6.76 (d, *J =* 8.7 Hz, 1H), 5.06 (d, *J* = 6.8 Hz, 2H), 4.76 (d, *J =* 6.0 Hz, 2H), 4.55 (t, *J =* 6.6 Hz, 2H), 4.45 (d, *J =* 6.2, 3.1 Hz, 2H), 3.54-3.43 (m, 7H), 2.98-2.86 (m, 4H), 2.78-2.65 (m, 2H).

### Preparation of compounds 11-a and 11-b:

Compound 11 was subjected to chiral resolution to give compound **11-a** and compound **11-b.** The resolution conditions were as follows: a YMC Amylose-SA preparative column (30 × 250 mm, 10 µm) was used; gradient elution was performed with methanol-dichloromethane (0%-30%/0-60 min), and the retention times were 23.5 min (11-a) and 25.6 min (11-b), respectively.

Compound 11-a LC-MS: m/z 692.11 (M+H)⁺.

Compound 11-b LC-MS: m/z 692.15 (M+H)⁺.

Compound 11-a had a shorter retention time in the chiral chromatography column than compound 11-b, and compound 11-b had a longer retention time in the chiral chromatography column than compound 11-a.

### Example 12: Preparation of Compounds 12, 12-a, and 12-b

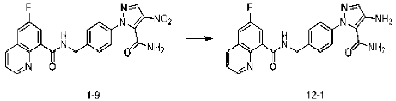

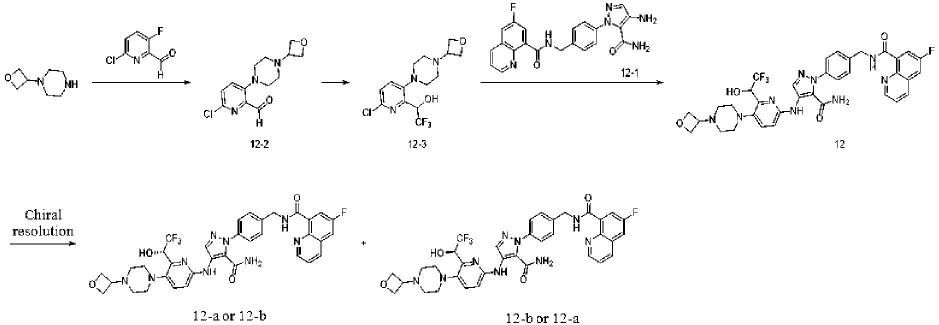

### Preparation of intermediate 12-1:

Intermediate **1-9** (10 g) was added to tetrahydrofuran (800 mL), and palladium on carbon (10%, 5 g) was added. The reaction system was purged three times with hydrogen and stirred at room temperature for 2 h.

After the reaction was completed, the reaction solution was filtered through celite, and the filtrate was concentrated under reduced pressure at 40 °C to give intermediate **12-1** (6.3 g).

LC-MS: m/z 405.16 (M+H)⁺.

### Preparation of intermediate 12-2:

2-Chloro-5-fluoropyridine-6-carbaldehyde (2.97 g) and 1-(3-oxetanyl)piperazine (2.64 g) were added to N,N-dimethylformamide (50 mL), and potassium carbonate (7.6 g) was added. The mixture was stirred with the temperature controlled at 85 °C for 1 h.

After the reaction was completed, the reaction solution was extracted with purified water (100 mL) and EA (100 mL). The organic phase was dried over anhydrous sodium sulfate and filtered, and the filtrate was concentrated under reduced pressure to give intermediate **12-2** (3.93 g).

LC-MS: m/z 282.10 (M+H)⁺.

### Preparation of intermediate 12-3:

Intermediate **12-2** (990 mg) was added to THF (5 mL), and (trifluoromethyl)trimethylsilane (750 mg) and tetrabutylammonium fluoride (1 M, 1 mL) were added sequentially and dropwise under nitrogen atmosphere. After the dropwise addition was completed, the mixture was stirred at room temperature for 3 h. After the reaction was completed, a saturated ammonium chloride solution (5 mL) was added to quench the reaction, and the resulting mixture was extracted with ethyl acetate (60 mL × 3). The organic phases were combined, washed with a saturated sodium chloride solution, dried over anhydrous sodium sulfate, and purified by column chromatography (dichloromethane/methanol = 50/1). The eluate was concentrated under reduced pressure to give intermediate **12-3** (552 mg).

LC-MS: m/z 352.14 (M+H)⁺.

### Preparation of compound 12:

Intermediate **12-1** (100 mg) and intermediate **12-3** (95 mg) were added to tert-butanol (8 mL), methanesulfonato(2-di-tert-butylphosphino-2',4',6'-triisopropyl-1,1'-biphenyl)(2'-amino-1,1'-biphenyl-2-yl)palladium(II) (35 mg), 2-di-tert-butylphosphino-2',4',6'-triisopropylbiphenyl (40 mg), and lithium bis(trimethylsilyl)amide (1 M, 0.6 mL) were added, and the mixture was stirred at 80 °C for 2 h under nitrogen atmosphere.

After the reaction was completed, ethyl acetate (50 mL) and purified water (50 mL) were added, and the mixture was stirred for 10 min and left to stand for liquid separation. The aqueous phase was extracted with ethyl acetate (50 mL × 3). The organic phases were combined, washed with saturated brine, dried over anhydrous sodium sulfate, and filtered, and the filtrate was concentrated under reduced pressure to give a concentrate, which was separated by preparative liquid chromatography to give compound **12.**

LC-MS: m/z 720.29 (M+H)⁺.

¹H NMR (500 MHz, DMSO-*d*₆) δ 11.24 (t, *J =* 6.0 Hz, 1H), 9.05 (dd, *J* = 4.3, 1.8 Hz, 1H), 8.57 (dd, *J* = 8.4, 1.8 Hz, 1H), 8.51 (s, 1H), 8.42-8.30 (m, 2H), 8.07 (dd, *J =* 8.5, 3.1 Hz, 1H), 7.78-7.66 (m, 2H), 7.53 (d, *J =* 8.5 Hz, 2H), 7.48-7.41 (m, 2H), 6.95 (d, *J* = 8.9 Hz, 1H), 6.73 (d, *J* = 7.6 Hz, 1H), 5.61 (p, *J =* 7.5 Hz, 1H), 4.76 (d, *J =* 6.0 Hz, 2H), 4.56 (t, *J =* 6.5 Hz, 2H), 4.46 (t, *J =* 6.1 Hz, 2H), 3.48 (p, *J =* 6.3 Hz, 1H), 3.34 (s, 4H), 2.89 (dt, *J =* 10.3, 4.3 Hz, 2H), 2.74 (dt, *J =* 10.9, 4.5 Hz, 2H), 2.41 (s, 2H).

### Preparation method for compounds 12-a and 12-b:

Compound 12 was subjected to chiral resolution to give compound **12-a** and compound **12-b.** The resolution conditions were as follows: a Cellulose-SC preparative column (30 × 250 mm, 10 µm) was used; by taking n-hexane as mobile phase A and ethanol as mobile phase B, gradient elution was performed at a flow rate of 0.8 mL/min; the detection wavelength was 254 nm, and the retention times were 53 min (12-a) and 60 min (12-b), respectively.

Compound 12-a LC-MS: m/z 720.29 (M+H)⁺.

¹H NMR (500 MHz DMSO-*d*₆) δ 11.24 (t, *J =* 5.5 Hz, 1H), 9.05 (d*, J =* 3.0 Hz, 1H), 8.57 (*d, J =* 8.0 Hz, 1H), 8.51 (s, 1H), 8.36 (m, 2H), 8.07 (dd, *J =* 8.5, 2.5 Hz, 1H), 7.72 (m, 2H), 7.53 (d, *J =* 8.0 Hz, 2H), 7.45 (d, *J =* 8.0 Hz, 2H), 6.95 (d, *J =* 9.0 Hz, 1H), 6.72 (d, *J =* 7.5 Hz, 1H), 5.61 (m, 1H), 4.76 (d, *J =* 6.0 Hz, 2H), 4.56 (t, *J =* 6.0 Hz, 2H), 4.46 (t, *J =* 6.0 Hz, 2H), 3.48 (m, 1H), 2.89 (m, 2H), 2.74 (m, 2H), 2.41 (m, 4H).

Compound 12-b LC-MS: m/z 720.31 (M+H)⁺.

¹H NMR (500 MHz DMSO-*d*₆) δ 11.24 (t, *J =* 6.0 Hz, 1H), 9.05 (d, *J* = 4.0*,* 1.5 Hz, 1H), 8.57 (d, *J =* 8.5, 1.5 Hz, 1H), 8.51 (s, 1H), 8.36 (m, 2H), 8.07 (dd, *J* = 8.5, 2.5 Hz, 1H), 7.72 (m, 2H), 7.53 (d, *J* = 8.0 Hz, 2H), 7.45 (d,*J =* 8.0 Hz, 2H), 6.95 (d, *J* = 9.0 Hz, 1H), 6.72 (d, *J =* 7.5 Hz, 1H), 5.61 (m, 1H), 4.76 (d, *J* = 6.0 Hz, 2H), 4.56 (t,*J =* 6.0 Hz, 2H), 4.46 (t, *J =* 6.0 Hz, 2H), 3.48 (m, 1H), 2.89 (m, 2H), 2.74 (m, 2H), 2.41 (m, 4H).

Compound 12-a had a shorter retention time in the chiral chromatography column than compound 12-b, and compound 12-b had a longer retention time in the chiral chromatography column than compound 12-a.

### Example 13: Preparation of Compounds 13, 13-a, and 13-b

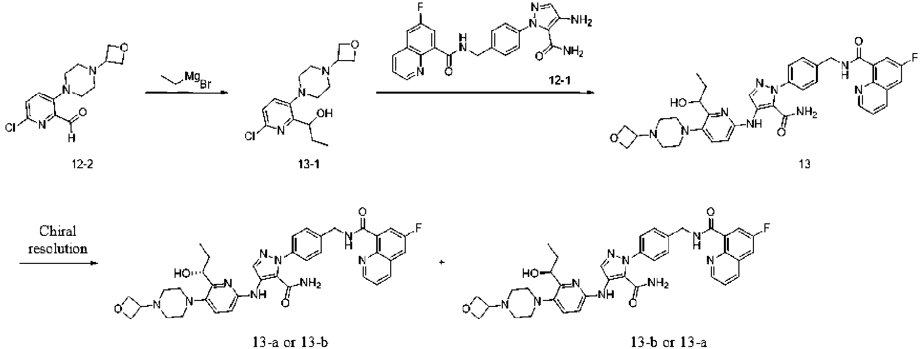

### Preparation of Intermediate 13-1:

Referring to the preparation steps of intermediate **5-2** in Example **5,** intermediate **5-1** was replaced with intermediate **12-2,** and cyclopropylmagnesium bromide was replaced with ethylmagnesium bromide. After the reaction was completed, ethyl acetate was added, and the mixture was concentrated under reduced pressure to give intermediate **13-1** (1 g).

LC-MS: m/z 312.17 (M+H)⁺.

### Preparation of compound 13:

Referring to the preparation steps of compound **3** in Example **3,** intermediate **3-5** was replaced with intermediate **12-1,** intermediate **1-3** was replaced with intermediate **13-1,** and cesium carbonate was replaced with potassium carbonate. After the reaction was completed, the reaction solution was filtered and concentrated to give a concentrate, which was separated by preparative liquid chromatography to give compound **13.**

LC-MS: m/z 680.38 (M+H)⁺.

¹H NMR (500 MHz, DMSO-*d*₆) δ 11.25 (t, *J =* 6.0 Hz, 1H), 9.05 (dd, *J* = 4.2, 1.8 Hz, 1H), 8.57 (dd, *J* = 8.4, 1.8 Hz, 1H), 8.36 (dd, J= 9.6, 3.1 Hz, 1H), 8.32 (s, 1H), 8.25 (d, *J =* 7.3 Hz, 2H), 8.07 (dd, J= 8.5, 3.1 Hz, 1H), 7.73 (dd, *J =* 8.4, 4.3 Hz, 1H), 7.53 (t, *J =* 8.9 Hz, 3H), 7.48-7.39 (m, 2H), 6.74 (d, *J =* 8.7 Hz, 1H), 4.82 (dd, *J =* 7.3, 5.5 Hz, 1H), 4.76 (d, *J =* 6.0 Hz, 2H), 4.55 (t, *J =* 6.5 Hz, 2H), 4.45 (td, *J =* 6.2, 1.4 Hz, 2H), 2.92 (dt, *J =* 10.2, 4.4 Hz, 2H), 2.72 (dt, *J =* 10.5, 4.2 Hz, 2H), 2.39 (s, 3H), 1.71 (ddd, *J* = 16.0, 13.2, 7.0 Hz, 2H), 1.24 (d*, J =* 6.7 Hz, 1H), 0.84 (t, *J* = 7.4 Hz, 3H).

### Preparation method for compounds 13-a and 13-b:

Compound **13** was subjected to chiral resolution to give compound **13-a** and compound **13-b.** The resolution conditions were as follows: a Cellulose-SC preparative column (30 × 250 mm, 10 µm) was used; by taking n-hexane as mobile phase A and ethanol as mobile phase B, gradient elution was performed at a flow rate of 0.8 mL/min; the detection wavelength was 254 nm, and the retention times were 24 min (13-a) and 27 min (13-b), respectively.

Compound 13-a LC-MS: m/z 680.35 (M+H)⁺.

Compound 13-b LC-MS: m/z 680.31 (M+H)⁺.

Compound 13-a had a shorter retention time in the chiral chromatography column than compound 13-b, and compound 13-b had a longer retention time in the chiral chromatography column than compound 13-a.

### Example 14: Preparation of Compound 14

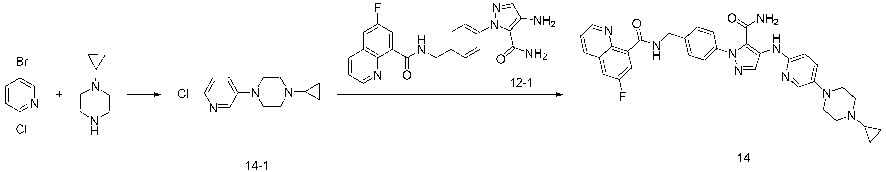

### Preparation of intermediate 14-1:

5-Bromo-2-chloropyridine (2.28 g), 1-cyclopropylpiperazine (1.49 g), tris(dibenzylideneacetone)dipalladium(0) (542 mg), 4,5-bis-diphenylphosphino-9,9-dimethylxanthene (675 mg), and cesium carbonate (5.4 g) were added to 1,4-dioxane (50 mL). The mixture was stirred at 85 °C for 2 h under nitrogen atmosphere, filtered, and purified by column chromatography (ethyl acetate:petroleum ether = 1:1). The resulting fraction was concentrated under reduced pressure to give intermediate **14-1** (2 g).

LC-MS: m/z 238.16 (M+H)⁺.

### Preparation of compound 14:

Referring to the preparation steps of compound **3** in Example **3,** intermediate **3-5** was replaced with intermediate **12-1,** intermediate **1-3** was replaced with intermediate **14-1,** and cesium carbonate was replaced with lithium bis(trimethylsilyl)amide. After the reaction was completed, the reaction solution was filtered and concentrated to give a concentrate, which was separated by preparative liquid chromatography to give compound **14.**

LC-MS: m/z 606.37 (M+H)⁺.

¹H NMR (500 MHz, DMSO-*d*₆) δ 11.24 (t*, J =* 6.0 Hz, 1H), 9.05 (dd, *J* = 4.3, 1.8 Hz, 1H), 8.57 (dd, *J* = 8.4, 1.8 Hz, 1H), 8.36 (dd, *J* = 9.6*,* 3.2 Hz, 1H), 8.16-7.99 (m, 3H), 7.79 (d, *J* = 2.9 Hz, 1H), 7.73 (dd, *J =* 8.4, 4.2 Hz, 1H), 7.52 (d, *J =* 8.3 Hz, 2H), 7.46-7.39 (m, 2H), 7.32 (dd, *J* = 9.0, 3.0 Hz, 1H), 6.77 (d, *J =* 9.0 Hz, 1H), 5.32 (t, *J =* 5.0 Hz, 1H), 4.76 (d, *J =* 6.0 Hz, 2H), 2.97 (t, *J =* 5.0 Hz, 4H), 2.66 (t, *J =* 4.9 Hz, 4H), 0.47-0.40 (m, 2H), 0.33 (p, *J* = 4.0 Hz, 2H).

### Example 15: Preparation of Compound 15

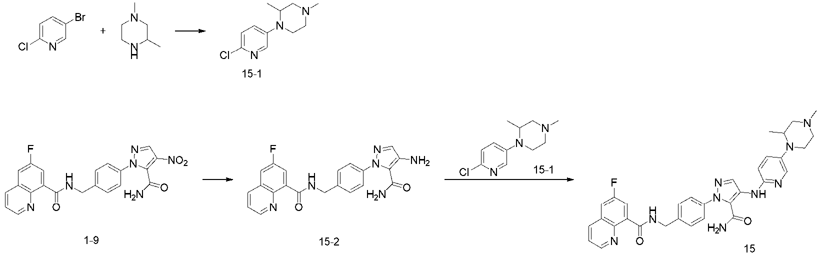

### Preparation of intermediate 15-1:

5-Bromo-2-chloropyridine (2.2 g) and 1,3-dimethylpiperazine (1.5 g) were added to 1,4-dioxane (100 mL), then potassium tert-butoxide (2.5 g) was added, and palladium(II) acetate (0.5 g) and 2-(dicyclohexylphosphino)-2',4',6'-triisopropylbiphenyl (0.1 g) were added. The mixture was stirred for 4 h with the temperature controlled at 85 °C.

After the reaction was completed, the reaction solution was filtered, and the filtrate was poured into purified water (250 mL). The resulting mixture was extracted with ethyl acetate (150 mL), followed by layer separation and liquid separation. The organic phase was washed with saturated brine, followed by layer separation and liquid separation. The organic phase was dried over anhydrous sodium sulfate and filtered, and the filtrate was concentrated under reduced pressure and subjected to column chromatography to give intermediate **15-1** (1.2 g).

LC-MS: m/z 226.12 (M+H)⁺.

### Preparation of intermediate 15-2:

Intermediate **1-9** (8 g) was added to tetrahydrofuran (160 mL), and palladium on carbon (10%, 500 mg) was added. The mixture was stirred at room temperature for 2 h under hydrogen atmosphere. After the reaction was completed, the reaction solution was filtered through celite, and the filtrate was concentrated under reduced pressure to give intermediate **15-2** (6 g).

LC-MS: m/z 405.12 (M+H)⁺.

### Preparation of compound 15:

Intermediate **15-2** (150 mg) and intermediate **15-1** (150 mg) were added to tert-butanol (38 mL), lithium bis(trimethylsilyl)amide (1 M, 375 µL), 2-(dicyclohexylphosphino)-3,6-dimethoxy-2'-4'-6'-tri-I-propyl-11'-biphenyl (80 mg), and chloro(2-dicyclohexylphosphino-3,6-dimethoxy-2',4',6'-triisopropyl-1,1'-biphenyl)[2-(2-aminoethylphenyl)]palladium(II) (60 mg) were added, and the mixture was reacted at 85 °C for 1 h under nitrogen atmosphere.

After the reaction was completed, the reaction solution was filtered, and the filtrate was extracted with ethyl acetate (100 mL) and purified water (100 mL). The mixture was stirred for 10 min and then left to stand for liquid separation. The aqueous phase was extracted with ethyl acetate (50 mL × 3), and the organic phases were combined, washed with saturated brine, dried over anhydrous sodium sulfate, and filtered. The filtrate was concentrated under reduced pressure to give a concentrate, which was separated by preparative liquid chromatography to give compound **15.**

LC-MS: m/z 594.45 (M+H)⁺.

¹H NMR (500 MHz, DMSO-*d*₆) δ 11.25 (s, 1H), 9.04 (d, *J =* 4.2 Hz, 1H), 8.55 (d, *J =* 8.5 Hz, 1H), 8.35-7.72 (m, 5H), 7.48 (dd, *J =* 43.9, 8.1 Hz, 2H), 7.32 (dd, *J =* 9.0, 2.8 Hz, 2H), 6.79 (d, *J =* 9.0 Hz, 2H), 4.76 (d, *J =* 6.0 Hz, 2H), 3.05-2.85 (m, 6H), 2.29-2.18 (m, 6H).

### Example 16: Preparation of Compound 16

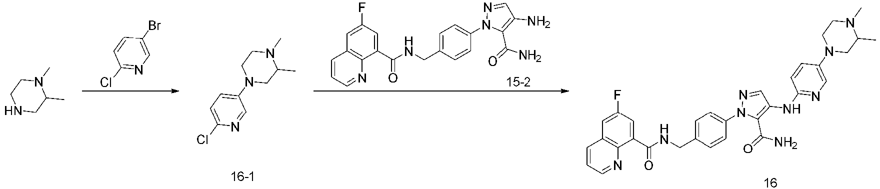

### Preparation of intermediate 16-1:

1,2-Dimethylpiperazine (1.37 g), 2-chloro-5-bromopyridine (1.92 g), tris(dibenzylideneacetone)palladium(0) (229 mg), 4,5-bis-diphenylphosphino-9,9-dimethylxanthene (579 mg), and cesium carbonate (6.52 g) were added to dioxane (100 mL), and the mixture was stirred at 100-105 °C for 15 h under nitrogen atmosphere.

After the reaction was completed, the reaction solution was cooled to room temperature. Purified water (300 mL) and ethyl acetate (300 mL) were added, and the mixture was stirred for extraction and left to stand for liquid separation. The organic phase was dried over anhydrous sodium sulfate and filtered under vacuum, and the filtrate was concentrated under reduced pressure to give intermediate **16-1** (2.26 g).

LC-MS: m/z 226.18 (M+H)⁺.

### Preparation of compound 16:

Intermediate **16-1** (113 mg), intermediate **15-2** (100 mg), chloro(2-dicyclohexylphosphino-3,6-dimethoxy-2',4',6'-triisopropyl-1,1'-biphenyl)[2-(2-aminoethylphenyl)palladium(II) (40 mg), 2-(dicyclohexylphosphino)-3,6-dimethoxy-2'-4'-6'-tri-I-propyl-11'-biphenyl (54 mg), and potassium bis(trimethylsilyl)amide (1 M, 1 mL) were added to tert-butanol (20 mL). The mixture was stirred at 100 °C under microwave at 150 W for 1.5 h under nitrogen atmosphere.

After the reaction was completed, ethyl acetate (100 mL) was added for dilution, and the mixture was filtered. The filtrate was concentrated under reduced pressure to give a concentrate, which was purified by preparative liquid chromatography to give compound **16.**

LC-MS: m/z 594.39 (M+H)⁺.

¹H NMR (500 MHz, DMSO-*d*₆) δ 11.25 (t, *J =* 6.0 Hz, 1H), 9.11-8.94 (m, 1H), 8.57 (dd, *J =* 8.5, 1.9 Hz, 1H), 8.36 (dd, *J* = 9.6, 3.1 Hz, 1H), 8.15-8.01 (m, 3H), 7.80 (d, *J* = 2.9 Hz, 1H), 7.73 (dd, *J =* 8.4, 4.3 Hz, 1H), 7.47 (dd,*J =* 45.7, 8.1 Hz, 5H), 7.32 (dd, *J =* 9.0, 3.0 Hz, 1H), 6.78 (d, *J =* 8.9 Hz, 1H), 4.76 (d, *J =* 6.0 Hz, 2H), 2.79 (dt, *J =* 11.3, 3.0 Hz, 1H), 2.67 (td, *J =* 11.5, 3.0 Hz, 1H), 2.40-2.24 (m, 2H), 2.21 (d, *J =* 7.2 Hz, 3H), 2.14 (ddt, *J =* 9.7, 6.4, 3.2 Hz, 1H), 1.22 (d, *J =* 13.1 Hz, 1H), 1.04 (dd, *J* = 17.6, 6.2 Hz, 3H).

### Example 17: Preparation of Compounds 17, 17-a, and 17-b

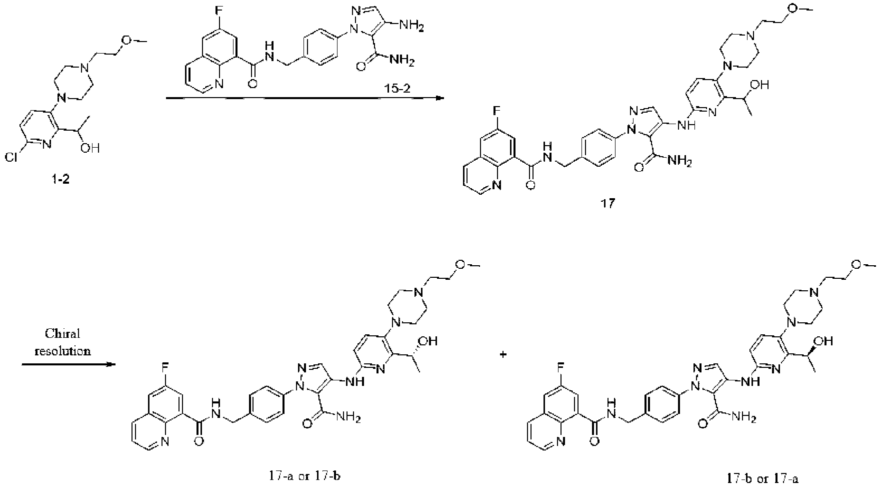

### Preparation of compound 17:

Intermediate **1-2** (150 mg), intermediate **15-2** (100 mg), chloro(2-dicyclohexylphosphino-3,6-dimethoxy-2',4',6'-triisopropyl-1,1'-biphenyl)[2-(2-aminoethylphenyl)]palladium(II) (40 mg), 2-(dicyclohexylphosphino)-3,6-dimethoxy-2'-4'-6'-tri-I-propyl-11'-biphenyl (54 mg), and potassium carbonate (138 mg) were added to tert-butanol (25 mL). The mixture was stirred at 100 °C under microwave at 150 W for 1.5 h under nitrogen atmosphere.

After the reaction was completed, ethyl acetate (100 mL) was added, and the reaction solution was filtered. The filtrate was concentrated under reduced pressure to give a concentrate, which was purified by preparative liquid chromatography to give compound **17.**

LC-MS: m/z 668.43 (M+H)⁺.

### Preparation of compounds 17-a and 17-b:

Compound **17** was subjected to chiral resolution to give compound **17-a** and compound **17-b.** The resolution conditions were as follows: a YMC-Amylose-SA preparative column (30 × 150 mm, 10 µm) was used; by taking n-hexane as mobile phase A and 0.2% diethylamine in ethanol as mobile phase B, gradient elution was performed at a flow rate of 40 mL/min; the detection wavelength was 254 nm, and the retention times were 18.0 min **(17-a)** and 24.0 min **(17-b),** respectively.

Compound 17-a LC-MS: m/z 668.41 (M+H)⁺.

Compound 17-b LC-MS: m/z 668.44 (M+H)⁺.

Compound 17-a had a shorter retention time in the chiral chromatography column than compound 17-b, and compound 17-b had a longer retention time in the chiral chromatography column than compound 17-a.

### Example 18: Preparation of Compound 18

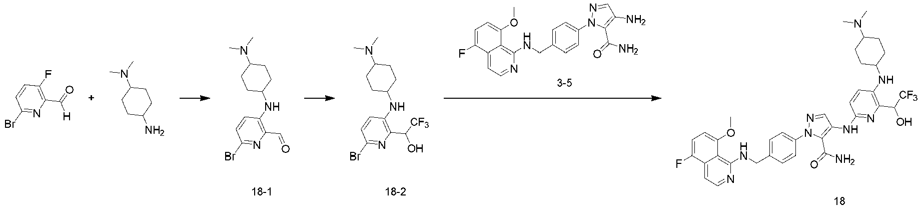

### Preparation of compound 18-1:

2-Bromo-5-fluoropyridine-6-carbaldehyde (3 g) and N,N-dimethyl-1,4-cyclohexanediamine (3.2 g) were added to N,N-dimethylformamide (40 mL), potassium carbonate (8.1 g) was added, and the mixture was stirred for 12 h with the temperature controlled at 120 °C.

After the reaction was completed, the reaction solution was extracted with purified water (200 mL) and ethyl acetate (200 mL). The organic phase was dried over anhydrous sodium sulfate and filtered, and the filtrate was concentrated under reduced pressure to give intermediate **18-1** (6.4 g).

LC-MS: m/z 326.14 (M+H)⁺.

### Preparation of intermediate 18-2:

Referring to the preparation steps of intermediate **10-2** in Example **10,** intermediate **10-1** was replaced with intermediate **18-1,** and after the reaction was completed, intermediate **18-2** (384 mg) was obtained.

LC-MS: m/z 396.21 (M+H)⁺.

### Preparation of compound 18:

Referring to the preparation steps of compound **3** in Example **3,** intermediate **1-3** was replaced with intermediate **18-2,** and after the reaction was completed, compound **18** was obtained.

LC-MS: m/z 722.14 (M+H)⁺.

¹H NMR (500 MHz DMSO-*d*₆) δ 8.46 (t, J = 6.0 Hz, 1H), 8.23 (m, 2H), 8.06 (s, 1H), 7.90 (d, J = 5.8 Hz, 1H), 7.43 (m, 2H), 7.38 (m, 3H), 7.17 (d, J = 9.0 Hz, 1H), 6.94 (dd, J = 9.0, 4.0 Hz, 1H), 6.87 (d, J = 5.7 Hz, 1H), 6.84 (d, J = 9.0 Hz, 1H), 5.27 (m, 1H), 4.81 (m, 3H), 4.00 (s, 3H), 3.14 (m, 1H), 2.42 (m, 1H), 2.31 (m, 6H), 1.99 (m, 2H), 1.86 (m, 2H), 1.35 (m, 2H), 1.16 (m, 2H).

### Example 19: Preparation of Compound 19

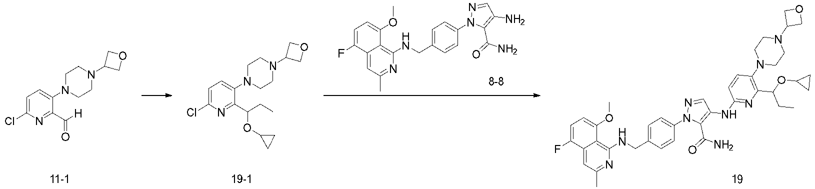

### Preparation of intermediate 19-1:

Intermediate **11-1** (281 mg) was added to THF (10 mL), and cyclopropanol (78 µL) was added dropwise under nitrogen atmosphere. After the dropwise addition was completed, sodium hydride (48 mg) was added with the temperature controlled at 0 °C. After the mixture was stirred for 3 h, ethylmagnesium bromide (2 M, 1.0 mL) was added dropwise directly. The mixture was stirred for 3 h. After the reaction was completed, a saturated sodium chloride solution (10 mL) was added to quench the reaction, and the reaction system was extracted with ethyl acetate (10 mL × 3). The organic phases were combined, washed with a saturated sodium chloride solution, dried over anhydrous sodium sulfate, and filtered under vacuum. The filtrate was concentrated under reduced pressure and purified by column chromatography to give intermediate **19-1** (90 mg).

LC-MS: m/z 352.25 (M+H)⁺.

### Preparation of compound 19:

Referring to the preparation steps of compound **3** in Example **3,** intermediate **1-3** was replaced with intermediate **19-1,** and intermediate **3-5** was replaced with intermediate **8-8.**

After the reaction was completed, the reaction solution was separated by preparative liquid chromatography to give compound **19.**

LC-MS: m/z 736.42 (M+H)⁺.

¹H NMR (500 MHz, DMSO-*d*₆) δ 8.34 (q, J = 5.2 Hz, 1H), 8.09 (s, 1H), 8.04 (s, 1H), 7.48 (d, J = 8.4 Hz, 2H), 7.41-7.28 (m, 4H), 6.82 (dd, J = 8.8, 4.2 Hz, 1H), 6.75-6.58 (m, 3H), 4.82-4.75 (m, 2H), 4.55 (t, J = 6.5 Hz, 2H), 4.45 (t, J = 6.0 Hz, 2H), 3.97 (s, 3H), 3.90 (s, 1H), 3.47 (t, J = 6.4 Hz, 1H), 2.83 (s, 3H), 2.48 (q, J = 1.9 Hz, 2H), 2.39 (s, 3H), 2.34 (s, 2H), 2.04-1.96 (m, 1H), 1.93 (d, J = 1.3 Hz, 2H), 1.56-1.44 (m, 2H), 1.24 (s, 3H), 0.83 (q, J = 8.4 Hz, 3H).

### Example 20: Preparation of Compound 20

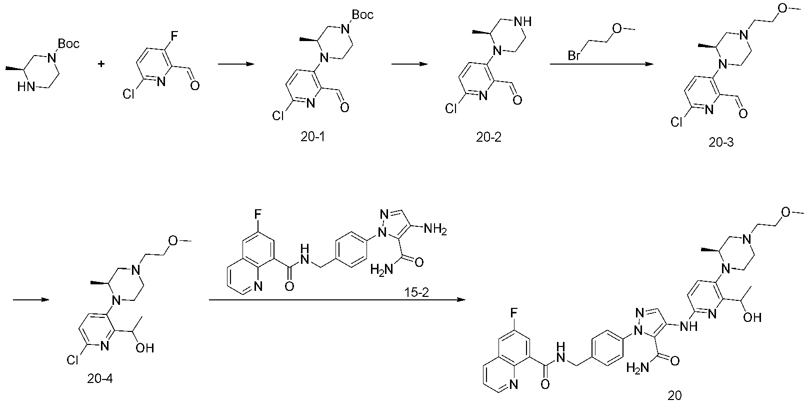

### Preparation of intermediate 20-1:

(S)-4-N-tert-butoxycarbonyl-2-methylpiperazine (4.4 g), 2-chloro-5-fluoropyridin-6-aldehyde (3.19 g) and potassium carbonate (8.28 g) were added to N,N-dimethylformamide (40 mL), and the mixture was stirred at 80 °C for 1.5 h under nitrogen atmosphere. After the reaction was completed, purified water (400 mL) and ethyl acetate (400 mL) were added, and the mixture was stirred for extraction and then left to stand for liquid separation. The organic phase was washed with an aqueous citric acid solution, washed with saturated brine, dried over anhydrous sodium sulfate, and filtered, and the filtrate was concentrated under reduced pressure to give intermediate **20-1** (3.6 g). LC-MS: m/z 284.0 (M-C(CH₃)₃+2H)⁺.

### Preparation of intermediate 20-2:

Intermediate **20-1** (1.8 g) was added to a solution of hydrogen chloride in ethyl acetate (4 M, 10 mL), and the mixture was stirred at room temperature for 3 h. After the reaction was completed, the reaction solution was concentrated under reduced pressure to give intermediate **20-2** (2.4 g).

LC-MS: m/z 240.1 (M+H)⁺.

### Preparation of compound 20-3:

Intermediate **20-2** (2.4 g), 2-bromoethyl methyl ether (736 mg), and potassium carbonate (2.92 g) were added to N,N-dimethylformamide (10 mL), and the mixture was stirred at 80 °C for 15 h under nitrogen atmosphere. After the reaction was completed, purified water (200 mL) and ethyl acetate (200 mL) were added, and the mixture was stirred for extraction and then left to stand for liquid separation. The aqueous phase was adjusted to pH 8-9 with an aqueous sodium carbonate solution, extracted with ethyl acetate (200 mL), and then left to stand for liquid separation. The resulting mixture was dried over anhydrous sodium sulfate, and filtered, and the filtrate was concentrated under reduced pressure to give intermediate **20-3** (1.7 g).

LC-MS: m/z 298.1 (M+H)⁺.

### Preparation of intermediate 20-4:

Intermediate **20-3** (1.7 g) was added to THF (50 mL). The mixture was stirred with the temperature controlled at - 20 °C, and a methylmagnesium bromide solution (3 M, 2.85 mL) was slowly added. After the dropwise addition was completed, the mixture was stirred for 4 h with the temperature controlled at -20 °C.

After the reaction was completed, an aqueous ammonium chloride solution (100 mL) was added to quench the reaction, ethyl acetate (100 mL) was added, and the mixture was extracted and then left to stand for liquid separation. The organic phase was dried over anhydrous sodium sulfate and filtered under vacuum, and the filtrate was concentrated under reduced pressure to give intermediate **20-4** (0.47 g).

LC-MS: m/z 314.18 (M+H)⁺.

### Preparation of compound 20:

Intermediate **20-4** (157 mg), intermediate **15-2** (100 mg), chloro(2-dicyclohexylphosphino-3,6-dimethoxy-2',4',6'-triisopropyl-1,1'-biphenyl)[2-(2-aminoethylphenyl)]palladium(II) (40 mg), 2-(dicyclohexylphosphino)-3,6-dimethoxy-2'-4'-6'-tri-I-propyl-11'-biphenyl (54 mg), and potassium carbonate (138 mg) were added to tert-butanol (25 mL). The mixture was stirred at 100 °C under microwave at 150 W under nitrogen atmosphere.

After the reaction was completed, ethyl acetate (100 mL) was added for dilution, and the mixture was filtered. The filtrate was concentrated under reduced pressure to give a concentrate, which was purified by preparative liquid chromatography to give compound **20.**

LC-MS: m/z 682.36 (M+H)⁺.

¹H NMR (500 MHz, DMSO-*d*₆) δ 11.25 (t, *J =* 6.0 Hz, 1H), 9.05 (dd, *J* = 4.2, 1.8 Hz, 1H), 8.57 (dd, *J* = 8.4, 1.8 Hz, 1H), 8.36 (td, *J =* 4.9, 3.1 Hz, 2H), 8.29 (s, 1H), 8.07 (dd, *J =* 8.5, 3.1 Hz, 1H), 7.73 (dd, *J =* 8.4, 4.2 Hz, 2H), 7.63-7.39 (m, 6H), 6.76 (d, *J =* 8.8 Hz, 1H), 5.32 (d, *J* = 5.1 Hz, 1H), 5.14 (q, *J =* 6.4 Hz, 1H), 4.76 (d, *J =* 6.0 Hz, 2H), 3.46 (t, *J =* 5.8 Hz, 3H), 3.10-3.02 (m, 1H), 2.97-2.77 (m, 3H), 2.76-2.65 (m, 1H), 2.22 (t, *J =* 10.2 Hz, 1H), 1.99-1.81 (m, 1H), 1.35 (d, *J =* 6.3 Hz, 3H), 1.24 (s, 2H), 0.70 (d, *J =* 6.2 Hz, 3H).

### Example 21: Preparation of Compound 21

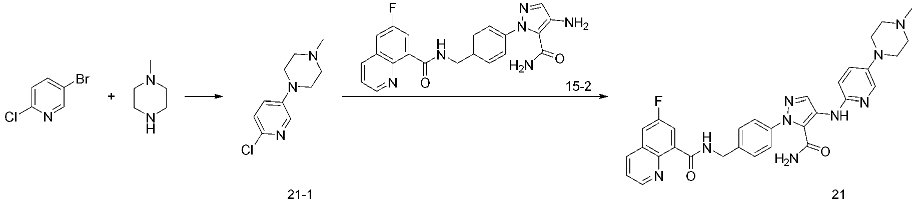

### Preparation of intermediate 21-1:

2-Chloro-5-bromopyridine (3 g), N-methylpiperazine (1.88 g), tris(dibenzylideneacetone)dipalladium (0.72 g), 4,5-bis-diphenylphosphino-9,9-dimethylxanthene (0.91 g), and cesium carbonate (15.2 g) were added to dioxane (30 mL), and the mixture was stirred at 85 °C for 16 h under nitrogen atmosphere.

After the reaction was completed, ethyl acetate (50 mL) and purified water (50 mL) were added, and the mixture was stirred for 10 min and left to stand for liquid separation. The aqueous phase was extracted with ethyl acetate (50 mL × 2). The organic phases were combined, washed with saturated brine, dried over anhydrous sodium sulfate, and filtered, and the filtrate was concentrated under reduced pressure. The residue was purified by column chromatography to give intermediate **21-1** (0.82 g).

LC-MS: m/z 212.16 (M+H)⁺.

### Preparation of compound 21:

Intermediate **21-1** (95 mg), intermediate **15-2** (100 mg), chloro(2-dicyclohexylphosphino-3,6-dimethoxy-2',4',6'-triisopropyl-1,1'-biphenyl)[2-(2-aminoethylphenyl)]palladium(II) (39.5 mg), 2-(dicyclohexylphosphino)-3,6-dimethoxy-2'-4'-6'-tri-I-propyl-11'-biphenyl (53.1 mg), and potassium bis(trimethylsilyl)amide (0.5 mL) were added to tert-butanol (10 mL). The mixture was stirred at 100 °C for 2 h under nitrogen atmosphere.

After the reaction was completed, ethyl acetate (20 mL) and purified water (20 mL) were added, and the mixture was stirred for 10 min and left to stand for liquid separation. The aqueous phase was extracted with ethyl acetate (20 mL × 2). The organic phases were combined, washed with saturated brine, dried over anhydrous sodium sulfate, and filtered, and the filtrate was concentrated under reduced pressure to give a concentrate, which was purified by preparative liquid chromatography to give compound **21.**

LC-MS: m/z 580.33 (M+H)⁺.

¹H NMR (500 MHz, DMSO-*d*₆) δ 11.24 (t, J = 6.1 Hz, 1H), 9.05 (dd, J = 4.3, 1.8 Hz, 1H), 8.57 (dd, J = 8.4, 1.8 Hz, 1H), 8.36 (dd, J = 9.6, 3.1 Hz, 1H), 8.11 (s, 1H), 8.09-8.04 (m, 2H), 7.81 (d, J = 3.0 Hz, 1H), 7.73 (dd, J = 8.4, 4.2 Hz, 1H), 7.52 (d, J = 8.3 Hz, 2H), 7.43 (d, J = 8.5 Hz, 2H), 7.32 (dd, J = 9.0, 3.0 Hz, 1H), 6.78 (d, J = 8.9 Hz, 1H), 4.76 (d, J = 6.0 Hz, 2H), 3.01 (t, J = 4.9 Hz, 4H), 2.44 (t, J = 4.9 Hz, 4H), 2.21 (s, 3H).

### Example 22: Preparation of Compound 22

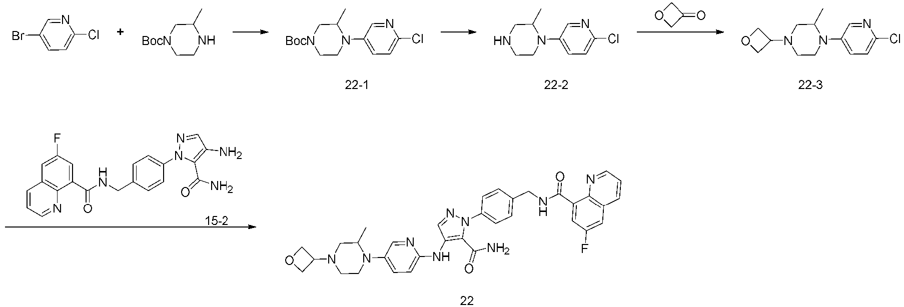

### Preparation of intermediate 22-1:

Referring to the preparation of intermediate **14-1** in Example **14,** 1-cyclopropylpiperazine was replaced with 4-tert-butoxycarbonyl-2-methylpiperazine. After the reaction was completed, ethyl acetate (30 mL) and purified water (20 mL) were added, and the mixture was stirred for 10 min and left to stand for liquid separation. The aqueous phase was extracted with ethyl acetate (30 mL × 3). The organic phases were combined, washed with saturated brine, dried over anhydrous sodium sulfate, and filtered, and the filtrate was concentrated under reduced pressure to give intermediate **22-1** (1.1 g).

LC-MS: m/z 312.15 (M+H)⁺.

### Preparation of intermediate 22-2:

Intermediate 22-1 (1.1 g) was added to ethyl acetate (10 mL), and a solution of hydrogen chloride in ethyl acetate (5 mL, 4 M) was added. The mixture was stirred at room temperature overnight. After the reaction was completed, a saturated NaHCO₃ solution was added to adjust the pH > 7, and n-butanol (30 mL) and purified water (20 mL) were added. The mixture was stirred for 10 min and then left to stand for liquid separation. The aqueous phase was extracted with n-butanol (30 mL × 3). The organic phases were combined, washed with saturated brine, dried over anhydrous sodium sulfate, and filtered, and the filtrate was concentrated under reduced pressure to give intermediate **22-2** (564 mg).

LC-MS: m/z 212.14 (M+H)⁺.

### Preparation of intermediate 22-3:

Referring to the preparation of compound **4** in Example **4,** intermediate **4-2** was replaced with intermediate **22-2,** and the reaction solution was stirred at room temperature for 12 h. After the reaction was completed, DCM (30 mL) and purified water (20 mL) were added, and the mixture was stirred for 10 min and then left to stand for liquid separation. The aqueous phase was extracted with DCM (20 mL × 3), and the organic phases were combined, washed with saturated brine, dried over anhydrous sodium sulfate, and filtered. The filtrate was concentrated under reduced pressure to give a concentrate, which was separated by preparative liquid chromatography to give intermediate **22-3** (326 mg).

LC-MS: m/z 268.14 (M+H)⁺.

### Preparation of compound 22:

Referring to the preparation steps of compound **3** in Example **3,** intermediate **3-5** was replaced with intermediate **15-2,** intermediate **1-3** was replaced with intermediate **22-3,** and cesium carbonate was replaced with lithium bis(trimethylsilyl)amide. After the reaction was completed, the reaction solution was filtered and concentrated to give a concentrate, which was separated by preparative liquid chromatography to give compound **22.**

LC-MS: m/z 636.40 (M+H)⁺.

### Example 23: Preparation of Compound 23

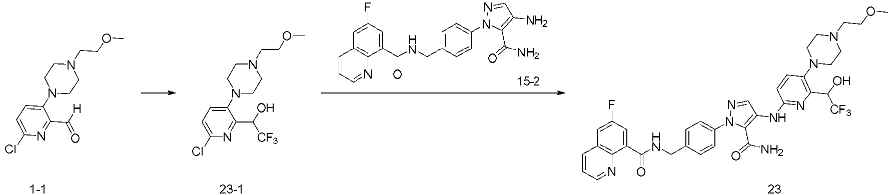

### Preparation of intermediate 23-1:

Intermediate 1-1 (2 g), cesium fluoride (1.61 g), and (trifluoromethyl)trimethylsilane (1.51 g) were added to tetrahydrofuran (30 mL), and the mixture was stirred overnight at room temperature.

After the reaction was completed, a saturated ammonium chloride solution (50 mL) and dichloromethane (50 mL) were added. The mixture was stirred for 10 min and left to stand for liquid separation. The aqueous phase was extracted with dichloromethane (50 mL × 2), and the organic phases were combined, washed with saturated brine, dried over anhydrous sodium sulfate, and filtered. The filtrate was concentrated under reduced pressure to give intermediate **23-1** (2.56 g).

LC-MS: m/z 354.15 (M+H)⁺.

### Preparation of compound 23:

Intermediate **23-1** (183 mg), intermediate **15-2** (150 mg), chloro(2-dicyclohexylphosphino-3,6-dimethoxy-2',4',6'-triisopropyl-1,1'-biphenyl)[2-(2-aminoethylphenyl)]palladium(II) (59.3 mg), 2-(dicyclohexylphosphino)-3,6-dimethoxy-2'-4'-6'-tri-I-propyl-11'-biphenyl (79.7 mg), and potassium bis(trimethylsilyl)amide (0.37 mL) were added to tert-butanol (80 mL). The mixture was stirred at 85 °C for 1 h under nitrogen atmosphere.

After the reaction was completed, ethyl acetate (20 mL) and purified water (20 mL) were added, and the mixture was stirred for 10 min and left to stand for liquid separation. The aqueous phase was extracted with ethyl acetate (20 mL × 2). The organic phases were combined, washed with saturated brine, dried over anhydrous sodium sulfate, and filtered, and the filtrate was concentrated under reduced pressure to give a concentrate, which was purified by preparative liquid chromatography to give compound **23.**

LC-MS: m/z 722.34 (M+H)⁺.

¹H NMR (500 MHz, DMSO-*d*₆) δ 11.24 (t, J = 6.0 Hz, 1H), 9.05 (dd, J = 4.3, 1.8 Hz, 1H), 8.57 (dd, J = 8.5, 1.8 Hz, 1H), 8.50 (s, 1H), 8.36 (d, J = 9.7 Hz, 2H), 8.07 (dd, J = 8.6, 3.1 Hz, 1H), 7.73 (dd, J = 8.4, 4.3 Hz, 1H), 7.69 (d, J = 8.9 Hz, 1H), 7.53 (d, J = 8.4 Hz, 2H), 7.45 (d, J = 8.5 Hz, 2H), 6.94 (d, J = 8.9 Hz, 1H), 6.81 (d, J = 7.5 Hz, 1H), 5.60 (p, J = 7.6 Hz, 1H), 4.76 (d, J = 6.0 Hz, 2H), 3.46 (t, J = 5.8 Hz, 2H), 3.25 (s, 3H), 2.89-2.83 (m, 2H), 2.74-2.68 (m, 2H), 2.53 (t, J = 5.9 Hz, 5H).

### Example 24: Preparation of Compounds 24, 24-a, and 24-b

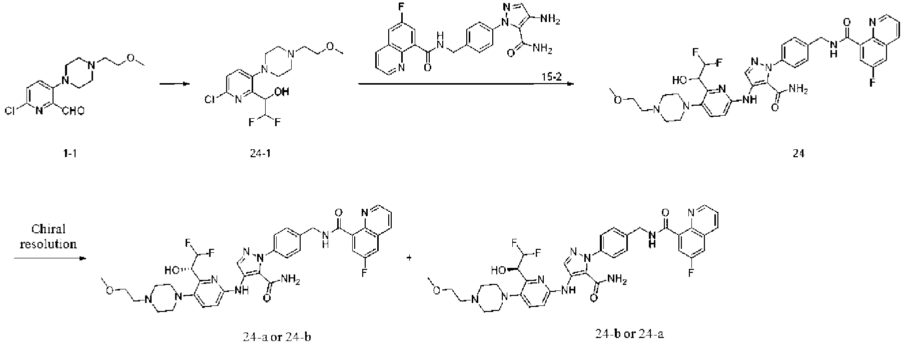

### Preparation of intermediate 24-1:

Intermediate 1-1 (917 mg) and (difluoromethyl)trimethylsilane (8.03 g) were added to tetrahydrofuran (20 mL), and tetrabutylammonium fluoride (2 mL, 1 M) was added slowly and dropwise. The mixture was reacted at room temperature for 1 h. After the reaction was completed, ethyl acetate (30 mL) and purified water (20 mL) were added, and the mixture was stirred for 10 min and left to stand for liquid separation. The aqueous phase was extracted with ethyl acetate (30 mL × 3). The organic phases were combined, washed with saturated brine, dried over anhydrous sodium sulfate, and filtered, and the filtrate was concentrated under reduced pressure to give intermediate **24-1** (700 mg). LC-MS: m/z 336.15 (M+H)⁺.

### Preparation of compound 24:

Referring to the preparation steps of compound **3** in Example **3,** intermediate **3-5** was replaced with intermediate **15-2,** intermediate **1-3** was replaced with intermediate **24-1,** and cesium carbonate was replaced with lithium bis(trimethylsilyl)amide. After the reaction was completed, the reaction solution was filtered and concentrated to give a concentrate, which was separated by preparative liquid chromatography to give compound **24.**

LC-MS: m/z 704.34 (M+H)⁺.

¹H NMR (500 MHz, DMSO-*d*₆) δ 11.24 (t, *J =* 6.0 Hz, 1H), 9.05 (dd, *J* = 4.3, 1.8 Hz, 1H), 8.57 (dd, *J* = 8.4, 1.8 Hz, 1H), 8.42-8.33 (m, 2H), 8.18 (s, 1H), 8.07 (dd, *J* = 8.5, 3.1 Hz, 1H), 7.73 (dd, *J* = 8.4, 4.2 Hz, 2H), 7.61 (d, *J=* 8.9 Hz, 1H), 7.52 (d, *J=* 8.5 Hz, 2H), 7.48-7.41 (m, 2H), 6.84 (d*, J =* 8.9 Hz, 1H), 6.23-6.04 (m, 2H), 5.13 (dd, *J* = 12.8, 6.6 Hz, 1H), 4.76 (d, *J =* 6.0 Hz, 2H), 3.46 (s, 2H), 3.25 (s, 3H), 2.89 (s, 2H), 2.73 (s, 2H), 2.07 (s, 1H).

### Preparation method for compounds 24-a and 24-b:

Compound **24** was subjected to chiral resolution to give compound **24-a** and compound **24-b.** The resolution conditions were as follows: a Cellulose-SC preparative column (30 × 250 mm, 10 µm) was used; by taking n-hexane as mobile phase A and ethanol as mobile phase B, gradient elution was performed at a flow rate of 0.8 mL/min; the detection wavelength was 254 nm, and the retention times were 36 min (24-a) and 45 min (24-b), respectively.

Compound 24-a LC-MS: m/z 704.25 (M+H)⁺.

Compound 24-b LC-MS: m/z 704.14 (M+H)⁺.

Compound 24-a had a shorter retention time in the chiral chromatography column than compound 24-b, and compound 24-b had a longer retention time in the chiral chromatography column than compound 24-a.

### Example 25: Preparation of Compound 25

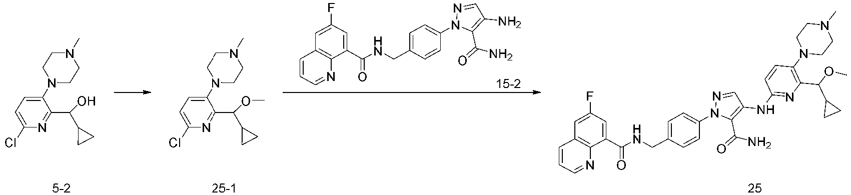

### Preparation of intermediate 25-1:

Intermediate **5-2** (0.8 g) was added to THF (20 mL), and the mixture was stirred with the temperature controlled at 0 °C. Sodium hydride (60%, 0.17 g) was slowly added, and the mixture was heated to room temperature and stirred for 1 h. Then iodomethane (0.36 g) was added, and the mixture was transferred to room temperature and stirred for 2 h.

After the reaction was completed, purified water (20 mL) was added to quench the reaction, and the reaction solution was stirred for 10 min, extracted with ethyl acetate (20 mL), and then left to stand for liquid separation. The organic phase was dried over anhydrous sodium sulfate and filtered under vacuum, and the filtrate was concentrated under reduced pressure to give intermediate **25-1.**

LC-MS: m/z 296.11 (M+H)⁺.

### Preparation of compound 25:

Intermediate **25-1** (125 mg), intermediate **15-2** (120 mg), chloro(2-dicyclohexylphosphino-3,6-dimethoxy-2',4',6'-triisopropyl-1,1'-biphenyl)[2-(2-aminoethylphenyl)]palladium(II) (47.2 mg), 2-(dicyclohexylphosphino)-3,6-dimethoxy-2'-4'-6'-tri-I-propyl-11'-biphenyl (63.5 mg), and potassium bis(trimethylsilyl)amide (0.3 mL) were added to tert-butanol (12 mL). The mixture was stirred at 85 °C for 0.5 h under nitrogen atmosphere.

After the reaction was completed, ethyl acetate (20 mL) and purified water (20 mL) were added, and the mixture was stirred for 10 min and left to stand for liquid separation. The aqueous phase was extracted with ethyl acetate (20 mL × 2). The organic phases were combined, washed with saturated brine, dried over anhydrous sodium sulfate, and filtered, and the filtrate was concentrated under reduced pressure to give a concentrate, which was purified by preparative liquid chromatography to give compound **25.**

LC-MS: m/z 664.39 (M+H)⁺.

¹H NMR (500 MHz, DMSO-*d*₆) δ 11.14 (s, 1H), 8.91 (s, 1H), 8.42 (d, J = 8.3 Hz, 1H), 8.34-8.12 (m, 3H), 7.93 (d, J = 8.5 Hz, 1H), 7.59 (s, 2H), 7.46-7.35 (m, 3H), 7.30 (d, J = 8.2 Hz, 2H), 6.67 (d, J = 8.5 Hz, 1H), 4.63 (s, 2H), 4.01 (d, J = 8.7 Hz, 1H), 3.01 (s, 3H), 2.60 (d, J *=* 45.3 Hz, 4H), 2.29 (s, 4H), 2.08 (s, 3H), 0.49 (s, 1H), 0.34 (s, 1H), 0.20 (s, 1H).

### Example 26: Preparation of Compound 26

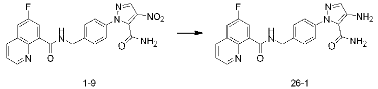

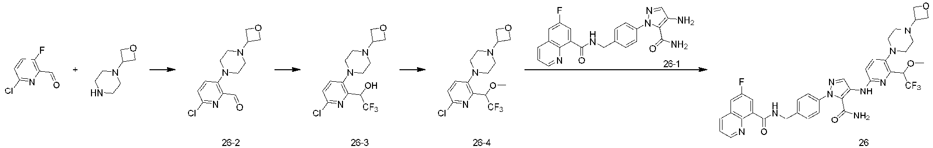

### Preparation of intermediate 26-1:

1-9 (8 g) was added to tetrahydrofuran (800 mL), and palladium on carbon (10%, 4 g) was added. The reaction system was purged three times with hydrogen and stirred at room temperature for 2 h.

After the reaction was completed, the reaction solution was filtered through celite, and the filtrate was concentrated under reduced pressure at 40 °C to give intermediate **26-1** (4.5 g).

LC-MS: m/z 405.16 (M+H)⁺.

### Preparation of intermediate 26-2:

2-Chloro-5-fluoropyridine-6-carbaldehyde (5 g) and 1-(3-oxetanyl)piperazine (4.5 g) were added to N,N-dimethylformamide (80 mL), and potassium carbonate (17.3 g) was added. The mixture was stirred with the temperature controlled at 80 °C for 6 h.

After the reaction was completed, the reaction solution was extracted with purified water (200 mL) and ethyl acetate (200 mL). The organic phase was dried over anhydrous sodium sulfate and filtered, and the filtrate was concentrated under reduced pressure to give intermediate **26-2** (6.4 g).

LC-MS: m/z 282.34 (M+H)⁺.

### Preparation of intermediate 26-3:

Referring to the preparation steps of intermediate **10-2** in Example **10,** intermediate **10-1** was replaced with intermediate **26-2,** and after the reaction was completed, intermediate **26-3** (1.1 g) was obtained.

LC-MS: m/z 352.41 (M+H)⁺.

### Preparation of intermediate 26-4:

Referring to the preparation steps of intermediate **10-3** in Example **10,** intermediate **10-2** was replaced with intermediate **26-3,** and after the reaction was completed, intermediate **26-4** (150 mg) was obtained.

LC-MS: m/z 366.22 (M+H)⁺.

### Preparation of compound 26:

Intermediate **26-4** (137 mg) and intermediate **26-1** (70 mg) were added to tert-butanol (5 mL) and 1,4-dioxane (5 mL), methanesulfonato(2-di-tert-butylphosphino-2',4',6'-triisopropyl-1,1'-biphenyl)(2'-amino-1,1'-biphenyl-2-yl)palladium(II) (28 mg), 2-di-tert-butylphosphino-2',4',6'-triisopropylbiphenyl (15 mg), and lithium bis(trimethylsilyl)amide (1 M, 0.5 mL) were added, and the mixture was stirred at 80 °C for 2 h under nitrogen atmosphere.

After the reaction was completed, ethyl acetate (100 mL) and purified water (100 mL) were added, and the mixture was stirred for 10 min and left to stand for liquid separation. The aqueous phase was extracted with ethyl acetate (50 mL × 3). The organic phases were combined, washed with saturated brine, dried over anhydrous sodium sulfate, and filtered, and the filtrate was concentrated under reduced pressure to give a concentrate, which was separated by preparative liquid chromatography to give compound **26.**

LC-MS: m/z 734.15 (M+H)⁺.

¹H NMR (500 MHz, DMSO-*d*₆) δ 11.25 (s, 1H), 9.05 (dd, J = 4.3, 1.8 Hz, 1H), 8.64-8.52 (m, 2H), 8.36 (d, J = 9.0 Hz, 2H), 8.07 (dd, J = 8.5, 3.1 Hz, 1H), 7.85-7.65 (m, 3H), 7.58-7.40 (m, 5H), 7.01 (d, J = 8.9 Hz, 1H), 5.57 (q, J = 7.3 Hz, 1H), 4.77 (d, J *=* 6.0 Hz, 2H), 4.63-4.37 (m, 4H), 3.59-3.43 (m, 1H), 2.94-2.66 (m, 4H), 2.43 (s, 3H).

### Example 27: Preparation of Compound 27

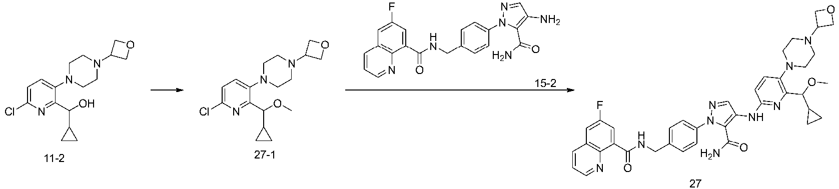

### Preparation of intermediate 27-1:

Intermediate **11-2** (2 g) was added to THF (30 mL). The mixture was stirred with the temperature controlled at 0 °C, and sodium hydride (60%, 296 mg) was slowly added. After the mixture was stirred for 20 min, iodomethane (269 µL) was added, and the mixture was transferred to room temperature and stirred for 12 h.

After the reaction was completed, purified water (50 mL) was added to quench the reaction, and the reaction system was stirred for 10 min. Ethyl acetate (100 mL) was added, and the reaction system was extracted and then left to stand for liquid separation. The organic phase was dried over anhydrous sodium sulfate and filtered under vacuum, and the filtrate was concentrated under reduced pressure to give intermediate **27-1** (1.9 g).

LC-MS: m/z 338.15 (M+H)⁺.

### Preparation of compound 27:

Intermediate **27-1** (150 mg) and intermediate **15-2** (150 mg) were added to tert-butanol (38 mL), lithium bis(trimethylsilyl)amide (1 M, 375 µL), 2-(dicyclohexylphosphino)-3,6-dimethoxy-2'-4'-6'-tri-I-propyl-11'-biphenyl (80 mg), and chloro(2-dicyclohexylphosphino-3,6-dimethoxy-2',4',6'-triisopropyl-1,1'-biphenyl)[2-(2-aminoethylphenyl)]palladium(II) (60 mg) were added, and the mixture was reacted at 85 °C for 1 h under nitrogen atmosphere.

After the reaction was completed, the reaction solution was filtered, and the filtrate was extracted with ethyl acetate (100 mL) and purified water (100 mL). The mixture was stirred for 10 min and left to stand for liquid separation. The aqueous phase was extracted with ethyl acetate (50 mL × 3), and the organic phases were combined, washed with saturated brine, dried over anhydrous sodium sulfate, and filtered. The filtrate was concentrated under reduced pressure to give a concentrate, which was separated by preparative liquid chromatography to give compound **27.**

LC-MS: m/z 706.12 (M+H)⁺.

### Example 28: Preparation of Compound 28

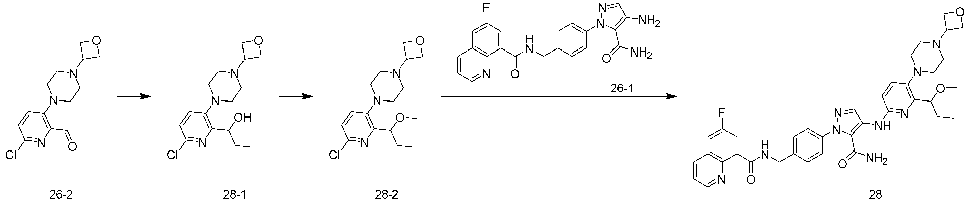

### Preparation of intermediate 28-1:

Intermediate **26-2** (1 g) was added to THF (20 mL), and ethylmagnesium bromide (3 M, 4 mL) was added dropwise with the temperature controlled at -20 °C under nitrogen atmosphere. After the dropwise addition was completed, the mixture was stirred for 3 h with the temperature controlled at 0 °C. After the reaction was completed, a saturated ammonium chloride solution (20 mL) was added to quench the reaction, and the resulting mixture was extracted with ethyl acetate (50 mL × 3). The organic phases were combined, washed with a saturated sodium chloride solution, dried over anhydrous sodium sulfate, and filtered under vacuum, and the filtrate was concentrated under reduced pressure to give intermediate **28-1** (800 mg).

LC-MS: m/z 312.24 (M+H)⁺.

### Preparation of intermediate 28-2:

Referring to the preparation steps of intermediate **10-3** in Example **10,** intermediate **10-2** was replaced with intermediate **28-1,** and after the reaction was completed, intermediate **28-2** (550 mg) was obtained.

LC-MS: m/z 326.41 (M+H)⁺.

### Preparation of compound 28:

Referring to the preparation steps of compound **26** in Example **26,** intermediate **26-4** was replaced with intermediate **28-2,** and after the reaction was completed, compound **28** was obtained.

LC-MS: m/z 694.32 (M+H)⁺.

### Example 29: Preparation of Compound 29

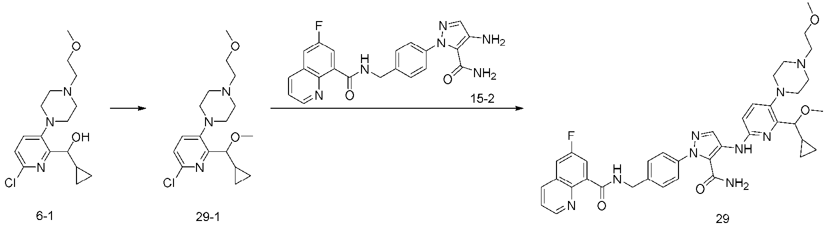

### Preparation of intermediate 29-1:

Intermediate **6-1** (1.1 g) was added to THF (30 mL), and the mixture was stirred with the temperature controlled at 0 °C. Sodium hydride (60%, 0.2 g) was slowly added, and the mixture was heated to room temperature and stirred for 1 h. Then iodomethane (0.38 g) was added, and the mixture was transferred to room temperature and stirred for 2 h.

After the reaction was completed, purified water (20 mL) was added to quench the reaction, and the reaction solution was stirred for 10 min, extracted with ethyl acetate (20 mL), and then left to stand for liquid separation. The organic phase was dried over anhydrous sodium sulfate and filtered under vacuum, and the filtrate was concentrated under reduced pressure to give intermediate **29-1.**

LC-MS: m/z 340.18(M+H)⁺.

### Preparation of compound 29:

Intermediate **29-1** (150 mg), intermediate **15-2** (120 mg), chloro(2-dicyclohexylphosphino-3,6-dimethoxy-2',4',6'-triisopropyl-1,1'-biphenyl)[2-(2-aminoethylphenyl)]palladium(II) (47.2 mg), 2-(dicyclohexylphosphino)-3,6-dimethoxy-2'-4'-6'-tri-I-propyl-11'-biphenyl (63.5 mg), and potassium bis(trimethylsilyl)amide (0.3 mL) were added to tert-butanol (12 mL). The mixture was stirred at 85 °C for 0.5 h under nitrogen atmosphere.

After the reaction was completed, ethyl acetate (20 mL) and purified water (20 mL) were added, and the mixture was stirred for 10 min and left to stand for liquid separation. The aqueous phase was extracted with ethyl acetate (20 mL × 2). The organic phases were combined, washed with saturated brine, dried over anhydrous sodium sulfate, and filtered, and the filtrate was concentrated under reduced pressure to give a concentrate, which was purified by preparative liquid chromatography to give compound **29.**

LC-MS: m/z 708.44(M+H)⁺.

¹H NMR (500 MHz, DMSO-*d*₆) δ 11.12 (t, *J =* 6.1 Hz, 1H), 8.90 (d, *J =* 4.3 Hz, 1H), 8.41 (d, *J* = 8.4 Hz, 1H), 8.21 (d, *J =* 12.7 Hz, 3H), 7.91 (dd, *J* = 8.4, 3.2 Hz, 1H), 7.58 (dd, *J* = 8.4, 4.3 Hz, 1H), 7.42 (s, 1H), 7.41-7.37 (m, 2H), 7.31 (d*, J =* 8.1 Hz, 2H), 6.66 (d*, J =* 8.7 Hz, 1H), 4.63 (d*, J =* 6.0 Hz, 2H), 4.01 (d, *J =* 8.8 Hz, 1H), 3.30 (t, *J* = 5.8 Hz, 2H), 3.10 (s, 3H), 3.01 (s, 3H), 2.64 (q, *J =* 5.3 Hz, 2H), 2.59-2.51 (m, 2H), 1.27-1.17 (m, 1H), 1.10 (d, *J =* 17.2 Hz, 1H), 0.48 (dd, *J* = 9.4, 5.0 Hz, 1H), 0.33 (dd, *J* = 9.6, 4.9 Hz, 1H), 0.19 (dt, *J* = 9.3, 4.5 Hz, 1H).

### Example 30: Preparation of Compound 30

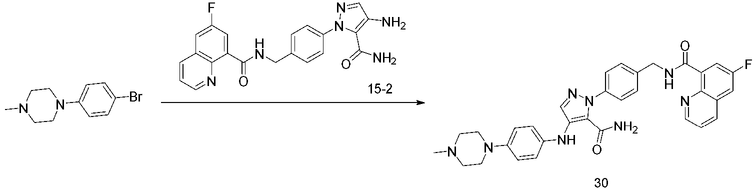

### Preparation of compound 30:

1-(4-Bromophenyl)-4-methylpiperazine (63 mg), intermediate **15-2** (100 mg), methanesulfonato(2-dicyclohexylphosphino-2',4',6'-triisopropyl-1,1'-biphenyl)(2'-amino-1,1'-biphenyl-2-yl)palladium(II) (41 mg), cesium carbonate (160 mg), and 1,4-dioxane (10 mL) were added. The mixture was reacted at 80 °C for 3 h under nitrogen atmosphere. After the reaction was completed, the reaction solution was filtered and concentrated to give a concentrate, which was separated by preparative liquid chromatography to give compound **30.**

LC-MS: m/z 579.28 (M+H)⁺.

### Example 31: Preparation of Compound 31

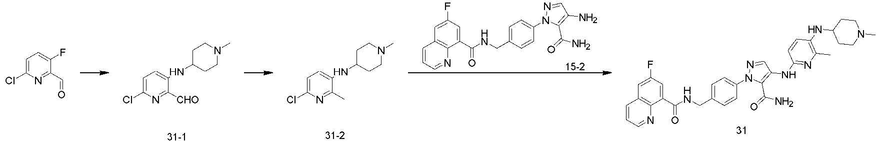

### Preparation of intermediate 31-1:

2-Chloro-5-fluoropyridine-6-carbaldehyde (1.6 g), 4-amino-1-methylpiperidine (1.14 g), and potassium carbonate (4.2 g) were added to DMF (40 mL), and the mixture was stirred at 80 °C for 2 h, and then cooled to room temperature. Ethyl acetate (40 mL) and purified water (30 mL) were added, and the mixture was stirred for 10 min and left to stand for liquid separation. The aqueous phase was extracted with ethyl acetate (40 mL × 2), the organic phases were combined, washed with saturated brine, dried over anhydrous sodium sulfate, and filtered, and the filtrate was concentrated under reduced pressure to give intermediate **31-1** (2.1 g).

LC-MS: m/z 254.21 (M+H)⁺.

### Preparation of intermediate 31-2:

Intermediate **31-1** (2.1 g), hydrazine hydrate (85%, 1.8 g), KOH (1.9 g), and ethylene glycol (30 mL) were added to a reaction flask, and the reaction system was purged three times with nitrogen and stirred at 140 °C for 2 h.

After the reaction was completed, the reaction solution was cooled to room temperature, and ethyl acetate (30 mL) and purified water (30 mL) were added. The resulting mixture was stirred for 10 min and then left to stand for liquid separation, and the aqueous phase was extracted with ethyl acetate (30 mL × 2). The organic phases were combined, washed with saturated brine, dried over anhydrous sodium sulfate, and filtered, and the filtrate was concentrated under reduced pressure and purified by column chromatography (dichloromethane/methanol = 10/1) to give intermediate **31-2** (1.3 g).

LC-MS: m/z 240.27 (M+H)⁺.

### Preparation of compound 31:

Intermediate **31-2** (86 mg) and intermediate **15-2** (100 mg) were added to tert-butanol (20 mL), methanesulfonato(2-di-tert-butylphosphino-2',4',6'-triisopropyl-1,1'-biphenyl)(2'-amino-1,1'-biphenyl-2-yl)palladium(II) (69 mg), 2-di-tert-butylphosphino-2',4',6'-triisopropylbiphenyl (79 mg), and potassium carbonate (150 mg) were added, and the mixture was stirred at 80 °C for 3 h under nitrogen atmosphere.

After the reaction was completed, ethyl acetate (30 mL) and purified water (30 mL) were added, and the mixture was stirred for 10 min and then left to stand for liquid separation. The aqueous phase was extracted with ethyl acetate (30 mL × 3). The organic phases were combined, washed with saturated brine, dried over anhydrous sodium sulfate, and filtered, and the filtrate was concentrated under reduced pressure to give a concentrate, which was separated by preparative liquid chromatography to give compound **31.**

LC-MS: m/z 608.37 (M+H)⁺.

### Example 32: Preparation of Compound 32

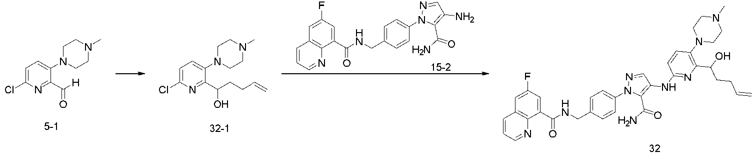

### Preparation of intermediate 32-1:

Intermediate **5-1** (1.2 g) was added to THF (24 mL), and the mixture was stirred with the temperature controlled at 0 °C. A solution of 3-butenylmagnesium bromide in tetrahydrofuran (1 M, 6 mL) was slowly added. After the dropwise addition was completed, the mixture was transferred to room temperature and stirred overnight.

After the reaction was completed, the reaction solution was transferred to an ice-water bath at 0 °C for cooling, and a saturated ammonium chloride solution (50 mL) was added slowly and dropwise to quench the reaction. The mixture was stirred for 10 min with the temperature maintained. DCM (100 mL) was added, and the reaction system was extracted and then left to stand for liquid separation. The organic phase was dried over anhydrous sodium sulfate and filtered under vacuum, and the filtrate was concentrated under reduced pressure and subjected to column chromatography to give intermediate **32-1** (550 mg).

LC-MS: m/z 296.21 (M+H)⁺.

### Preparation of compound 32:

Intermediate **5-1** (120 mg) and intermediate **15-2** (120 mg) were added to tert-butanol (30 mL), lithium bis(trimethylsilyl)amide (1 M, 300 µL), 2-(dicyclohexylphosphino)-3,6-dimethoxy-2'-4'-6'-tri-I-propyl-11'-biphenyl (64 mg), and chloro(2-dicyclohexylphosphino-3,6-dimethoxy-2',4',6'-triisopropyl-1,1'-biphenyl)[2-(2-aminoethylphenyl)]palladium(II) (48 mg) were added, and the mixture was reacted at 85 °C for 1 h under nitrogen atmosphere.

After the reaction was completed, the reaction solution was filtered, and the filtrate was extracted with ethyl acetate (100 mL) and purified water (100 mL). The mixture was stirred for 10 min and left to stand for liquid separation. The aqueous phase was extracted with ethyl acetate (50 mL × 3), and the organic phases were combined, washed with saturated brine, dried over anhydrous sodium sulfate, and filtered. The filtrate was concentrated under reduced pressure to give a concentrate, which was separated by preparative liquid chromatography to give compound **32.**

LC-MS: m/z 664.17 (M+H)⁺.

¹H NMR (500 MHz, DMSO-*d*₆) δ 11.25 (s, 1H), 9.10-8.88 (m, 2H), 8.56 (dd, *J =* 8.4, 1.8 Hz, 1H), 8.40-8.20 (m, 5H), 8.06 (dd, *J* = 8.5, 3.1 Hz, 2H), 7.72 (dd, *J* = 8.4, 4.2 Hz, 2H), 6.74 *(d, J =* 8.7 Hz, 2H), 5.22-4.86 (m, 5H), 4.76 (d, *J =* 6.0 Hz, 4H), 2.88-2.67 (m, 4H), 2.43 (s, 3H), 2.05 (dd, *J* = 15.1, 7.8 Hz, 2H).

### Example 33: Preparation of Compound 33

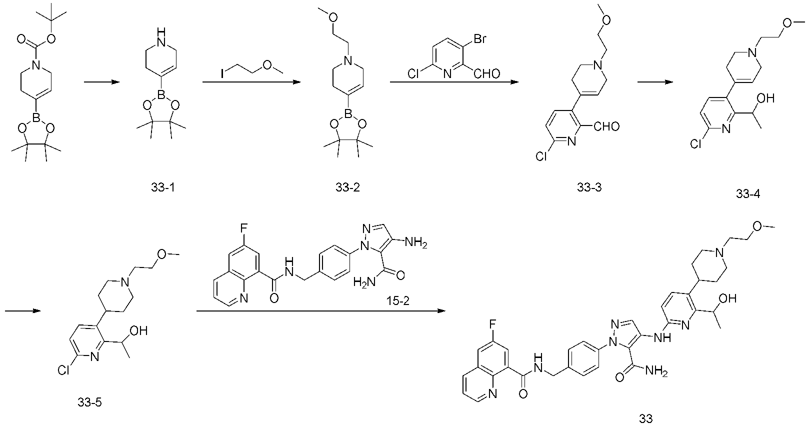

### Preparation of intermediate 33-1:

N-Boc-1,2,5,6-tetrahydropyridine-4-boronic acid pinacol ester (7 g) and a hydrochloric acid-ethyl acetate solution (50 mL) were added to ethyl acetate (15 mL), and the mixture was stirred at room temperature for 1 h.

After the reaction was completed, the reaction mixture was directly concentrated to dryness to give intermediate **33-1.**

LC-MS: m/z 210.18 (M+H)⁺.

### Preparation of intermediate 33-2:

Intermediate **33-1** (5.3 g), 1-iodo-2-methoxyethane (5 g), and triethylamine (6.9 g) were added to acetonitrile (70 mL), and the mixture was stirred at room temperature overnight.

After the reaction was completed, ethyl acetate (50 mL) and purified water (50 mL) were added, and the mixture was stirred for 10 min and left to stand for liquid separation. The aqueous phase was extracted with ethyl acetate (50 mL × 2). The organic phases were combined, washed with saturated brine, dried over anhydrous sodium sulfate, and filtered, and the filtrate was concentrated under reduced pressure to give intermediate **33-2.**

LC-MS: m/z 268.18 (M+H)⁺.

### Preparation of intermediate 33-3:

Intermediate **33-2** (8.2 g), 3-bromo-6-chloropyridine-2-carbaldehyde (4.5 g), DPPF palladium dichloride (1.5 g), and potassium carbonate (9.5 g) were added to dioxane (150 mL) and purified water (30 mL), and the mixture was reacted at 80 °C for 1 h.

After the reaction was completed, ethyl acetate (50 mL) and purified water (50 mL) were added, and the mixture was stirred for 10 min and left to stand for liquid separation. The aqueous phase was extracted with ethyl acetate (50 mL × 2). The organic phases were combined, washed with saturated brine, dried over anhydrous sodium sulfate, and filtered, and the filtrate was concentrated under reduced pressure to give intermediate **33-3.**

LC-MS: m/z 281.10(M+H)⁺.

### Preparation of intermediate 33-4:

Referring to the preparation steps of compound **7-1** in Example **7,** intermediate **5-1** was replaced with intermediate **33-3,** and after the reaction was completed, intermediate **33-4** was obtained.

LC-MS: m/z 297.14 (M+H)⁺.

### Preparation of intermediate 33-5:

Intermediate **33-4** (5.14 g) and platinum dioxide (1.67 g) were added to methanol (100 mL). The reaction system was purged 3 times with hydrogen and reacted at room temperature overnight.

After the reaction was completed, the reaction solution was filtered, and the filtrate was concentrated under reduced pressure to give intermediate **33-5.**

LC-MS: m/z 299.14 (M+H)⁺.

### Preparation of compound 33:

Intermediate **33-5** (114 mg), intermediate **15-2** (120 mg), chloro(2-dicyclohexylphosphino-3,6-dimethoxy-2',4',6'-triisopropyl-1,1'-biphenyl)[2-(2-aminoethylphenyl)]palladium(II) (47.2 mg), 2-(dicyclohexylphosphino)-3,6-dimethoxy-2'-4'-6'-tri-I-propyl-11'-biphenyl (63.5 mg), and potassium bis(trimethylsilyl)amide (0.3 mL) were added to tert-butanol (15 mL). The mixture was stirred at 85 °C for 0.5 h under nitrogen atmosphere.

After the reaction was completed, ethyl acetate (20 mL) and purified water (20 mL) were added, and the mixture was stirred for 10 min and left to stand for liquid separation. The aqueous phase was extracted with ethyl acetate (20 mL × 2). The organic phases were combined, washed with saturated brine, dried over anhydrous sodium sulfate, and filtered, and the filtrate was concentrated under reduced pressure to give a concentrate, which was purified by preparative liquid chromatography to give compound **33.**

LC-MS: m/z 667.40 (M+H)⁺.

¹H NMR (500 MHz, DMSO-*d*₆) δ 11.27 (s, 1H), 9.05 (s, 1H), 8.57 (s, 1H), 8.37 (s, 1H), 8.27 (d, *J =* 25.0 Hz, 2H), 8.07 (s, 1H), 7.73 (s, 2H), 7.51 (d, *J* = 32.2 Hz, 6H), 6.73 (s, 1H), 4.93 (d, *J =* 30.9 Hz, 2H), 4.84-4.73 (m, 2H), 3.45 (s, 3H), 3.25 (s, 3H), 2.97 (s, 2H), 2.79 (s, 1H), 2.07 (s, 2H), 1.60 (d, *J* = 27.0 Hz, 3H), 1.44-1.33 (m, 3H).

### Example 34: Preparation of Compound 34

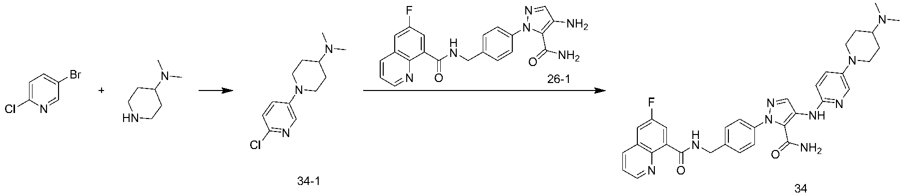

### Preparation of intermediate 34-1:

5-Bromo-2-chloropyridine (2 g), 4-dimethylaminopiperidine (1.4 g), tris(dibenzylideneacetone)dipalladium (952 mg), 4,5-bis-diphenylphosphino-9,9-dimethylxanthene (902 mg), and cesium carbonate (6.8 g) were added to 1,4-dioxane (50 mL). The mixture was purged three times with nitrogen and stirred at 85 °C for 4 h.

After the reaction was completed, ethyl acetate (100 mL) and purified water (100 mL) were added. The mixture was stirred for 10 min and then left to stand for liquid separation. The aqueous phase was extracted with ethyl acetate (50 mL × 3), and the organic phases were combined, washed with saturated brine, dried over anhydrous sodium sulfate, and filtered. The filtrate was concentrated under reduced pressure to give intermediate **34-1** (3.5 g). LC-MS: m/z 240.23 (M+H)⁺.

### Preparation of compound 34:

Referring to the preparation steps of compound **26** in Example **26,** intermediate **26-4** was replaced with intermediate **34-1,** and after the reaction was completed, compound **34** was obtained.

LC-MS: m/z 608.41 (M+H)⁺.

### Example 35: Preparation of Compound 35

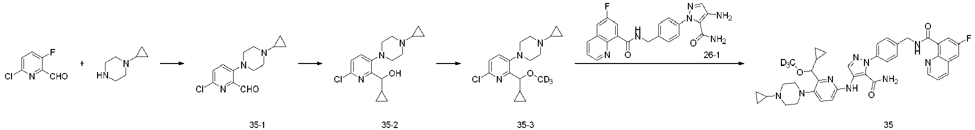

### Preparation of intermediate 35-1:

Referring to the preparation steps of intermediate **5-1** in Example **5,** N-methylpiperazine was replaced with 1-cyclopropylpiperazine. After the reaction was completed, the reaction solution was filtered, and the filtrate was extracted with purified water (100 mL) and ethyl acetate (100 mL). The organic phase was dried over anhydrous sodium sulfate and filtered, and the filtrate was concentrated under reduced pressure to give intermediate **35-1** (4.37 g). LC-MS: m/z 266.15 (M+H)⁺.

### Preparation of intermediate 35-2:

Referring to the preparation steps of intermediate **1-2** in Example **1,** intermediate **1-1** was replaced with intermediate **35-1,** and a methylmagnesium bromide solution was replaced with a cyclopropylmagnesium bromide solution. After the reaction was completed, purified water (150 mL) was added to quench the reaction, and the reaction solution was stirred for 10 min. Ethyl acetate (200 mL) was added, and the reaction system was extracted and then left to stand for liquid separation. The organic phase was dried over anhydrous sodium sulfate and filtered under vacuum, and the filtrate was concentrated under reduced pressure to give intermediate **35-2** (4 g).

LC-MS: m/z 308.18 (M+H)⁺.

### Preparation of intermediate 35-3:

Referring to the preparation steps of intermediate **1-3** in Example **1,** intermediate **1-2** was replaced with intermediate **35-2,** and iodomethane was replaced with deuterated iodomethane. After the reaction was completed, purified water (150 mL) was added to quench the reaction, and the reaction solution was stirred for 10 min. Ethyl acetate (200 mL) was added, and the reaction system was extracted and then left to stand for liquid separation. The organic phase was dried over anhydrous sodium sulfate and filtered under vacuum, and the filtrate was concentrated under reduced pressure to give intermediate **35-3** (312 mg).

LC-MS: m/z 325.25 (M+H)⁺.

### Preparation of compound 35:

Referring to the preparation steps of compound **3** in Example **3,** intermediate **3-5** was replaced with intermediate **26-1,** intermediate **1-3** was replaced with intermediate **35-3,** and cesium carbonate was replaced with potassium carbonate. After the reaction was completed, the reaction solution was filtered and concentrated to give a concentrate, which was separated by preparative liquid chromatography to give compound **35.**

LC-MS: m/z 693.50 (M+H)⁺.

### Example 36: Preparation of Compound 36

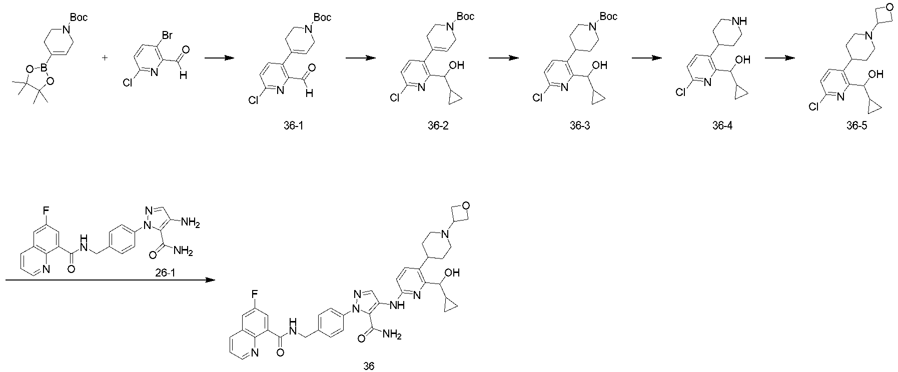

### Preparation of intermediate 36-1:

N-tert-butoxycarbonyl-1,2,5,6-tetrahydropyridine-4-boronic acid pinacol ester (500 mg), 2-chloro-5-bromopyridine-6-carbaldehyde (710 mg), [1,1'-bis(diphenylphosphino)ferrocene]palladium dichloride (120 mg), and potassium carbonate (1.2 g) were added to 1,4-dioxane (10 mL) and purified water (2 mL), and the mixture was purged three times with nitrogen and stirred at 95 °C for 4 h.

After the reaction was completed, ethyl acetate (100 mL) and purified water (100 mL) were added. The mixture was stirred for 10 min and left to stand for liquid separation. The aqueous phase was extracted with ethyl acetate (50 mL × 3), and the organic phases were combined, washed with saturated brine, dried over anhydrous sodium sulfate, and filtered. The filtrate was concentrated under reduced pressure to give intermediate **36-1** (600 mg).

LC-MS: m/z 267.15 (M-C(CH₃)₃+2H)⁺.

### Preparation of intermediate 36-2:

Referring to the preparation steps of intermediate **6-1** in Example **6,** intermediate **1-1** was replaced with intermediate **36-1,** and after the reaction was completed, intermediate **36-2** (800 mg) was obtained.

LC-MS: m/z 365.21 (M+H)⁺.

### Preparation of intermediate 36-3:

Intermediate **36-2** (800 mg) was added to methanol (15 mL), and platinum dioxide (500 mg) was added. The mixture was purged three times with hydrogen and stirred at room temperature for 12 h.

After the reaction was completed, the reaction solution was filtered through celite, and the filtrate was concentrated under reduced pressure at 40 °C to give intermediate **36-3** (1 g).

LC-MS: m/z 367.17 (M+H)⁺.

### Preparation of intermediate 36-4:

Intermediate **36-3** (1 g) was added to dichloromethane (10 mL), and trifluoroacetic acid (1 mL) was added. The mixture was reacted at room temperature for 2 h.

After the reaction was completed, the reaction solution was added to water, and an aqueous sodium bicarbonate solution (1 M, 30 mL) was added to adjust the pH to about 7-8. The reaction solution was extracted with dichloromethane (40 mL). The organic phase was dried over anhydrous sodium sulfate and filtered, and the filtrate was concentrated under reduced pressure to give intermediate **36-4** (100 mg).

LC-MS: m/z 267.14 (M+H)⁺.

### Preparation of intermediate 36-5:

Intermediate **36-4** (100 mg) and 3-oxetanone (34 mg) were dissolved in methanol (4 mL) and dichloromethane (4 mL), and sodium triacetoxyborohydride (159 mg) was added. The mixture was reacted at room temperature for 12 h.

After the reaction was completed, ethyl acetate (50 mL) and purified water (50 mL) were added to the reaction solution, and the mixture was stirred for 10 min and left to stand for liquid separation. The aqueous phase was extracted with ethyl acetate (50 mL × 3). The organic phases were combined, washed with saturated brine, dried over anhydrous sodium sulfate, and filtered, and the filtrate was concentrated under reduced pressure to give intermediate **36-5.**

LC-MS: m/z 323.13 (M+H)⁺.

### Preparation of compound 36:

Referring to the preparation steps of compound **26** in Example **26,** intermediate **26-4** was replaced with intermediate **36-5,** and after the reaction was completed, compound **36** was obtained.

LC-MS: m/z 691.25 (M+H)⁺.

### Example 37: Preparation of Compounds 37-a and 37-b

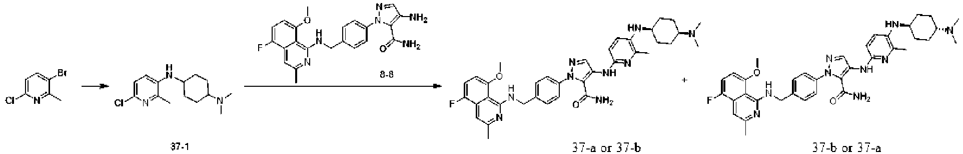

### Preparation of intermediate 37-1:

2-Methyl-3-bromo-5-chloropyridine (2.27 g), 4-dimethylaminocyclohexylamine hydrochloride (2.52 g), tris(dibenzylideneacetone)dipalladium (850 mg), 4,5-bis-diphenylphosphino-9,9-dimethylxanthene (1.18 g), sodium tert-butoxide (5.1 g), and toluene (180 mL) were added to a reaction flask. The mixture was purged three times with nitrogen and stirred at 100 °C for 2 h.

After the reaction was completed, the reaction solution was cooled to room temperature, and ethyl acetate (80 mL) and purified water (60 mL) were added. The resulting mixture was stirred for 10 min and left to stand for liquid separation, and the aqueous phase was extracted with ethyl acetate (580 mL × 2). The organic phases were combined, washed with saturated brine, dried over anhydrous sodium sulfate, and filtered, and the filtrate was concentrated under reduced pressure and purified by column chromatography (dichloromethane/methanol = 20/1) to give intermediate **37-1** (1.4 g).

LC-MS: m/z 268.29 (M+H)⁺.

### Preparation of compounds 37-a and 37-b:

Intermediate **37-1** (170 mg) and intermediate **8-8** (260 mg) were added to tert-butanol (40 mL), methanesulfonato(2-di-tert-butylphosphino-2',4',6'-triisopropyl-1,1'-biphenyl)(2'-amino-1,1'-biphenyl-2-yl)palladium(II) (160 mg), 2-di-tert-butylphosphino-2',4',6'-triisopropylbiphenyl (220 mg), and lithium bis(trimethylsilyl)amide (1 M, 770 µL) were added, and the mixture was stirred at 80 °C for 3 h under nitrogen atmosphere.

After the reaction was completed, ethyl acetate (30 mL) and purified water (30 mL) were added, and the mixture was stirred for 10 min and then left to stand for liquid separation. The aqueous phase was extracted with ethyl acetate (30 mL × 3). The organic phases were combined, washed with saturated brine, dried over anhydrous sodium sulfate, and filtered, and the filtrate was concentrated under reduced pressure to give a concentrate, which was separated by preparative liquid chromatography to give compounds **37-a** and **37-b.** The preparative conditions were as follows: a Chiral AM-4 chromatography column (20 × 250 mm, 10 µm) was used; gradient elution was performed with 0.2 diethylamine-methanol-25% dichloromethane-n-hexane (5%-50%/0-40 min) at a flow rate of 25 mL/min; the detection wavelength was 254 nm; compounds **37-a** (retention time: 15.2 min) and **37-b** (retention time: 17.1 min) were obtained in sequence.

Compound **37-a** LC-MS: m/z 652.41 (M+H)⁺.

Compound **37-b** LC-MS: m/z 652.39 (M+H)⁺.

Compound **37-b** ¹H NMR (500 MHz, DMSO-*d*₆) δ 8.02 (s, 1H), 7.51 (d, *J =* 8.5 Hz, 3H), 7.45 (d, *J =* 8.4 Hz, 2H), 7.24 (s, 2H), 7.14 (s, 2H), 7.04 (s, 2H), 6.85 (s, 1H), 6.75 (d, *J* = 9.0 Hz, 1H), 4.93 (s, 2H), 2.76 (d, *J* = 4.7 Hz, 7H), 2.40 (d, *J* = 6.2 Hz, 6H), 1.90 (d, *J =* 13.6 Hz, 2H), 1.78 (t, *J =* 11.8 Hz, 5H), 1.67-1.53 (m, 3H), 1.24 (s, 2H). Compound 37-a had a shorter retention time in the chiral chromatography column than compound 37-b, and compound 37-b had a longer retention time in the chiral chromatography column than compound 37-a.

### Example 38: Preparation of Compound 38

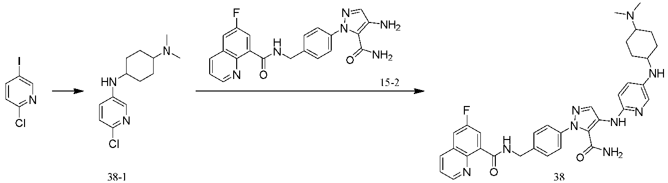

### Preparation of intermediate 38-1:

2-Chloro-5-iodopyridine (1 g), N,N-dimethyl-1,4-cyclohexanediamine (1.4 g), tris(dibenzylideneacetone)dipalladium (193 mg), 4,5-bis-diphenylphosphino-9,9-dimethylxanthene (243 mg), and a sodium tert-butoxide solution (1 M, 21 mL) were added to dioxane (45 mL), and the mixture was stirred at 85 °C for 5 h under nitrogen atmosphere.

After the reaction was completed, the reaction solution was filtered through celite, and purified water (50 mL) and ethyl acetate (50 mL) were added to the filtrate. The mixture was stirred for 10 min and then left to stand for liquid separation. The aqueous phase was extracted with ethyl acetate (30 mL × 2). The organic phases were combined, washed with saturated brine, dried over anhydrous sodium sulfate, and filtered under vacuum, and the filtrate was concentrated under reduced pressure to give intermediate **38-1** (940 mg).

LC-MS: m/z 254.21 (M+H)⁺.

### Preparation of compound 38:

Referring to the preparation steps of compound **9** in Example **9,** intermediate **8-8** was replaced with intermediate **15-2,** and intermediate **9-2** was replaced with intermediate **38-1;** after the reaction was completed, the system was separated by preparative liquid chromatography to give compound **38.**

LC-MS: m/z 622.44 (M+H)⁺.

¹H NMR (500 MHz, DMSO-*d*₆) δ 11.24 (t, *J =* 6.0 Hz, 1H), 9.04 (dd, *J* = 4.3, 1.9 Hz, 1H), 8.57 (dd, *J* = 8.4, 1.9 Hz, 1H), 8.36 (dd, *J* = 9.6, 3.1 Hz, 1H), 8.23 (s, 1H), 8.09-8.05 (m, 1H), 7.83 (d, *J =* 12.7 Hz, 1H), 7.73 (dd, *J* = 8.4, 4.2 Hz, 1H), 7.59-7.49 (m, 3H), 7.45-7.37 (m, 2H), 6.96 (dd, *J =* 8.9, 2.9 Hz, 1H), 6.69 (t, *J =* 8.6 Hz, 1H), 4.76 (d, *J =* 6.1 Hz, 2H), 2.23 (d, *J =* 7.6 Hz, 6H), 2.04-1.96 (m, 2H), 1.83 (d, *J =* 12.6 Hz, 2H), 1.46 (s, 1H), 1.34-1.27 (m, 2H), 1.24 (d, *J* = 5.8 Hz, 2H), 1.10 (d, *J =* 12.4 Hz, 1H).

### Example 39: Preparation of Compound 39

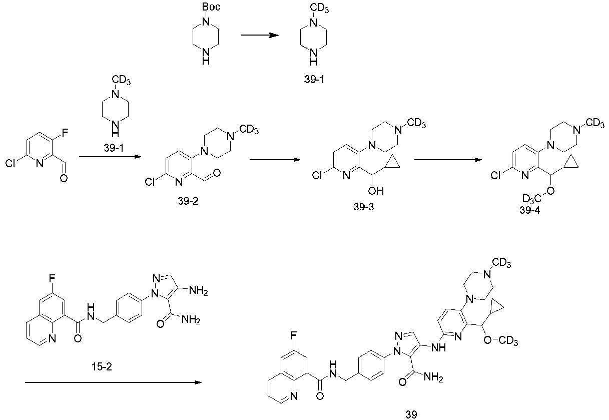

### Preparation of intermediate 39-1:

N-tert-butoxycarbonyl-piperazine (10 g) was added to THF (200 mL), and deuterated lithium aluminum hydride (4.5 g) was added with the temperature controlled at 0 °C under nitrogen atmosphere. The mixture was stirred overnight at room temperature. After the reaction was completed in an ice bath, sodium sulfate decahydrate (21 g) was added to quench the reaction, and the mixture was filtered under vacuum. The filtrate was concentrated under reduced pressure to give intermediate **39-1** (3.58 g).

LC-MS: m/z 104.13 (M+H)⁺.

### Preparation of intermediate 39-2:

2-Chloro-5-fluoropyridine-6-carbaldehyde (5.54 g), **39-1** (3.58 g), and potassium carbonate (14.4 g) were added to N,N-dimethylformamide (55 mL), and the mixture was stirred with the temperature controlled at 80 °C for 2 h. After the reaction was completed, the reaction solution was filtered, and the filtrate was extracted with purified water (100 mL) and ethyl acetate (100 mL). The organic phase was dried over anhydrous sodium sulfate and filtered, and the filtrate was concentrated under reduced pressure to give intermediate **39-2** (6 g).

LC-MS: m/z 243.14 (M+H)⁺.

### Preparation of compound 39-3:

Intermediate **39-2** (6 g) was added to THF (40 mL). The mixture was stirred with the temperature controlled at - 20 °C under nitrogen atmosphere, and a solution of cyclopropylmagnesium bromide (1 M, 30 mL) was added slowly and dropwise. After the dropwise addition was completed, the reaction system was slowly heated to 0 °C and stirred for 3 h.

After the reaction was completed, purified water (100 mL) was added to quench the reaction, and the reaction system was stirred for 10 min. Ethyl acetate (100 mL) was added, and the reaction system was extracted and then left to stand for liquid separation. The organic phase was dried over anhydrous sodium sulfate and filtered under vacuum, and the filtrate was concentrated under reduced pressure to give intermediate **39-3** (6.2 g).

LC-MS: m/z 285.18 (M+H)⁺.

### Preparation of intermediate 39-4:

Intermediate **39-3** (6.2 g) was added to THF (100 mL), and the mixture was stirred with the temperature controlled at 0 °C. Sodium hydride (60%, 980 mg) was slowly added, and after the mixture was stirred for 20 min, deuterated iodomethane (3.4 g) was added, and the mixture was transferred to room temperature and stirred for 4 h.

After the reaction was completed, purified water (100 mL) was added to quench the reaction, and the reaction system was stirred for 10 min. Ethyl acetate (150 mL) was added, and the reaction system was extracted and then left to stand for liquid separation. The organic phase was dried over anhydrous sodium sulfate and filtered under vacuum, and the filtrate was concentrated under reduced pressure to give intermediate **39-4** (5.8 g).

LC-MS: m/z 302.17 (M+H)⁺.

### Preparation of compound 39:

Intermediate **39-4** (90 mg) and intermediate **15-2** (92 mg) were added to tert-butanol (6 mL), methanesulfonato(2-di-tert-butylphosphino-2',4',6'-triisopropyl-1,1'-biphenyl)(2'-amino-1,1'-biphenyl-2-yl)palladium(II) (39 mg), 2-di-tert-butylphosphino-2',4',6'-triisopropylbiphenyl (51 mg), and lithium bis(trimethylsilyl)amide (504 mg) were added, and the mixture was stirred at 85 °C for 2 h under nitrogen atmosphere. After the reaction was completed, ethyl acetate (100 mL) and purified water (1 M, 80 µL) were added, and the mixture was stirred for 10 min and left to stand for liquid separation. The aqueous phase was extracted with ethyl acetate (10 mL × 3), and the organic phases were combined, washed with saturated brine, dried over anhydrous sodium sulfate, and filtered. The filtrate was concentrated under reduced pressure to give a concentrate, which was separated by preparative liquid chromatography to give compound **39.**

LC-MS: m/z 670.41 (M+H)⁺.

### Example 40: Preparation of Compound 40

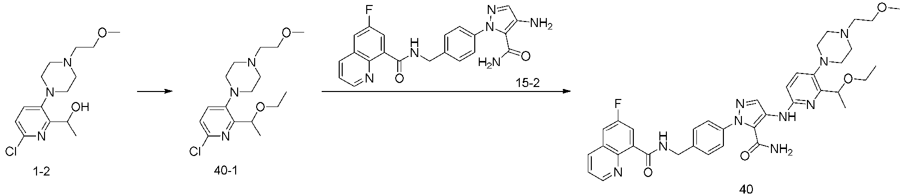

### Preparation of intermediate 40-1:

Referring to the preparation steps of compound **1-3** in Example **1,** iodomethane was replaced with iodoethane to give intermediate **40-1.**

LC-MS: m/z 328.24 (M+H)⁺.

### Preparation of compound 40:

Intermediate **40-1** (218 mg), intermediate **15-2** (150 mg), chloro(2-dicyclohexylphosphino-3,6-dimethoxy-2',4',6'-triisopropyl-1,1'-biphenyl)[2-(2-aminoethylphenyl)]palladium(II) (59.3 mg), 2-(dicyclohexylphosphino)-3,6-dimethoxy-2'-4'-6'-tri-I-propyl-11'-biphenyl (63.5 mg), and potassium bis(trimethylsilyl)amide (0.3 mL) were added to tert-butanol (15 mL). The mixture was stirred at 85 °C for 0.5 h under nitrogen atmosphere.

After the reaction was completed, ethyl acetate (20 mL) and purified water (20 mL) were added, and the mixture was stirred for 10 min and left to stand for liquid separation. The aqueous phase was extracted with ethyl acetate (20 mL × 2). The organic phases were combined, washed with saturated brine, dried over anhydrous sodium sulfate, and filtered, and the filtrate was concentrated under reduced pressure to give a concentrate, which was purified by preparative liquid chromatography to give compound **40.**

LC-MS: m/z 696.48 (M+H)⁺.

### Example 41: Preparation of Compound 41

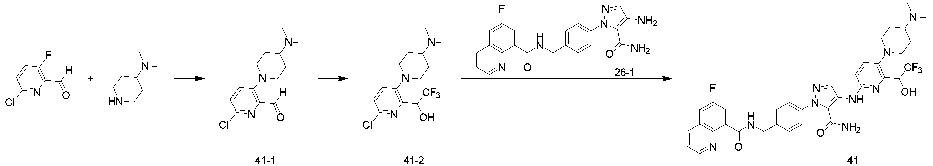

### Preparation of intermediate 41-1:

2-Chloro-5-fluoropyridine-6-carbaldehyde (1 g) and 4-dimethylaminopiperidine (844 mg) were added to N,N-dimethylformamide (30 mL), and potassium carbonate (2.6 g) was added. The mixture was stirred with the temperature controlled at 80 °C for 2 h.

After the reaction was completed, the reaction solution was extracted with purified water (200 mL) and ethyl acetate (200 mL). The organic phase was dried over anhydrous sodium sulfate and filtered, and the filtrate was concentrated under reduced pressure to give intermediate **41-1** (1.5 g).

LC-MS: m/z 268.31 (M+H)⁺.

### Preparation of intermediate 41-2:

Referring to the preparation steps of intermediate **10-2** in Example **10,** intermediate **10-1** was replaced with intermediate **41-1,** and after the reaction was completed, intermediate **41-2** (1.1 g) was obtained.

LC-MS: m/z 338.22 (M+H)⁺.

### Preparation of compound 41:

Referring to the preparation steps of compound **26** in Example **26,** intermediate **26-4** was replaced with intermediate **41-2,** and after the reaction was completed, compound 41 was obtained.

LC-MS: m/z 706.35 (M+H)⁺.

¹H NMR (500 MHz DMSO-*d*₆) δ 11.24 (t, *J =* 6.0 Hz, 1H), 9.05 (dd, *J =* 4.0, 2.0 Hz, 1H), 8.57 (dd, *J* = 8.5, 1.5 Hz, 1H), 8.48 (s, 1H), 8.36 (m, 2H), 8.07 (dd, *J* = 8.5, 3.0 Hz, 1H), 7.73 (dd, *J =* 8.5, 4.0 Hz, 1H), 7.66 (d, *J =* 9.0 Hz, 1H), 7.53 (d, *J =* 8.5 Hz, 2H), 7.44 (d, *J =* 8.5 Hz, 2H), 6.92 (m, 2H), 5.57 (m, 1H), 4.76 (d, *J =* 6.0 Hz, 2H), 2.94 (m, 1H), 2.79 (m, 2H), 2.66 (m, 1H), 2.21 (s, 6H), 1.83 (m, 2H), 1.50 (m, 2H), 1.25 (m, 2H).

### Example 42: Preparation of Compound 42

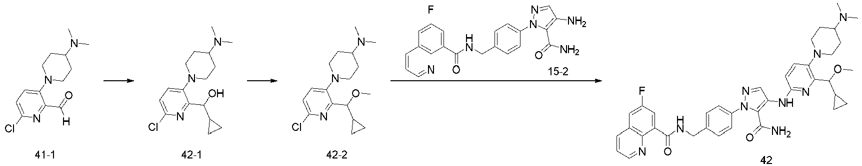

### Preparation of intermediate 42-1:

Intermediate **41-1** (5.36 g) was added to THF (100 mL). The mixture was stirred with the temperature controlled at -20 °C, and a solution of cyclopropylmagnesium bromide (1 M, 40 mL) was slowly added. After the dropwise addition was completed, the mixture was stirred with the temperature controlled at -20 °C.

After the reaction was completed, an aqueous ammonium chloride solution (100 mL) was added to quench the reaction, ethyl acetate (200 mL) was added, and the mixture was extracted and then left to stand for liquid separation. The organic phase was dried over anhydrous sodium sulfate and filtered under vacuum, and the filtrate was concentrated under reduced pressure to give intermediate **42-1** (6.2 g).

LC-MS: m/z 310.18 (M+H)⁺.

### Preparation of intermediate 42-2:

Intermediate **42-1** (0.93 g) was added to THF (30 mL), and the mixture was stirred in an ice bath. Sodium hydride (60%, 0.18 g) was slowly added, and after the mixture was stirred homogeneously, iodomethane (213 mg) was added. The mixture was transferred to room temperature and stirred.

After the reaction was completed, purified water (50 mL) was added to quench the reaction. Ethyl acetate (50 mL) was added, and the reaction system was extracted and then left to stand for liquid separation. The organic phase was dried over anhydrous sodium sulfate and filtered under vacuum, and the filtrate was concentrated under reduced pressure. The concentrate was purified by column chromatography to give intermediate **42-2** (400 mg).

LC-MS: m/z 324.22 (M+H)⁺.

### Preparation of compound 42:

Intermediate **42-2** (81 mg), intermediate **15-2** (100 mg), chloro(2-dicyclohexylphosphino-3,6-dimethoxy-2',4',6'-triisopropyl-1,1'-biphenyl)[2-(2-aminoethylphenyl)]palladium(II) (40 mg), 2-(dicyclohexylphosphino)-3,6-dimethoxy-2'-4'-6'-tri-I-propyl-11'-biphenyl (54 mg), and lithium bis(trimethylsilyl)amide (1 M, 250 µL) were added to tert-butanol (50 mL). The mixture was stirred at 85 °C for 0.5 h under nitrogen atmosphere.

After the reaction was completed, ethyl acetate (100 mL) was added for dilution, and the mixture was filtered. The filtrate was concentrated under reduced pressure to give a concentrate, which was purified by preparative liquid chromatography to give compound **42.**

LC-MS: m/z 692.44 (M+H)⁺.

¹H NMR (500 MHz, DMSO-*d*₆) δ 11.25 (t, *J =* 6.1 Hz, 1H), 9.05 (dd, *J* = 4.3, 1.8 Hz, 1H), 8.57 (dd, *J* = 8.4, 1.8 Hz, 1H), 8.47-8.19 (m, 3H), 8.07 (dd, *J* = 8.5, 3.1 Hz, 1H), 7.73 (dd, *J* = 8.4, 4.3 Hz, 1H), 7.52 (dd, *J* = 8.6, 3.7 Hz, 3H), 7.46-7.41 (m, 2H), 6.78 (d, *J =* 8.7 Hz, 1H), 4.76 (d, *J =* 6.0 Hz, 2H), 4.14 (d, *J =* 8.8 Hz, 1H), 3.22-3.16 (m, 1H), 3.15 (s, 2H), 2.88 (d, *J =* 11.1 Hz, 1H), 2.77 (d, *J =* 11.1 Hz, 1H), 2.73-2.57 (m, 2H), 2.20 (s, 5H), 2.18-2.10 (m, 1H), 1.81 (d, *J* = 12.1 Hz, 2H), 1.48 (tq, *J* = 12.6, 5.8 Hz, 2H), 1.35 (ddt, *J* = 13.2, 8.5, 4.2 Hz, 1H), 1.28-1.21 (m, 2H), 0.62 (tt, *J* = 9.0, 4.3 Hz, 1H), 0.49 (dq, *J =* 9.6, 4.8 Hz, 1H), 0.34 (tdd, *J* = 9.0, 5.7, 4.1 Hz, 1H), 0.14 (dq,*J* = 9.6, 4.7 Hz, 1H).

### Example 43: Preparation of Compound 43

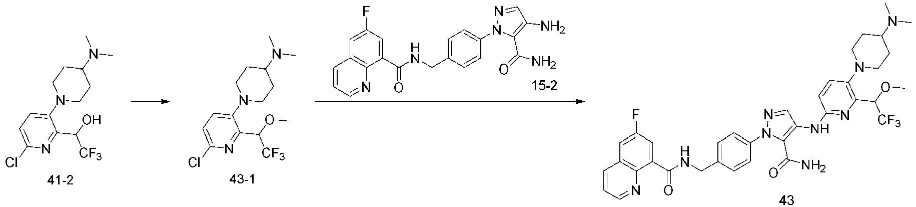

### Preparation of intermediate 43-1:

Intermediate **41-2** (1 g) was added to THF (20 mL), and sodium hydride (60%, 144 mg) was slowly added at room temperature. The mixture was stirred homogeneously, then iodomethane (213 mg) was added, and the mixture was stirred at room temperature.

After the reaction was completed, purified water was added to quench the reaction. The reaction solution was concentrated under reduced pressure, and the concentrate was purified by column chromatography to give intermediate **43-1.**

LC-MS: m/z 352.16 (M+H)⁺.

### Preparation of compound 43:

Intermediate **43-1** (132 mg), intermediate **15-2** (150 mg), chloro(2-dicyclohexylphosphino-3,6-dimethoxy-2',4',6'-triisopropyl-1,1'-biphenyl)[2-(2-aminoethylphenyl)]palladium(II) (60 mg), 2-(dicyclohexylphosphino)-3,6-dimethoxy-2'-4'-6'-tri-I-propyl-11'-biphenyl (81 mg), and lithium bis(trimethylsilyl)amide (1 M, 375 µL) were added to tert-butanol (75 mL), and the mixture was stirred at 85 °C under nitrogen atmosphere.

After the reaction was completed, the reaction solution was diluted with ethyl acetate (100 mL) and filtered, and the filtrate was concentrated under reduced pressure to give a concentrate, which was purified by preparative liquid chromatography to give compound **43.**

LC-MS: m/z 720.50 (M+H)⁺.

### Example 44: Preparation of Compound 44

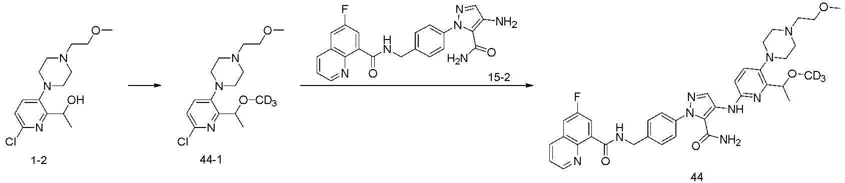

### Preparation of intermediate 44-1:

Referring to the preparation steps of compound **1-3** in Example 1, iodomethane was replaced with deuterated iodomethane to give intermediate **44-1.**

LC-MS: m/z 317.21 (M+H)⁺.

### Preparation of compound 44:

Intermediate **44-1** (164 mg), intermediate **15-2** (150 mg), chloro(2-dicyclohexylphosphino-3,6-dimethoxy-2',4',6'-triisopropyl-1,1'-biphenyl)[2-(2-aminoethylphenyl)]palladium(II) (59.3 mg), 2-(dicyclohexylphosphino)-3,6-dimethoxy-2'-4'-6'-tri-I-propyl-11'-biphenyl (63.5 mg), and potassium bis(trimethylsilyl)amide (0.3 mL) were added to tert-butanol (15 mL). The mixture was stirred at 85 °C for 0.5 h under nitrogen atmosphere.

After the reaction was completed, ethyl acetate (20 mL) and purified water (20 mL) were added, and the mixture was stirred for 10 min and left to stand for liquid separation. The aqueous phase was extracted with ethyl acetate (20 mL × 2). The organic phases were combined, washed with saturated brine, dried over anhydrous sodium sulfate, and filtered, and the filtrate was concentrated under reduced pressure to give a concentrate, which was purified by preparative liquid chromatography to give compound **44.**

LC-MS: m/z 685.48 (M+H)⁺.

¹H NMR (500 MHz, DMSO-*d*₆) δ 11.24 (t*, J =* 6.1 Hz, 1H), 9.05 (dd, *J* = 4.3, 1.8 Hz, 1H), 8.57 (dd, *J* = 8.4, 1.8 Hz, 1H), 8.37 (d, *J =* 3.1 Hz, 1H), 8.32 (s, 1H), 8.31 (s, 1H), 8.07 (dd, *J* = 8.5, 3.1 Hz, 1H), 7.73 (dd, *J* = 8.4, 4.3 Hz, 1H), 7.55-7.50 (m, 3H), 7.45 (s, 1H), 7.44 (d, *J =* 1.8 Hz, 1H), 6.79 (d, *J =* 8.7 Hz, 1H), 4.97 (q, *J =* 6.4 Hz, 1H), 4.76 (d, *J =* 6.0 Hz, 2H), 3.46 (t, *J =* 5.8 Hz, 2H), 3.25 (s, 3H), 2.81-2.68 (m, 4H), 1.36 (d, *J =* 6.4 Hz, 3H), 1.31-1.13 (m, 4H).

### Example 45: Preparation of Compound 45

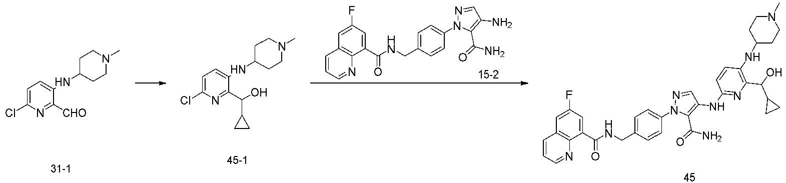

### Preparation of intermediate 45-1:

Intermediate **31-1** (2.7 g) was added to THF (150 mL), and cyclopropylmagnesium bromide (1 M, 30 mL) was added dropwise with the temperature controlled at -10 °C to 0 °C under nitrogen atmosphere. After the dropwise addition was completed, the mixture was stirred at the same temperature for 2 h. After the reaction was completed, a saturated ammonium chloride solution (50 mL) was added to quench the reaction, and the reaction system was extracted with ethyl acetate (50 mL × 3). The organic phases were combined, washed with saturated sodium chloride solution, dried over anhydrous sodium sulfate, and filtered under vacuum, and the filtrate was concentrated under reduced pressure and purified by column chromatography (DCM:methanol = 12:1) to give intermediate **45-1** (0.46 g). LC-MS: m/z 296.25 (M+H)⁺.

### Preparation of compound 45:

Intermediate **45-1** (297 mg) and intermediate **15-2** (278 mg) were added to tert-butanol (50 mL), methanesulfonato(2-di-tert-butylphosphino-2',4',6'-triisopropyl-1,1'-biphenyl)(2'-amino-1,1'-biphenyl-2-yl)palladium(II) (140 mg), 2-di-tert-butylphosphino-2',4',6'-triisopropylbiphenyl (170 mg), and lithium bis(trimethylsilyl)amide (1 M, 740 µL) were added, and the mixture was stirred at 80 °C for 2 h under nitrogen atmosphere.

After the reaction was completed, ethyl acetate (30 mL) and purified water (30 mL) were added, and the mixture was stirred for 10 min and left to stand for liquid separation. The aqueous phase was extracted with ethyl acetate (30 mL × 3). The organic phases were combined, washed with saturated brine, dried over anhydrous sodium sulfate, and filtered, and the filtrate was concentrated under reduced pressure to give a concentrate, which was separated by preparative liquid chromatography to give compound 45.

LC-MS: m/z 664.37 (M+H)⁺.

¹H NMR (500 MHz, DMSO-*d*₆) δ 11.24 (t*, J =* 6.0 Hz, 1H), 9.04 (dd, *J* = 4.3, 1.8 Hz, 1H), 8.56 (dd, *J* = 8.4, 1.8 Hz, 1H), 8.36 (dd, *J* = 9.6, 3.1 Hz, 1H), 8.24 (s, 1H), 8.07 (dd, *J =* 8.5, 3.1 Hz, 1H), 7.91 (s, 1H), 7.73 (dd, *J =* 8.4, 4.3 Hz, 1H), 7.55-7.49 (m, 2H), 7.45-7.40 (m, 2H), 7.04 (d*, J =* 8.8 Hz, 1H), 6.68 (d*, J =* 8.7 Hz, 1H), 5.50 (s, 1H), 5.05 (s, 1H), 4.76 (d, *J =* 6.0 Hz, 2H), 4.00 (d, *J =* 7.9 Hz, 1H), 2.69 (d, *J =* 11.3 Hz, 2H), 2.19 (s, 3H), 2.14-2.03 (m, 2H), 1.91 (s, 3H), 1.46-1.33 (m, 3H), 0.50 (ddd, *J* = 11.4, 8.0, 5.6 Hz, 1H), 0.41 (tq, *J =* 6.0, 2.5 Hz, 2H), 0.33-0.22 (m, 1H).

### Example 46: Preparation of Compound 46

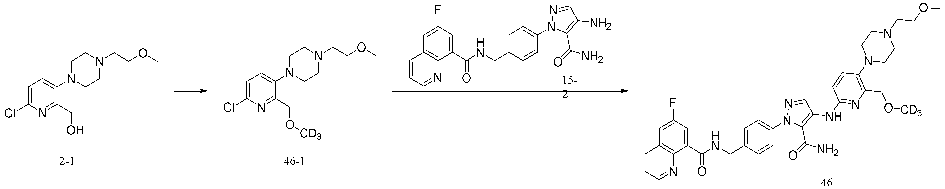

### Preparation of intermediate 46-1:

Intermediate **2-1** (700 mg) was added to THF (70 mL), and the mixture was stirred with the temperature controlled at 0 °C. Sodium hydride (60%, 91 mg) was slowly added, and after the mixture was stirred for 20 min, deuterated iodomethane (357 mg) was added, and the mixture was transferred to room temperature and stirred for 4 h.

After the reaction was completed, purified water (50 mL) was added to quench the reaction, and the reaction system was stirred for 10 min. Ethyl acetate (100 mL) was added, and the reaction system was extracted and then left to stand for liquid separation. The organic phase was dried over anhydrous sodium sulfate and filtered under vacuum, and the filtrate was concentrated under reduced pressure to give intermediate **46-1** (208 mg).

LC-MS: m/z 303.14 (M+H)⁺.

### Preparation of compound 46:

Referring to the preparation steps of compound **9** in Example **9,** intermediate **8-8** was replaced with intermediate **15-2,** intermediate **9-2** was replaced with intermediate **46-1,** and the reaction system was separated by preparative liquid chromatography to give compound **46.**

LC-MS: m/z 671.43 (M+H)⁺.

### Example 47: Preparation of Compound 47

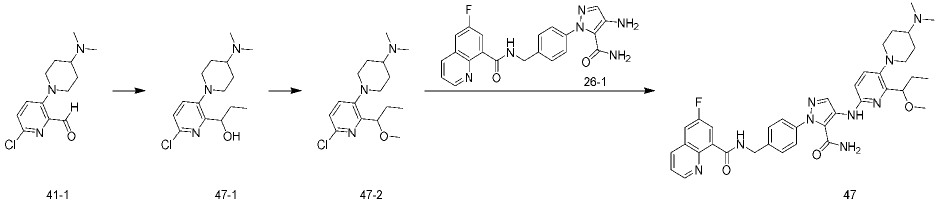

### Preparation of intermediate 47-1:

Referring to the preparation steps of intermediate **28-1** in Example **28,** intermediate **26-2** was replaced with intermediate **41-1,** and after the reaction was completed, intermediate **47-1** (3.3 g) was obtained.

LC-MS: m/z 298.25 (M+H)⁺.

### Preparation of intermediate 47-2:

Referring to the preparation steps of intermediate **10-3** in Example **10,** intermediate **10-2** was replaced with intermediate **47-1,** and after the reaction was completed, intermediate **47-2** (400 mg) was obtained.

LC-MS: m/z 312.25 (M+H)⁺.

### Preparation of compound 47:

Referring to the preparation steps of compound **26** in Example **26,** intermediate **26-4** was replaced with intermediate **47-2,** and after the reaction was completed, compound **47** was obtained.

LC-MS: m/z 680.34 (M+H)⁺.

¹H NMR (500 MHz DMSO-*d*₆) δ 11.25 (t, *J =* 6.0 Hz, 1H), 9.05 (dd, *J* = 4.2, 1.5 Hz, 1H), 8.57 (dd, *J* = 8.5, 1.5 Hz, 1H), 8.52 (s, 1H), 8.48 (s, 1H), 8.36 (dd, *J* = 9.5, 3.0 Hz, 1H), 8.27 (s, 1H), 8.08 (dd, *J* = 8.5, 3.0 Hz, 1H), 7.73 (dd, *J =* 8.5, 4.0 Hz, 1H), 7.65 (brs, 1H), 7.52 (d*, J =* 8.5 Hz, 2H), 7.48 (d*, J =* 9.0 Hz, 1H), 7.44 (d, *J* = 8.5 Hz, 2H), 6.77 (d, *J =* 8.5 Hz, 1H), 4.85 (t, *J =* 6.5 Hz, 1H), 4.76 (d, *J =* 6.0 Hz, 2H), 3.22 (m, 2H), 3.07 (s, 9H), 2.96 (m, 1H), 2.79 (m, 1H), 2.62 (m, 1H), 2.18 (m, 2H), 1.80 (m, 4H), 0.85 (t, *J =* 7.0 Hz, 3H).

### Example 48: Preparation of Compounds 48-a and 48-b

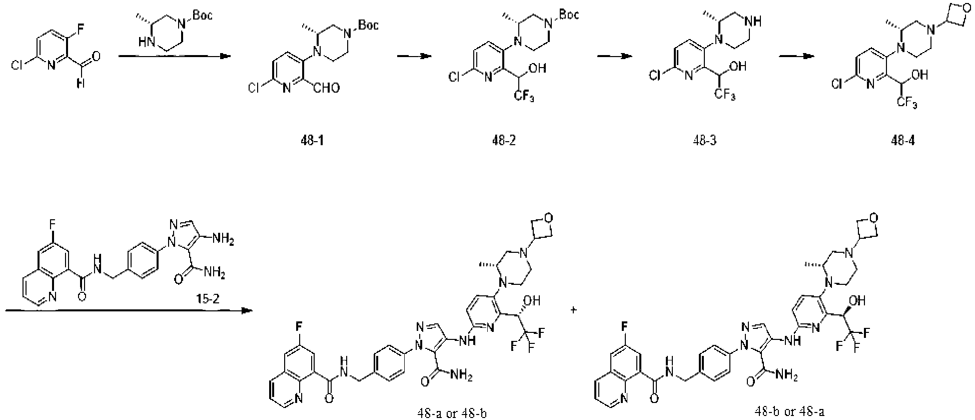

### Preparation of intermediate 48-1:

2-Chloro-5-fluoropyridine-6-carbaldehyde (3.6 g), (R)-4-Boc-2-methylpiperazine (5.10 g), and potassium carbonate (6.1 g) were added to DMF (100 mL), and the mixture was stirred at 80 °C for 3 h, and then cooled to room temperature. Ethyl acetate (50 mL) and purified water (30 mL) were added, and the mixture was stirred for 10 min and left to stand for liquid separation. The aqueous phase was extracted with ethyl acetate (40 mL × 2), the organic phases were combined, washed with saturated brine, dried over anhydrous sodium sulfate, and filtered, and the filtrate was concentrated under reduced pressure to give intermediate **48-1** (4.1 g).

LC-MS: m/z 340.21 (M+H)⁺.

### Preparation of intermediate 48-2:

Intermediate **48-1** (1.52 g), THF (150 mL), and CsF (2.36 g) were added to a reaction flask, and TMSCF₃ (2.40 g) was added with stirring. The mixture was purged three times with nitrogen and then stirred at room temperature for 3 h.

After the reaction was completed, ethyl acetate (50 mL) and purified water (30 mL) were added, and the mixture was stirred for 10 min and left to stand for liquid separation. The aqueous phase was extracted with ethyl acetate (50 mL × 2). The organic phases were combined, washed with saturated brine, dried over anhydrous sodium sulfate, and filtered, and the filtrate was concentrated under reduced pressure to give intermediate **48-2** (1.32 g).

LC-MS: m/z 410.27 (M+H)⁺.

### Preparation of intermediate 48-3:

Intermediate **48-2** (1.32 g) and ethyl acetate (30 mL) were added to a reaction flask, hydrogen chloride/ethyl acetate (2 M, 30 mL) was added with stirring, and the mixture was stirred at room temperature for 3 h. After the reaction was completed, the reaction solution was concentrated under reduced pressure to give intermediate **48-3** (1.12 g). LC-MS: m/z 310.12 (M+H)⁺.

### Preparation of intermediate 48-4:

Intermediate **48-3** (1.12 g), zinc chloride (120 mg), triethylamine (3.6 g), methanol (200 mL), and cyclobutanone (1.20 g) were added to a reaction flask, and the mixture was purged three times with nitrogen and stirred at 65 °C for 6 h. The reaction system was cooled to room temperature, sodium cyanoborohydride (1.24 g) was added in portions, and the mixture was stirred overnight. Ethyl acetate (80 mL) and purified water (60 mL) were added, and the mixture was stirred for 10 min and left to stand for liquid separation. The aqueous phase was extracted with ethyl acetate (60 mL × 2). The organic phases were combined, washed with saturated brine, dried over anhydrous sodium sulfate, and filtered, and the filtrate was concentrated under reduced pressure to give intermediate **48-4** (1.24 g).

LC-MS: m/z 366.06 (M+H)⁺.

### Preparation of compounds 48-a and 48-b:

Intermediate **48-4** (150 mg) and intermediate **15-2** (158 mg) were added to tert-butanol (30 mL), methanesulfonato(2-di-tert-butylphosphino-2',4',6'-triisopropyl-1,1'-biphenyl)(2'-amino-1,1'-biphenyl-2-yl)palladium(II) (70 mg), 2-di-tert-butylphosphino-2',4',6'-triisopropylbiphenyl (90 mg), and lithium bis(trimethylsilyl)amide (1 M, 385 µL) were added, and the mixture was stirred at 80 °C for 3 h under nitrogen atmosphere.

After the reaction was completed, ethyl acetate (30 mL) and purified water (30 mL) were added, and the mixture was stirred for 10 min and left to stand for liquid separation. The aqueous phase was extracted with ethyl acetate (30 mL × 3). The organic phases were combined, washed with saturated brine, dried over anhydrous sodium sulfate, and filtered, and the filtrate was concentrated under reduced pressure to give a concentrate, which was separated by preparative liquid chromatography to give compound **48-a** and compound **48-b.** The preparative conditions were as follows: a YMC AQ C18 preparative column (50 × 250 mm, 10 µm) was used; by taking 0.5% formic acid-water as mobile phase A and acetonitrile as mobile phase B, gradient elution was performed at a flow rate of 50 mL/min; the detection wavelength was 254 nm, and the retention times were 45.23 min (**48-a**) and 49.52 min (**48-b**), respectively.

Compound **48-a** LC-MS: m/z 734.27 (M+H)⁺.

¹H NMR (500 MHz, DMSO-*d*₆) δ 11.25 (t, *J =* 6.0 Hz, 1H), 9.12-9.01 (m, 1H), 8.62-8.53 (m, 2H), 8.40 (s, 1H), 8.36 (dd, *J* = 9.6, 3.1 Hz, 1H), 8.06 (dd, *J* = 8.5, 3.2 Hz, 1H), 7.75-7.69 (m, 2H), 7.53 (d, *J =* 8.5 Hz, 2H), 7.48-7.43 (m, 2H), 6.98 (d, *J* = 8.9 Hz, 1H), 6.36 (d, *J* = 7.4 Hz, 1H), 5.83 (p, *J =* 7.0 Hz, 1H), 4.77 (d, *J =* 6.0 Hz, 2H), 4.56 (q, *J =* 6.4 Hz, 2H), 4.46 (dt, *J* = 9.2, 6.1 Hz, 2H), 3.46 (t*, J =* 6.3 Hz, 1H), 3.01 (dqd, *J* = 9.1, 6.1, 2.5 Hz, 1H), 2.87 (td, *J =* 11.3, 2.7 Hz, 1H), 2.81-2.69 (m, 2H), 2.60 (dt, *J =* 11.3, 2.8 Hz, 1H), 2.02 (td*, J =* 11.1, 3.1 Hz, 1H), 1.73 (t, *J* = 10.2 Hz, 1H), 1.24 (d, *J =* 4.7 Hz, 1H), 0.70 (d, *J =* 6.3 Hz, 3H).

Compound **48-b** LC-MS: m/z 734.26 (M+H)⁺.

¹H NMR (500 MHz, DMSO-*d*₆) δ 11.25 (q, *J =* 5.0 Hz, 1H), 9.13-9.00 (m, 1H), 8.63-8.53 (m, 2H), 8.45-8.33 (m, 2H), 8.06 (dd, *J =* 8.5, 3.2 Hz, 1H), 7.75-7.68 (m, 2H), 7.53 (d, *J =* 8.3 Hz, 2H), 7.45 (d, *J* = 8.3 Hz, 2H), 6.98 (d, *J* = 8.9 Hz, 1H), 5.67 (q, *J* = 7.7 Hz, 1H), 4.77 (d, *J =* 6.0 Hz, 2H), 4.56 (td, *J =* 6.5, 3.2 Hz, 2H), 4.47 (dt, *J =* 10.2, 6.1 Hz, 2H), 3.15 (ddt, *J* = 9.5, 6.3, 3.4 Hz, 1H), 2.89-2.68 (m, 4H), 2.09 (dt, *J =* 10.9, 6.2 Hz, 1H), 1.74 (t, *J =* 10.3 Hz, 1H), 0.70 (d, *J =* 6.2 Hz, 3H).

Compound 48-a had a shorter retention time in the chiral chromatography column than compound 48-b, and compound 48-b had a longer retention time in the chiral chromatography column than compound 48-a.

### Example 49: Preparation of Compounds 49-a and 49-b

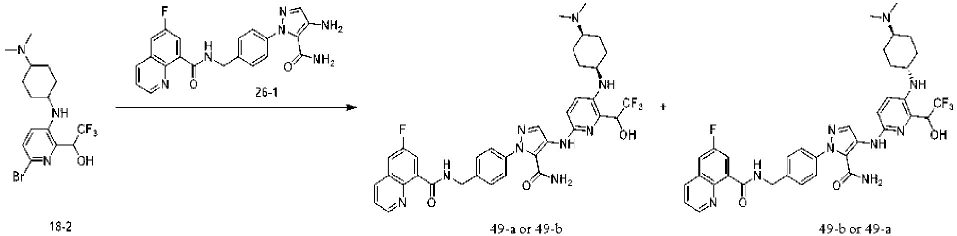

### Preparation of compounds 49-a and 49-b:

Referring to the preparation steps of compound **26** in Example **26,** intermediate **26-4** was replaced with intermediate **18-2.**

After the reaction was completed, the reaction system was subjected to preparative liquid chromatography to give compounds **49-a** and **49-b.** The conditions were as follows: a YMC AQ C18 preparative column (30 × 250 mm, 10 µm) was used; by taking 0.1% acetic acid as mobile phase A and acetonitrile as mobile phase B, gradient elution was performed at a flow rate of 30 mL/min; the detection wavelength was 254 nm, and the retention times were 33 min (**49-a**) and 35 min (**49-b**), respectively.

Compound **49-a** LC-MS: m/z 720.23 (M+H)⁺.

Compound **49-b** LC-MS: m/z 720.21 (M+H)⁺.

Compound **49-b** ¹H NMR (500 MHz DMSO-*d*₆) δ 11.25 (t, *J =* 6.0 Hz, 1H), 9.04 (dd, *J =* 4.2, 1.5 Hz, 1H), 8.57 (dd, *J* = 8.5, 1.5 Hz, 1H), 8.36 (dd, *J =* 9.5, 3.0 Hz, 1H), 8.22 (s, 1H), 8.07 (dd, *J =* 8.5, 3.0 Hz, 1H), 8.02 (s, 1H), 7.73 (dd, *J* = 8.5, 4.0 Hz, 1H), 7.52 (d, *J =* 8.5 Hz, 2H), 7.43 (d, *J* = 8.5 Hz, 2H), 7.19 (brs, 1H), 7.13 (d, *J =* 9.0 Hz, 1H), 6.83 (d, *J =* 9.0 Hz, 1H), 6.64 (m, 1H), 5.32 (m, 1H), 5.24 (m, 1H), 4.76 (d, *J =* 6.0 Hz, 2H), 3.45 (m, 1H), 2.17 (s, 6H), 2.01 (m, 2H), 1.58 (m, 8H).

Compound 49-a had a shorter retention time in the chiral chromatography column than compound 49-b, and compound 49-b had a longer retention time in the chiral chromatography column than compound 49-a.

### Example 50: Preparation of Compound 50

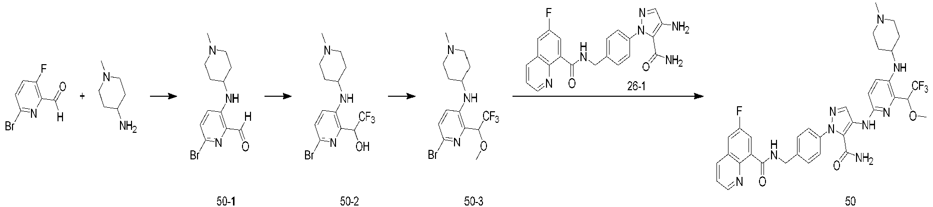

### Preparation of intermediate 50-1:

2-Bromo-5-fluoropyridine-6-carbaldehyde (18 g) and 4-amino-1-methylpiperidine (10.1 g) were added to N,N-dimethylformamide (200 mL), and potassium carbonate (36 g) was added. The mixture was stirred with the temperature controlled at 80 °C for 2 h.

After the reaction was completed, the reaction solution was extracted with purified water (400 mL) and ethyl acetate (400 mL). The organic phase was dried over anhydrous sodium sulfate and filtered, and the filtrate was concentrated under reduced pressure to give intermediate **50-1** (22 g).

LC-MS: m/z 298.15 (M+H)⁺.

### Preparation of intermediate 50-2:

Referring to the preparation steps of intermediate **10-2** in Example **10,** intermediate **10-1** was replaced with intermediate **50-1,** and after the reaction was completed, intermediate **50-2** (21 g) was obtained.

LC-MS: m/z 368.42 (M+H)⁺.

### Preparation of intermediate 50-3:

Referring to the preparation steps of intermediate **10-3** in Example **10,** intermediate **10-2** was replaced with intermediate **50-2,** and after the reaction was completed, intermediate **50-3** (510 mg) was obtained.

LC-MS: m/z 382.16 (M+H)⁺.

### Preparation of compound 50:

Referring to the preparation steps of compound **26** in Example **26,** intermediate **26-4** was replaced with intermediate **50-3,** and after the reaction was completed, compound **50** was obtained.

LC-MS: m/z 706.11 (M+H)⁺.

¹H NMR (500 MHz DMSO-*d*₆) δ 11.24 (t, *J =* 6.0 Hz, 1H), 9.04 (dd, *J* = 4.2, 1.5 Hz, 1H), 8.57 (dd, *J* = 8.5, 1.5 Hz, 1H), 8.36 (dd, *J =* 9.5, 3.0 Hz, 1H), 8.32 (s, 1H), 8.12 (s, 1H), 8.07 (dd, *J* = 8.5, 3.0 Hz, 1H), 7.73 (dd, *J =* 8.5, 4.0 Hz, 1H), 7.52 (d, *J =* 8.5 Hz, 2H), 7.43 (d, *J =* 8.5 Hz, 2H), 7.21 (d, *J* = 9.0 Hz, 1H), 6.89 (d, *J =* 9.0 Hz, 1H), 5.24 (m, 1H), 4.76 (d, *J =* 6.0 Hz, 2H), 4.71 (d, *J =* 8.0 Hz, 1H), 3.38 (s, 3H), 3.23 (m, 1H), 2.72 (m, 2H), 2.19 (s, 3H), 2.05 (m, 2H), 1.85 (m, 2H), 1.39 (m, 2H).

### Example 51: Preparation of Compound 51

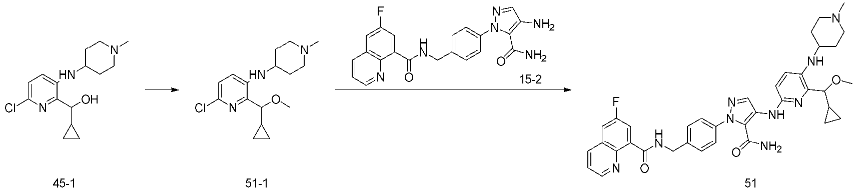

### Preparation of intermediate 51-1:

Intermediate **45-1** (180 mg) and 2,6-dimethylpyridine (210 mg) were added to DCM (20 mL). The mixture was purged three times with nitrogen, and trifluoromethanesulfonic anhydride (540 mg) was added dropwise with the temperature controlled at -78 °C to -70 °C. After the dropwise addition was completed, the mixture was stirred at the same temperature for 2 h. Methanol (20 mL) was added, and the mixture was heated to room temperature and stirred overnight. A saturated ammonium chloride solution (30 mL) was added, and the mixture was extracted with ethyl acetate (50 mL × 3). The organic phases were combined, washed with a saturated sodium chloride solution, dried over anhydrous sodium sulfate, and filtered under vacuum, and the filtrate was concentrated under reduced pressure to give intermediate **51-1** (180 mg).

LC-MS: m/z 310.25 (M+H)⁺.

### Preparation of compound 51:

Intermediate **51-1** (180 mg) and intermediate **15-2** (265 mg) were added to tert-butanol (40 mL), methanesulfonato(2-di-tert-butylphosphino-2',4',6'-triisopropyl-1,1'-biphenyl)(2'-amino-1,1'-biphenyl-2-yl)palladium(II) (106 mg), 2-di-tert-butylphosphino-2',4',6'-triisopropylbiphenyl (144 mg), and lithium bis(trimethylsilyl)amide (1 M, 670 µL) were added, and the mixture was stirred at 80 °C for 2 h under nitrogen atmosphere.

After the reaction was completed, ethyl acetate (30 mL) and purified water (30 mL) were added, and the mixture was stirred for 10 min and left to stand for liquid separation. The aqueous phase was extracted with ethyl acetate (30 mL × 3). The organic phases were combined, washed with saturated brine, dried over anhydrous sodium sulfate, and filtered, and the filtrate was concentrated under reduced pressure to give a concentrate, which was separated by preparative liquid chromatography to give compound **51.**

LC-MS: m/z 678.40 (M+H)⁺.

¹H NMR (500 MHz, DMSO-*d*₆) δ 11.24 (t*, J =* 6.1 Hz, 1H), 9.04 (dd, *J* = 4.3, 1.8 Hz, 1H), 8.56 (dd, *J* = 8.4, 1.8 Hz, 1H), 8.36 (dd, *J* = 9.6, 3.1 Hz, 1H), 8.23 (s, 1H), 8.07 (dd, *J =* 8.5, 3.1 Hz, 1H), 7.94 (s, 1H), 7.73 (dd, *J =* 8.4, 4.2 Hz, 1H), 7.54-7.50 (m, 2H), 7.45-7.41 (m, 2H), 7.08 (d, *J =* 8.9 Hz, 1H), 6.73 (d, *J* = 8.7 Hz, 1H), 4.77 (t, *J* = 7.7 Hz, 3H), 3.68 (d, *J =* 8.6 Hz, 1H), 3.20 (s, 3H), 2.68 (d, *J =* 10.4 Hz, 2H), 2.18 (s, 3H), 2.10 (d, *J =* 8.3 Hz, 2H), 1.95-1.85 (m, 2H), 1.44-1.33 (m, 3H), 1.27-1.22 (m, 1H), 0.66-0.54 (m, 1H), 0.51-0.34 (m, 2H), 0.33-0.21 (m, 1H).

### Example 52: Preparation of Compound 52

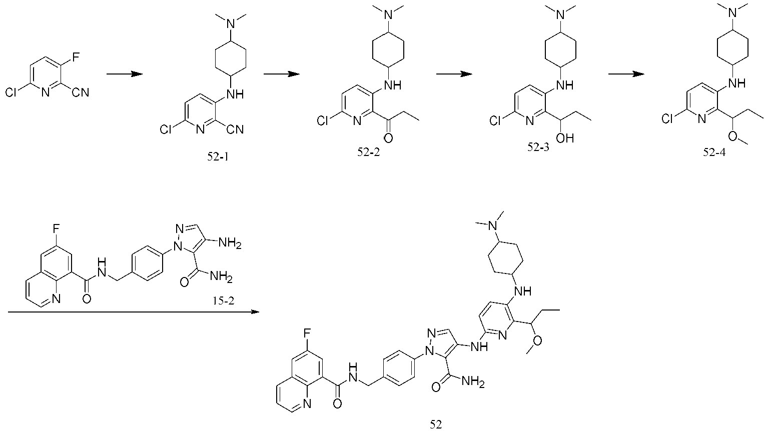

### Preparation of intermediate 52-1:

5-Chloro-2-fluorobenzonitrile (3 g), N,N-dimethyl-1,4-cyclohexanediamine (3.21 g), and potassium carbonate (6.21 g) were added to N,N-dimethylformamide (30 mL), and the mixture was stirred with the temperature controlled at 85 °C for 2 h.

After the reaction was completed, the reaction solution was filtered, and the filtrate was extracted with purified water (50 mL) and ethyl acetate (100 mL). The organic phase was dried over anhydrous sodium sulfate and filtered, and the filtrate was concentrated under reduced pressure to give intermediate **52-1** (4.6 g).

LC-MS: m/z 279.22 (M+H)⁺.

### Preparation of intermediate 52-2:

Intermediate **52-1** (1 g) was added to THF (10 mL), and ethylmagnesium bromide (3 M, 1.8 mL) was added dropwise with the temperature controlled at -20 °C under nitrogen atmosphere. After the dropwise addition was completed, the mixture was stirred for 3 h with the temperature controlled at 0 °C. After the reaction was completed, a saturated ammonium chloride solution (10 mL) was added to quench the reaction, and the reaction system was extracted with ethyl acetate (20 mL × 3). The organic phases were combined, washed with a saturated sodium chloride solution, dried over anhydrous sodium sulfate, and filtered under vacuum, and the filtrate was concentrated under reduced pressure to give intermediate **52-2** (925 mg).

LC-MS: m/z 310.22 (M+H)⁺.

### Preparation of intermediate 52-3:

Intermediate **52-2** (1 g) was added to methanol (10 mL), and sodium borohydride (244 mg) was slowly added. The mixture was stirred at room temperature for 3 h.

After the reaction was completed, purified water (10 mL) was added to quench the reaction, and the reaction solution was stirred for 10 min. DCM (30 mL) was added, and the reaction system was extracted and then left to stand for liquid separation. The organic phase was dried over anhydrous sodium sulfate and filtered under vacuum, and the filtrate was concentrated under reduced pressure to give intermediate **52-3** (700 mg).

LC-MS: m/z 312.42 (M+H)⁺.

### Preparation of intermediate 52-4:

Intermediate **52-3** (100 mg) was added to THF (5 mL), and the mixture was stirred with the temperature controlled at 0 °C. Sodium hydride (60%, 50 mg) was slowly added, and after the mixture was stirred for 20 min, a chloromethane solution (2 M, 2.5 mL) was added. The mixture was transferred to room temperature and stirred for 12 h.

After the reaction was completed, purified water (10 mL) was added to quench the reaction, and the reaction solution was stirred for 10 min. Ethyl acetate (20 mL) was added, and the reaction system was extracted and then left to stand for liquid separation. The organic phase was dried over anhydrous sodium sulfate and filtered under vacuum, and the filtrate was concentrated under reduced pressure to give intermediate **52-4** (94 mg).

LC-MS: m/z 326.36 (M+H)⁺.

### Preparation of compound 52:

Referring to the preparation steps of compound **9** in Example **9,** intermediate **8-8** was replaced with intermediate **15-2,** intermediate **9-2** was replaced with intermediate **52-4,** and the reaction system was separated by preparative liquid chromatography to give compound **52.**

LC-MS: m/z 694.56 (M+H)⁺.

¹H NMR (500 MHz, DMSO-*d*₆) δ 11.24 (t*, J =* 6.1 Hz, 1H), 9.04 (dd, *J* = 4.3, 1.8 Hz, 1H), 8.56 (dd, *J* = 8.4, 1.8 Hz, 1H), 8.36 (dd, *J* = 9.6, 3.1 Hz, 1H), 8.21 (s, 1H), 8.07 (dd, *J* = 8.5, 3.1 Hz, 1H), 7.93 (s, 1H), 7.73 (dd, *J* = 8.3, 4.2 Hz, 1H), 7.54-7.50 (m, 2H), 7.44-7.41 (m, 2H), 7.08 (d, *J* = 8.9 Hz, 1H), 6.71 (d, *J* = 8.7 Hz, 1H), 4.76 (d, *J =* 6.0 Hz, 2H), 4.63 (d, *J* = 8.7 Hz, 1H), 4.25 (t, *J* = 7.1 Hz, 1H), 3.18 (s, 3H), 2.17 (s, 6H), 2.01 (td, *J =* 11.5, 3.4 Hz, 2H), 1.31-1.27 (m, 2H), 1.25 (d, *J =* 12.5 Hz, 4H), 1.14-1.05 (m, 2H), 0.87 (t, *J =* 7.4 Hz, 4H).

### Example 53: Preparation of Compound 53

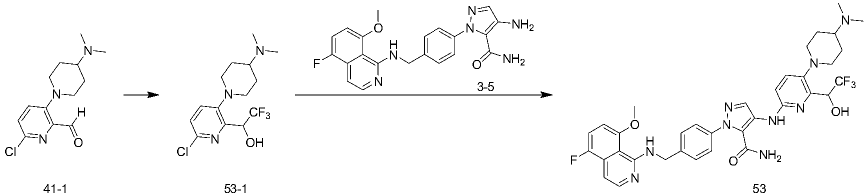

### Preparation of intermediate 53-1:

Referring to the preparation steps of intermediate **10-2** in Example 10, intermediate **10-1** was replaced with intermediate **41-1,** and after the reaction was completed, intermediate **53-1** (800 mg) was obtained.

LC-MS: m/z 338.23 (M+H)⁺.

### Preparation of compound 53:

Intermediate **53-1** (110 mg) and intermediate 3-5 (120 mg) were added to tert-butanol (5 mL) and 1,4-dioxane (5 mL), methanesulfonato(2-di-tert-butylphosphino-2',4',6'-triisopropyl-1,1'-biphenyl)(2'-amino-1,1'-biphenyl-2-yl)palladium(II) (47 mg), 2-di-tert-butylphosphino-2',4',6'-triisopropylbiphenyl (38 mg), and lithium bis(trimethylsilyl)amide (1 M, 0.3 mL) were added, and the mixture was stirred at 80 °C for 2 h under nitrogen atmosphere.

After the reaction was completed, ethyl acetate (50 mL) and purified water (60 mL) were added, and the mixture was stirred for 10 min and left to stand for liquid separation. The aqueous phase was extracted with ethyl acetate (50 mL × 3). The organic phases were combined, washed with saturated brine, dried over anhydrous sodium sulfate, and filtered, and the filtrate was concentrated under reduced pressure to give a concentrate, which was separated by preparative liquid chromatography to give compound **53.**

LC-MS: m/z 708.31 (M+H)⁺.

### Example 54: Preparation of Compounds 54, 54-a, and 54-b

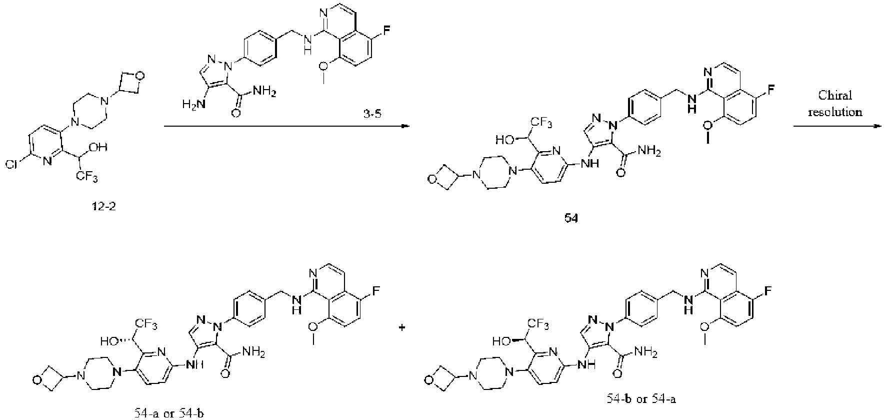

### Preparation of compound 54:

Referring to the preparation steps of compound **3** in Example **3,** intermediate **1-3** was replaced with intermediate **12-2,** and cesium carbonate was replaced with potassium carbonate. After the reaction was completed, the reaction solution was filtered and concentrated to give a concentrate, which was separated by preparative liquid chromatography to give compound **54.**

LC-MS: m/z 722.41 (M+H)⁺.

¹H NMR (500 MHz, DMSO-*d*₆) δ 8.51 (s, 1H), 8.46 (t, *J =* 6.0 Hz, 1H), 8.34 (s, 1H), 8.15 (s, 2H), 7.91 (d, *J* = 5.8 Hz, 1H), 7.71 (d, *J =* 8.9 Hz, 2H), 7.52-7.32 (m, 6H), 6.94 (dd, *J =* 8.8, 4.0 Hz, 2H), 6.87 (d, *J =* 5.8 Hz, 1H), 5.60 (q, *J* = 7.6 Hz, 1H), 4.81 (d, *J =* 5.9 Hz, 2H), 4.56 (t, *J =* 6.5 Hz, 2H), 4.46 (t, *J =* 6.1 Hz, 2H), 4.00 (s, 3H), 3.48 (p, *J =* 6.4 Hz, 1H), 2.89 (dt, *J* = 9.9, 4.5 Hz, 2H), 2.74 (d, *J =* 13.3 Hz, 2H), 2.40 (s, 3H).

### Preparation method for compounds 54-a and 54-b:

Compound **54** was subjected to chiral resolution to give compound **54-a** and compound **54-b.** The resolution conditions were as follows: a Cellulose-SC preparative column (30 × 250 mm, 10 µm) was used; by taking n-hexane as mobile phase A and ethanol as mobile phase B, gradient elution was performed at a flow rate of 0.8 mL/min; the detection wavelength was 254 nm, and the retention times were 14 min (54-a) and 16 min (54-b), respectively.

Compound 54-a LC-MS: m/z 722.41 (M+H)⁺.

Compound 54-b LC-MS: m/z 722.43 (M+H)⁺.

Compound 54-a had a shorter retention time in the chiral chromatography column than compound 54-b, and compound 54-b had a longer retention time in the chiral chromatography column than compound 54-a.

### Example 55: Preparation of Compound 55

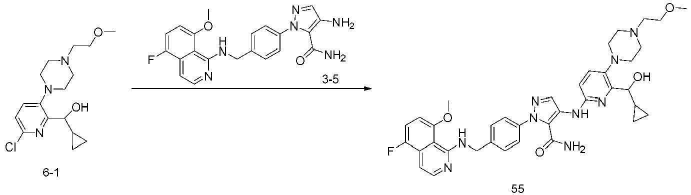

### Preparation of compound 55:

Intermediate **6-1** (256 mg), intermediate **3-5** (200 mg), chloro(2-dicyclohexylphosphino-3,6-dimethoxy-2',4',6'-triisopropyl-1,1'-biphenyl)[2-(2-aminoethylphenyl)]palladium(II) (78.7 mg), 2-(dicyclohexylphosphino)-3,6-dimethoxy-2'-4'-6'-tri-I-propyl-11'-biphenyl (105.8 mg), and potassium bis(trimethylsilyl)amide (0.5 mL) were added to tert-butanol (20 mL), and the mixture was stirred at 80 °C for 0.5 h under nitrogen atmosphere.

After the reaction was completed, ethyl acetate (20 mL) and purified water (20 mL) were added, and the mixture was stirred for 10 min and left to stand for liquid separation. The aqueous phase was extracted with ethyl acetate (20 mL × 2). The organic phases were combined, washed with saturated brine, dried over anhydrous sodium sulfate, and filtered, and the filtrate was concentrated under reduced pressure to give a concentrate, which was purified by preparative liquid chromatography to give compound **55.**

LC-MS: m/z 696.50 (M+H)⁺.

¹H NMR (500 MHz, DMSO-*d*₆) δ 8.46 (t, *J =* 6.0 Hz, 1H), 8.32 (s, 1H), 8.27 (s, 1H), 7.91 (d, *J =* 5.9 Hz, 1H), 7.55 (d, *J* = 8.8 Hz, 1H), 7.44 (d, *J =* 8.5 Hz, 2H), 7.40 (dt, *J =* 8.7, 5.0 Hz, 3H), 6.94 (dd, *J =* 8.8, 4.2 Hz, 1H), 6.87 (d, *J =* 5.7 Hz, 1H), 6.74 (d, *J* = 8.8 Hz, 1H), 4.81 (d, *J* = 5.9 Hz, 2H), 4.39 (d, *J =* 7.2 Hz, 1H), 4.00 (s, 3H), 3.45 (t, *J* = 5.8 Hz, 2H), 2.92-2.84 (m, 2H), 2.72-2.65 (m, 2H), 2.58-2.51 (m, 4H), 1.34-1.18 (m, 3H), 0.49-0.37 (m, 2H), 0.37-0.25 (m, 2H).

### Example 56: Preparation of Compound 56

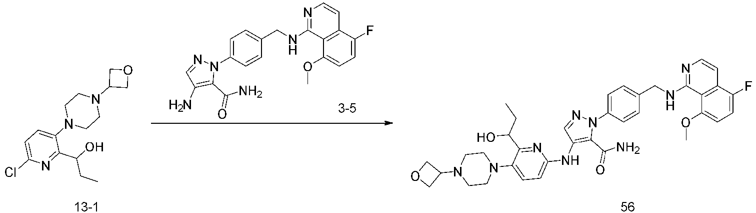

### Preparation of compound 56:

Referring to the preparation steps of compound **3** in Example **3,** intermediate **1-3** was replaced with intermediate **13-1,** and cesium carbonate was replaced with potassium carbonate. After the reaction was completed, the reaction solution was filtered and concentrated to give a concentrate, which was separated by preparative liquid chromatography to give compound **56.**

LC-MS: m/z 682.37 (M+H)⁺.

¹H NMR (500 MHz, DMSO-*d*₆) δ 8.46 (t, *J =* 5.9 Hz, 1H), 8.31 (s, 1H), 8.25 (s, 1H), 8.17 (s, 1H), 7.91 (d, *J =* 5.8 Hz, 1H), 7.54 (d, *J =* 8.7 Hz, 1H), 7.50-7.24 (m, 6H), 6.94 (dd, *J =* 8.8, 4.2 Hz, 1H), 6.87 (d, *J =* 5.8 Hz, 1H), 6.74 (d, *J =* 8.7 Hz, 1H), 4.81 (d, *J =* 6.0 Hz, 3H), 4.55 (t, *J =* 6.5 Hz, 2H), 4.45 (td, *J =* 6.2, 1.3 Hz, 2H), 4.00 (s, 3H), 3.47 (p, *J =* 6.3 Hz, 2H), 2.92 (dt, *J =* 10.2, 4.3 Hz, 2H), 2.71 (dt, *J =* 10.4, 4.4 Hz, 2H), 2.39 (s, 3H), 1.81-1.65 (m, 2H), 1.24 (s, 1H), 0.84 (t, *J =* 7.4 Hz, 3H).

### Example 57: Preparation of Compounds 57, 57-a, and 57-b

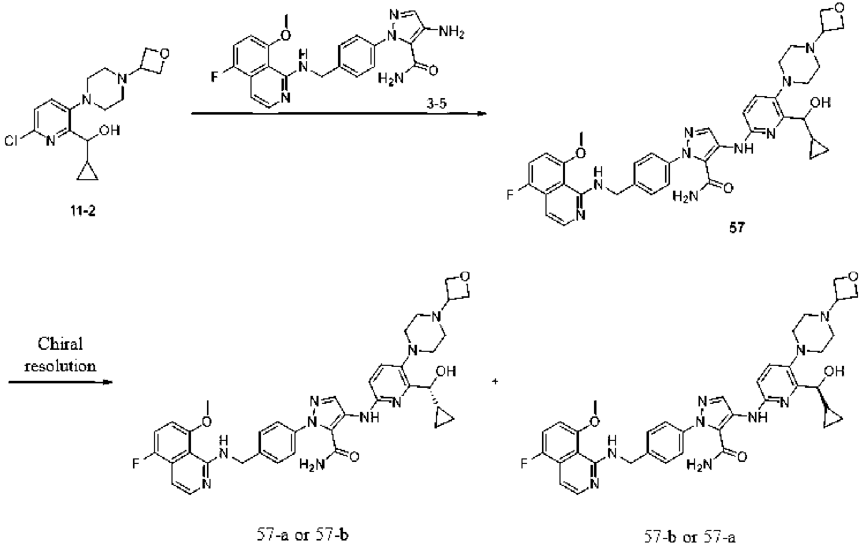

### Preparation of compound 57:

Intermediate **11-2** (200 mg) and intermediate **3-5** (200 mg) were added to tert-butanol (50 mL), lithium bis(trimethylsilyl)amide (1 M, 500 µL), 2-(dicyclohexylphosphino)-3,6-dimethoxy-2'-4'-6'-tri-I-propyl-11'-biphenyl (106 mg), and chloro(2-dicyclohexylphosphino-3,6-dimethoxy-2',4',6'-triisopropyl-1,1'-biphenyl)[2-(2-aminoethylphenyl)]palladium(II) (80 mg) were added, and the mixture was reacted at 85 °C for 1 h under nitrogen atmosphere.

After the reaction was completed, the reaction solution was filtered, and the filtrate was extracted with ethyl acetate (100 mL) and purified water (100 mL). The mixture was stirred for 10 min and left to stand for liquid separation. The aqueous phase was extracted with ethyl acetate (50 mL × 3), and the organic phases were combined, washed with saturated brine, dried over anhydrous sodium sulfate, and filtered. The filtrate was concentrated under reduced pressure to give a concentrate, which was subjected to preparative liquid chromatography to give compound **57.**

¹H NMR (500 MHz, DMSO-*d*₆) δ 8.45 (t, *J =* 6.0 Hz, 2H), 8.33-8.27 (m, 2H), 7.91 (d, *J =* 5.8 Hz, 1H), 7.56 (d, *J =* 8.7 Hz, 1H), 7.45 (d, *J =* 8.6 Hz, 2H), 7.42-7.36 (m, 2H), 6.93 (dd, *J* = 8.8, 4.2 Hz, 1H), 6.87 (d, *J =* 5.8 Hz, 1H), 6.75 (d, *J =* 8.7 Hz, 1H), 5.15-4.97 (m, 2H), 4.81 (d, *J =* 5.9 Hz, 2H), 4.55 (t, *J =* 6.5 Hz, 2H), 4.45 (dd, *J* = 6.1, 2.9 Hz, 2H), 4.41 (s, 3H), 4.34-4.00 (m, 8H), 2.95-2.64 (m, 4H).

### Preparation of compounds 57-a and 57-b:

Compound 57 was subjected to chiral resolution to give compound **57-a** and compound **57-b.** The resolution conditions were as follows: a YMC Cellulose SC preparative column (30 × 250 mm, 10 µm) was used; by taking dichloromethane as mobile phase A and methanol as mobile phase B, gradient elution was performed at a flow rate of 30 mL/min; the detection wavelength was 254 nm, and the retention times were 27 min (57-a) and 28 min (57-b), respectively.

Compound **57-a** LC-MS: m/z 694.11 (M+H)⁺.

Compound **57-b** LC-MS: m/z 694.15 (M+H)⁺.

Compound 57-a had a shorter retention time in the chiral chromatography column than compound 57-b, and compound 57-b had a longer retention time in the chiral chromatography column than compound 57-a.

### Example 58: Preparation of Compounds 58, 58-a, and 58-b

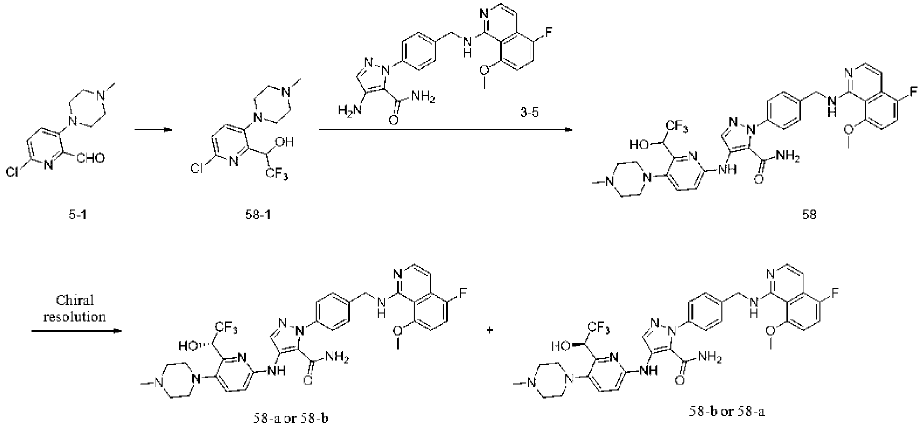

### Preparation of intermediate 58-1:

Referring to the preparation steps of intermediate **12-3** in Example **12,** intermediate **12-2** was replaced with intermediate **5-1,** and after the reaction was completed, a saturated ammonium chloride solution (5 mL) was added to quench the reaction, and ethyl acetate (60 mL × 3) was added for extraction. The organic phases were combined, washed with saturated sodium chloride solution, dried over anhydrous sodium sulfate, and filtered. The filtrate was concentrated to give intermediate **58-1** (500 mg).

LC-MS: m/z 310.08 (M+H)⁺.

### Preparation of compound 58:

Referring to the preparation steps of compound **3** in Example **3,** intermediate **1-3** was replaced with intermediate **58-1,** and cesium carbonate was replaced with potassium carbonate. After the reaction was completed, the reaction solution was filtered and concentrated to give a concentrate, which was separated by preparative liquid chromatography to give compound **58.**

LC-MS: m/z 680.35 (M+H)⁺.

¹H NMR (500 MHz, DMSO-*d*₆) δ 8.50 (s, 1H), 8.46 (t*, J =* 6.0 Hz, 1H), 8.34 (s, 1H), 7.91 (d, *J =* 5.8 Hz, 1H), 7.68 (d, *J =* 8.9 Hz, 2H), 7.48-7.36 (m, 6H), 6.94 (dd, *J* = 8.9, 3.7 Hz, 2H), 6.86 (d, *J =* 5.7 Hz, 1H), 6.78 (d, *J =* 7.5 Hz, 1H), 5.60 (s, 1H), 4.81 (d, *J =* 5.9 Hz, 2H), 4.00 (s, 3H), 2.85 (d, *J =* 8.7 Hz, 2H), 2.72 *(d, J =* 8.3 Hz, 2H), 2.46 (s, 3H), 2.23 (s, 3H).

### Preparation method for compounds 58-a and 58-b:

Compound **58** was subjected to chiral resolution to give compound **58-a** and compound **58-b.** The resolution conditions were as follows: a Daicel preparative column (20 × 250 mm, 10 µm) was used; by taking n-hexane as mobile phase A and ethanol as mobile phase B, gradient elution was performed at a flow rate of 30 mL/min; the detection wavelength was 254 nm, and the retention times were 18.4 min (58-a) and 22.4 min (58-b), respectively. Compound **58-a** LC-MS: m/z 680.25 (M+H)⁺.

Compound **58-b** LC-MS: m/z 680.27 (M+H)⁺.

Compound 58-a had a shorter retention time in the chiral chromatography column than compound 58-b, and compound 58-b had a longer retention time in the chiral chromatography column than compound 58-a.

### Example 59: Preparation of Compounds 59-a and 59-b

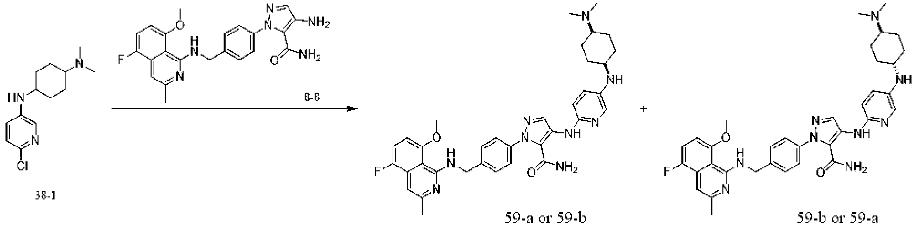

### Preparation of compounds 59-a and 59-b:

Referring to the preparation steps of compound **9** in Example **9,** intermediate **9-2** was replaced with intermediate **38-1,** and the reaction system was separated by preparative liquid chromatography to give compound **59-a** and compound **59-b.** The conditions were as follows: a YMC-AQ-C18 preparative column (50 × 250 mm, 10.0 µm) was used; by taking methanol as mobile phase A and aqueous ammonium acetate solution as mobile phase B, gradient elution was performed at a flow rate of 50 mL/min; the detection wavelength was 254 nm, and the retention times were 25.0 min (**59-a**) and 32.0 min (**59-b**), respectively.

Compound **59-a** LC-MS: m/z 638.43 (M+H)⁺.

Compound **59-b** LC-MS: m/z 638.43 (M+H)⁺.

Compound 59-a had a shorter retention time in the chiral chromatography column than compound 59-b, and compound 59-b had a longer retention time in the chiral chromatography column than compound 59-a.

### Example 60: Preparation of Compound 60

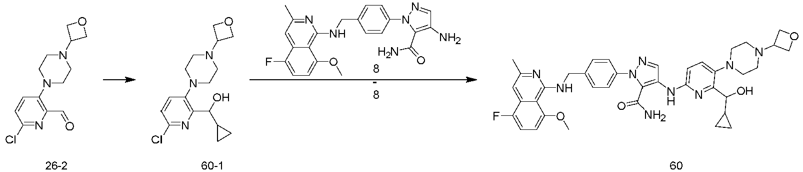

### Preparation of intermediate 60-1:

Referring to the preparation steps of intermediate **6-1** in Example **6,** intermediate **1-1** was replaced with intermediate **26-2,** and after the reaction was completed, compound **60-1** (500 mg) was obtained.

LC-MS: m/z 324.25 (M+H)⁺.

### Preparation of compound 60:

Referring to the preparation steps of compound **10** in Example **10,** intermediate **10-3** was replaced with intermediate **60-1,** and after the reaction was completed, compound **60** was obtained.

LC-MS: m/z 708.35 (M+H)⁺.

¹H NMR (500 MHz, DMSO-*d*₆) δ 8.40-8.31 (m, 2H), 8.27 (s, 1H), 7.57 (d, *J =* 8.7 Hz, 1H), 7.52-7.46 (m, 2H), 7.45-7.38 (m, 2H), 7.32 (dd, *J* = 10.0, 8.7 Hz, 1H), 6.82 (dd, *J* = 8.8, 4.2 Hz, 1H), 6.77-6.69 (m, 2H), 5.07 (d*, J =* 6.7 Hz, 1H), 4.80 (d, *J* = 5.9 Hz, 2H), 3.97 (s, 3H), 2.91 (dt, *J =* 10.1, 4.3 Hz, 2H), 2.72 (dt, *J =* 10.3, 4.4 Hz, 2H), 2.34 (s, 8H), 1.37-1.20 (m, 7H), 0.52-0.19 (m, 4H).

### Example 61: Preparation of Compounds 61, 61-a, and 61-b

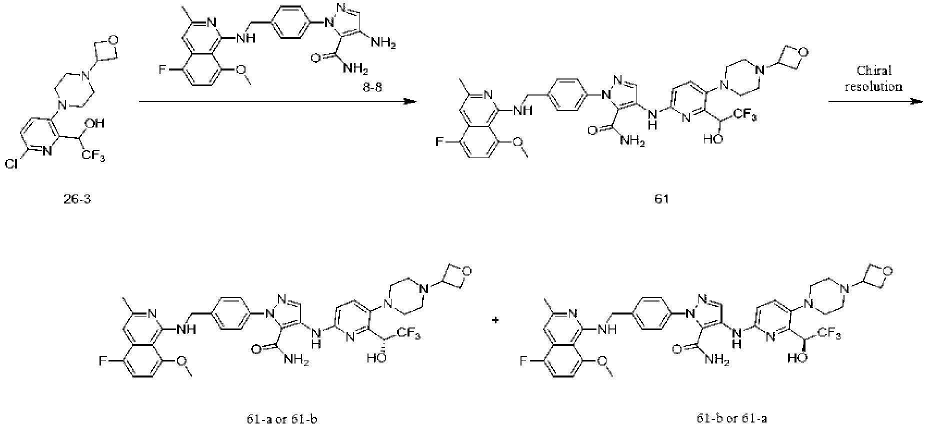

### Preparation of compound 61:

Referring to the preparation steps of compound **10** in Example **10,** intermediate **10-3** was replaced with intermediate **26-3,** and after the reaction was completed, compound **61** was obtained.

LC-MS: m/z 736.41 (M+H)⁺.

### Preparation of compounds 61-a and 61-b:

Compound **61** was subjected to chiral resolution to give compound **61-a** and compound **61-b.** The resolution conditions were as follows: a CHIRALPAK IC preparative column (4.6 × 250 mm, 5.0 µm) was used; by taking n-hexane as mobile phase A and ethanol as mobile phase B, gradient elution was performed at a flow rate of 0.5 mL/min; the detection wavelength was 254 nm, and the retention times were 34.010 min (61-a) and 46.978 min (61-b), respectively.

Compound 61-a LC-MS: m/z 736.45 (M+H)⁺.

¹H NMR (500 MHz DMSO-*d*₆) δ 8.51 (s, 1H), 8.35 (m, 2H), 7.71 (d, *J =* 9.0 Hz, 1H), 7.49 (d, *J =* 8.5 Hz, 2H), 7.40 (d, *J =* 8.5 Hz, 2H), 7.32 (dd, *J =* 9.0, 10.0 Hz, 1H), 6.94 (d, *J =* 9.0 Hz, 1H), 6.82 (dd, *J =* 9.0, 4.0 Hz, 1H), 6.72 (m, 2H), 5.60 (m, 1H), 4.80 (d, *J =* 6.0 Hz, 2H), 4.56 (t, *J =* 6.5 Hz, 2H), 4.46 (t, *J =* 6.0 Hz, 2H), 3.97 (s, 3H), 3.48 (m, 1H), 2.89 (m, 2H), 2.74 (m, 2H), 2.40 (m, 4H), 2.34 (s, 3H).

Compound 61-b LC-MS: m/z 736.41 (M+H)⁺.

¹H NMR (500 MHz DMSO-*d*₆) δ 8.51 (s, 1H), 8.35 (m, 2H), 7.71 (d, *J =* 9.0 Hz, 1H), 7.49 (d, *J =* 8.5 Hz, 2H), 7.40 (d, *J =* 8.5 Hz, 2H), 7.32 (m, 1H), 6.94 (d, *J =* 9.0 Hz, 1H), 6.82 (dd, *J =* 9.0, 4.0 Hz, 1H), 6.72 (m, 2H), 5.60 (m, 1H), 4.80 (d, *J =* 6.0 Hz, 2H), 4.56 (t, *J =* 6.5 Hz, 2H), 4.46 (t, *J =* 6.0 Hz, 2H), 3.97 (s, 3H), 3.48 (m, 1H), 2.89 (m, 2H), 2.74 (m, 2H), 2.40 (m, 4H), 2.34 (s, 3H).

Compound 61-a had a shorter retention time in the chiral chromatography column than compound 61-b, and compound 61-b had a longer retention time in the chiral chromatography column than compound 61-a.

### Example 62: Preparation of Compounds 62, 62-a, and 62-b

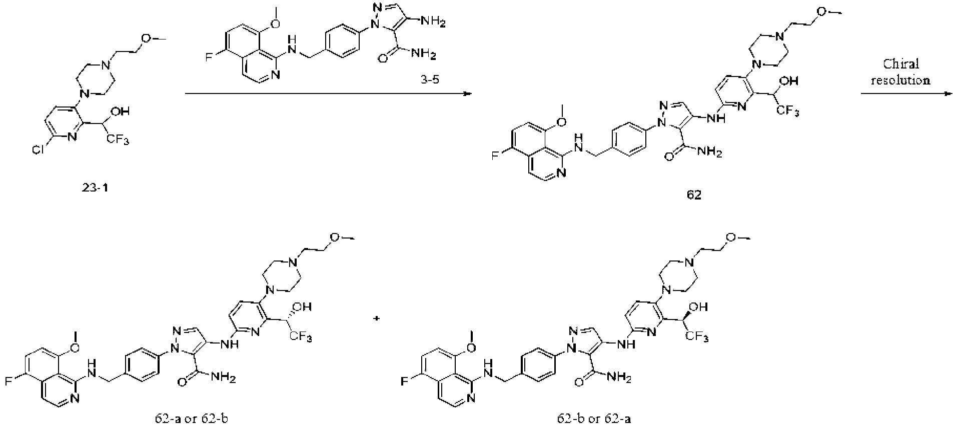

### Preparation of compound 62:

Intermediate **23-1** (171 mg), intermediate **3-5** (150 mg), chloro(2-dicyclohexylphosphino-3,6-dimethoxy-2',4',6'-triisopropyl-1,1'-biphenyl)[2-(2-aminoethylphenyl)]palladium(II) (59 mg), 2-(dicyclohexylphosphino)-3,6-dimethoxy-2'-4'-6'-tri-I-propyl-11'-biphenyl (79 mg), and potassium bis(trimethylsilyl)amide (0.4 mL) were added to tert-butanol (20 mL). The mixture was stirred at 80 °C for 0.5 h under nitrogen atmosphere.

After the reaction was completed, ethyl acetate (20 mL) and purified water (20 mL) were added, and the mixture was stirred for 10 min and left to stand for liquid separation. The aqueous phase was extracted with ethyl acetate (20 mL × 2). The organic phases were combined, washed with saturated brine, dried over anhydrous sodium sulfate, and filtered, and the filtrate was concentrated under reduced pressure to give a concentrate, which was purified by preparative liquid chromatography to give compound **62.**

LC-MS: m/z 724.43(M+H)⁺.

### Preparation of compounds 62-a and 62-b:

Compound **62** was subjected to chiral resolution to give compound **62-a** and compound **62-b.** The resolution conditions were as follows: a Daicel ID preparative column (20 × 250 mm, 10 µm) was used; gradient elution was performed with ethanol-n-hexane:dichloromethane = 3:1 (5%-60%/0-55 min) at a flow rate of 30 mL/min; the detection wavelength was 254 nm, and the retention times were 23.2 min (62-a) and 27.0 min (62-b).

Compound 62-a LC-MS: m/z 724.39 (M+H)⁺.

Compound 62-b LC-MS: m/z 724.36 (M+H)⁺.

Compound 62-b ¹H NMR (500 MHz, DMSO *-d*₆) δ 8.53-8.43 (m, 2H), 8.34 (s, 1H), 7.91 (d, *J =* 5.8 Hz, 1H), 7.69 (d, *J =* 8.8 Hz, 1H), 7.51-7.34 (m, 6H), 6.94 (dd, *J* = 8.9, 4.8 Hz, 2H), 6.87 (d, *J =* 5.8 Hz, 1H), 6.81 (d, *J* = 7.6 Hz, 1H), 5.60 (t, *J =* 7.6 Hz, 1H), 4.81 (d, *J =* 6.0 Hz, 2H), 4.00 (s, 3H), 3.46 (t, *J =* 5.8 Hz, 2H), 3.25 (s, 3H), 2.85 (d, *J =* 9.5 Hz, 2H), 2.75-2.68 (m, 2H).

Compound 62-a had a shorter retention time in the chiral chromatography column than compound 62-b, and compound 62-b had a longer retention time in the chiral chromatography column than compound 62-a.

### Example 63: Preparation of Compounds 63, 63-a, and 63-b

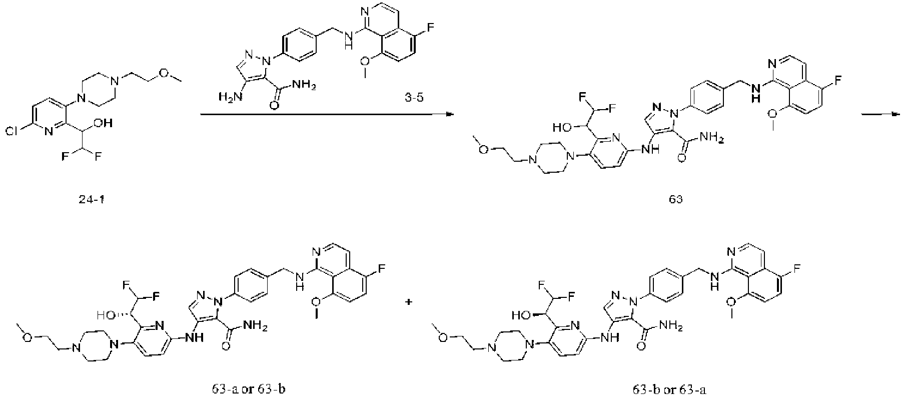

### Preparation of compound 63:

Referring to the preparation steps of compound **3** in Example **3,** intermediate **1-3** was replaced with intermediate **24-1,** and cesium carbonate was replaced with potassium carbonate. After the reaction was completed, the reaction solution was filtered and concentrated to give a concentrate, which was separated by preparative liquid chromatography to give compound **63.**

LC-MS: m/z 706.37 (M+H)⁺.

¹H NMR (500 MHz, DMSO-*d*₆) δ 8.46 (t, *J =* 6.0 Hz, 1H), 8.40 (s, 1H), 8.17 (s, 1H), 7.91 (d, *J* = 5.8 Hz, 1H), 7.60 (d, *J* = 8.8 Hz, 1H), 7.53-7.34 (m, 6H), 6.94 (dd, *J =* 8.8, 4.3 Hz, 1H), 6.89-6.79 (m, 2H), 6.23-6.03 (m, 2H), 5.11 (dq, *J =* 12.2, 5.7 Hz, 1H), 4.81 (d, *J =* 5.9 Hz, 2H), 4.00 (s, 3H), 3.47 (d, *J =* 6.3 Hz, 2H), 3.25 (s, 3H), 2.90 (s, 2H), 2.74 (s, 2H), 1.36-1.18 (m, 4H), 0.91 (t, *J =* 7.3 Hz, 2H).

### Preparation method for compounds 63-a and 63-b:

Compound **63** was subjected to chiral resolution to give compound **63-a** and compound **63-b.** The resolution conditions were as follows: a Cellulose-SC preparative column (30 × 250 mm, 10 µm) was used; by taking n-hexane as mobile phase A and ethanol as mobile phase B, gradient elution was performed at a flow rate of 0.8 mL/min; the detection wavelength was 254 nm, and the retention times were 23 min (63-a) and 29 min (63-b), respectively.

Compound 63-a LC-MS: m/z 706.35 (M+H)⁺.

Compound 63-b LC-MS: m/z 706.37 (M+H)⁺.

Compound 63-a had a shorter retention time in the chiral chromatography column than compound 63-b, and compound 63-b had a longer retention time in the chiral chromatography column than compound 63-a.

### Example 64: Preparation of Compound 64

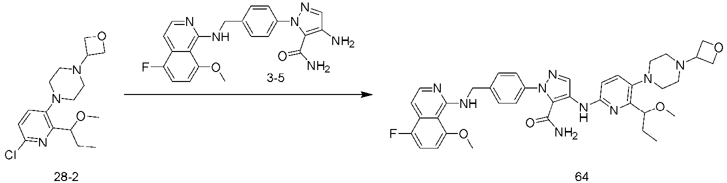

### Preparation of compound 64:

Referring to the preparation steps of compound **53** in Example **53,** intermediate **53-1** was replaced with intermediate **28-2,** and after the reaction was completed, the reaction system was purified by preparative liquid chromatography to give compound **64.**

LC-MS: m/z 696.23 (M+H)⁺.

### Example 65: Preparation of Compound 65

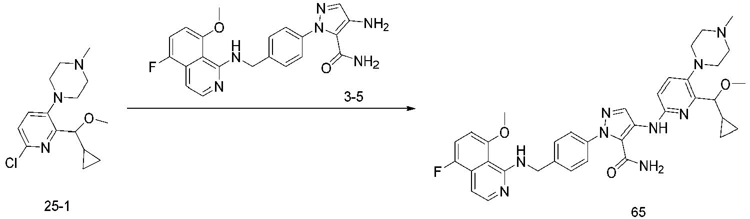

### Preparation of compound 65:

Intermediate **25-1** (113 mg), intermediate **3-5** (120 mg), chloro(2-dicyclohexylphosphino-3,6-dimethoxy-2',4',6'-triisopropyl-1,1'-biphenyl)[2-(2-aminoethylphenyl)]palladium(II) (47 mg), 2-(dicyclohexylphosphino)-3,6-dimethoxy-2'-4'-6'-tri-I-propyl-11'-biphenyl (63 mg), and potassium bis(trimethylsilyl)amide (0.3 mL) were added to tert-butanol (20 mL). The mixture was stirred at 80 °C for 0.5 h under nitrogen atmosphere.

After the reaction was completed, ethyl acetate (20 mL) and purified water (20 mL) were added, and the mixture was stirred for 10 min and left to stand for liquid separation. The aqueous phase was extracted with ethyl acetate (20 mL × 2). The organic phases were combined, washed with saturated brine, dried over anhydrous sodium sulfate, and filtered, and the filtrate was concentrated under reduced pressure to give a concentrate, which was purified by preparative liquid chromatography to give compound **65.**

LC-MS: m/z 666.42 (M+H)⁺.

¹H NMR (500 MHz, DMSO-*d*₆) δ 8.46 (t, *J =* 6.0 Hz, 1H), 8.36 (s, 1H), 8.32 (s, 1H), 7.91 (d, *J =* 5.8 Hz, 1H), 7.54 (d, *J =* 8.7 Hz, 1H), 7.45 (s, 1H), 7.44 (s, 1H), 7.42-7.37 (m, 3H), 6.94 (dd, *J* = 8.8, 4.2 Hz, 1H), 6.87 (d*, J =* 5.7 Hz, 1H), 6.79 (d, *J =* 8.7 Hz, 1H), 4.81 (d, *J =* 5.9 Hz, 2H), 4.14 (d, *J =* 8.7 Hz, 1H), 4.00 (s, 3H), 3.14 (s, 3H), 2.82-2.75 (m, 2H), 2.70 (d, *J =* 6.2 Hz, 2H), 2.42 (s, 3H), 2.21 (s, 3H), 1.39-1.32 (m, 1H), 1.27-1.22 (m, 1H), 0.62 (qd, *J* = 8.4, 4.5 Hz, 1H), 0.47 (dq, *J =* 9.6, 4.9 Hz, 1H), 0.33 (dp, *J =* 12.8, 4.3 Hz, 1H), 0.13 (dq, *J =* 9.6, 4.8 Hz, 1H).

### Example 66: Preparation of Compound 66

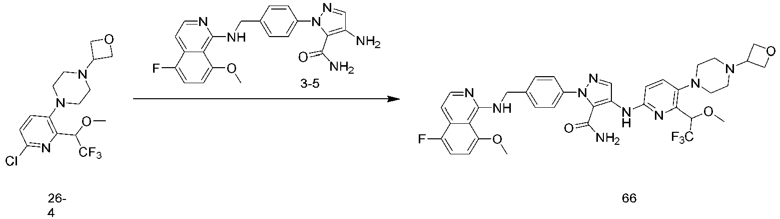

### Preparation of compound 66:

Referring to the preparation steps of compound **53** in Example **53,** intermediate **53-1** was replaced with intermediate **26-4,** and after the reaction was completed, the reaction system was purified by preparative liquid chromatography to give compound **66.**

LC-MS: m/z 736.15 (M+H)⁺.

### Example 67: Preparation of Compound 67

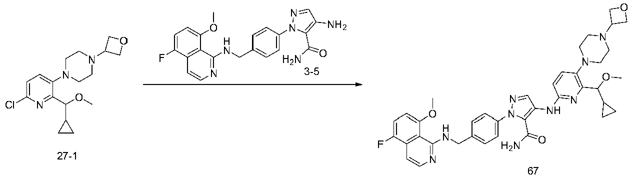

### Preparation of compound 67:

Intermediate **27-1** (150 mg) and intermediate **3-5** (150 mg) were added to tert-butanol (38 mL), lithium bis(trimethylsilyl)amide (1 M, 375 µL), 2-(dicyclohexylphosphino)-3,6-dimethoxy-2'-4'-6'-tri-I-propyl-11'-biphenyl (80 mg), and chloro(2-dicyclohexylphosphino-3,6-dimethoxy-2',4',6'-triisopropyl-1,1'-biphenyl)[2-(2-aminoethylphenyl)]palladium(II) (60 mg) were added, and the mixture was reacted at 85 °C for 1 h under nitrogen atmosphere.

After the reaction was completed, the reaction solution was filtered, and the filtrate was extracted with ethyl acetate (100 mL) and purified water (100 mL). The mixture was stirred for 10 min and left to stand for liquid separation. The aqueous phase was extracted with ethyl acetate (50 mL × 3), and the organic phases were combined, washed with saturated brine, dried over anhydrous sodium sulfate, and filtered. The filtrate was concentrated under reduced pressure to give a concentrate, which was separated by preparative liquid chromatography to give compound 67.

LC-MS: m/z 708.23 (M+H)⁺.

### Example 68: Preparation of Compounds 68, 68-a, and 68-b

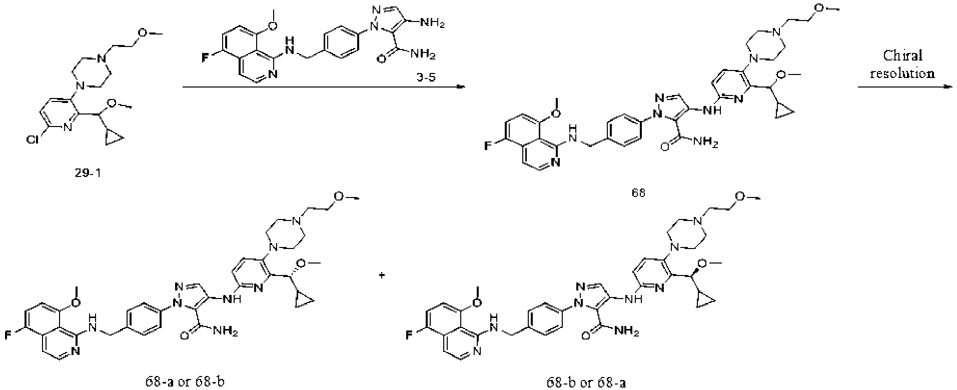

### Preparation of compound 68:

Intermediate **29-1** (130 mg), intermediate **3-5** (120 mg), chloro(2-dicyclohexylphosphino-3,6-dimethoxy-2',4',6'-triisopropyl-1,1'-biphenyl)[2-(2-aminoethylphenyl)]palladium(II) (47 mg), 2-(dicyclohexylphosphino)-3,6-dimethoxy-2'-4'-6'-tri-I-propyl-11'-biphenyl (63 mg), and potassium bis(trimethylsilyl)amide (0.3 mL) were added to tert-butanol (20 mL). The mixture was stirred at 80 °C for 0.5 h under nitrogen atmosphere.

After the reaction was completed, ethyl acetate (20 mL) and purified water (20 mL) were added, and the mixture was stirred for 10 min and left to stand for liquid separation. The aqueous phase was extracted with ethyl acetate (20 mL × 2). The organic phases were combined, washed with saturated brine, dried over anhydrous sodium sulfate, and filtered, and the filtrate was concentrated under reduced pressure to give a concentrate, which was purified by preparative liquid chromatography to give compound **68.**

LC-MS: m/z 710.38 (M+H)⁺.

### Preparation of compounds 68-a and 68-b:

Compound **68** was subjected to chiral resolution to give compound **68-a** and compound **68-b.** The resolution conditions were as follows: a YMC Cellulose SC chromatography column (30 mm × 250 mm, 10 µm) was used; gradient elution was performed with ethanol-n-hexane (10%-60%/0-40 min) at a flow rate of 40 mL/min; the detection wavelength was 254 nm, and the retention times were 23.5 min (68-a) and 29.5 min (68-b), respectively. Compound 68-a LC-MS: m/z 710.40 (M+H)⁺.

Compound 68-b LC-MS: m/z 710.46 (M+H)⁺.

Compound 68-b ¹H NMR (500 MHz, DMSO-*d*₆) δ 8.46 (t, *J =* 6.0 Hz, 1H), 8.35 (s, 1H), 8.32 (s, 1H), 7.91 (d, *J =* 5.8 Hz, 1H), 7.55 (d, *J =* 8.8 Hz, 1H), 7.44 (d, *J =* 8.4 Hz, 2H), 7.42-7.38 (m, 3H), 6.94 (dd, *J =* 8.8, 4.2 Hz, 1H), 6.87 (d, *J =* 5.7 Hz, 1H), 6.79 (d, *J =* 8.8 Hz, 1H), 4.81 (d, *J =* 5.9 Hz, 2H), 4.15 (d, *J =* 8.8 Hz, 1H), 4.00 (s, 3H), 3.45 (t, *J =* 5.8 Hz, 2H), 3.32 (s, 5H), 3.14 (s, 3H), 2.82-2.75 (m, 2H), 2.73-2.66 (m, 2H), 1.36 (td, *J =* 8.4, 4.0 Hz, 1H), 1.25 (d, *J =* 12.9 Hz, 1H), 0.66-0.59 (m, 1H), 0.47 (dq, *J =* 9.6, 4.8 Hz, 1H), 0.37-0.29 (m, 1H), 0.13 (dq, *J =* 9.5, 5.0 Hz, 1H).

Compound 68-a had a shorter retention time in the chiral chromatography column than compound 68-b, and compound 68-b had a longer retention time in the chiral chromatography column than compound 68-a.

### Example 69: Preparation of Compound 69

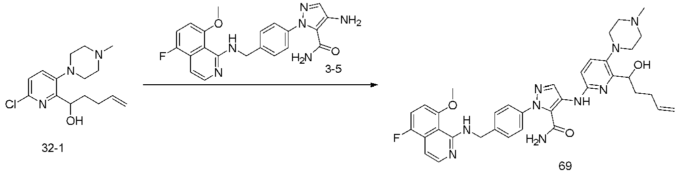

### Preparation of compound 69:

Intermediate **32-1** (120 mg) and intermediate **3-5** (120 mg) were added to tert-butanol (30 mL), lithium bis(trimethylsilyl)amide (1 M, 300 µL), 2-(dicyclohexylphosphino)-3,6-dimethoxy-2'-4'-6'-tri-I-propyl-11'-biphenyl (64 mg), and chloro(2-dicyclohexylphosphino-3,6-dimethoxy-2',4',6'-triisopropyl-1,1'-biphenyl)[2-(2-aminoethylphenyl)]palladium(II) (48 mg) were added, and the mixture was reacted at 85 °C for 1 h under nitrogen atmosphere.

After the reaction was completed, the reaction solution was filtered, and the filtrate was extracted with ethyl acetate (100 mL) and purified water (100 mL). The mixture was stirred for 10 min and left to stand for liquid separation. The aqueous phase was extracted with ethyl acetate (50 mL × 3), and the organic phases were combined, washed with saturated brine, dried over anhydrous sodium sulfate, and filtered. The filtrate was concentrated under reduced pressure to give a concentrate, which was separated by preparative liquid chromatography to give compound **69.**

LC-MS: m/z 666.13 (M+H)⁺.

### Example 70: Preparation of Compound 70

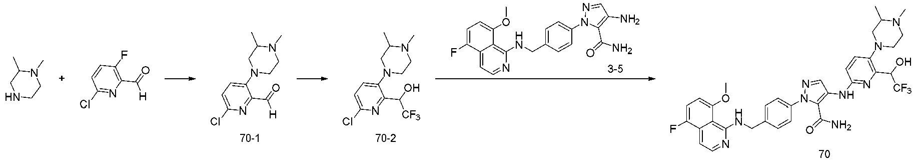

### Preparation of intermediate 70-1:

1,2-Dimethylpiperazine (2.3 g) and 2-chloro-5-fluoropyridine-6-carbaldehyde (3.19 g) were added to N,N-dimethylformamide (20 mL), and potassium carbonate (11 g) was added. The mixture was stirred with the temperature controlled at 80-90 °C under nitrogen atmosphere.

After the reaction was completed, the reaction solution was extracted with purified water (200 mL) and dichloromethane (200 mL). The organic phase was dried over anhydrous sodium sulfate and filtered, and the filtrate was concentrated under reduced pressure to give intermediate **70-1** (5.1 g).

LC-MS: m/z 254.16 (M+H)⁺.

### Preparation of intermediate 70-2:

Intermediate **70-1** (5.1 g), cesium fluoride (4.56 g), and (trifluoromethyl)trimethylsilane (4.27 g) were added to tetrahydrofuran (100 mL), and the mixture was stirred at 20-30 °C under nitrogen atmosphere.

After the reaction was completed, a saturated ammonium chloride solution (100 mL) and dichloromethane (100 mL) were added. The mixture was stirred for extraction and left to stand for liquid separation. The organic phase was dried over anhydrous sodium sulfate and filtered, and the filtrate was concentrated under reduced pressure to give intermediate **70-2** (6.4 g).

LC-MS: m/z 324.1 (M+H)⁺.

### Preparation of compound 70:

Intermediate **70-2** (50 mg), intermediate **3-5** (50 mg), chloro(2-dicyclohexylphosphino-3,6-dimethoxy-2',4',6'-triisopropyl-1,1'-biphenyl)[2-(2-aminoethylphenyl)]palladium(II) (20 mg), 2-(dicyclohexylphosphino)-3,6-dimethoxy-2'-4'-6'-tri-I-propyl-11'-biphenyl (26.6 mg), and potassium carbonate (34.2 mg) were added to tert-butanol (25 mL). The mixture was reacted at 85 °C under microwave at 150 W under nitrogen atmosphere until the reaction was completed.

Ethyl acetate (50 mL) was added for dilution, and the mixture was filtered. The filtrate was concentrated under reduced pressure to give a concentrate, which was purified by preparative liquid chromatography to give compound **70.**

LC-MS: m/z 694.36 (M+H)⁺.

### Example 71: Preparation of Compounds 71-a and 71-b

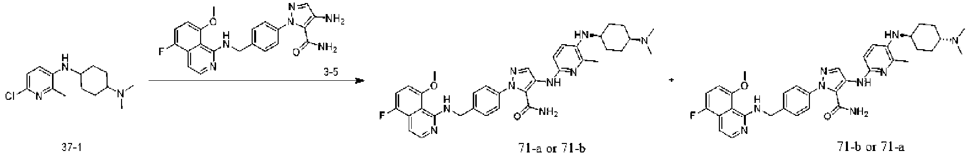

### Preparation of compounds 71-a and 71-b:

Intermediate **37-1** (98 mg) and intermediate **3-5** (106 mg) were added to tert-butanol (40 mL), methanesulfonato(2-di-tert-butylphosphino-2',4',6'-triisopropyl-1,1'-biphenyl)(2'-amino-1,1'-biphenyl-2-yl)palladium(II) (48 mg), 2-di-tert-butylphosphino-2',4',6'-triisopropylbiphenyl (74 mg), and potassium carbonate (120 mg) were added, and the mixture was stirred at 80 °C for 6 h under nitrogen atmosphere.

After the reaction was completed, ethyl acetate (30 mL) and purified water (30 mL) were added, and the mixture was stirred for 10 min and then left to stand for liquid separation. The aqueous phase was extracted with ethyl acetate (30 mL × 3). The organic phases were combined, washed with saturated brine, dried over anhydrous sodium sulfate, and filtered, and the filtrate was concentrated under reduced pressure to give a concentrate, which was separated by preparative liquid chromatography to give compounds **71-a** and **71-b.**

The preparative conditions were as follows: a YMC TA C18 chromatography column (30 × 250 mm, 10 µm) was used; gradient elution was performed with acetonitrile-0.1% aqueous formic acid solution (5%-50%/0-60 min) at a flow rate of 40 mL/min; the detection wavelength was 254 nm; compound **71-a** (retention time: 23.8 min) and compound **71-b** (retention time: 32.5 min) were obtained in sequence.

Compound **71-a** LC-MS: m/z 638.32 (M+H)⁺.

Compound **71-b** LC-MS: m/z 638.30 (M+H)⁺.

Compound **71-b** ¹H NMR (500 MHz, DMSO-*d*₆) δ 8.46 (t, *J =* 6.0 Hz, 1H), 8.12 (s, 1H), 7.91 (d, *J =* 5.8 Hz, 1H), 7.80 (s, 1H), 7.46-7.42 (m, 2H), 7.42-7.36 (m, 3H), 6.94 (dd, *J* = 8.7, 5.0 Hz, 2H), 6.87 (d, *J =* 5.7 Hz, 1H), 6.57 (d, *J* = 8.6 Hz, 1H), 4.81 (d, *J =* 6.0 Hz, 2H), 4.00 (s, 3H), 3.04 (tt, *J =* 10.6, 3.7 Hz, 1H), 2.24 (s, 3H), 2.18 (s, 6H), 2.01-1.94 (m, 2H), 1.90 (s, 3H), 1.85-1.78 (m, 2H), 1.33-1.15 (m, 4H).

Compound 71-a had a shorter retention time in the chiral chromatography column than compound 71-b, and compound 71-b had a longer retention time in the chiral chromatography column than compound 71-a.

### Example 72: Preparation of Compound 72

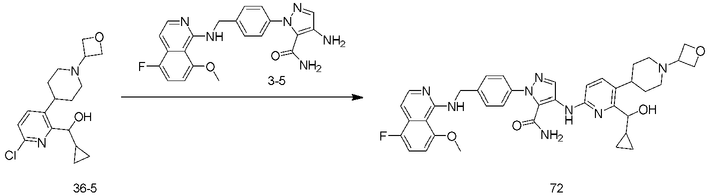

### Preparation of compound 72:

Referring to the preparation steps of compound **53** in Example **53,** intermediate **53-1** was replaced with intermediate **36-5,** and after the reaction was completed, compound **72** was obtained.

LC-MS: m/z 693.21 (M+H)⁺.

### Example 73: Preparation of Compounds 73-a and 73-b

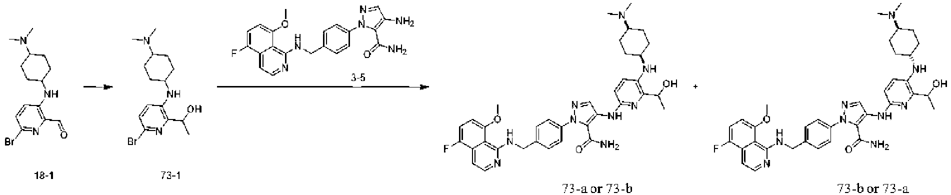

### Preparation of intermediate 73-1:

Intermediate **18-1** (2.5 g) was added to THF (50 mL), and methylmagnesium bromide (3 M, 2.5 mL) was added dropwise with the temperature controlled at -20 °C under nitrogen atmosphere. After the dropwise addition was completed, the mixture was stirred for 3 h with the temperature controlled at 0 °C. After the reaction was completed, a saturated ammonium chloride solution (20 mL) was added to quench the reaction, and the reaction system was extracted with ethyl acetate (50 mL × 3). The organic phases were combined, washed with saturated sodium chloride solution, dried over anhydrous sodium sulfate, and filtered under vacuum, and the filtrate was concentrated under reduced pressure. The residue was purified by preparative chromatography to give intermediate **73-1** (600 mg).

LC-MS: m/z 342.14 (M+H)⁺.

### Preparation of compounds 73-a and 73-b:

Referring to the preparation steps of compound **53** in Example **53,** intermediate **53-1** was replaced with intermediate **73-1.**

After the reaction was completed, the reaction system was subjected to preparative liquid chromatography to give compounds **73-a** and **73-b.** The conditions were as follows: a YMC AQ C18 preparative column (30 × 250 mm, 10 µm) was used; by taking 0.1% acetic acid as mobile phase A and acetonitrile as mobile phase B, gradient elution was performed at a flow rate of 30 mL/min; the detection wavelength was 254 nm, and the retention times were 29 min (**73-a**) and 32 min (**73-b**), respectively.

Compound 73-a LC-MS: m/z 668.25 (M+H)⁺.

¹H NMR (500 MHz DMSO-*d*₆) δ 8.46 (t, *J =* 6.0 Hz, 1H), 8.18 (s, 1H), 7.90 (d, *J =* 6.0Hz, 1H), 7.89 (s, 1H), 7.43 (m, 2H), 7.38 (m, 3H), 7.03 (d, *J* = 9.0 Hz, 1H), 6.94 (dd, *J =* 9.0, 4.0 Hz, 1H), 6.86 (d, *J =* 5.5 Hz, 1H), 6.66 (d, *J* = 8.5 Hz, 1H), 5.40 (m, 1H), 4.90 (m, 1H), 4.78 (m, 3H), 4.00 (s, 3H), 3.09 (m, 2H), 2.17 (m, 8H), 2.01 (m, 2H), 1.79 (m, 2H), 1.40 (d, *J =* 4.5 Hz, 3H), 1.12 (m, 2H).

Compound 73-b LC-MS: m/z 668.21 (M+H)⁺.

¹H NMR (500 MHz DMSO-*d*₆) δ 8.46 (t, *J =* 6.0 Hz, 1H), 8.18 (s, 1H), 7.90 (d, *J =* 6.0 Hz, 1H), 7.87 (s, 1H), 7.43 (m, 2H), 7.38 (m, 3H), 6.99 (d, *J* = 9.0 Hz, 1H), 6.94 (dd, *J =* 9.0, 4.0 Hz, 1H), 6.87 (d, *J =* 5.5 Hz, 1H), 6.66 (d, *J* = 8.5 Hz, 1H), 5.60 (m, 1H), 5.36 (m, 1H), 4.81 (m, 3H), 4.00 (s, 3H), 3.45 (m, 2H), 2.16 (m, 8H), 1.71 (m, 2H), 1.57 (m, 2H), 1.50 (m, 2H), 1.45 (d, *J =* 6.5 Hz, 3H).

Compound 73-a had a shorter retention time in the chiral chromatography column than compound 73-b, and compound 73-b had a longer retention time in the chiral chromatography column than compound 73-a.

### Example 74: Preparation of Compounds 74, 74-a, and 74-b

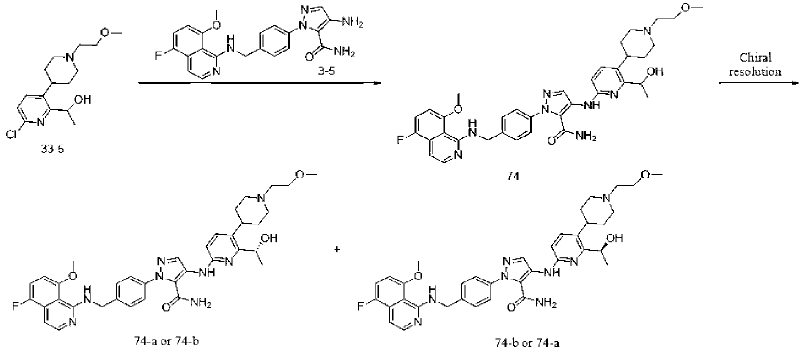

### Preparation of compound 74:

Intermediate **33-5** (131 mg), intermediate **3-5** (120 mg), chloro(2-dicyclohexylphosphino-3,6-dimethoxy-2',4',6'-triisopropyl-1,1'-biphenyl)[2-(2-aminoethylphenyl)]palladium(II) (47 mg), 2-(dicyclohexylphosphino)-3,6-dimethoxy-2'-4'-6'-tri-I-propyl-11'-biphenyl (63 mg), and potassium bis(trimethylsilyl)amide (0.3 mL) were added to tert-butanol (20 mL). The mixture was stirred at 80 °C for 0.5 h under nitrogen atmosphere.

After the reaction was completed, ethyl acetate (20 mL) and purified water (20 mL) were added, and the mixture was stirred for 10 min and left to stand for liquid separation. The aqueous phase was extracted with ethyl acetate (20 mL × 2). The organic phases were combined, washed with saturated brine, dried over anhydrous sodium sulfate, and filtered, and the filtrate was concentrated under reduced pressure to give a concentrate, which was purified by preparative liquid chromatography to give compound **74.**

LC-MS: m/z 669.36 (M+H)⁺.

### Preparation of compounds 74-a and 74-b:

Compound **74** was subjected to chiral resolution to give compound **74-a** and compound **74-b.** The resolution conditions were as follows: an AM-4 preparative column (20 × 250 mm, 10 µm) was used; by taking n-hexane as mobile phase A and 0.2% diethylamine-ethanol as mobile phase B, gradient elution was performed at a flow rate of 20 mL/min; the detection wavelength was 254 nm, and the retention times were 27.52 min (74-a) and 30.15 min (74-b), respectively.

Compound 74-a LC-MS: m/z 669.42 (M+H)⁺.

Compound 74-b LC-MS: m/z 669.48 (M+H)⁺.

Compound 74-a had a shorter retention time in the chiral chromatography column than compound 74-b, and compound 74-b had a longer retention time in the chiral chromatography column than compound 74-a.

### Example 75: Preparation of Compounds 75, 75-a, and 75-b

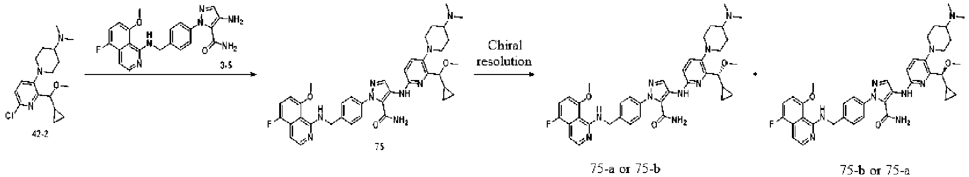

### Preparation of compound 75:

Intermediate **42-2** (120 mg), intermediate **3-5** (100 mg), chloro(2-dicyclohexylphosphino-3,6-dimethoxy-2',4',6'-triisopropyl-1,1'-biphenyl)[2-(2-aminoethylphenyl)]palladium(II) (40 mg), 2-(dicyclohexylphosphino)-3,6-dimethoxy-2'-4'-6'-tri-I-propyl-11'-biphenyl (54 mg), and lithium bis(trimethylsilyl)amide (1 M, 250 µL) were added to tert-butanol (50 mL). The mixture was stirred at 85 °C for 0.5 h under nitrogen atmosphere.

After the reaction was completed, the reaction solution was diluted with ethyl acetate (100 mL) and filtered, and the filtrate was concentrated under reduced pressure to give a concentrate, which was purified by preparative liquid chromatography to give compound **75.**

LC-MS: m/z 694.41 (M+H)⁺.

### Preparation of compounds 75-a and 75-b:

Compound **75** was subjected to chiral resolution to give compound **75-a** and compound **75-b.** The resolution conditions were as follows: a YMC Amylose-SA preparative column (30 × 250 mm, 10 µm) was used; by taking n-hexane as mobile phase A and ethanol as mobile phase B, gradient elution was performed at a flow rate of 30 mL/min; the detection wavelength was 254 nm, and the retention times were 10 min (**75-a**) and 13 min (**75-b**), respectively. Compound 75-a LC-MS: m/z 694.53 (M+H)⁺.

¹H NMR (500 MHz, DMSO-*d*₆) δ 8.46 (t, *J =* 5.7 Hz, 1H), 8.34 (d, *J =* 8.3 Hz, 2H), 7.91 (d, *J =* 5.9 Hz, 1H), 7.51 (dd, *J= 8.9,* 2.6 Hz, 1H), 7.46 (d, *J =* 8.2 Hz, 2H), 7.41 (d, *J =* 8.5 Hz, 3H), 6.93 (dd, *J =* 8.9, 4.3 Hz, 1H), 6.87 (d, *J =* 5.7 Hz, 1H), 6.79 (d, *J =* 8.6 Hz, 1H), 4.82 (d, *J =* 5.9 Hz, 2H), 4.15 (d, *J =* 8.6 Hz, 1H), 4.00 (d, *J =* 2.5 Hz, 3H), 2.88 (d, *J =* 11.1 Hz, 1H), 2.77 (d, *J =* 10.9 Hz, 1H), 2.73-2.58 (m, 2H), 2.19 (d, *J* = 2.5 Hz, 6H), 2.16-2.08 (m, 1H), 1.86-1.74 (m, 2H), 1.56-1.17 (m, 7H), 0.62 (tt, *J =* 8.9, 4.7 Hz, 1H), 0.49 (dq, *J* = 9.6*,* 4.8 Hz, 1H), 0.35 (dq, *J* = 9.8, 4.7 Hz, 1H), 0.15 (dq, *J* = 9.7, 4.8 Hz, 1H).

Compound 75-b LC-MS: m/z 694.45 (M+H)⁺.

¹H NMR (500 MHz, DMSO-*d*₆) δ 8.45 (t, *J* = 5.9 Hz, 1H), 8.32 (d, *J =* 6.1 Hz, 2H), 7.91 (d, *J =* 5.8 Hz, 1H), 7.51 (d, *J =* 8.7 Hz, 1H), 7.45 (d, *J =* 8.2 Hz, 2H), 7.42-7.37 (m, 3H), 6.94 (dd, *J =* 8.8, 4.2 Hz, 1H), 6.87 (d, *J =* 5.8 Hz, 1H), 6.78 (d, *J =* 8.7 Hz, 1H), 4.82 (d*, J =* 6.0 Hz, 2H), 4.14 (d, *J =* 8.7 Hz, 1H), 4.00 (s, 3H), 3.15 (s, 2H), 2.88 (d, *J* = 11.2 Hz, 1H), 2.81-2.73 (m, 1H), 2.72-2.59 (m, 2H), 2.19 (s, 5H), 2.13 (ddt, *J =* 10.8, 7.4, 3.5 Hz, 1H), 1.84-1.75 (m, 2H), 1.47 (qt, *J =* 11.1, 4.5 Hz, 2H), 1.40-1.29 (m, 2H), 1.25 (d, *J =* 12.1 Hz, 3H), 0.62 (tt, *J* = 8.1, 4.5 Hz, 1H), 0.49 (dq, *J* = 9.6, 4.9 Hz, 1H), 0.39-0.29 (m, 1H), 0.14 (dq, *J =* 9.6, 4.8 Hz, 1H).

Compound 75-a had a shorter retention time in the chiral chromatography column than compound 75-b, and compound 75-b had a longer retention time in the chiral chromatography column than compound 75-a.

### Example 76: Preparation of Compound 76

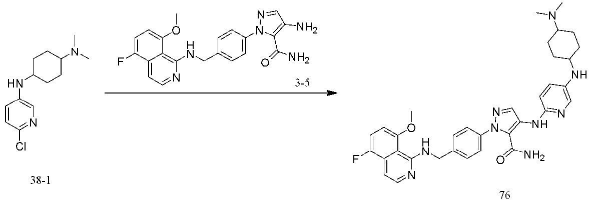

### Preparation of compound 76:

Referring to the preparation steps of compound **9** in Example **9,** intermediate **8-8** was replaced with intermediate **3-5,** and intermediate **9-2** was replaced with intermediate **38-1;** after the reaction was completed, the system was separated by preparative liquid chromatography to give compound **76.**

LC-MS: m/z 624.35 (M+H)⁺.

¹H NMR (500 MHz, DMSO-*d*₆) δ 8.45 (t, *J =* 5.9 Hz, 1H), 8.07 (s, 1H), 7.91 (d, *J =* 5.7 Hz, 1H), 7.83 (s, 1H), 7.57 (d, *J* = 2.9 Hz, 1H), 7.44 (d, *J* = 8.2 Hz, 2H), 7.38 *(t, J=* 7.3 Hz, 3H), 6.95 (ddd, *J* = 12.8, 8.8, 3.6 Hz, 2H), 6.87 (d, *J =* 5.7 Hz, 1H), 6.68 (d, *J =* 8.9 Hz, 1H), 4.81 (d, *J =* 5.9 Hz, 2H), 4.00 (s, 3H), 2.21 (s, 6H), 2.00 (dd, *J =* 12.5, 4.5 Hz, 2H), 1.90 (s, 2H), 1.81 (d, *J =* 11.6 Hz, 2H), 1.32-1.25 (m, 2H), 1.15-1.06 (m, 2H).

### Example 77: Preparation of Compound 77

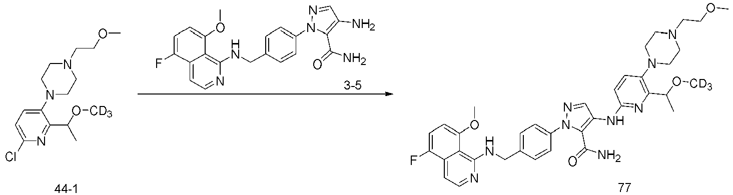

### Preparation of compound 77:

Intermediate **44-1** (141 mg), intermediate 3-5 (120 mg), chloro(2-dicyclohexylphosphino-3,6-dimethoxy-2',4',6'-triisopropyl-1,1'-biphenyl)[2-(2-aminoethylphenyl)]palladium(II) (47 mg), 2-(dicyclohexylphosphino)-3,6-dimethoxy-2'-4'-6'-tri-I-propyl-11'-biphenyl (63 mg), and potassium bis(trimethylsilyl)amide (0.3 mL) were added to tert-butanol (20 mL). The mixture was stirred at 80 °C for 0.5 h under nitrogen atmosphere.

After the reaction was completed, ethyl acetate (20 mL) and purified water (20 mL) were added, and the mixture was stirred for 10 min and left to stand for liquid separation. The aqueous phase was extracted with ethyl acetate (20 mL × 2). The organic phases were combined, washed with saturated brine, dried over anhydrous sodium sulfate, and filtered, and the filtrate was concentrated under reduced pressure to give a concentrate, which was purified by preparative liquid chromatography to give compound **77.**

LC-MS: m/z 687.49 (M+H)⁺.

¹H NMR (500 MHz, DMSO-*d*₆) δ 8.46 (t, J = 6.0 Hz, 1H), 8.32 (s, 1H), 8.30 (s, 1H), 7.91 (d, J = 5.8 Hz, 1H), 7.52 (d, J = 8.8 Hz, 1H), 7.45 (s, 1H), 7.44 (d, J *=* 1.9 Hz, 1H), 7.42-7.38 (m, 3H), 6.94 (dd, J *=* 8.8, 4.2 Hz, 1H), 6.87 (d, J = 5.8 Hz, 1H), 6.78 (d, J = 8.8 Hz, 1H), 4.96 (q, J = 6.4 Hz, 1H), 4.81 (d, J = 6.0 Hz, 2H), 4.00 (s, 3H), 3.46 (t, J = 5.8 Hz, 2H), 3.25 (s, 3H), 2.76 (qd, J = 10.7, 5.0 Hz, 4H), 1.36 (d, J = 6.3 Hz, 3H).

### Example 78: Preparation of Compound 78

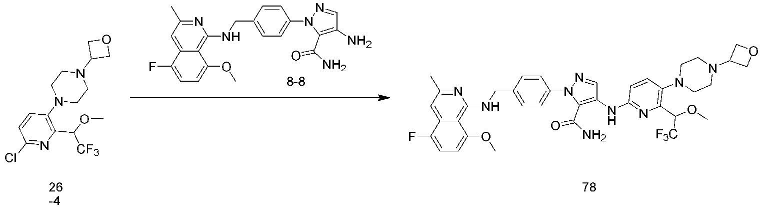

### Preparation of compound 78:

Referring to the preparation steps of compound **10** in Example **10,** intermediate **10-3** was replaced with intermediate **26-4,** and after the reaction was completed, compound **78** was obtained.

LC-MS: m/z 750.17 (M+H)⁺.

¹H NMR (500 MHz DMSO-*d*₆) δ 8.58 (s, 1H), 8.36 (s, 2H), 7.75 (d, *J =* 8.0 Hz, 1H), 7.49 (d, *J =* 7.0 Hz, 2H), 7.41 (d, *J =* 7.0 Hz, 2H), 7.32 (t, *J* = 9.0 Hz, 1H), 7.00 (d, *J =* 8.0 Hz, 1H), 6.82 (m, 1H), 6.72 (s, 1H), 5.57 (m, 1H), 4.80 (brs, 2H), 4.56 (m, 2H), 4.46 (m, 2H), 3.97 (s, 3H), 3.49 (m, 1H), 2.79 (m, 4H), 2.42 (m, 12H).

### Example 79: Preparation of Compound 79

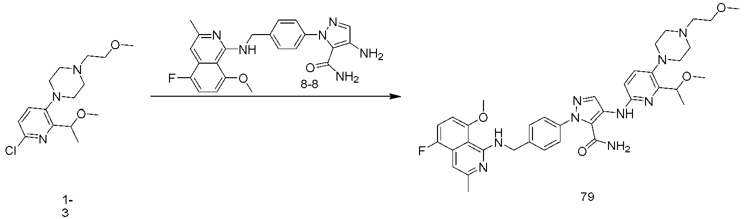

### Preparation of compound 79:

Referring to the preparation steps of compound **10** in Example 10, intermediate **10-3** was replaced with intermediate **1-3,** and after the reaction was completed, compound **79** was obtained.

LC-MS: m/z 698.45 (M+H)⁺.

¹H NMR (500 MHz DMSO-*d*₆) δ 8.35 (t, *J =* 6.0 Hz, 1H), 8.33 (s, 1H), 8.30 (s, 1H), 7.52 (d, *J =* 8.5 Hz, 1H), 7.49 (d, *J* = 8.5 Hz, 2H), 7.40 (d*, J =* 8.5 Hz, 2H), 7.32 (m, 1H), 6.82 (dd, *J* = 9.0, 4.0 Hz, 1H), 6.78 (d, *J =* 8.5 Hz, 1H), 6.72 (s, 1H), 4.97 (m, 1H), 4.80 (d, *J =* 6.0 Hz, 2H), 3.97 (s, 3H), 3.46 (t, *J =* 6.0 Hz, 2H), 3.25 (s, 3H), 3.11 (s, 3H), 2.76 (m, 4H), 2.56 (m, 6H), 2.34 (s, 3H), 1.36 (d, *J =* 6.0 Hz, 3H).

### Example 80: Preparation of Compounds 80-a and 80-b

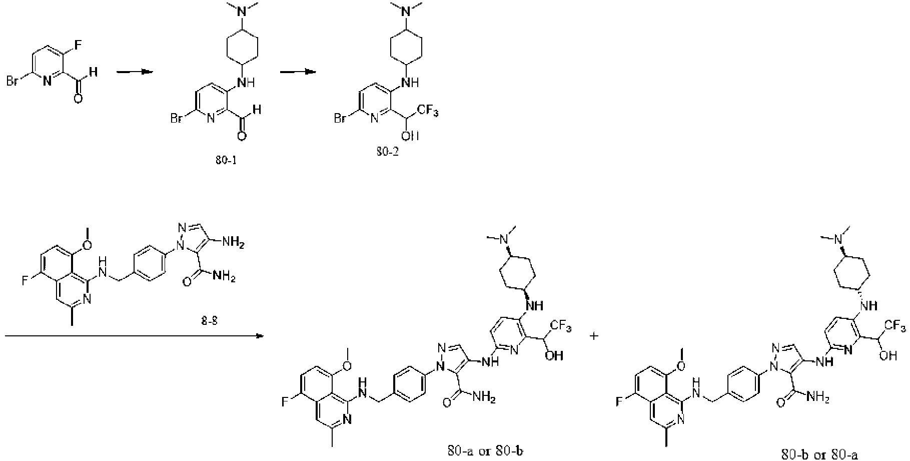

### Preparation of intermediate 80-1:

5-Bromo-2-fluorobenzaldehyde (2 g), N,N-dimethyl-1,4-cyclohexanediamine (1.4 g), and potassium carbonate (4.1 g) were added to N,N-dimethylformamide (20 mL), and the mixture was stirred for 2 h with the temperature controlled at 85 °C.

After the reaction was completed, the reaction solution was filtered, and the filtrate was extracted with purified water (20 mL) and ethyl acetate (40 mL). The organic phase was dried over anhydrous sodium sulfate and filtered, and the filtrate was concentrated under reduced pressure to give intermediate **80-1** (2.4 g).

LC-MS: m/z 326.08 (M+H)⁺.

### Preparation of intermediate 80-2:

Intermediate **80-2** (300 mg) was added to DCM (5 mL), and the mixture was stirred with the temperature controlled at 0 °C. (Trifluoromethyl)trimethylsilane (200 mg) was slowly added, and after the mixture was stirred for 20 min, tetrabutylammonium fluoride (131 mg) was added. The mixture was transferred to room temperature and stirred for 5 h.

After the reaction was completed, a saturated ammonium chloride solution (10 mL) was added to quench the reaction, and the reaction system was stirred for 10 min. Ethyl acetate (20 mL × 2) was added, and the reaction system was extracted and then left to stand for liquid separation. The organic phase was dried over anhydrous sodium sulfate and filtered under vacuum, and the filtrate was concentrated under reduced pressure and purified by column chromatography to give intermediate **80-2** (120 mg).

LC-MS: m/z 396.11 (M+H)⁺.

### Preparation of compounds 80-a and 80-b:

Referring to the preparation steps of compound **9** in Example **9,** intermediate **9-2** was replaced with intermediate **80-2,** and the reaction system was separated by preparative liquid chromatography to give compound **80-a** and compound **80-b.** The conditions were as follows: a YMC-AQ-C18 preparative column (30 × 250 mm, 10.0 µm) was used; by taking acetonitrile as mobile phase A and 0.1% aqueous acetic acid solution as mobile phase B, gradient elution was performed at a flow rate of 20 mL/min; the detection wavelength was 254 nm, and the retention times were 24.8 min (**80-a**) and 30.1 min (**80-b**), respectively.

Compound **80-a** LC-MS: m/z 736.42 (M+H)⁺.

Compound **80-b** LC-MS: m/z 736.42 (M+H)⁺.

Compound **80-b** ¹H NMR (500 MHz, DMSO-*d*₆) δ 8.34 (t, *J =* 5.9 Hz, 1H), 8.21 (s, 1H), 8.02 (s, 1H), 7.49 (d, *J =* 8.3 Hz, 2H), 7.41-7.37 (m, 2H), 7.31 (dd, *J =* 10.0, 8.7 Hz, 1H), 7.13 (d, *J =* 9.2 Hz, 1H), 6.84-6.80 (m, 2H), 6.72 (s, 1H), 5.23 (q, *J =* 7.9 Hz, 1H), 5.10 (s, 1H), 4.80 (d, *J =* 5.8 Hz, 2H), 3.97 (s, 3H), 2.34 (s, 3H), 2.17 (s, 6H), 1.69 (q, *J* = 5.9 Hz, 2H), 1.61-1.49 (m, 6H), 1.24 (s, 2H).

Compound 80-a had a shorter retention time in the chiral chromatography column than compound 80-b, and compound 80-b had a longer retention time in the chiral chromatography column than compound 80-a.

### Example 81: Preparation of Compound 81

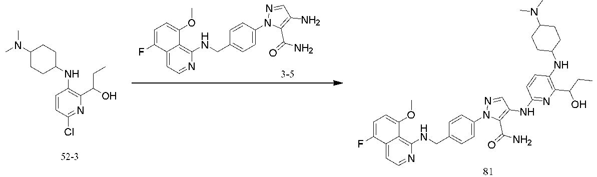

### Preparation of compound 81:

Referring to the preparation steps of compound **9** in Example **9,** intermediate **8-8** was replaced with intermediate **3-5,** and intermediate **9-2** was replaced with intermediate **52-3;** after the reaction was completed, the system was separated by preparative liquid chromatography to give compound **81.** Compound **81-a** LC-MS: m/z 682.51 (M+H)⁺. LC-MS: m/z 682.51 (M+H)⁺.

¹H NMR (500 MHz, DMSO-*d*₆) δ 8.45 (t, *J =* 6.0 Hz, 1H), 8.17 (s, 1H), 7.93-7.85 (m, 2H), 7.46-7.36 (m, 5H), 6.99 (d, *J* = 8.8 Hz, 1H), 6.94 (dd, *J =* 8.8, 4.2 Hz, 1H), 6.87 (d, *J =* 5.8 Hz, 1H), 6.65 (d, *J =* 8.7 Hz, 1H), 5.60 (s, 1H), 5.33 (s, 1H), 4.81 (d, *J =* 6.0 Hz, 2H), 4.53 (t, *J =* 6.7 Hz, 1H), 4.00 (s, 3H), 2.22 (s, 6H), 1.83 (dq, *J =* 23.4, 6.7 Hz, 2H), 1.74 (d, *J =* 11.3 Hz, 2H), 1.54 (dd, *J =* 20.7, 9.2 Hz, 6H), 1.24 (s, 1H), 0.90 (t, *J =* 7.4 Hz, 3H).

### Example 82: Preparation of Compounds 82-a and 82-b

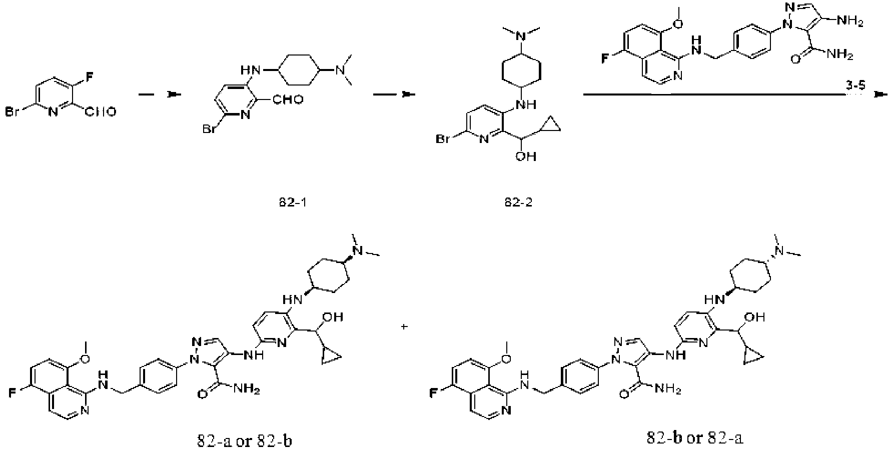

### Preparation of intermediate 82-1:

2-Bromo-5-fluoropyridine-6-carbaldehyde (2.0 g), 4-dimethylaminocyclohexylamine (2.38 g), and potassium carbonate (5.60 g) were added to DMF (180 mL), and the mixture was stirred at 80 °C for 3 h, and then cooled to room temperature. Ethyl acetate (80 mL) and purified water (50 mL) were added, and the mixture was stirred for 10 min and left to stand for liquid separation. The aqueous phase was extracted with ethyl acetate (60 mL × 2), the organic phases were combined, washed with saturated brine, dried over anhydrous sodium sulfate, and filtered, and the filtrate was concentrated under reduced pressure to give intermediate **82-1** (2.58 g).

LC-MS: m/z 326.14 (M+H)⁺.

### Preparation of intermediate 82-2:

Intermediate **82-1** (1.0 g) was added to THF (100 mL), and cyclopropylmagnesium bromide (1 M, 8 mL) was added dropwise with the temperature controlled at -10 °C to 0 °C under nitrogen atmosphere. After the dropwise addition was completed, the mixture was stirred at the same temperature for 2 h. After the reaction was completed, a saturated ammonium chloride solution (40 mL) was added to quench the reaction, and the reaction system was extracted with ethyl acetate (40 mL × 3). The organic phases were combined, washed with a saturated sodium chloride solution, dried over anhydrous sodium sulfate, and filtered under vacuum, and the filtrate was concentrated under reduced pressure to give intermediate **82-2** (1.14 g).

LC-MS: m/z 368.26 (M+H)⁺.

### Preparation of compounds 82-a and 82-b:

Intermediate **82-2** (160 mg) and intermediate **3-5** (177 mg) were added to tert-butanol (30 mL), methanesulfonato(2-di-tert-butylphosphino-2',4',6'-triisopropyl-1,1'-biphenyl)(2'-amino-1,1'-biphenyl-2-yl)palladium(II) (128 mg), 2-di-tert-butylphosphino-2',4',6'-triisopropylbiphenyl (180 mg), and lithium bis(trimethylsilyl)amide (1 M, 480 µL) were added, and the mixture was stirred at 80 °C for 2 h under nitrogen atmosphere.

After the reaction was completed, ethyl acetate (30 mL) and purified water (30 mL) were added, and the mixture was stirred for 10 min and left to stand for liquid separation. The aqueous phase was extracted with ethyl acetate (30 mL × 3). The organic phases were combined, washed with saturated brine, dried over anhydrous sodium sulfate, and filtered, and the filtrate was concentrated under reduced pressure to give a concentrate, which was separated by preparative liquid chromatography to give compound **82-a** and compound **82-b.** The preparative conditions were as follows: a YMC AQ C18 (30 × 250 mm, 10 µm) chromatography column was used; by taking 0.1% acetic acid as mobile phase A and 5%-40% acetonitrile as mobile phase B, gradient elution was performed for 0-70 min at a flow rate of 30 mL/min; the wavelength was 254 nm, and the retention times were 35 min (**82-a**) and 43 min (**82-b**), respectively.

Compound **82-a** LC-MS: m/z 694.42 (M+H)⁺.

Compound **82-b** LC-MS: m/z 694.40 (M+H)⁺.

Compound 82-a had a shorter retention time in the chiral chromatography column than compound 82-b, and compound 82-b had a longer retention time in the chiral chromatography column than compound 82-a.

### Example 83: Preparation of Compound 83

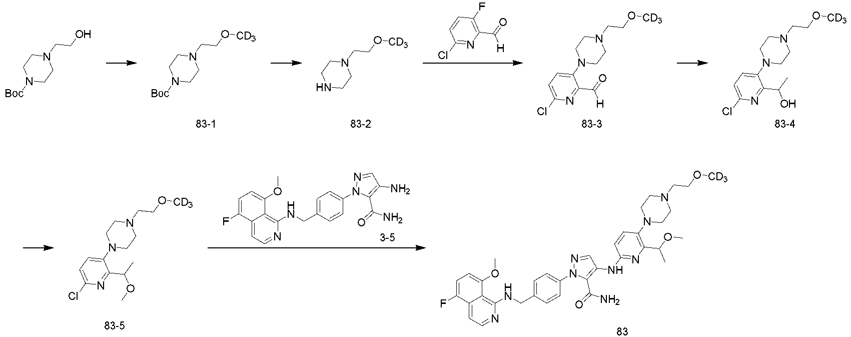

### Preparation of intermediate 83-1:

Tert-butyl-4-(2-hydroxyethyl)piperazine-1-carboxylate (2.3 g) was added to THF (50 mL), sodium hydride (60%, 0.6 g) was slowly added at room temperature, and the mixture was stirred uniformly. Deuterated iodomethane (1.45 g) was then added, and the mixture was stirred overnight at room temperature. Deuterated iodomethane (0.29 g) was then supplemented, and the mixture was stirred at room temperature.

After the reaction was completed, purified water was added to quench the reaction, and the reaction solution was concentrated under reduced pressure to give intermediate **83-1** (3.1 g).

### Preparation of intermediate 83-2:

Intermediate **83-1** (3.1 g) was added to a solution of hydrogen chloride in dioxane (4 M, 5 mL), and the mixture was stirred at room temperature.

After the reaction was completed, the reaction solution was concentrated under reduced pressure to give intermediate **83-2** (2.8 g).

### Preparation of intermediate 83-3:

Intermediate **83-2** (2.8 g) and 2-chloro-5-fluoropyridine-6-carbaldehyde (1.6 g) were added to N,N-dimethylformamide (40 mL), potassium carbonate (8.28 g) was added, and the mixture was stirred with the temperature controlled at 80-90 °C under nitrogen atmosphere.

After the reaction was completed, the reaction solution was extracted with purified water (200 mL) and dichloromethane (200 mL). The organic phase was dried over anhydrous sodium sulfate and filtered, and the filtrate was concentrated under reduced pressure to give intermediate **83-3** (2.8 g).

LC-MS: m/z 287.11 (M+H)⁺.

### Preparation of intermediate 83-4:

Intermediate 83-3 (2 g) was added to THF (50 mL). The mixture was stirred with the temperature controlled at - 20 °C, and a methylmagnesium bromide solution (3 M, 4.7 mL) was slowly added. After the dropwise addition was completed, the mixture was stirred with the temperature controlled at -20 °C.

After the reaction was completed, an aqueous ammonium chloride solution (100 mL) was added to quench the reaction, ethyl acetate (100 mL) was added, and the mixture was extracted and then left to stand for liquid separation. The organic phase was dried over anhydrous sodium sulfate and filtered under vacuum, and the filtrate was concentrated under reduced pressure to give intermediate 83-4 (2.12 g).

LC-MS: m/z 303.17 (M+H)⁺.

### Preparation of intermediate 83-5:

Intermediate **83-4** (1.06 g) was added to THF (50 mL), sodium hydride (60%, 210 mg) was slowly added in an ice bath, and the mixture was stirred homogeneously, followed by addition of iodomethane (250 mg). The resulting mixture was stirred in an ice bath.

After the reaction was completed, purified water was added to quench the reaction. The reaction solution was concentrated under reduced pressure, and the concentrate was purified by column chromatography to give intermediate **83-5.**

LC-MS: m/z 317.18 (M+H)⁺.

### Preparation of compound 83:

Intermediate **83-5** (158 mg), intermediate **3-5** (100 mg), chloro(2-dicyclohexylphosphino-3,6-dimethoxy-2',4',6'-triisopropyl-1,1'-biphenyl)[2-(2-aminoethylphenyl)]palladium(II) (40 mg), 2-(dicyclohexylphosphino)-3,6-dimethoxy-2'-4'-6'-tri-I-propyl-11'-biphenyl (54 mg), and lithium bis(trimethylsilyl)amide (1 M, 250 µL) were added to tert-butanol (50 mL). The mixture was stirred at 85 °C for 0.5 h under nitrogen atmosphere.

After the reaction was completed, the reaction solution was diluted with ethyl acetate (100 mL) and filtered, and the filtrate was concentrated under reduced pressure to give a concentrate, which was purified by preparative liquid chromatography to give compound **83.**

LC-MS: m/z 687.41 (M+H)⁺.

¹H NMR (500 MHz, DMSO-*d*₆) δ 8.46 (t, *J* = 6.0 Hz, 1H), 8.31 (d, *J =* 10.5 Hz, 2H), 7.91 (d, *J =* 5.8 Hz, 1H), 7.75-7.6 (br, 1H), 7.52 (d, *J =* 8.6 Hz, 1H), 7.45 (d, *J =* 8.1 Hz, 2H), 7.39 (d, *J =* 8.0 Hz, 3H), 6.94 (dd, *J* = 8.7, 4.4 Hz, 1H), 6.87 (d, *J* = 5.8 Hz, 1H), 6.78 (d, *J =* 8.7 Hz, 1H), 4.97 (q, *J =* 6.4 Hz, 1H), 4.81 (d, *J =* 6.0 Hz, 2H), 4.00 (s, 3H), 3.45 (*t, J =* 5.8 Hz, 2H), 3.11 (s, 3H), 2.77 (tt*, J =* 12.5, 4.7 Hz, 4H), 2.58-2.51 (m, 6H), 1.36 (d, *J =* 6.4 Hz, 3H).

### Example 84: Preparation of Compound 84

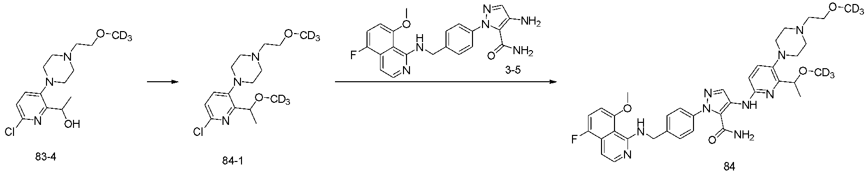

### Preparation of intermediate 84-1:

Intermediate **83-4** (1.06 g) was added to THF (50 mL), sodium hydride (60%, 210 mg) was slowly added in an ice bath, and the mixture was stirred uniformly, followed by addition of deuterated iodomethane (250 mg). The resulting mixture was stirred in an ice bath.

After the reaction was completed, purified water was added to quench the reaction. The reaction solution was concentrated under reduced pressure, and the concentrate was purified by column chromatography to give intermediate **84-1.**

LC-MS: m/z 320.20 (M+H)⁺.

### Preparation of compound 84:

Intermediate **84-1** (160 mg), intermediate **3-5** (100 mg), chloro(2-dicyclohexylphosphino-3,6-dimethoxy-2',4',6'-triisopropyl-1,1'-biphenyl)[2-(2-aminoethylphenyl)]palladium(II) (40 mg), 2-(dicyclohexylphosphino)-3,6-dimethoxy-2'-4'-6'-tri-I-propyl-11'-biphenyl (54 mg), and lithium bis(trimethylsilyl)amide (1 M, 250 µL) were added to tert-butanol (50 mL). The mixture was stirred at 85 °C for 0.5 h under nitrogen atmosphere.

After the reaction was completed, the reaction solution was diluted with ethyl acetate (100 mL) and filtered, and the filtrate was concentrated under reduced pressure to give a concentrate, which was purified by preparative liquid chromatography to give compound **84.**

LC-MS: m/z 690.45 (M+H)⁺.

¹H NMR (500 MHz, DMSO-*d*₆) δ 8.46 (t, *J =* 6.0 Hz, 1H), 8.31 (d, *J =* 10.6 Hz, 2H), 7.91 (d, *J =* 5.8 Hz, 1H), 7.75-7.6 (br, 1H), 7.52 (d, *J =* 8.8 Hz, 1H), 7.45 (d, *J =* 8.2 Hz, 2H), 7.39 (d, *J =* 8.0 Hz, 3H), 6.94 (dd, *J =* 8.8, 4.2 Hz, 1H), 6.87 (d, *J =* 5.8 Hz, 1H), 6.78 (d, *J =* 8.7 Hz, 1H), 4.96 (q, *J =* 6.4 Hz, 1H), 4.81 (d, *J =* 6.0 Hz, 2H), 4.00 (s, 3H), 3.46 (t, *J =* 5.8 Hz, 2H), 2.75 (dt, *J =* 12.4, 5.1 Hz, 4H), 2.59-2.51 (m, 6H), 1.36 (d, *J =* 6.4 Hz, 3H).

### Example 85: Preparation of Compound 85

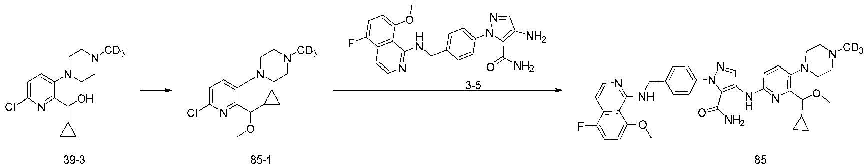

### Preparation of intermediate 85-1:

Intermediate **39-3** (6.2 g) was added to THF (100 mL), and the mixture was stirred with the temperature controlled at 0 °C. Sodium hydride (60%, 980 mg) was slowly added, and after the mixture was stirred for 20 min, iodomethane (3.4 g) was added. The mixture was transferred to room temperature and stirred for 4 h.

After the reaction was completed, purified water (100 mL) was added to quench the reaction, and the reaction system was stirred for 10 min. Ethyl acetate (150 mL) was added, and the reaction system was extracted and then left to stand for liquid separation. The organic phase was dried over anhydrous sodium sulfate and filtered under vacuum, and the filtrate was concentrated under reduced pressure to give intermediate **85-1** (5.8 g).

LC-MS: m/z 299.12 (M+H)⁺.

### Preparation of compound 85:

Referring to the preparation steps of compound **3** in Example **3,** intermediate **1-3** was replaced with intermediate **85-1,** and the reaction system was purified to give compound **85** (5 mg).

LC-MS: m/z 669.40 (M+H)⁺.

### Example 86: Preparation of Compound 86

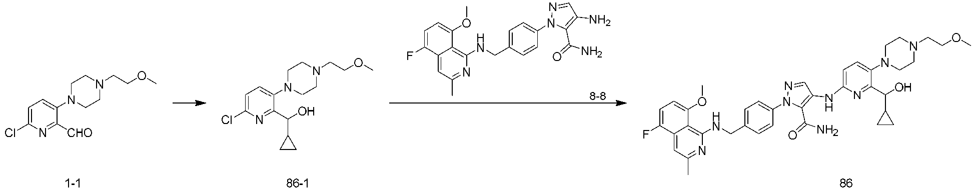

### Preparation of intermediate 86-1:

Intermediate **1-1** (0.60 g) was added to THF (50 mL), and cyclopropylmagnesium bromide (1 M, 4.4 mL) was added dropwise with the temperature controlled at -10 °C to 0 °C under nitrogen atmosphere. After the dropwise addition was completed, the mixture was stirred at the same temperature for 2 h. After the reaction was completed, a saturated ammonium chloride solution (40 mL) was added to quench the reaction, and the reaction system was extracted with ethyl acetate (40 mL × 3). The organic phases were combined, washed with a saturated sodium chloride solution, dried over anhydrous sodium sulfate, and filtered under vacuum, and the filtrate was concentrated under reduced pressure to give intermediate **86-1** (0.54 g).

LC-MS: m/z 326.26 (M+H)⁺.

### Preparation of intermediate 86:

Intermediate **86-1** (70 mg) and intermediate **8-8** (122 mg) were added to tert-butanol (24 mL), methanesulfonato(2-di-tert-butylphosphino-2',4',6'-triisopropyl-1,1'-biphenyl)(2'-amino-1,1'-biphenyl-2-yl)palladium(II) (50 mg), 2-ditert-butylphosphino-2',4',6'-triisopropylbiphenyl (72 mg), and lithium bis(trimethylsilyl)amide (1 M, 36 µL) were added, and the mixture was stirred at 80 °C for 2 h under nitrogen atmosphere.

After the reaction was completed, ethyl acetate (30 mL) and purified water (30 mL) were added, and the mixture was stirred for 10 min and left to stand for liquid separation. The aqueous phase was extracted with ethyl acetate (30 mL × 3). The organic phases were combined, washed with saturated brine, dried over anhydrous sodium sulfate, and filtered, and the filtrate was concentrated under reduced pressure to give a concentrate, which was separated by preparative liquid chromatography to give compound **86.**

LC-MS: m/z 710.42 (M+H)⁺.

### Example 87: Preparation of Compound 87

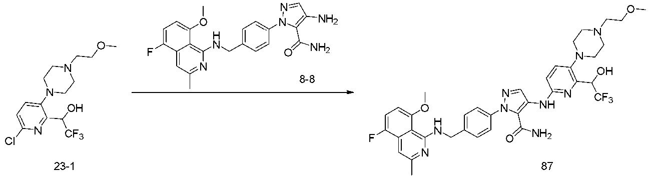

### Preparation of compound 87:

Intermediate **23-1** (177 mg), intermediate **8-8** (100 mg), chloro(2-dicyclohexylphosphino-3,6-dimethoxy-2',4',6'-triisopropyl-1,1'-biphenyl)[2-(2-aminoethylphenyl)]palladium(II) (40 mg), 2-(dicyclohexylphosphino)-3,6-dimethoxy-2'-4'-6'-tri-I-propyl-11'-biphenyl (54 mg), and lithium bis(trimethylsilyl)amide (1 M, 250 µL) were added to tert-butanol (50 mL). The mixture was stirred at 85 °C for 0.5 h under nitrogen atmosphere.

After the reaction was completed, the reaction solution was diluted with ethyl acetate (100 mL) and filtered, and the filtrate was concentrated under reduced pressure to give a concentrate, which was purified by preparative liquid chromatography to give compound **87.**

LC-MS: m/z 738.39 (M+H)⁺.

### Example 88: Preparation of Compounds 88, 88-a, and 88-b

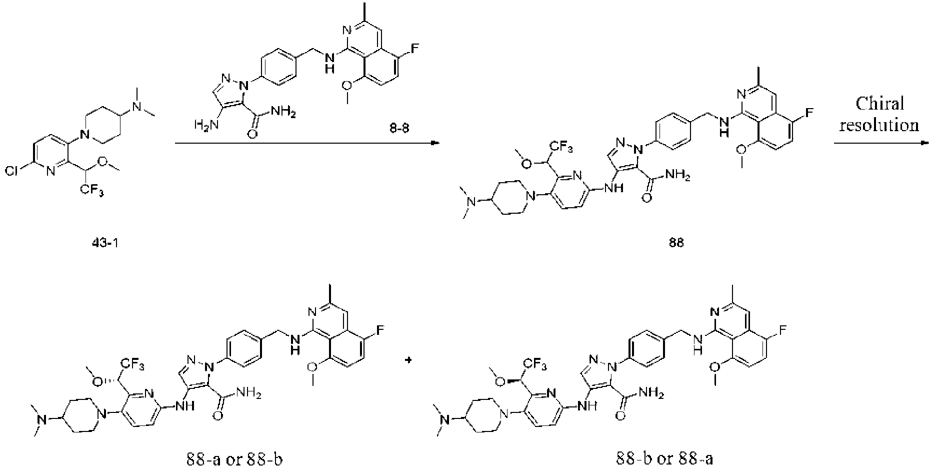

### Preparation of compound 88:

Referring to the preparation steps of compound **3** in Example **3,** intermediate **3-5** was replaced with intermediate **8-8,** intermediate **1-3** was replaced with intermediate **43-1,** and cesium carbonate was replaced with potassium carbonate. After the reaction was completed, the reaction solution was filtered and concentrated to give a concentrate, which was separated by preparative liquid chromatography to give compound **88.**

LC-MS: m/z 736.55 (M+H)⁺.

¹H NMR (500 MHz, DMSO-*d*₆) δ 8.58 (s, 1H), 8.35 (d*, J =* 5.3 Hz, 2H), 8.21 (s, 2H), 7.69 (d, *J =* 8.9 Hz, 1H), 7.49 (d, *J =* 8.5 Hz, 2H), 7.44-7.37 (m, 2H), 7.32 (dd, *J =* 10.0, 8.7 Hz, 2H), 6.99 (d, *J =* 8.9 Hz, 1H), 6.82 (dd, *J =* 8.8, 4.2 Hz, 1H), 6.72 (s, 1H), 5.57 (q, *J =* 7.2 Hz, 1H), 4.80 (d*, J =* 5.9 Hz, 2H), 3.97 (s, 3H), 3.17 (s, 1H), 2.87 *(d, J=* 11.6 Hz, 1H), 2.83-2.69 (m, 3H), 2.63 (d, *J =* 11.8 Hz, 1H), 2.45 (s, 5H), 2.34 (s, 3H), 1.93 (d, *J =* 12.1 Hz, 2H), 1.68 (ddt, *J =* 15.9, 11.4, 6.2 Hz, 2H).

### Preparation method for compounds 88-a and 88-b:

Compound **88** was subjected to chiral resolution to give compound **88-a** and compound **88-b.** The resolution conditions were as follows: a YMC Amylose-SA preparative column (30 × 250 mm, 10 µm) was used; by taking n-hexane as mobile phase A and ethanol as mobile phase B, gradient elution was performed at a flow rate of 0.8 mL/min; the detection wavelength was 254 nm, and the retention times were 15.5 min (88-a) and 16.3 min (88-b), respectively. Compound 88-a LC-MS: m/z 736.45 (M+H)⁺.

Compound 88-b LC-MS: m/z 736.42 (M+H)⁺.

Compound 88-a had a shorter retention time in the chiral chromatography column than compound 88-b, and compound 88-b had a longer retention time in the chiral chromatography column than compound 88-a.

### Test Example 1: Inhibitory Activity Against In Vitro Cell Proliferation

### 1.1 Assay for inhibitory activity against OCI-LY10 cell proliferation

OCI-LY10 cells in a good growth state were collected into a centrifuge tube, adjusted to a cell density of 9 × 10⁴ cells/mL using an assay medium (1640 + 10% FBS), and seeded onto a 96-well plate (100 µL/well). The cells were incubated overnight in a cell incubator. Compounds were added using a nanoliter pipettor such that the final concentrations of the compounds were 1000 nM to 1.64 nM (the addition was performed in duplicate). Meanwhile, a control was set. After 72 h of incubation in the cell incubator, the assay reagent CCK-8 (manufacturer: Dojindo Laboratories; 10 µL/well) was added. After 4 h of incubation in the cell incubator, the absorbance values were measured at 450 nm on a PerkinElmer Envision microplate reader. A four-parameter analysis was performed, a dose-response curve was fitted, and IC₅₀ was calculated. The test results are shown in Table 1.

**Table 1. Inhibitory activity against OCI-LY10 cell proliferation**

| **Compound No.** | **IC₅₀(nM)** | **Compound No.** | **IC₅₀(nM)** |
|---|---|---|---|
| **Compound 1** | <10 | **Compound 48-b** | <10 |
| **Compound 1-b** | <10 | **Compound 49-a** | <10 |
| **Compound 2** | <10 | **Compound 49-b** | <10 |
| **Compound 3-b** | <10 | **Compound 50** | <10 |
| **Compound 5** | <10 | **Compound 51** | <10 |
| **Compound 5-a** | <10 | **Compound 52** | <10 |
| **Compound 5-b** | <10 | **Compound 53** | <10 |
| **Compound 6** | <10 | **Compound 55** | <10 |
| **Compound 7** | <10 | **Compound 56** | <10 |
| **Compound 8-b** | <10 | **Compound 57** | <10 |
| **Compound 10** | <10 | **Compound 58-b** | <10 |
| **Compound 11-a** | <10 | **Compound 60** | <10 |
| **Compound 11-b** | <10 | **Compound 61** | <10 |
| **Compound 12** | <10 | **Compound 61-a** | <10 |
| **Compound 12-a** | <10 | **Compound 62** | <10 |
| **Compound 13** | <10 | **Compound 62-b** | <10 |
| **Compound 13-a** | <10 | **Compound 63** | <10 |
| **Compound 13-b** | <10 | **Compound 64** | <10 |
| **Compound 17-b** | <10 | **Compound 65** | <10 |
| **Compound 18** | <10 | **Compound 68** | <10 |
| **Compound 19** | <10 | **Compound 68-a** | <10 |
| **Compound 20** | <10 | **Compound 69** | <10 |
| **Compound 22** | <10 | **Compound 70** | <10 |
| **Compound 23** | <10 | **Compound 71-b** | <10 |
| **Compound 24** | <10 | **Compound 72** | <10 |
| **Compound 24-a** | <10 | **Compound 73-a** | <10 |
| **Compound 25** | <10 | **Compound 73-b** | <10 |
| **Compound 26** | <10 | **Compound 74** | <10 |
| **Compound 27** | <10 | **Compound 74-b** | <10 |
| **Compound 28** | <10 | **Compound 75** | <10 |
| **Compound 29** | <10 | **Compound 75-a** | <10 |
| **Compound 32** | <10 | **Compound 77** | <10 |
| **Compound 33** | <10 | **Compound 79** | <10 |
| **Compound 34** | <10 | **Compound 81** | <10 |
| **Compound 35** | <10 | **Compound 82-a** | <10 |
| **Compound 36** | <10 | **Compound 82-b** | <10 |
| **Compound 38** | <10 | **Compound 83** | <10 |
| **Compound 39** | <10 | **Compound 84** | <10 |
| **Compound 40** | <10 | **Compound 85** | <10 |
| **Compound 41** | <10 | **Compound 86** | <10 |
| **Compound 42** | <10 | | |
| **Compound 43** | <10 | | |
| **Compound 44** | <10 | | |
| **Compound 45** | <10 | | |
| **Compound 46** | <10 | | |

### Test Example 2: Evaluation on In Vitro Stability in Liver Microsomes

300 µL of the final incubation system contains 30 µL of liver microsomes (protein concentration: 0.15 mg/mL), 30 µL of NADPH + MgCl₂, 3 µL of the test compound (in acetonitrile), and 237 µL of PBS buffer (pH 7.4). The proportion of the organic solvent (acetonitrile) was 1%. Samples were prepared in duplicate of 0.3 mL for each species. Tubes each containing 270 µL of a mixed solution of substrate and enzyme and NADPH were pre-incubated at 37 °C for 5 min. 30 µL of NADPH + MgCl₂ was added and the mixture was mixed. 50 µL of the mixture was taken at 0 min, 15 min, 30 min, and 60 min, and 300 µL of glacial acetonitrile containing an internal standard was added to terminate the reaction.

300 µL of glacial acetonitrile containing an internal standard (20 ng/mL diazepam) was added to 50 µL of the incubated sample for precipitation. The mixture was vortexed for 5 min, and centrifuged (12,000 rpm, 4 °C) for 10 min. 75 µL of supernatant was taken and diluted with 75 µL of ultrapure water. After being homogeneously mixed, 0.5 µL of the resulting sample was injected for analysis.

The compounds of the present application exhibit good *in vitro* stability in liver microsomes. The test results are shown in Table 2.

**Table 2**

| **Compound** | **Human liver microsome (Remaining amount % at 60 min)** | **Mouse liver microsome (Remaining amount % at 60 min)** |
|---|---|---|
| **9** | ≥60% | ≥50% |
| **18** | ≥60% | ≥50% |
| **31** | ≥60% | ≥50% |
| **34** | ≥60% | - |
| **42** | ≥60% | ≥50% |
| **43** | ≥60% | ≥50% |
| **45** | ≥60% | ≥50% |
| **47** | ≥60% | ≥50% |
| **49-a** | ≥60% | ≥50% |
| **50** | ≥60% | ≥50% |
| **51** | ≥60% | ≥50% |
| **53** | ≥60% | ≥50% |
| **73-a** | ≥60% | - |
| **73-b** | ≥60% | - |
| **75** | ≥60% | ≥50% |
| **80** | ≥60% | ≥50% |
| **81** | ≥60% | - |
| **82-a** | ≥60% | ≥50% |
| **82-b** | ≥60% | ≥50% |
| **88-b** | ≥60% | ≥50% |

### Test Example 3: In Vitro Inhibitory Activity Against Kinase

### 3.1 Assay for inhibitory activity against BTK(WT) kinase

BTK kinase solution (concentration: 0.003 ng/µL) was added to assay wells at 6 µL/well, and different compounds dissolved in DMSO were added to the assay wells separately using a nanoliter pipettor, such that the final concentrations of the compounds were 1000 nM to 0.244 nM. The wells were set in duplicate, and a control well was set. After a 30-min incubation of the above system, ATP (concentration: 50 µM) was mixed with ULight-poly GT substrate (manufacturer: PerkinElmer, concentration: 0.5 µM) in a ratio of 1:1, and the mixture was added to the assay wells at 4 µL/well. The resulting mixture was left to react at room temperature for 2 h, and then 5 µL of EDTA was added to terminate the reaction, followed by the addition of 5 µL of a detection antibody (manufacturer: PerkinElmer, concentration: 8 nM). The mixture was incubated at room temperature for 1 h. The assay was performed using a PerkinElmer Envision multifunctional microplate reader (excitation: 320 nm, emission: 615 nm/665 nm), and IC₅₀ was calculated by four-parameter fitting. The test results are shown in Table 3.

### 3.2 Assay for inhibitory activity against BTK(C481S) kinase

BTK(C481S) kinase solution (concentration: 0.006 ng/µL) was added to assay wells at 6 µL/well, and different compounds dissolved in DMSO were added to the assay wells separately using a nanoliter pipettor, such that the final concentrations of the compounds were 1000 nM to 0.244 nM. The wells were set in duplicate, and a control well was set. After a 30-min incubation of the above system, ATP (concentration: 50 µM) was mixed with ULight-poly GT substrate (manufacturer: PerkinElmer, concentration: 0.5 µM) in a ratio of 1:1, and the mixture was added to the assay wells at 4 µL/well. The resulting mixture was left to react at room temperature for 2 h, and then 5 µL of EDTA was added to terminate the reaction, followed by the addition of 5 µL of a detection antibody (manufacturer: PerkinElmer, concentration: 8 nM). The mixture was incubated at room temperature for 1 h. The assay was performed using a PerkinElmer Envision multifunctional microplate reader (excitation: 320 nm, emission: 615 nm/665 nm), and IC₅₀ was calculated by four-parameter fitting. The test results are shown in Table 3, where A represents: the IC₅₀ value ≤ 10 nM.

**Table 3**

| **Compound** | **BTK(WT) kinase inhibition** | **BTK(C481S) kinase inhibition** | **Compound** | **BTK(WT) kinase inhibition** | **BTK(C481S) kinase inhibition** |
|---|---|---|---|---|---|
| | **IC₅₀(nM )** | | | **IC₅₀(nM )** | |
| **1** | A | A | **48-b** | A | A |
| **1-b** | A | A | **49-a** | A | A |
| **2** | A | A | **49-b** | A | A |
| **3-b** | A | A | **50** | A | A |
| **5** | A | A | **51** | A | A |
| **6** | A | A | **52** | A | A |
| **7** | A | A | **53** | A | A |
| **10** | A | A | **54** | A | A |
| **11-a** | A | A | **54-a** | A | A |
| **11-b** | A | A | **55** | A | A |
| **12** | A | A | **56** | A | A |
| **13** | A | A | **57** | A | A |
| **14** | A | A | **58** | A | A |
| **17** | A | A | **59-a** | A | A |
| **18** | A | A | **59-b** | A | A |
| **21** | A | A | **61** | A | A |
| **22** | A | A | **62** | A | A |
| **23** | A | A | **63** | A | A |
| **24-a** | A | A | **70** | A | A |
| **26** | A | A | **71-b** | A | A |
| **30** | A | A | **73-b** | A | A |
| **31** | A | A | **74** | A | A |
| **32** | A | A | **75** | A | A |
| **33** | A | A | **76** | A | A |
| **34** | A | A | **77** | A | A |
| **37-b** | A | A | **79** | A | A |
| **38** | A | A | **80** | A | A |
| **39** | A | A | **81** | A | A |
| **40** | A | A | **82-a** | A | A |
| **41** | A | A | **82-b** | A | A |
| **42** | A | A | **83** | A | A |
| **43** | A | A | **84** | A | A |
| **44** | A | A | **85** | A | A |
| **45** | A | A | **86** | A | A |
| **46** | A | A | **87** | A | A |
| **47** | A | A | **88-b** | A | A |

### Test Example 4: In-Vivo Pharmacokinetic Study in Mice

ICR mice weighing 20-25 g were randomly grouped after 3-5 days of acclimatization, with 6 mice in each intravenous injection group and 3 mice in each intragastric administration group. The mice were intragastrically (IG) and intravenously (IV) administered the compound solutions of the present application at a dose of 5 mg/kg. Blood collection time points for intragastric administration were 30 min, 2 h, 8 h, and 24 h, and blood collection time points for intravenous injection were 0.083 h (5 min), 0.25 h (15 min), 1 h, 2 h, 4 h, 8 h, and 24 h. Blood was collected from the orbit to prepare plasma samples to be tested. 30 µL of each of the plasma samples to be tested and a standard curve sample were taken, and an acetonitrile solution containing an internal standard was added. Supernatants were obtained by protein precipitation, diluted, and then assayed by LC/MS/MS. Fitting was performed using a non-compartmental model, and the pharmacokinetic parameters are shown in Table 4.

**Table 4**

| **Compound** | **Compound 3-b** | | **Compound 12-a** | | **Compound 54-a** | | **Compound 14** | |
|---|---|---|---|---|---|---|---|---|
| **Route of administration** | IV | IG | IV | IG | IV | IG | IV | IG |
| **Dose** | 5mg/kg | 5mg/kg | 5mg/kg | 5mg/kg | 5mg/kg | 5mg/kg | 5mg/kg | 5mg/kg |
| **AUC(0-t) ng*h/mL** | 3602 | 3038 | 4705 | 2934 | 13855 | 10930 | 9322 | 4275 |
| **T_{1/2} h** | 12.1 | / | 4.24 | / | 9.54 | / | 3.17 | / |
| **Cₘₐₓ ng/mL** | / | 191 | / | 701 | / | 707 | / | 1874 |
| **Vₛₛ L/kg** | 16.5 | / | 3.015 | / | 3.779 | / | 1.12 | / |
| **CL L/h/kg** | 1.10 | / | 1.05 | / | 0.305 | / | 0.535 | / |

### Test Example 5: In Vivo Pharmacodynamic Study

### 5.1 Pharmacodynamic evaluation of OCI-LY10 cells in mouse subcutaneous xenograft tumor model

SPF-grade female NOD-SCID mice (source: Shanghai Lingchang Biotech Co., Ltd.) were subcutaneously inoculated at the right axilla with OCI-LY10 cells at 1 × 10⁷ cells/mouse. When the mean tumor volume reached about 180 mm³, the animals were grouped.

The day of grouping was defined as day 0, and intragastric administration was performed once daily from day 1 at a dose of 20 mpk or 40 mpk. The tumor volume was measured twice every week, and meanwhile, the mice were weighed, and the data were recorded. The general behavior of the mice was observed and recorded every day. After the experiment was completed, the tumors were extracted, weighed, and photographed.

The detection parameters and the calculation formulas are as follows: $Tumor volume TV \left({mm}^{3}\right) = 1 / 2 \times \left(a\times {b}^{2}\right) ,$ where a represents the long diameter of the tumor, and b represents the short diameter of the tumor. $Relative tumor volume RTV = {TV}_{t} {/ TV}_{0} ,$ where TV₀ represents the tumor volume on day 0, and TVₜ represents the tumor volume at each measurement. $Relative tumor proliferation rate T / C \left(%\right) = \left({T}_{RTV}{/ C}_{RTV}\right) \times 100 % ,$ where T_{RTV} represents RTV of the treatment group, and C_{RTV} represents RTV of the vehicle control group. $Tumor volume inhibition rate TGI \left(TV\right) \left(%\right) , TGI \left(%\right) = \left[1-\left(T-T0\right)/\left(C-0\right)\right] \times 100 % ,$ where T represents the mean tumor volume of each treatment group; T0 represents the mean tumor volume of each treatment group on d0; C represents the mean tumor volume of the control group; C0 represents the mean tumor volume of the control group on d0. $Tumor weight inhibition rate TGI \left(TW\right) \left(%\right) , TGI \left(%\right) = \left(1-TWt/TWc\right) \times 100 % ,$ where TWt represents the tumor weight of each treatment group; TWc represents the tumor weight of the control group. $Weight change rate WCR \left(%\right) = \left({Wt}_{t}-{Wt}_{0}\right) / {Wt}_{0} \times 100 % ,$ where Wt₀ represents the body weight of the mouse on day 0, and Wtt represents the body weight of the mouse at each measurement.

The compounds of the present application, including the example compounds, exhibit relatively high *in vivo* antitumor effects. For example, compound 3-b, compound 12-a, compound 54-a, and compound 14 had tumor volume inhibition rates greater than 95% and tumor weight inhibition rates greater than 92% on day 23 of administration.

## Claims

1. A compound of formula (I), a stereoisomer thereof, or a pharmaceutically acceptable salt thereof, 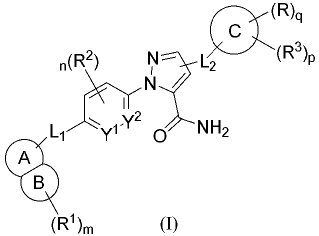 wherein,
Y¹ and Y² are each independently selected from the group consisting of CH and N;
ring A or ring B is each independently selected from the group consisting of phenyl and 5- to 6-membered heteroaryl; ring C is selected from the group consisting of 6- to 12-membered aryl, 4- to 12-membered heterocyclyl, and 5- to 12-membered heteroaryl;
each R¹ is independently selected from the group consisting of deuterium, hydroxy, amino, cyano, nitro, halogen, C₁₋₆ alkyl, hydroxy C₁₋₆ alkyl, 3- to 6-membered cycloalkyl, C₁₋₆ alkoxy, C₁₋₆ alkylthio, C₁₋₆ alkylamino, di-C₁₋₆ alkylamino, C₁₋₆ haloalkyl, C₁₋₆ haloalkoxy, C₁₋₆ haloalkylthio, C₁₋₆ haloalkylamino, and di-C₁₋₆ haloalkylamino; each R² is independently selected from the group consisting of deuterium, hydroxy, amino, cyano, halogen, C₁₋₆ alkyl, C₂₋₆ alkenyl, C₂₋₆ alkynyl, hydroxy C₁₋₆ alkyl, 3- to 6-membered cycloalkyl, C₁₋₆ alkoxy, C₁₋₆ alkylthio, C₁₋₆ alkylamino, di-C₁₋₆ alkylamino, C₁₋₆ haloalkyl, C₁₋₆ haloalkoxy, C₁₋₆ haloalkylthio, C₁₋₆ haloalkylamino, and di-C₁₋₆ haloalkylamino;
each R³ is independently selected from the group consisting of deuterium, hydroxy, amino, cyano, halogen, C₁₋₆ alkyl, C₁₋₆ alkoxy, C₁₋₆ alkylamino, di-C₁₋₆ alkylamino, C₂₋₈ alkenyl, and C₂₋₈ alkynyl, wherein the hydroxy, amino, C₁₋₆ alkyl, C₁₋₆ alkoxy, C₁₋₆ alkylamino, di-C₁₋₆ alkylamino, C₂₋₈ alkenyl, or C₂₋₈ alkynyl is optionally substituted with one or more R^{3a};
each R^{3a} is independently selected from the group consisting of deuterium, hydroxy, halogen, amino, cyano, =O, C₁₋₆ alkyl, C₁₋₆ alkoxy, C₁₋₆ alkylamino, di-C₁₋₆ alkylamino, 3- to 6-membered cycloalkyl, and 4- to 6-membered heterocyclyl, wherein the hydroxy, amino, C₁₋₆ alkyl, C₁₋₆ alkoxy, C₁₋₆ alkylamino, di-C₁₋₆ alkylamino, 3- to 6-membered cycloalkyl, or 4- to 6-membered heterocyclyl is optionally substituted with one or more R^{3aa};
each R^{3aa} is independently selected from the group consisting of deuterium, hydroxy, halogen, amino, cyano, =O, C₁₋₆ alkyl, C₁₋₆ alkoxy, -N(C₁₋₆ alkyl)₂, -NHC₁₋₆ alkyl, C₂₋₆ alkenyl, C₂₋₆ alkynyl, 3- to 6-membered cycloalkyl, and 4- to 6-membered heterocyclyl;
each R is independently selected from the group consisting of 3- to 12-membered cycloalkyl, 4- to 12-membered heterocyclyl, 6- to 12-membered aryl, and 5- to 12-membered heteroaryl, wherein the 3- to 12-membered cycloalkyl, 4- to 12-membered heterocyclyl, 6- to 12-membered aryl, or 5- to 12-membered heteroaryl is optionally substituted with one or more R^{a};
each R^{a} is independently selected from the group consisting of deuterium, hydroxy, halogen, amino, cyano, =O, - N(C₁₋₆ alkyl)₂, -NHC₁₋₆ alkyl, C₁₋₆ alkyl, hydroxy C₁₋₆ alkyl, C₁₋₆ haloalkyl, deuterated C₁₋₆ alkyl, amino C₁₋₆ alkyl, C₁₋₆ alkoxy C₁₋₆ alkylene, deuterated C₁₋₆ alkoxy C₁₋₆ alkylene, C₁₋₆ haloalkoxy C₁₋₆ alkylene, C₁₋₆ alkylamino C₁₋₆ alkylene, di-C₁₋₆ alkylamino C₁₋₆ alkylene, C₁₋₆ alkylthio C₁₋₆ alkylene, 3- to 6-membered cycloalkyl, 4- to 6-membered heterocyclyl, 3- to 6-membered cycloalkyl C₁₋₆ alkylene, and 4- to 6-membered heterocyclyl C₁₋₆ alkylene;
L₁ is selected from the group consisting of a bond, -C₁₋₆ alkylene-, -C(O)NHC₁₋₆ alkylene-, -NHC(O)C₁₋₆ alkylene-, and -NHC₁₋₆ alkylene-, wherein the -C₁₋₆ alkylene-, -C(O)NHC₁₋₆ alkylene-, -NHC(O)C₁₋₆ alkylene-, or -NHC₁₋₆ alkylene- is optionally substituted with one or more groups selected from the group consisting of deuterium, hydroxy, halogen, amino, cyano, =O, and C₁₋₆ alkyl;
L₂ is selected from the group consisting of a bond, -NH-, -N(C₁₋₃ alkyl)-, -O-, and -S-;
m is selected from the group consisting of 0, 1, 2, 3, 4, 5, and 6;
n is selected from the group consisting of 0, 1, 2, 3, and 4;
p is selected from the group consisting of 0, 1, 2, 3, and 4;
q is selected from the group consisting of 0, 1, and 2;
provided that p + q ≥ 1.

2. The compound of formula (I), the stereoisomer thereof, or the pharmaceutically acceptable salt thereof according to claim 1, wherein at least one of Y¹ and Y² is CH; or, Y¹ is CH and Y² is N; or, Y¹ is N and Y² is CH; or, Y¹ and Y² are both CH.

3. The compound of formula (I), the stereoisomer thereof, or the pharmaceutically acceptable salt thereof according to claim 1 or 2, wherein ring A or ring B is each independently selected from the group consisting of phenyl, pyridinyl, pyrimidinyl, pyridazinyl, pyrazinyl, pyranyl, furanyl, pyrrolyl, thienyl, pyrazolyl, imidazolyl, thiazolyl, oxazolyl, and isoxazolyl;
or, ring A or ring B is each independently selected from the group consisting of phenyl and 6-membered heteroaryl; or, ring A is selected from phenyl and ring B is selected from 5- to 6-membered heteroaryl; or, ring A is selected from phenyl and ring B is selected from 6-membered heteroaryl;
or, ring A is selected from the group consisting of phenyl and pyridinyl; or, ring B is selected from the group consisting of phenyl and pyridinyl;
or, the moiety 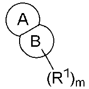 is selected from the group consisting of 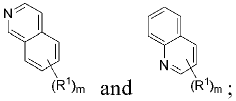 or, the moiety 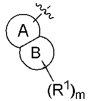 is selected from the group consisting of 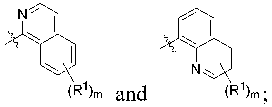
or, the moiety 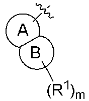 is selected from the group consisting of

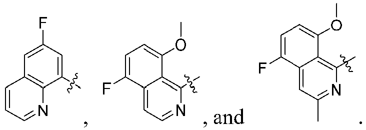

4. The compound of formula (I), the stereoisomer thereof, or the pharmaceutically acceptable salt thereof according to any one of claims 1 to 3, wherein ring C is selected from the group consisting of 6- to 10-membered aryl, 5- to 10-membered heterocyclyl, and 5- to 10-membered heteroaryl; or, ring C is selected from the group consisting of phenyl, 5- to 6-membered heterocyclyl, and 5- to 6-membered heteroaryl;
or, ring C is selected from the group consisting of 6- to 10-membered aryl and 5- to 6-membered heteroaryl; or, ring C is selected from the group consisting of phenyl and 5- to 6-membered heteroaryl;
or, ring C is selected from the group consisting of phenyl, pyridinyl, pyrimidinyl, pyridazinyl, pyrazinyl, and pyranyl;
or, ring C is selected from the group consisting of phenyl and pyridinyl;
or, ring C is selected from pyridinyl;
or, the moiety 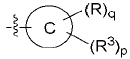 is selected from the group consisting of 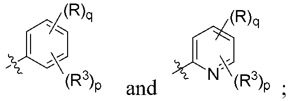 or, the moiety 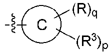 is selected from the group consisting of 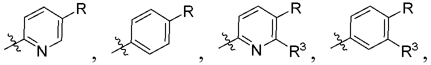 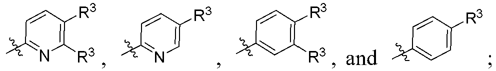 or, the moiety 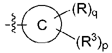 is selected from the group consisting of 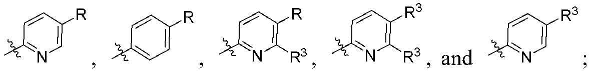 or, the moiety 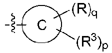 is selected from

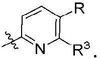

5. The compound of formula (I), the stereoisomer thereof, or the pharmaceutically acceptable salt thereof according to any one of claims 1 to 4, wherein each R¹ is independently selected from the group consisting of hydroxy, amino, cyano, halogen, C₁₋₄ alkyl, C₃₋₆ cycloalkyl, C₁₋₄ alkoxy, C₁₋₄ haloalkyl, C₁₋₄ alkylamino, and di-C₁₋₄ alkylamino;
or, each R¹ is independently selected from the group consisting of halogen, C₁₋₆ alkyl, and C₁₋₆ alkoxy;
or, each R¹ is independently selected from the group consisting of hydroxy, amino, cyano, fluoro, chloro, bromo, methyl, ethyl, n-propyl, isopropyl, n-butyl, isobutyl, tert-butyl, cyclopropyl, cyclobutyl, cyclopentyl, cyclohexyl, methoxy, ethoxy, trifluoromethyl, difluoromethyl, trifluoroethyl, dimethylamino, and diethylamino;
or, each R¹ is independently selected from the group consisting of fluorine, methyl, and methoxy.

6. The compound of formula (I), the stereoisomer thereof, or the pharmaceutically acceptable salt thereof according to any one of claims 1 to 5, wherein each R² is independently selected from the group consisting of hydroxy, amino, cyano, halogen, methyl, ethyl, n-propyl, isopropyl, trifluoromethyl, and difluoromethyl;
or, R² is selected from fluorine.

7. The compound of formula (I), the stereoisomer thereof, or the pharmaceutically acceptable salt thereof according to any one of claims 1 to 6, wherein each R³ is independently selected from the group consisting of deuterium, hydroxy, amino, cyano, halogen, C₁₋₄ alkyl, C₁₋₄ alkoxy, C₁₋₄ alkylamino, di-C₁₋₄ alkylamino, C₂₋₆ alkenyl, and C₂₋₆ alkynyl, wherein the hydroxy, amino, C₁₋₄ alkyl, C₁₋₄ alkoxy, C₁₋₄ alkylamino, di-C₁₋₄ alkylamino, C₂₋₆ alkenyl, or C₂₋₆ alkynyl is optionally substituted with one or more R^{3a};
or, each R³ is independently selected from the group consisting of amino, C₁₋₆ alkyl, and C₂₋₈ alkenyl, wherein the amino, C₁₋₆ alkyl, or C₂₋₈ alkenyl is optionally substituted with one or more R^{3a};
or, each R³ is independently selected from the group consisting of deuterium, cyano, fluorine, chlorine, and the following groups optionally substituted with one or more R^{3a}: hydroxy, amino, methyl, ethyl, n-propyl, isopropyl, n-butyl, isobutyl, tert-butyl, -CH=CH₂, -CH₂CH=CH₂, -CH₂CH₂CH=CH₂, -CH₂CH=CHCH₃, - CH₂CH₂CH₂CH=CH₂, -CH₂CH₂CH=CHCH₃, and -CH₂CH=CHCH₂CH₃;
or, each R³ is independently selected from the group consisting of the following groups optionally substituted with one or more R^{3a}: amino, methyl, ethyl, n-propyl, and -CH₂CH₂CH₂CH=CH₂;
or, each R³ is independently selected from the group consisting of 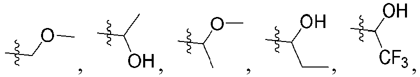 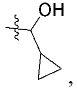 methyl, 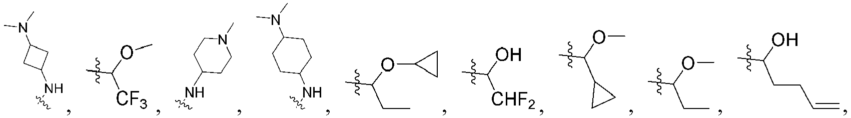 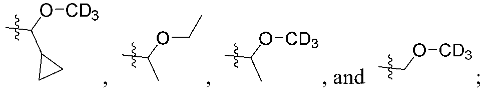
optionally, each R^{3a} is independently selected from the group consisting of deuterium, halogen, cyano, =O, hydroxy, amino, C₁₋₄ alkyl, C₁₋₄ alkoxy, C₁₋₄ alkylamino, di-C₁₋₄ alkylamino, 3- to 6-membered cycloalkyl, and 4- to 6-membered heterocycloalkyl, wherein the hydroxy, amino, C₁₋₆ alkyl, C₁₋₄ alkoxy, C₁₋₄ alkylamino, di-C₁₋₄ alkylamino, 3- to 6-membered cycloalkyl, or 4- to 6-membered heterocycloalkyl is optionally substituted with one or more R^{3aa};
or, each R^{3a} is independently selected from the group consisting of hydroxy, halogen, C₁₋₆ alkoxy, 3- to 6-membered cycloalkyl, and 4- to 6-membered heterocyclyl, wherein the hydroxy, C₁₋₆ alkoxy, 3- to 6-membered cycloalkyl, or 4- to 6-membered heterocyclyl is optionally substituted with one or more R^{3aa};
or, each R^{3a} is independently selected from the group consisting of hydroxy, halogen, amino, cyano, =O, methyl, ethyl, n-propyl, isopropyl, methoxy, ethoxy, methylamino, ethylamino, dimethylamino, diethylamino, cyclopropyl, cyclobutyl, cyclopentyl, cyclohexyl, azetidinyl, oxetanyl, tetrahydrofuranyl, tetrahydropyrrolyl, tetrahydrothienyl, piperidinyl, piperazinyl, and morpholinyl, wherein the hydroxy, amino, methyl, ethyl, n-propyl, isopropyl, methoxy, ethoxy, methylamino, ethylamino, dimethylamino, diethylamino, cyclopropyl, cyclobutyl, cyclopentyl, cyclohexyl, azetidinyl, oxetanyl, tetrahydrofuranyl, tetrahydropyrrolyl, tetrahydrothienyl, piperidinyl, piperazinyl, or morpholinyl is optionally substituted with one or more R^{3aa};
or, each R^{3a} is independently selected from the group consisting of fluoro, hydroxy, methoxy, ethoxy, cyclopropyl, cyclobutyl, cyclopentyl, cyclohexyl, and piperidinyl, wherein the hydroxy, methoxy, ethoxy, cyclopropyl, cyclobutyl, cyclopentyl, cyclohexyl, or piperidinyl is optionally substituted with one or more R^{3aa};
or, each R^{3a} is independently selected from the group consisting of hydroxy, fluoro, methoxy, ethoxy, -OCD₃, cyclopropyl, 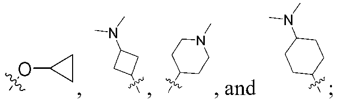
optionally, each R^{3aa} is independently selected from the group consisting of deuterium, hydroxy, halogen, amino, cyano, =O, -N(C₁₋₃ alkyl)₂, -NHC₁₋₃ alkyl, C₁₋₃ alkyl, 3- to 6-membered cycloalkyl, and 4- to 6-membered heterocycloalkyl;
or, each R^{3aa} is independently selected from the group consisting of deuterium, C₁₋₆ alkyl, -N(C₁₋₆ alkyl)₂, and 3- to 6-membered cycloalkyl;
or, each R^{3aa} is independently selected from the group consisting of deuterium, fluoro, -N(CH₃)₂, methyl, and cyclopropyl.

8. The compound of formula (I), the stereoisomer thereof, or the pharmaceutically acceptable salt thereof according to any one of claims 1 to 7, wherein each R is independently selected from the group consisting of the following groups optionally substituted with one or more R^{a}: 3- to 8-membered cycloalkyl, 4- to 8-membered heterocyclyl, 6- to 10-membered aryl, and 5- to 10-membered heteroaryl; or, each R is independently selected from the group consisting of the following groups optionally substituted with one or more R^{a}: 3- to 6-membered cycloalkyl, 4- to 6-membered heterocyclyl, phenyl, and 5- to 6-membered heteroaryl;
or, each R is independently selected from 4- to 12-membered heterocyclyl, wherein the 4- to 12-membered heterocyclyl is optionally substituted with one or more R^{a};
or, each R is independently selected from the group consisting of the following groups optionally substituted with one or more R^{a}: cyclopropyl, cyclobutyl, cyclopentyl, cyclohexyl, azetidinyl, oxetanyl, tetrahydrofuranyl, tetrahydropyrrolyl, tetrahydrothienyl, piperidinyl, tetrahydropyranyl, tetrahydrothiopyranyl, piperazinyl, morpholinyl, 1,4-dioxanyl, thiomorpholinyl, furanyl, pyrrolyl, pyrazolyl, imidazolyl, thienyl, thiazolyl, oxazolyl, isoxazolyl, phenyl, pyridinyl, pyrimidinyl, pyridazinyl, pyrazinyl, and pyranyl;
or, each R is independently selected from the group consisting of the following groups optionally substituted with one or more R^{a}: piperidinyl and piperazinyl;
or, each R is independently selected from the group consisting of 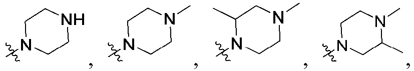 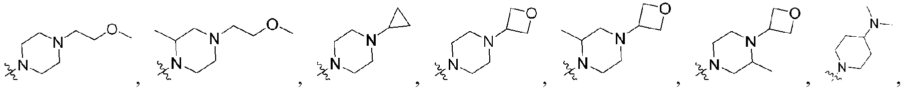 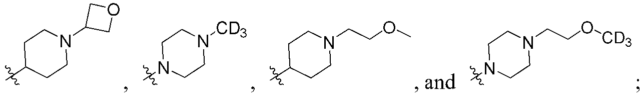
optionally, each R^{a} is independently selected from the group consisting of deuterium, hydroxy, halogen, amino, cyano, =O, -N(C₁₋₃ alkyl)₂, -NHC₁₋₃ alkyl, C₁₋₄ alkyl, hydroxy C₁₋₄ alkyl, C₁₋₄ haloalkyl, deuterated C₁₋₄ alkyl, amino C₁₋₄ alkyl, C₁₋₃ alkoxy C₁₋₃ alkylene, deuterated C₁₋₃ alkoxy C₁₋₃ alkylene, C₁₋₃ haloalkoxy C₁₋₃ alkylene, C₁₋₃ alkylamino C₁₋₃ alkylene, di-C₁₋₃ alkylamino C₁₋₃ alkylene, C₁₋₃ alkylthio C₁₋₃ alkylene, 3- to 6-membered cycloalkyl, 4- to 6-membered heterocycloalkyl, 3- to 6-membered cycloalkyl C₁₋₃ alkylene, and 4- to 6-membered heterocycloalkyl C₁₋₃ alkylene;
or, each R^{a} is independently selected from the group consisting of -N(C₁₋₆ alkyl)₂, C₁₋₆ alkyl, deuterated C₁₋₆ alkyl, C₁₋₆ alkoxy C₁₋₆ alkylene, deuterated C₁₋₆ alkoxy C₁₋₆ alkylene, 3- to 6-membered cycloalkyl, and 4- to 6-membered heterocyclyl;
or, each R^{a} is independently selected from the group consisting of C₁₋₃ alkoxy C₁₋₃ alkylene, C₁₋₃ alkylamino C₁₋₃ alkylene, di-C₁₋₃ alkylamino C₁₋₃ alkylene, 3- to 6-membered cycloalkyl, and 4- to 6-membered heterocycloalkyl; or, each R^{a} is independently selected from the group consisting of deuterium, hydroxy, halogen, amino, cyano, =O, -NHCH₃, -NHCH₂CH₃, -N(CH₃)₂, -N(CH₂CH₃)₂, methyl, ethyl, n-propyl, isopropyl, n-butyl, isobutyl, tert-butyl, hydroxymethyl, hydroxyethyl, trifluoromethyl, difluoromethyl, monofluoromethyl, trifluoroethyl, pentafluoroethyl, trideuteriomethyl, dideuteriomethyl, monodeuteriomethyl, -CH₂NH₂, -CH₂CH₂NH₂, -CH₂OCH₃, -CH₂OCH₂CH₃, - CH₂CH₂OCH₃, -CH₂CH₂OCH₂CH₃, -CH₂CH₂OCD₃, -CH₂NHCH₃, -CH₂CH₂NHCH₃, -CH₂N(CH₃)₂, - CH₂CH₂N(CH₃)₂, -CH₂SCH₃, -CH₂CH₂SCH₃, cyclopropyl, cyclobutyl, cyclopentyl, cyclohexyl, azetidinyl, oxetanyl, tetrahydrofuranyl, tetrahydropyrrolyl, tetrahydrothienyl, piperidinyl, piperazinyl, morpholinyl, cyclopropylmethyl, cyclobutylmethyl, cyclopentylmethyl, cyclohexylmethyl, azetidinylmethyl, oxetanylmethyl, tetrahydrofuranylmethyl, tetrahydropyrrolylmethyl, tetrahydrothienylmethyl, piperidinylmethyl, piperazinylmethyl, and morpholinylmethyl;
or, each R^{a} is independently selected from the group consisting of -N(CH₃)₂, methyl, trideuteriomethyl, - CH₂CH₂OCH₃, -CH₂CH₂OCD₃, cyclopropyl, and oxetanyl.

9. The compound of formula (I), the stereoisomer thereof, or the pharmaceutically acceptable salt thereof according to any one of claims 1 to 8, wherein L₁ is selected from the group consisting of a bond, -C₁₋₃ alkylene-, - C(O)NHC₁₋₃ alkylene-, -NHC(O)C₁₋₃ alkylene-, and -NHC₁₋₃ alkylene-, wherein the -C₁₋₃ alkylene-, -C(O)NHC₁₋₃ alkylene-, -NHC(O)C₁₋₃ alkylene-, or -NHC₁₋₃ alkylene- is optionally substituted with one or more groups selected from the group consisting of hydroxy, halogen, amino, cyano, =O, and C₁₋₃ alkyl; or L₁ is selected from the group consisting of -C(O)NHC₁₋₆ alkylene- and -NHC₁₋₆ alkylene-;
or, L₁ is selected from the group consisting of a bond, -methylene-, -C(O)NHCH₂-, -NHC(O)CH₂-, and -NHCH₂-; or, L₁ is selected from the group consisting of -C(O)NHCH₂- and -NHCH₂-;
optionally, L₂ is selected from -NH-.

10. The compound of formula (I), the stereoisomer thereof, or the pharmaceutically acceptable salt thereof according to any one of claims 1 to 9, wherein m is selected from the group consisting of 0, 1, 2, and 3; or, m is selected from the group consisting of 1 and 2;
optionally, n is selected from the group consisting of 0, 1, and 2; or, n is selected from 0;
optionally, p is selected from the group consisting of 0, 1, 2, and 3; or, p is selected from the group consisting of 1 and 2; or, p is selected from the group consisting of 0 and 1;
optionally, q is selected from the group consisting of 1 and 2; or, q is selected from 1.

11. The compound of formula (I), the stereoisomer thereof, or the pharmaceutically acceptable salt thereof according to any one of claims 1 to 10, wherein the compound of formula (I), the stereoisomer thereof, or the pharmaceutically acceptable salt thereof is selected from the group consisting of compounds of formula (I-A), formula (II), and formula (II-A), stereoisomers thereof, or pharmaceutically acceptable salts thereof, 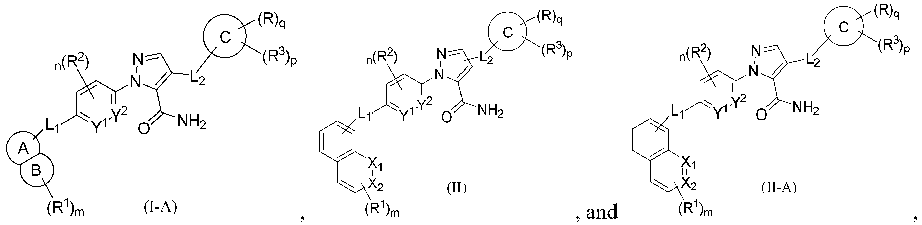 wherein R¹, m, L₁, Y₁, Y₂, R², n, L₂, ring A, ring B, ring C, R, q, R³, and p are as defined in any one of claims 1 to 10, and X₁ and X₂ are each independently selected from the group consisting of CH and N; or, X₁ is selected from N and X₂ is selected from CH; or, X₁ is selected from CH and X₂ is selected from N.

12. The compound of formula (I), the stereoisomer thereof, or the pharmaceutically acceptable salt thereof according to any one of claims 1 to 10, wherein the compound of formula (I), the stereoisomer thereof, or the pharmaceutically acceptable salt thereof is selected from the group consisting of compounds of formula (III-A) and formula (III-B), stereoisomers thereof, or pharmaceutically acceptable salts thereof, 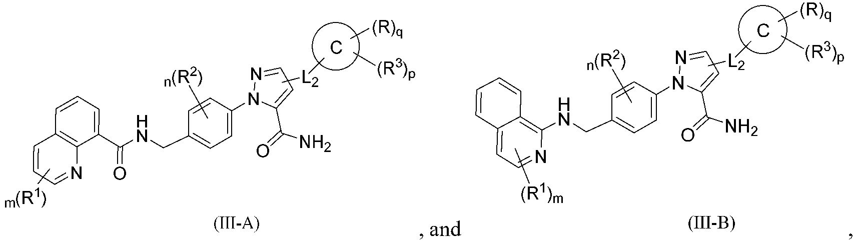 wherein R¹, m, R², n, L₂, ring C, R, q, R³, and p are as defined in any one of claims 1 to 11.

13. A compound of a formula selected from the group consisting of the following formulas, a stereoisomer thereof, or a pharmaceutically acceptable salt thereof: 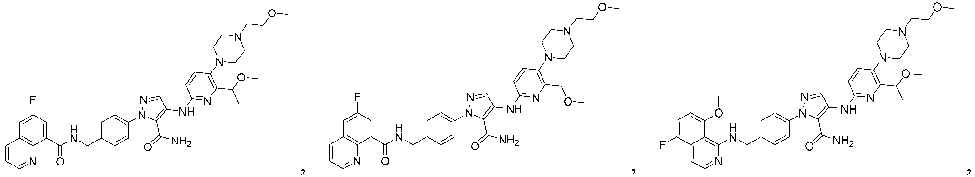 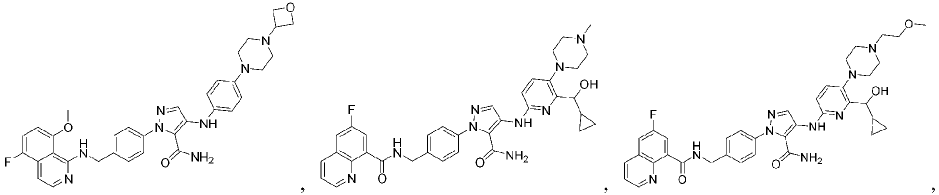 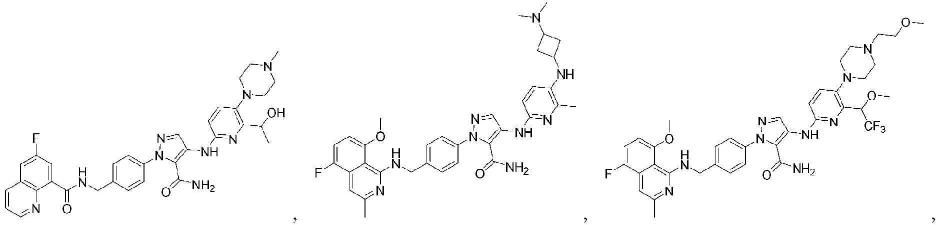 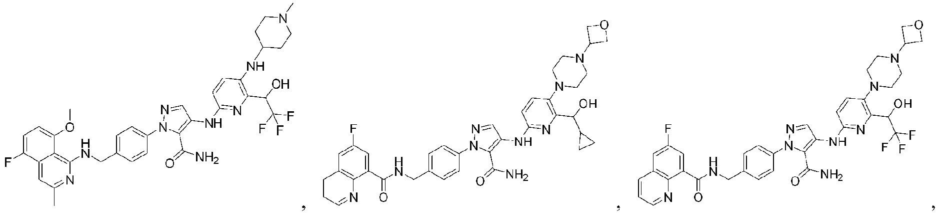 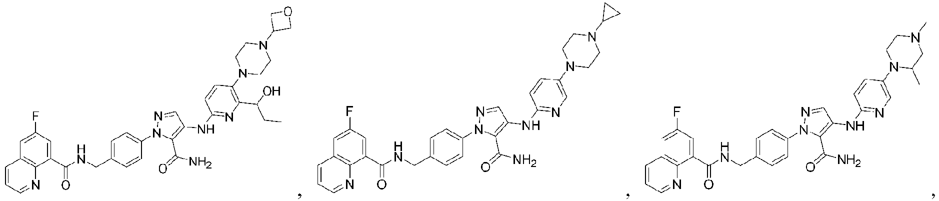 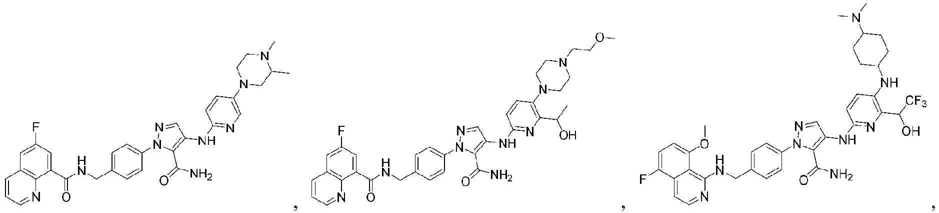 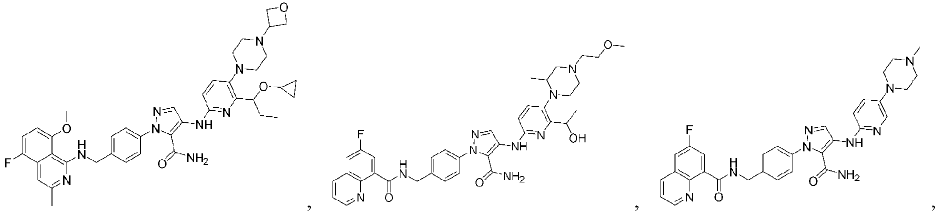 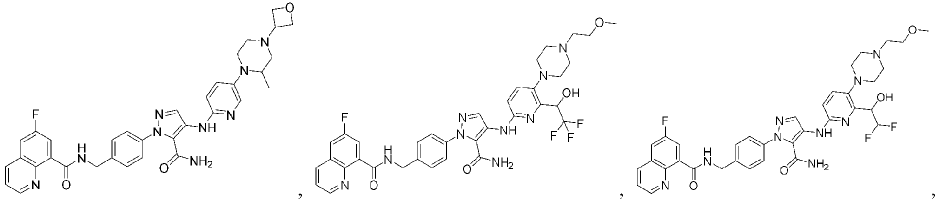 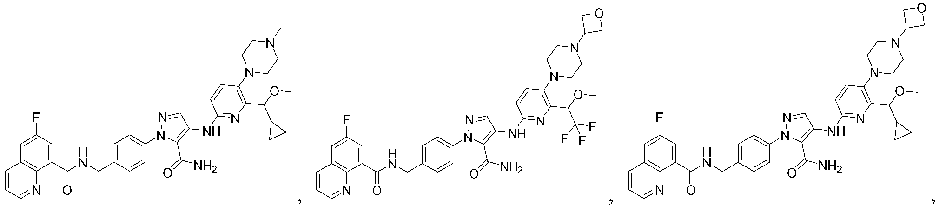 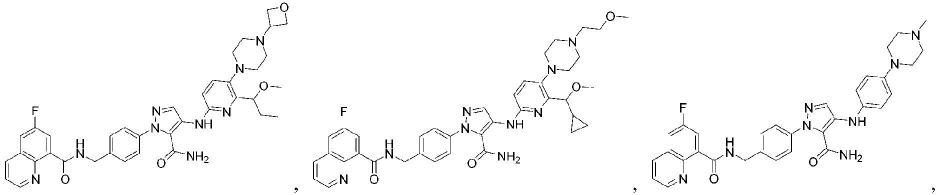 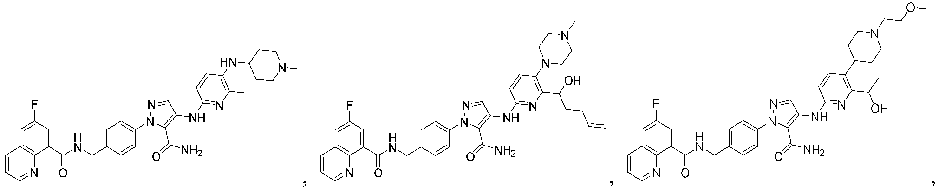 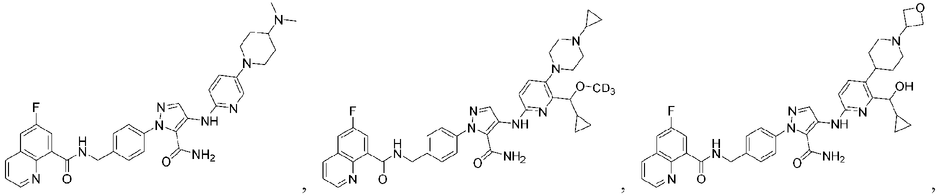 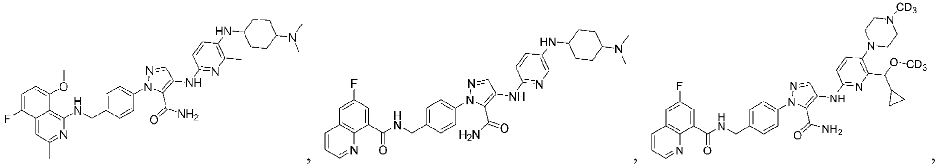 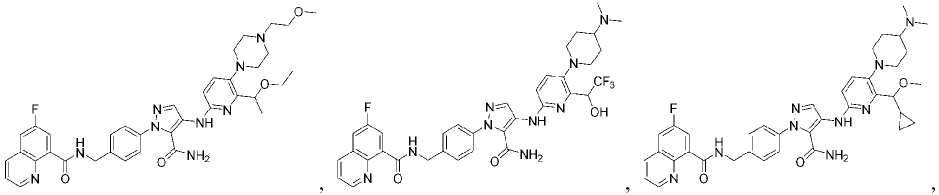 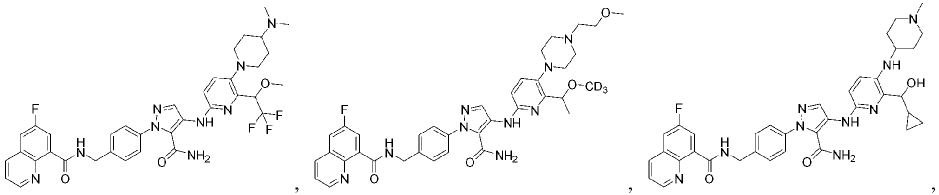 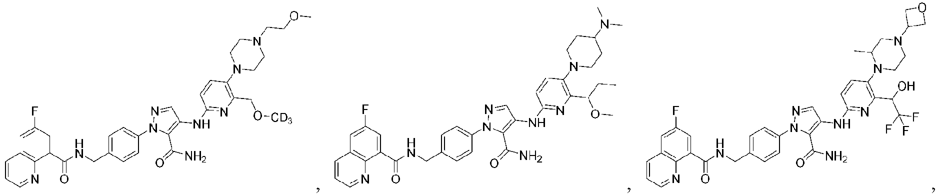 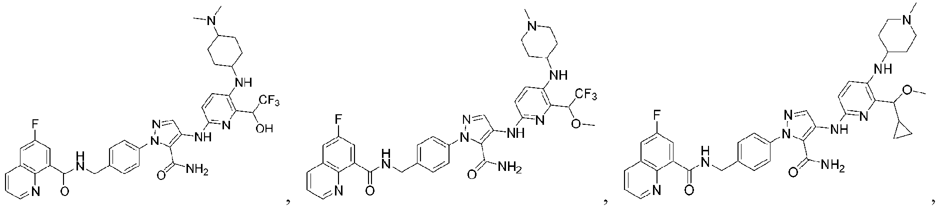 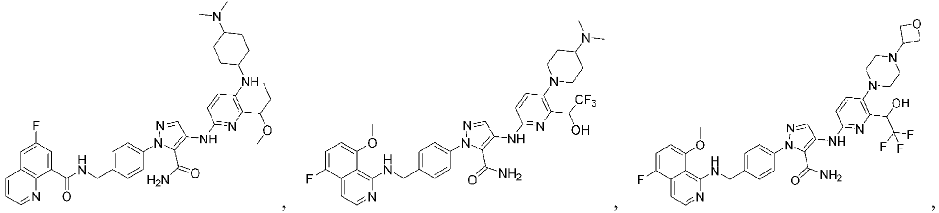 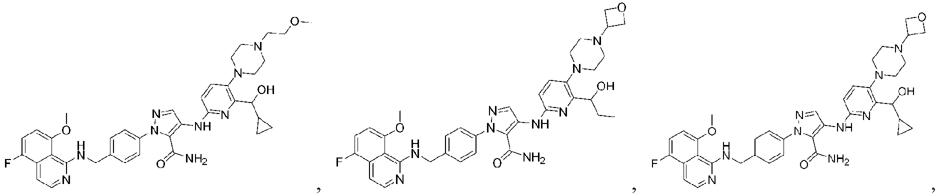 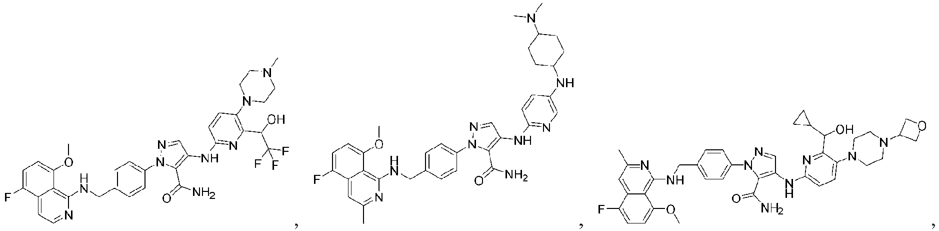 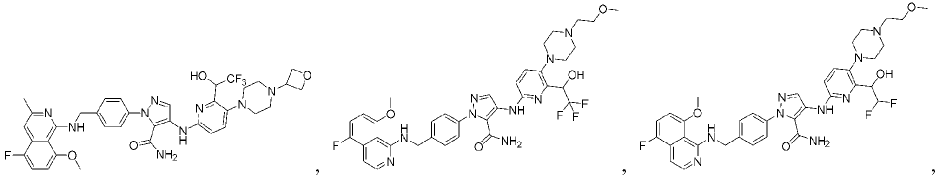 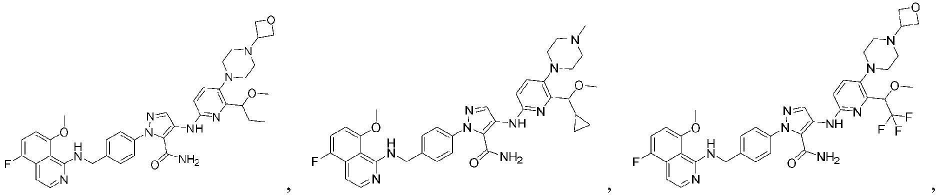 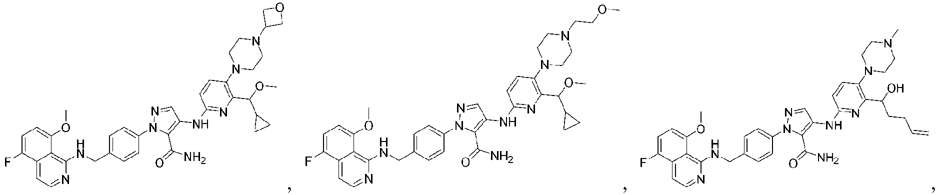 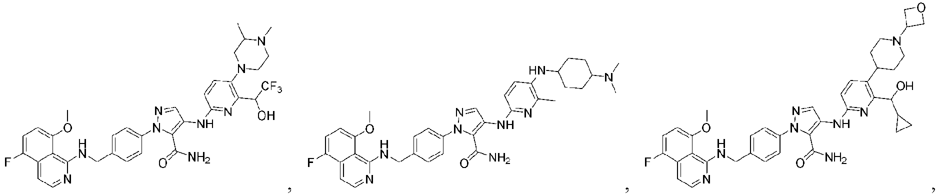 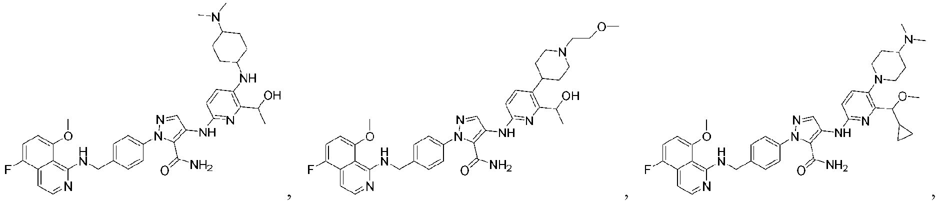 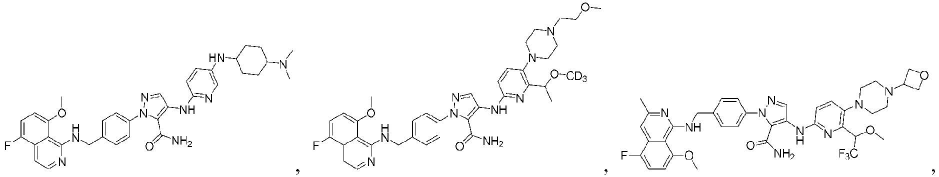 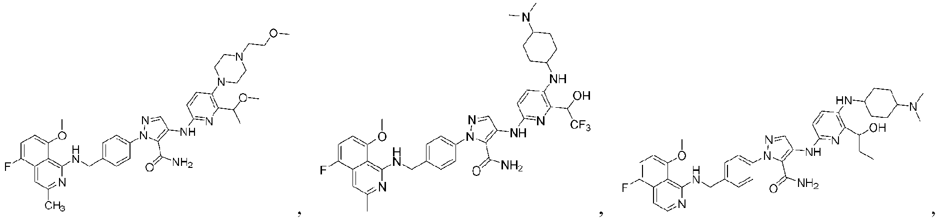 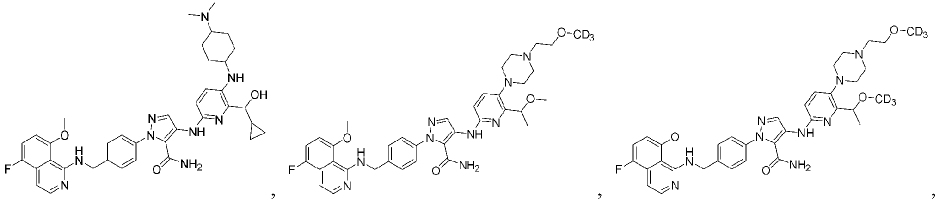 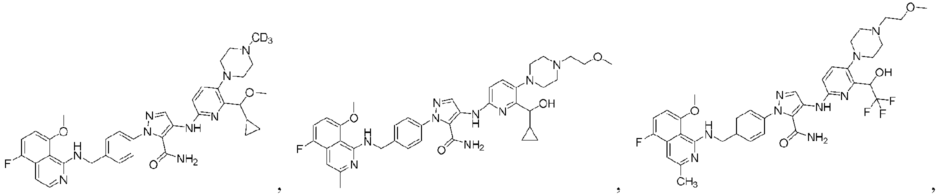 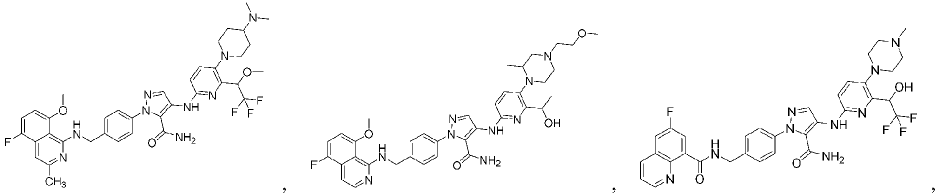 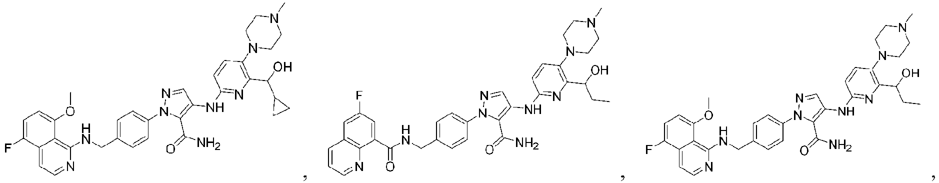 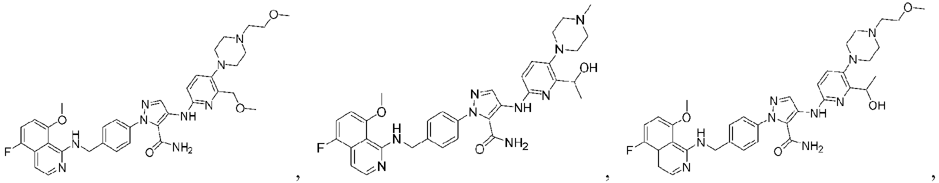 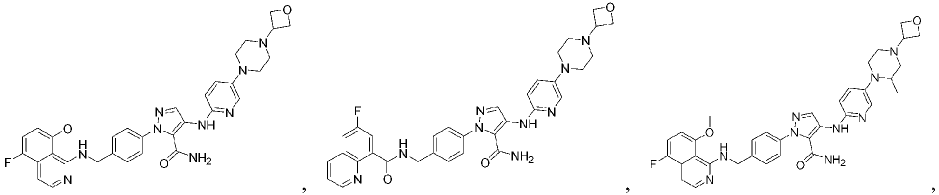 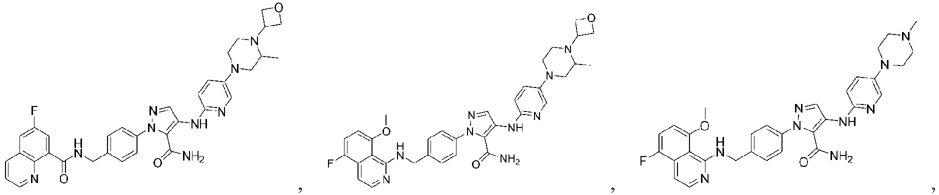 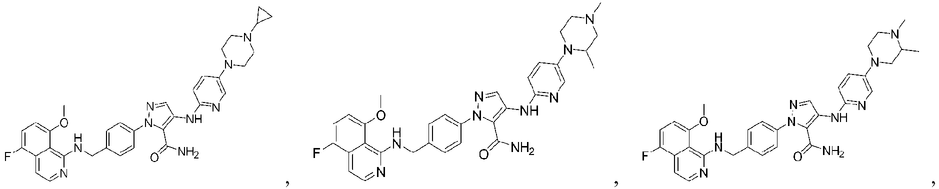 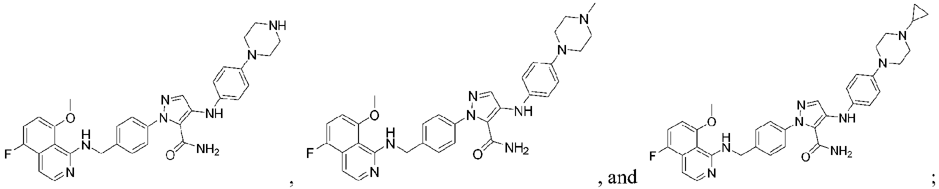 or, a compound of a formula selected from the group consisting of the following formulas, a stereoisomer thereof, or a pharmaceutically acceptable salt thereof:

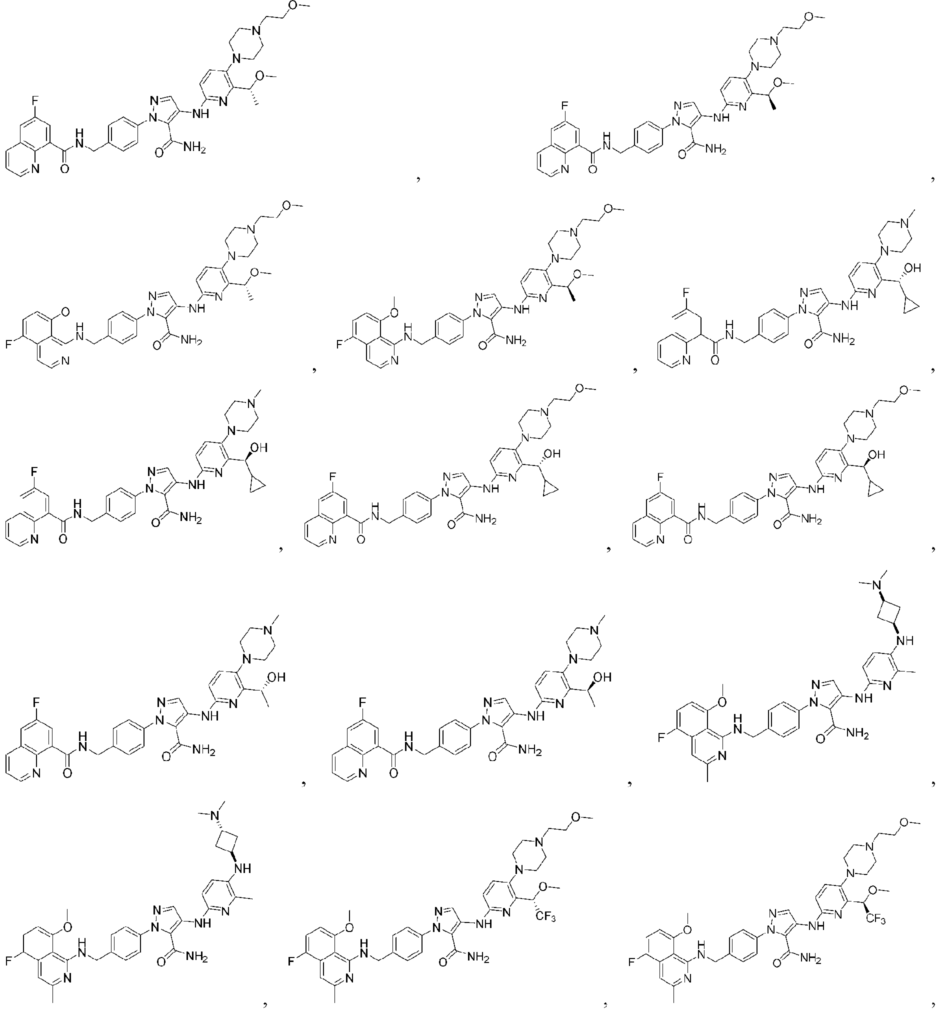

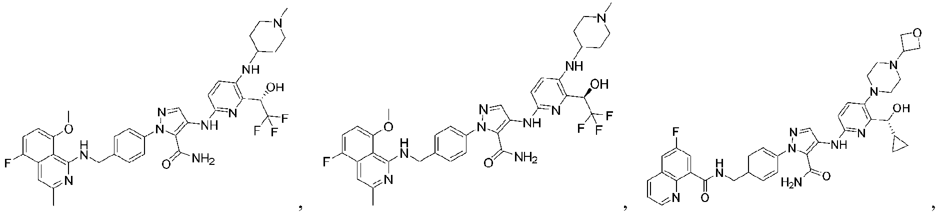

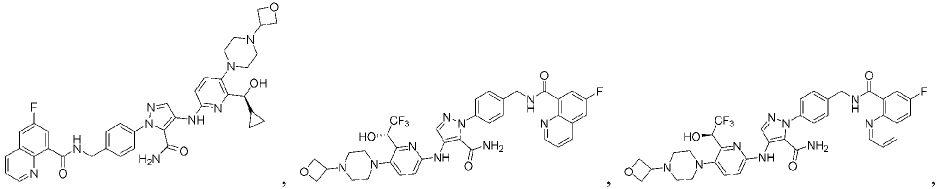

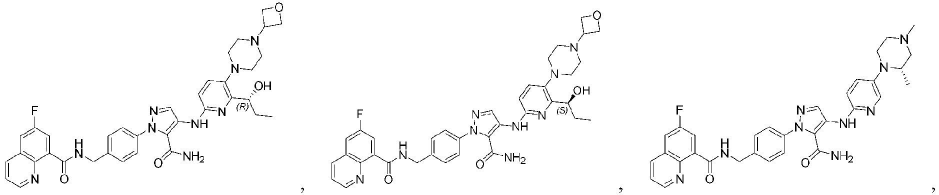

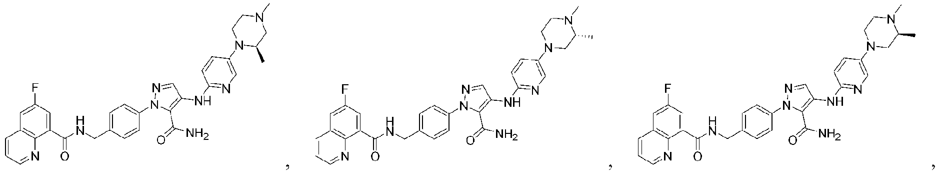

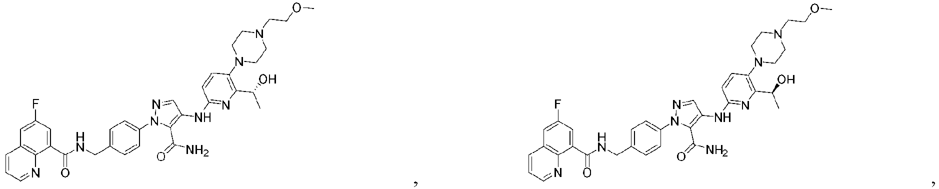

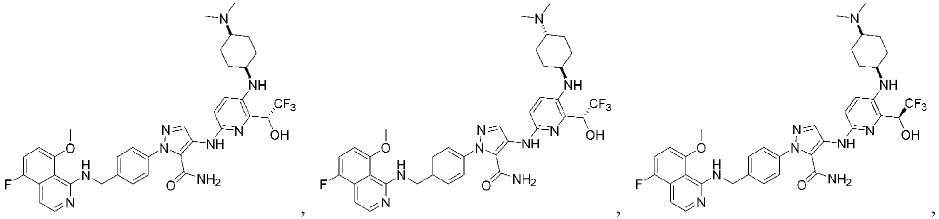

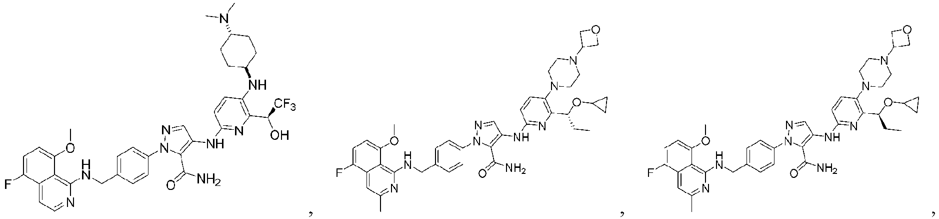

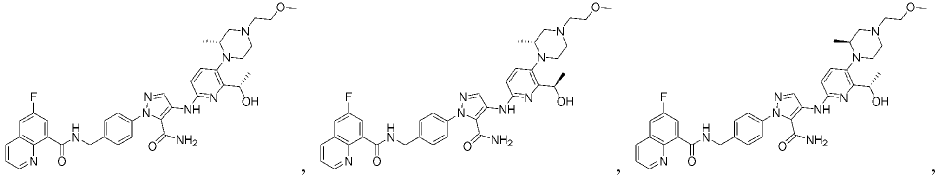

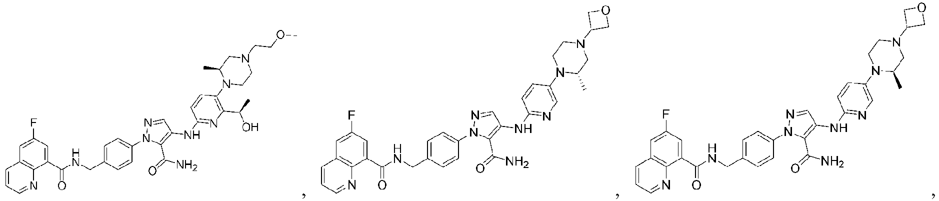

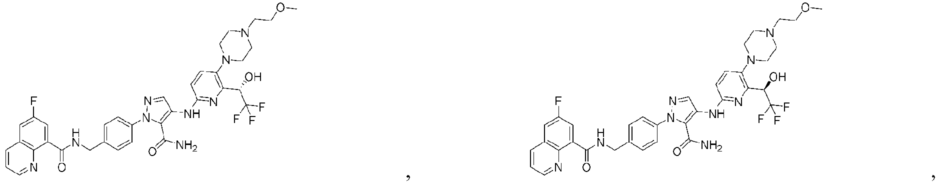

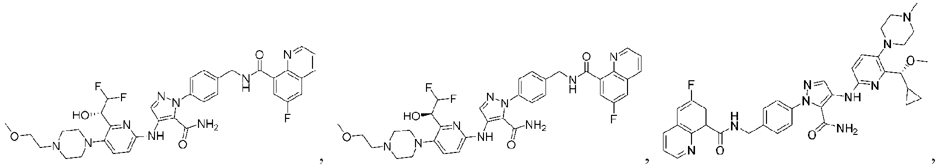

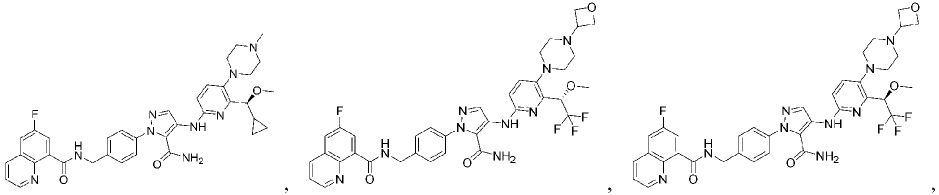

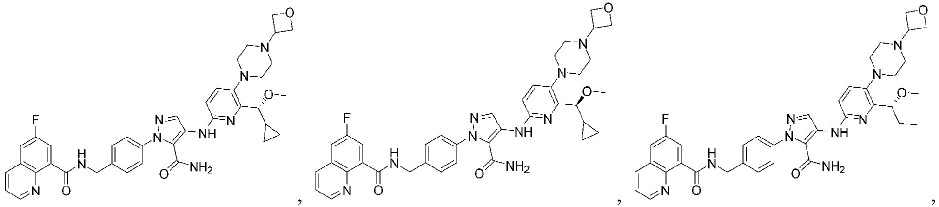

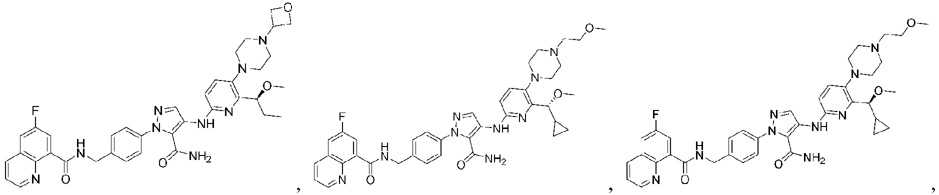

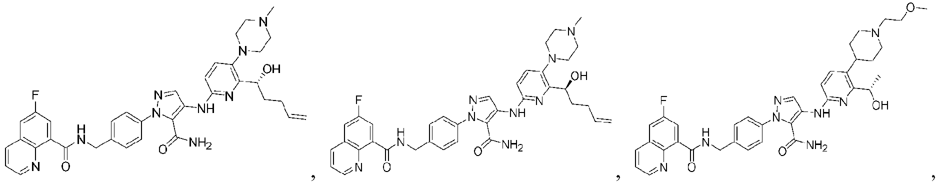

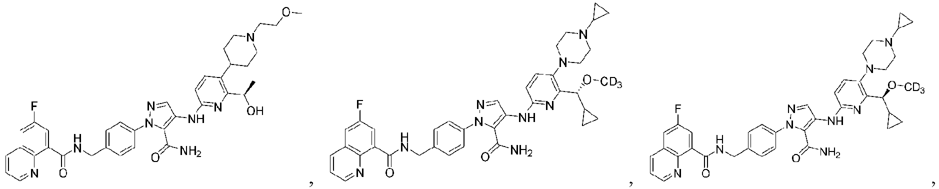

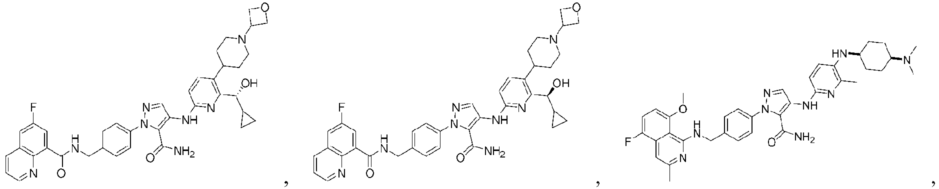

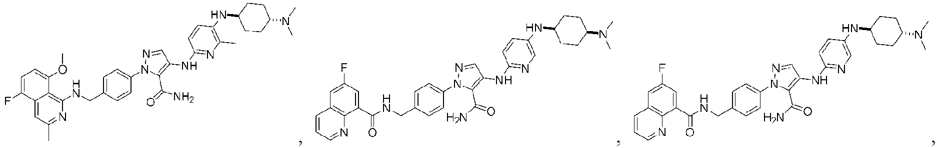

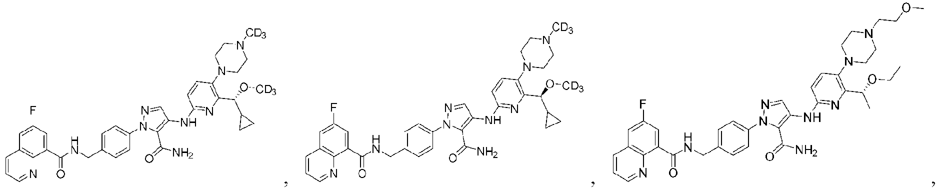

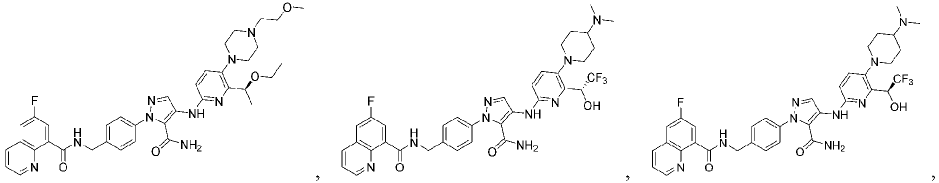

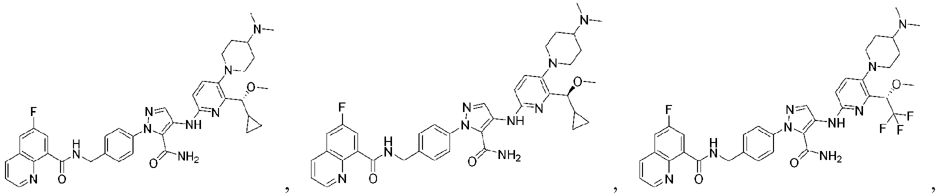

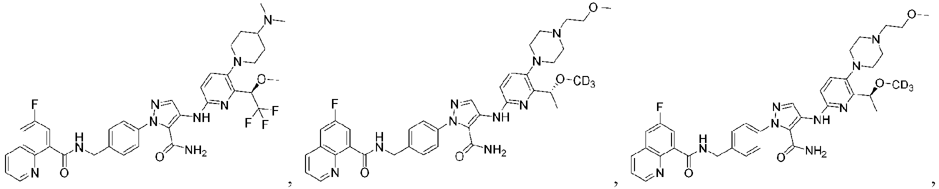

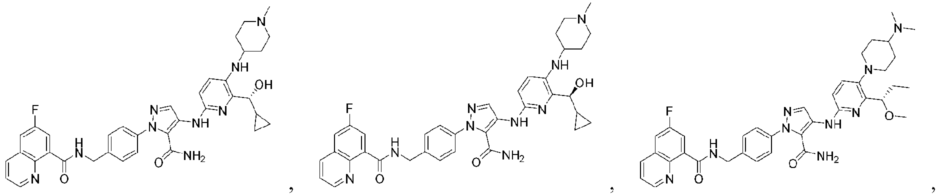

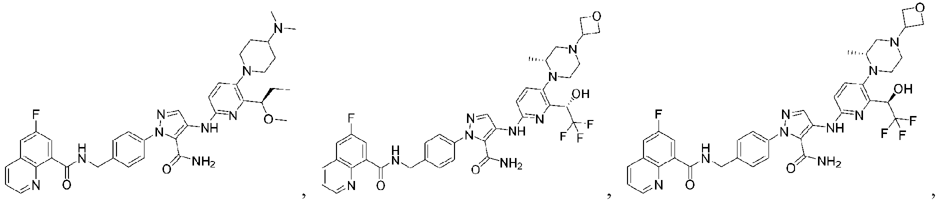

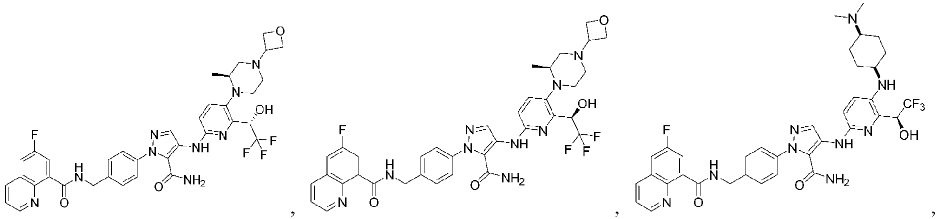

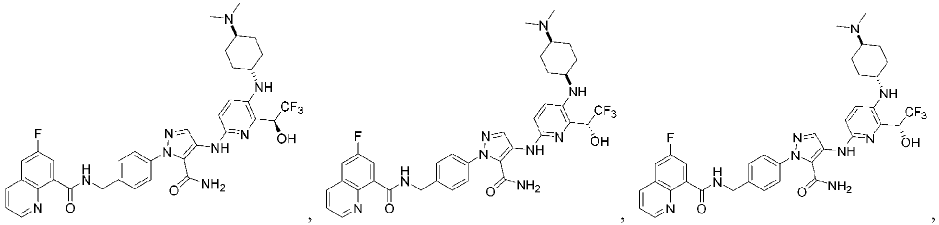

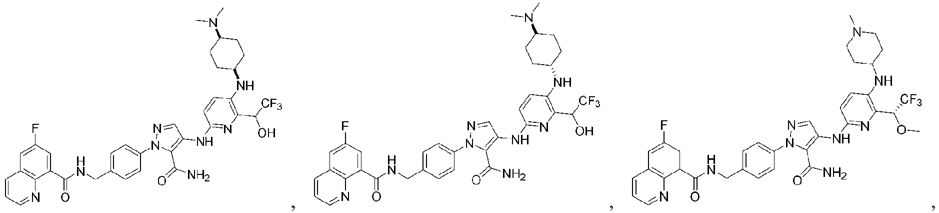

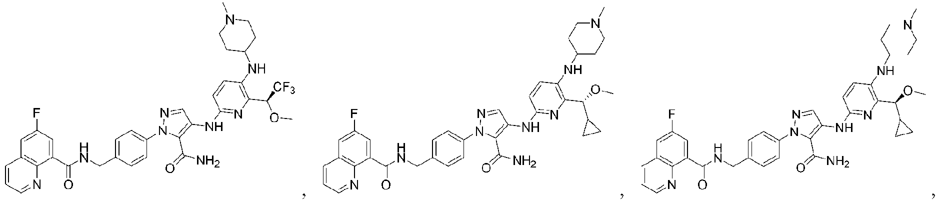

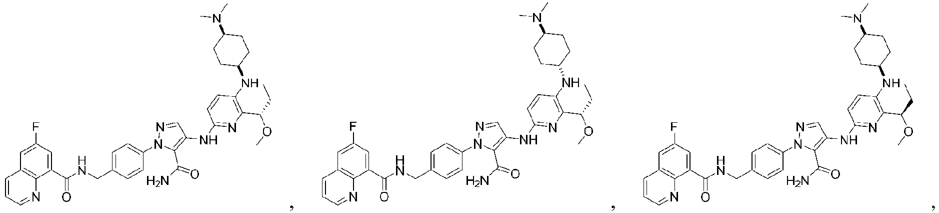

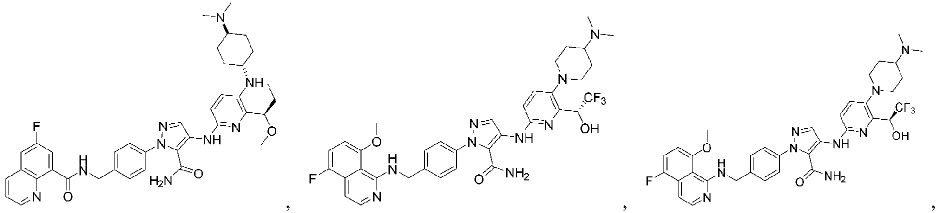

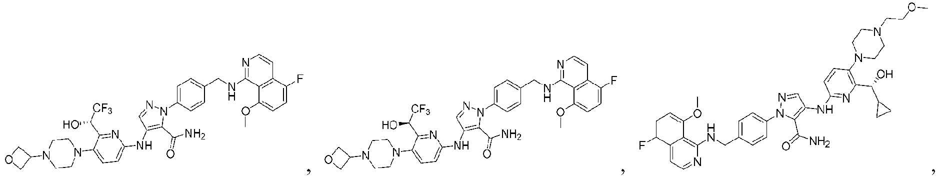

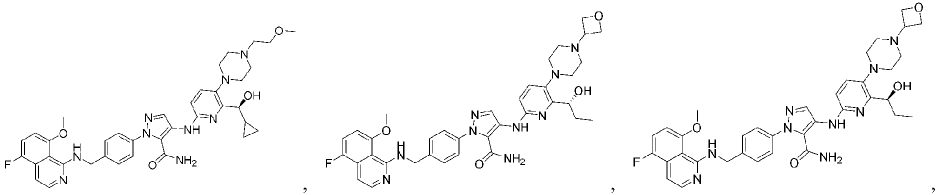

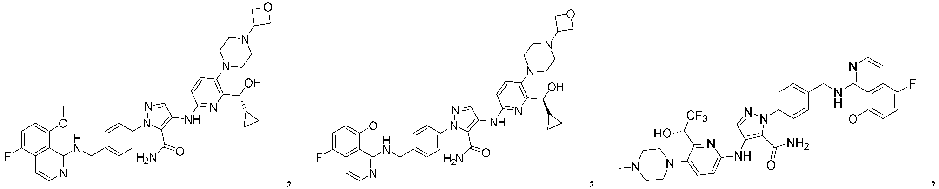

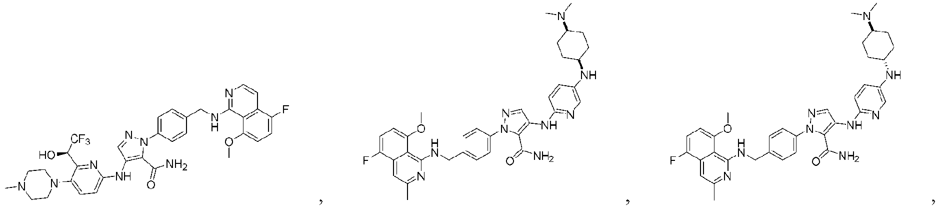

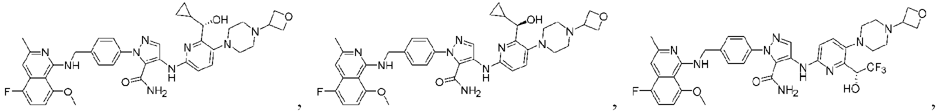

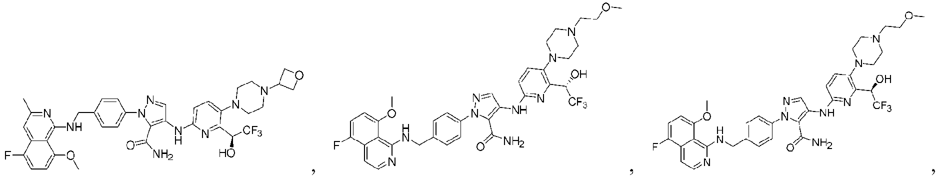

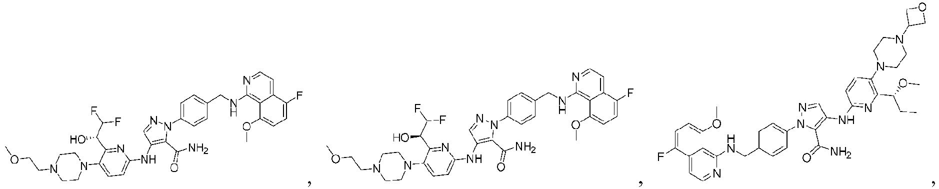

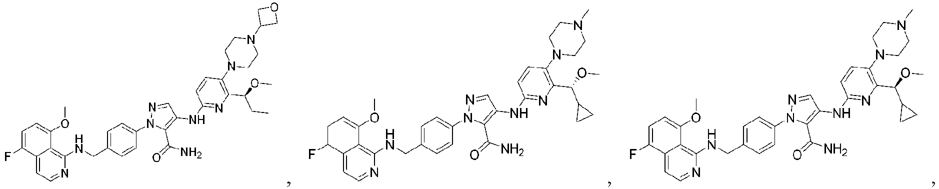

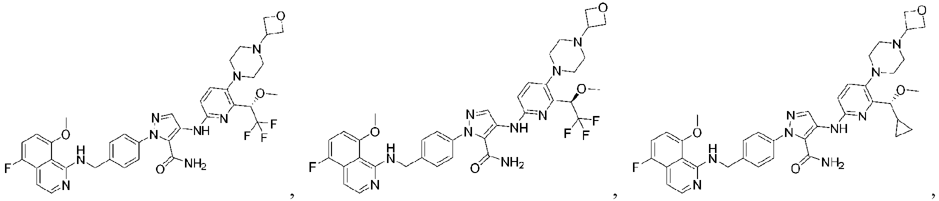

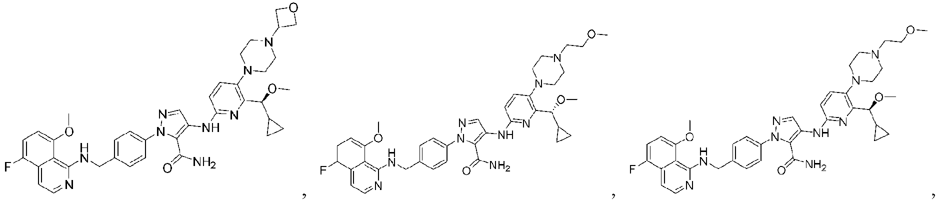

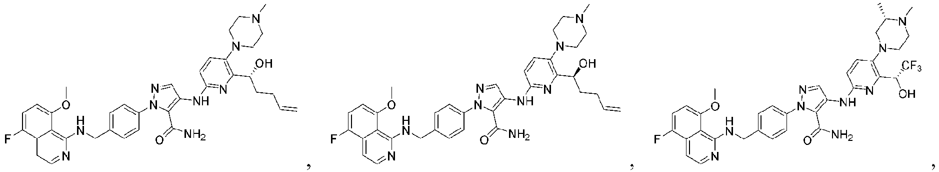

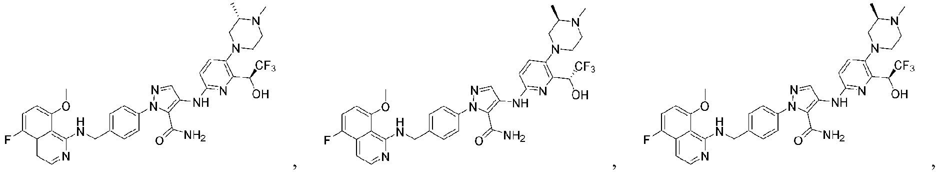

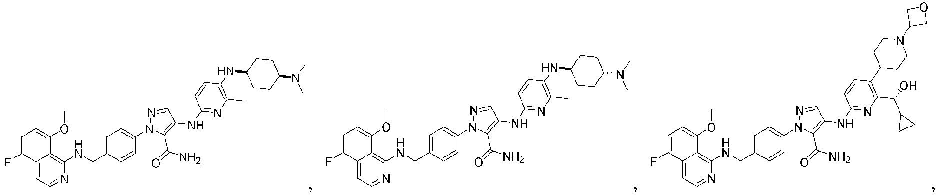

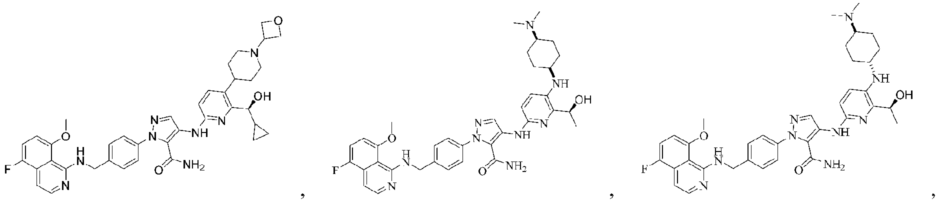

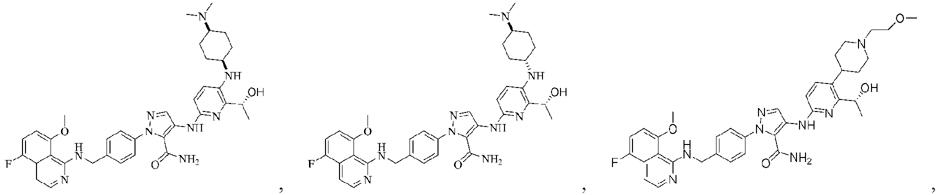

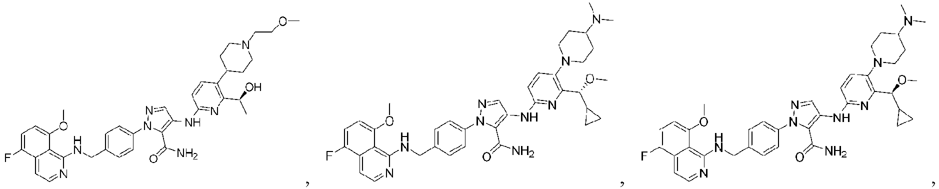

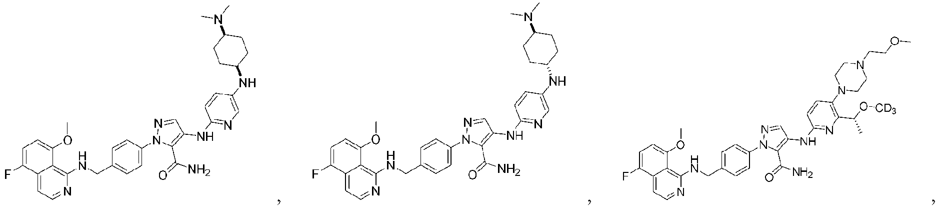

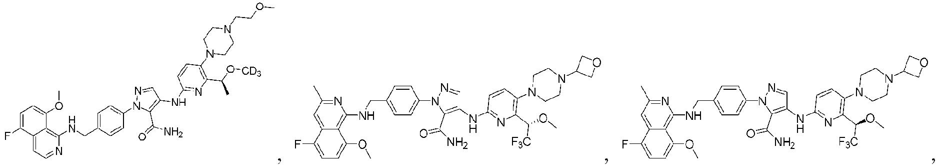

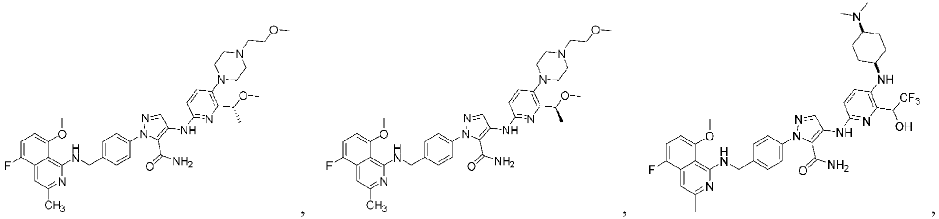

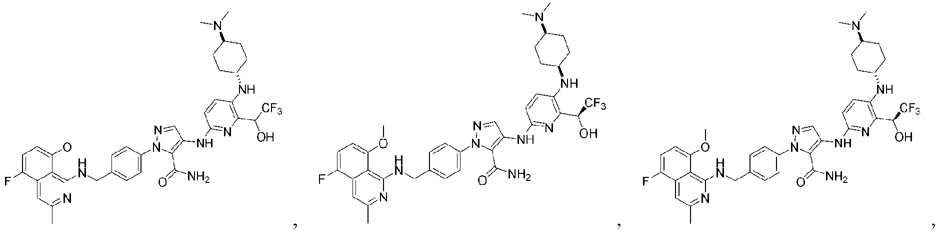

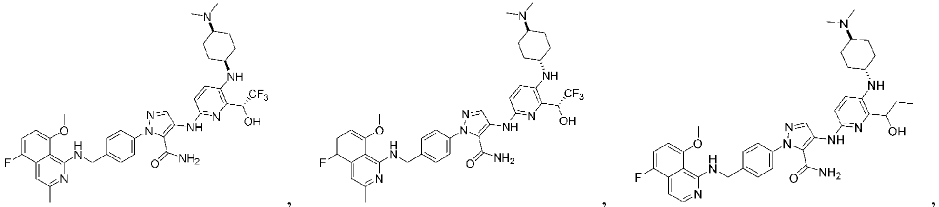

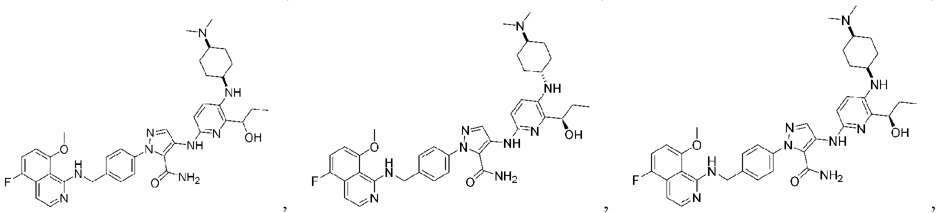

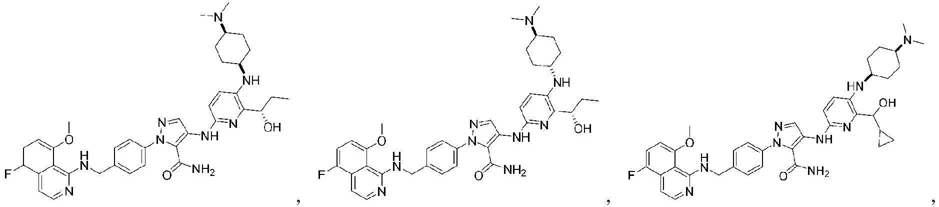

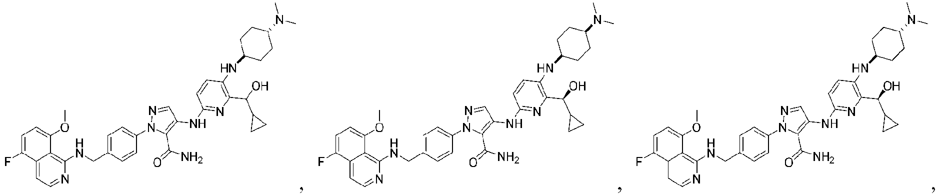

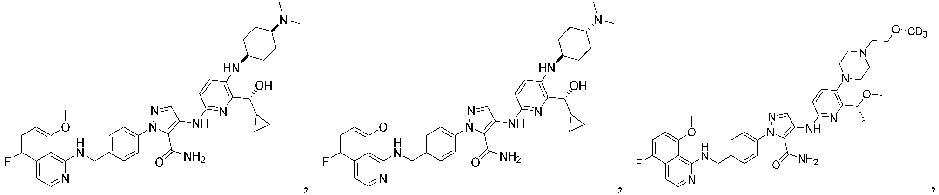

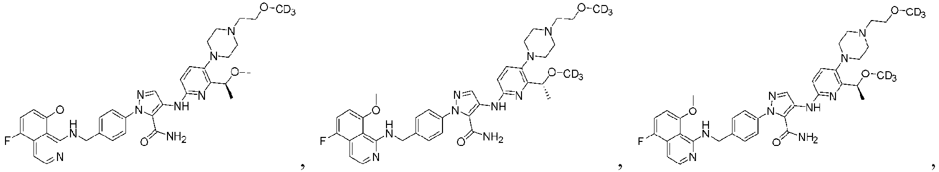

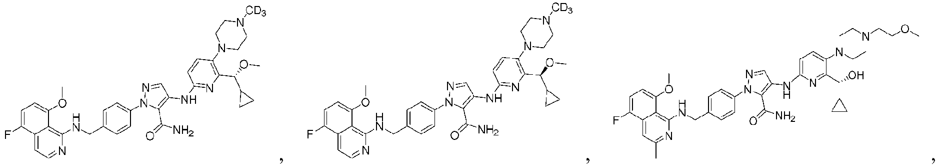

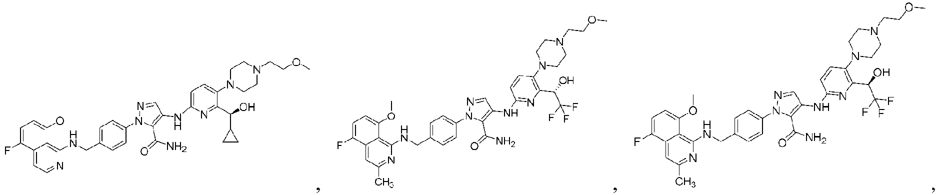

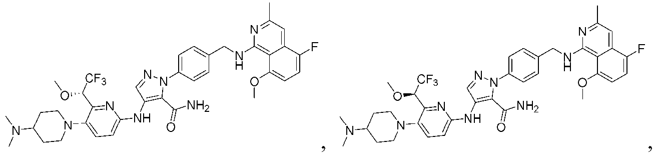

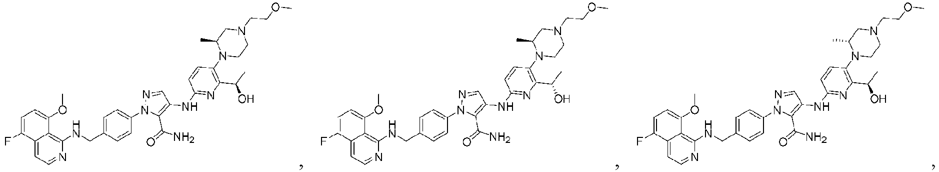

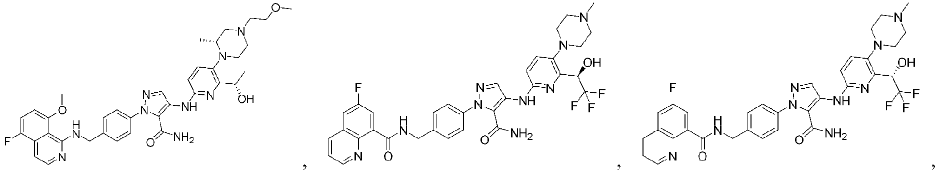

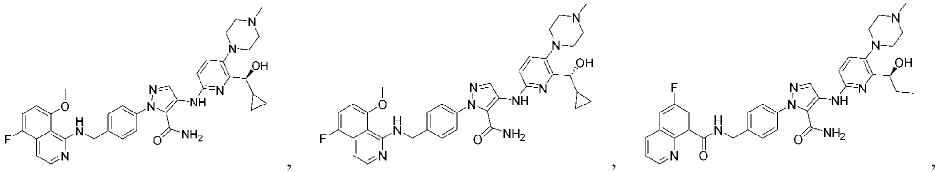

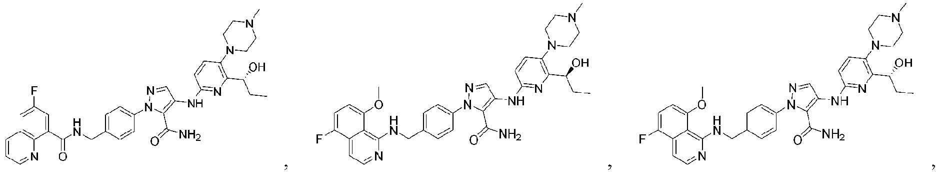

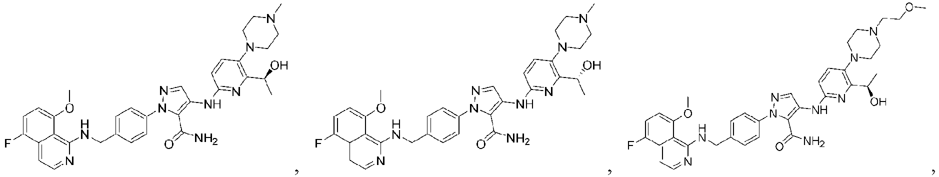

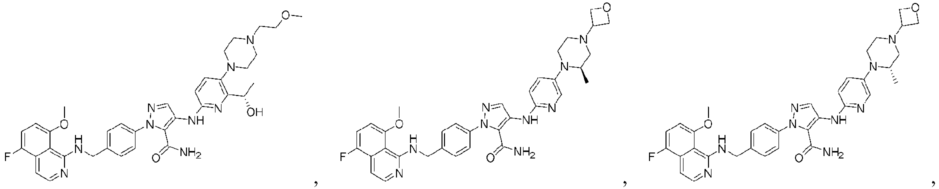

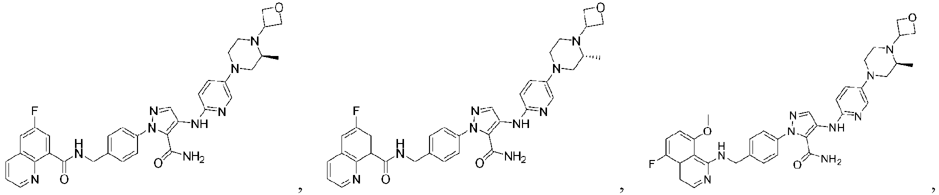

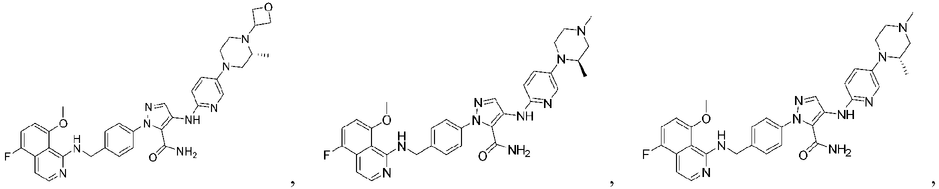

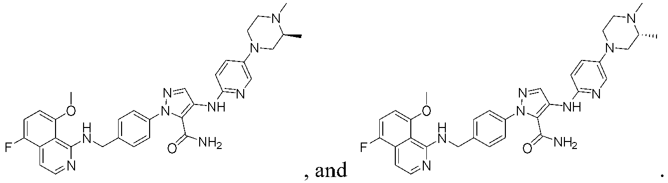

14. A pharmaceutical composition comprising the compound, the stereoisomer thereof, or the pharmaceutically acceptable salt thereof according to any one of claims 1 to 13.

15. Use of the compound, the stereoisomer thereof, or the pharmaceutically acceptable salt thereof according to any one of claims 1 to 13, or the pharmaceutical composition according to claim 14 for preparing a medicament for treating a BTK-related disease, wherein optionally, the BTK-related disease is selected from the group consisting of cancer and an immune disease; or, the BTK-related disease is selected from lymphoma; optionally, the lymphoma is selected from diffuse large B-cell lymphoma.
